# EUROPEAN PATENT SPECIFICATION

(11) **EP 2 316 459 B1**
(45) Date of publication and mention of the grant of the patent: **06.11.2013**
(21) Application number: 10010732.5
(22) Date of filing: 29.07.2003
(51) Int. Cl.: A61K 31/506, A61K 31/519, A61P 35/00, C07D 401/12, C07D 403/12, C07D 405/12, C07D 407/12, C07D 413/12, C07D 413/14, C07D 471/04, C07D 498/04

(54) **2,4-pyrimidinediamine compounds for use in methods of treating or preventing autoimmune diseases**
2,4-Pyrimidindiaminen zur Behandlung oder Vorbeugung von Autoimmunerkrankungen
Composants de 2,4-pyrimidinediamine pour être utilisés dans des procédés de traitement ou de prévention des maladies auto-immunes

(30) Priority: 29.07.2002 US 399673 P; 31.01.2003 US 443949 P; 06.03.2003 US 452339 P; 29.07.2003 US 631029
(43) Date of publication of application: 04.05.2011
(62) Divisional of application: 09004539.4
(73) Proprietor: Rigel Pharmaceuticals, Inc., South San Francisco, CA 94080 (US)
(72) Inventor: Singh, Rajinder, Belmont, CA 94002 (US); Argade, Ankush, Foster City, CA 94404 (US); Payan, Donald G., Hillsborough, CA 94010 (US); Clough, Jeffrey, Redwood City, CA 94063 (US); Keim, Holger, Irvine, CA 92620 (US); Sylvain, Catherine, San Mateo, CA 94401 (US); Li, Hui, Millbrae, CA 94030 (US); Bhamidipati, Somasekhar, Foster City, CA 94404 (US)
(74) Representative: Sexton, Jane Helen

(56) References cited:
- WO-A1-00/39101
- WO-A1-00/58305
- WO-A1-01/60816
- WO-A1-01/64656
- WO-A1-97/19065
- WO-A2-03/063794

## Description

### 1. CROSS REFERENCE TO RELATED APPLICATIONS

This application claims benefit under 35 U.S.C. § 119(e) to application Serial No. 60/399,673 filed July 29, 2002; Serial No. 60/443,949 filed January 31, 2003 and Serial No. 60/452,339 filed March 6, 2003.

### 2. FIELD OF THE INVENTION

The present invention relates generally to 2,4-pyrimidinediamine compounds, pharmaceutical compositions comprising the compounds, intermediates and synthetic methods of making the compounds and the compounds and compositions for use in a variety of contexts, such as in the treatment or prevention of autoimmune diseases and/or the symptoms associated therewith.

### 3. BACKGROUND OF THE INVENTION

Crosslinking of Fc receptors, such as the high affinity receptor for IgE (FceRI) and/or the high affinity receptor for IgG (FcγRI) activates a signaling cascade in mast, basophil and other immune cells that results in the release of chemical mediators responsible for numerous adverse events. For example, such crosslinking leads to the release of preformed mediators of Type I (immediate) anaphylactic hypersensitivity reactions, such as histamine, from storage sites in granules *via* degranulation. It also leads to the synthesis and release of other mediators, including leukotrienes, prostaglandins and platelet-activating factors (PAFs), that play important roles in inflammatory reactions. Additional mediators that are synthesized and released upon crosslinking Fc receptors include cytokines and nitric oxide.

The signaling cascade(s) activated by crosslinking Fc receptors such as FcεRI and/or FcγRI comprises an array of cellular proteins. Among the most important intracellular signal propagators are the tyrosine kinases. And, an important tyrosine kinase involved in the signal transduction pathways associated with crosslinking the FcεRI and/or FcγRI receptors, as well as other signal transduction cascades, is Syk kinase (*see* Valent et al., 2002, Intl. J. Hematol. 75(4):257-362 for review).

As the mediators released as a result of FcεRI and FcγRI receptor cross-linking are responsible for, or play important roles in, the manifestation of numerous adverse events, the availability of compounds capable of inhibiting the signaling cascade(s) responsible for their release would be highly desireable. Moreover, owing to the critical role that Syk kinase plays these and other receptor signaling cascade(s), the availability of compounds capable of inhibiting Syk kinase would also be highly desirable.

WO 01/600,816 describes inhibitors ofkinases.

### 1. SUMMARY OF THE INVENTION

2,4-Pyrimidinediaminc compounds, as will be discussed in more detail below, have myriad biological activities. 2,4-Pyrimidinediamine comprise a 2,4-pyrimidinediamine "core" having the following structure and numbering convention:

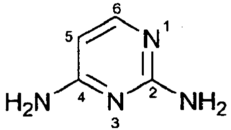

The compounds of the invention are of formula (1a) as defined below and are substituted at the C2 and C4 nitrogens (N2 and N4) to form secondary amines and are optionally further substituted at one or more of the following positions: the C5 position and/or the C6 position.
The present invention provides compounds of formula (1a) 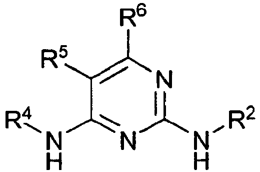 or a salt thereof wherein
R⁵ is selected from the group consisting of halogen, -OR^{d}, -SR^{d}, (C1-C3) haloalkyloxy, (C1-C3) perhaloalkyloxy, -NMR^{c}R^{c}, (C1-C3) haloalkyl, (C1-C3) perhaloalkyl, -CN, -NC, -OCN, -SCN, -NO, -NO₂, -N₃, -S(O)R^{d}, -S(O)₂R^{d}, -S(O)₂OR^{d}, -S(O)NR^{c}R^{c}; -S(O)₂NR^{c}R^{c}, -OS(O)R^{d}, -OS(O)₂R^{d}, -OS(O)₂OR^{d}, -OS(O)NR^{c}R^{c}, -OS(O)₂NR^{c}R^{c}, -C(O)R^{d}, -C(O)OR^{d}, -C(O)NR^{c}R^{c}, -C(NH)NR^{c}R^{c}, -OC(O)R^{d},
-SC(O)R^{d}, -OC(O)OR^{d}, -SC(O)OR^{d}, -OC(O)NR^{c}R^{c}, -SC(O)NR^{c}R^{c}, -OC(NH)NR^{c}R^{c}, -SC(NH)NR^{c}R^{c}, -[NHC(O)]*ₙ*R^{d}, -[NHC(O)]*ₙ*OR^{d}, -[NHC(O)]*ₙ*NR^{c}R^{c} and -[NHC(NH)]*ₙ*NR^{c}R^{c}, (C5-C10) aryl optionally substituted with one or more of the same or different R⁸ groups, (C6-C16) arylalkyl optionally substituted with one or more of the same or different R⁸ groups, 5-10 membered heteroaryl optionally substituted with one or more of the same or different R⁸ groups and 6-16 membered heteroarylalkyl optionally substituted with one or more of the same or different R⁸ groups, (C1-C6) alkyl optionally substituted with one or more of the same or different R⁸ groups, (C1-C4) alkanyl optionally substituted with one or more of the same or different R⁸ groups, (C2-C4) alkenyl optionally substituted with one or more of the same or different R⁸ groups and (C2-C4) alkynyl optionally substituted with one or more of the same or different R⁸ groups;
each R⁶ is independently selected from the group consisting of hydrogen, halogen, -OR^{d}, -SR^{d}, (C1-C3) haloalkyloxy, (C1-C3) perhaloalkyloxy, -NR^{c}R^{c}, (C1-C3) haloalkyl, (C1-C3) perhaloalkyl, -CN, -NC, -OCN, -SCN, -NO, -NO₂, -N₃, -S(O)R^{d}, -S(O)₂R^{d}, -S(O)₂OR^{d}, -S(O)NR^{c}R^{c}; -S(O)₂NR^{c}R^{c}, -OS(O)R^{d}, -OS(O)₂R^{d}, -OS(O)₂OR^{d}, -OS(O)NR^{c}R^{c}, -OS(O)₂NR^{c}R^{c}, -C(O)R^{d}, -C(O)OR^{d}, -C(O)NR^{c}R^{c}, -C(NH)NR^{c}R^{c}, -OC(O)R^{d}, -SC(O)R^{d}, -OC(O)OR^{d}, -SC(O)OR^{d}, -OC(O)NR^{c}R^{c}, -SC(O)NR^{c}R^{c}, -OC(NH)NR^{c}R^{c}, -SC(NH)NR^{c}R^{c}, -[NHC(O)]*ₙ*R^{d}, -[NHC(O)]*ₙ*OR^{d}, -[NHC(O)]*ₙ*NR^{c}R^{c} and-[NHC(NH)]*ₙ*NR^{c}R^{c}, (C5-C10) aryl optionally substituted with one or more of the same or different R⁸ groups, (C6-C16) arylalkyl optionally substituted with one or more of the same or different R⁸ groups, 5-10 membered heteroaryl optionally substituted with one or more of the same or different R⁸ groups and 6-16 membered heteroarylalkyl optionally substituted with one or more of the same or different R⁸ groups;
R⁸ is selected from the group consisting of R^{a}, R^{a} substituted with one or more of the same or different R^{a} or R^{b}, R^{b}, -OR^{a} substituted with one or more of the same or different R³ or R^{b}, -B(OR^{a})₂, -B(NR^{c}R^{c})₂, -(CH₂)*ₘ*-R^{b}, -(CHR^{a})*ₘ*-R^{b}, -O-(CH₂)*ₘ*-R^{b}, -S-(CH₂)*ₘ*-R^{b}, -O-CHR^{a}R^{b}, -O-CR^{a}(R^{b})₂, -O-(CHR^{a})*ₘ*-R^{b}, -O-(CH₂)*ₘ*-CH[(CH₂)*ₘ*R^{b}]R^{b}, -S-(CHR^{a})*ₘ*-R^{b}, -C(O)NH-(CH₂)*ₘ*-R^{b}, -C(O)NH-(CHR³)*ₘ*-R^{b}, -O-(CH₂)*ₘ*-C(O)NH-(CH₂)*ₘ*-R^{b}, -S-(CH₂)*ₘ*-C(O)NH-(CH₂)*ₘ*-R^{b}, -O-(CHR^{a})*ₘ*-C(O)NH-(CHR^{a})*ₘ*-R^{b}, -S-(CHR^{a})*ₘ*-C(O)NH-(CHR^{a})*ₘ*-R^{b}, -NH-(CH₂)*ₘ*-R^{b},
-NH-(CHR³)*ₘ*-R^{b}, -NH[(CH₂)*ₘ*R^{b}], -N[(CH₂)*ₘ*R^{b}]₂, -NH-C(O)-NH-(CH₂)*ₘ*-R^{b}, -NH-C(O)-(CH₂)*ₘ*-CHR^{b}R^{b} and -NH-(CH₂)*ₘ*-C(O)-NH-(CH₂)*ₘ*-R^{b};
each R^{a} is independently selected from the group consisting of (C1-C6) alkyl, hydrogen, (C3-C8) cycloalkyl, cyclohexyl, (C4-C11) cycloalkylalkyl, (C5-C10) aryl, (C6-C16) arylalkyl, 2-6 membered heteroalkyl, 3-8 membered cycloheteroalkyl, 4-11 membered cycloheteroalkylalkyl, 5-10 membered heteroaryl and 6-16 membered heteroarylalkyl;
each R^{b} is a suitable group independently selected from the group consisting of -C(O)R^{d}, -C(O)OR^{d}, =O, -OR^{d}, (C1-C3) haloalkyloxy, -OCF₃, =S, -SR^{d}, =NR^{d}, =NOR^{d}, -NR^{c}R^{c}, halogen, -CF₃, -CN, -NC, -OCN, -SCN, -NO, -NO₂, =N₂, -N₃, -S(O)R^{d}, -S(O)₂R^{d}, -S(O)₂OR^{d}, -S(O)NR^{c}R^{c}, -S(O)₂NR^{c}R^{c}, -OS(O)R^{d}, -OS(O)₂R^{d}, -OS(O)₂OR^{d}, -OS(O)₂NR^{c}R^{c}, -C(O)NR^{c}R^{c}, -C(NH)NR^{c}R^{c}, -C(NR^{a})NR^{c}R^{c}, -C(NOH)R^{a}, -C(NOH)NR^{c}R^{c}, -OC(O)R^{d}, -OC(O)OR^{d}, -OC(O)NR^{c}R^{c}, -OC(NH)NR^{c}R^{c}, -OC(NR^{a})NR^{c}R^{c}, -[NHC(O)]*ₙ*R^{d}, -[NR^{a}C(O)]*ₙ*R^{d}, -[NHC(O)]*ₙ*OR^{d}, -[NR^{a}C(O)]*ₙ*OR^{d}, -[NHC(O)]*ₙ*NR^{c}R^{c}, -[NR^{a}C(O)]*ₙ*NR^{c}R^{c}, -[NHC(NH)]*ₙ*NR^{c}R^{c} and -[NR^{a}C(NR⁸)]*ₙ*NR^{c}R^{c};
each R^{c} is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
each R^{d} is independently R^{a} or a protecting group;
each *m* is independently an integer from 1 to 3; and
each *n* is independently an integer from 0 to 3.
R² is selected from the group consisting (C1-C6) alkyl optionally substituted with one or more of the same or different R⁸ groups, (C3-C8) cycloalkyl optionally substituted with one or more of the same or different R⁸ groups, 3-8 membered cycloheteroalkyl optionally substituted with one or more of the same or different R⁸ groups, (C5-C15) aryl optionally substituted with one or more of the same or different R⁸ groups and 5-15 membered heteroaryl optionally substituted with one or more of the same or different R⁸ groups;
R⁴ is 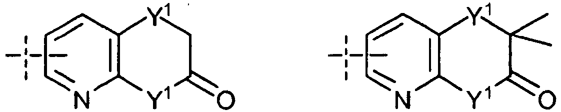 wherein:
   each Y¹ is independently selected from the group consisting of O, NR³⁷, S, SO, SO₂, SONR³⁶ and NH;
   R³⁶ is hydrogen or alkyl; and
   R³⁷ is selected from the group consisting of hydrogen or a progroup selected from the group consisting of -CH₂-PO(OR⁸)₂, aryl, arylalkyl, heteroaryl, R^{a}, R^{b}, -CR^{a}R^{b}-O-C(O)R⁸, -CR^{a}R^{b}-O-PO(OR⁸)₂, -CH₂-O-PO(OR⁸)₂, -C(O)-CR^{a}R^{b}-N(CH₃)₂, -CR^{a}R^{b}-O-C(O)-CR^{a}R^{b}-N(CH₃)₂, -C(O)R⁸, and -C(O)-NR⁸-C(O)R⁸.

When R⁵ is (C1-C3) haloalkyl it may in one embodiment be -CF³, -CH²CF³, or-CF²CF³.

When R⁵ is (C5-C10) aryl optionally substituted with one or more of the same or different R⁸ groups, it may in one embodiment be phenyl optionally substituted with one or more of the same or different R⁸ groups.

When R⁶ is (C1-C3) haloalkyl it may in one embodiment be -CF³, -CH²CF³, or-CF²CF³.

When R⁶ is (C5-C10) aryl optionally substituted with one or more of the same or different R⁸ groups, it may in one embodiment be phenyl optionally substituted with one or more of the same or different R⁸ groups.

When R^{a} is 3-8 membered cycloheteroalkyl it may in one embodiment be morpholinyl, piperazinyl, homopiperazinyl, or piperidinyl.

When R^{a} is (C5-C10) aryl, it may in one embodiment be phenyl.

When R^{a} is (C6-C16) arylalkyl, it may in one embodiment be benzyl.

When R² is (C5-C15) aryl optionally substituted with one or more of the same or different R⁸ groups it may in one embodiment be phenyl optionally substituted with one or more of the same or different R⁸ groups.

When R² is (C3-C8) cycloalkyl optionally substituted with one or more of the same or different R⁸ groups, it may in one embodiment be cyclohexyl optionally substituted with one or more of the same or different R⁸ groups.

When R³⁷ is -C(O)R⁸, it may in one embodiment be -C(O)CF₃.

Also described herein are prodrugs of the compounds of formula (Ia). Such prodrugs may be active in their prodrug form, or may be inactive until converted under physiological or other conditions of use to an active drug form. In the prodrugs, one or more functional groups of the compounds of formula (1a) are included in promoieties that cleave from the molecule under the conditions of use, typically by way of hydrolysis, enzymatic cleavage or some other cleavage mechanism, to yield the functional groups. For example, primary or secondary amino groups may be included in an amide promoiety that cleaves under conditions of use to generate the primary or secondary amino group. Thus, the prodrugs include special types of protecting groups, termed "progroups," masking one or more functional groups of the compounds of formula (Ia) that cleave under the conditions of use to yield an active drug compound of formula (Ia). Functional groups within the compounds of formula (Ia) that may be masked with progroups for inclusion in a promoiety include, but are not limited to, amines (primary and secondary), hydroxyls, sulfanyls (thiols), carboxyls, carbonyls, phenols, catechols, diols, alkynes, phosphates, etc. Myriad progroups suitable for masking such functional groups to yield promoieties that are cleavable under the desired conditions of use are known in the art. All of these progroups, alone or in combinations, may be included in the prodrugs. Specific examples of promoieties that yield primary or secondary amine groups that can be included in the prodrugs include, but are not limited to amides, carbamates, imines, ureas, phosphenyls, phosphoryls and sulfenyls. Specific examples of promoieties that yield sulfanyl groups that can be included in the prodrugs include, but are not limited to, thioethers, for example S-methyl derivatives (monothio, dithio, oxythio, aminothio acetals), silyl thioethers, thioesters, thiocarbonates, thiocarbamates, asymmetrical disulfides, etc. Specific examples of promoieties that cleave to yield hydroxyl groups that can be included in the prodrugs include, but are not limited to, sulfonates, esters and carbonates. Specific examples of promoieties that yield carboxyl groups that can be included in the prodrugs include, but are not limited to, esters (including silyl esters, oxamic acid esters and thioesters), amides and hydrazides.

The prodrugs disclosed herein include compounds according to structural formula (Ia) in which R^{c} and R^{d} are replaced by a progroup.

Replacing the hydrogens attached to N2 and N4 in the 2,4-pyrimidinediamines of structural formula (Ia) with substituents adversely affects the activity of the compounds. However, as will be appreciated by skilled artisans, these nitrogens may be included in promoieties that, under conditions of use, cleave to yield 2,4-pyrimidinediamines according to structural formula (Ia). Thus, the prodrugs include compounds according to structural formula (II): 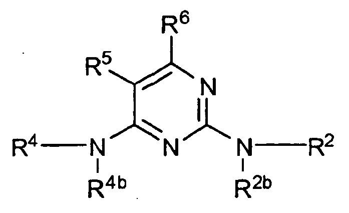
including salts, hydrates, solvates and N-oxides thereof, wherein:
R², R⁴, R⁵ and R⁶ are as previously defined for structural formula (Ia); and
R^{2b} and R^{4b} are each, independently of one another, a progroup.

In another aspect, the present invention provides compositions comprising one or more compounds of the invention and an appropriate carrier, excipient or diluent. The exact nature of the carrier, excipient or diluent will depend upon the desired use for the composition, and may range from being suitable or acceptable for veterinary uses to being suitable or acceptable for human use.

Also disclosed herein are intermediates useful for synthesizing the compounds of formula (Ia) and prodrugs thereof. The intermediates include 4-pyrimidineamines according to structural formula (III): 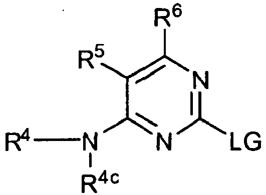 including salts, hydrates, solvates and N-oxides thereof, wherein R⁴, R⁵ and R⁶ are as previously defined for structural formula (Ia); LG is a leaving group such as, for example, - S(O)₂Me, -SMe or halo (*e.g*., F, Cl, Br, I); and R^{4c} is hydrogen or a progroup.

The intermediates also include 2-pyrimidineamines according to structural formula (IV): 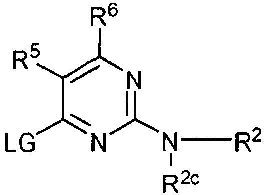 including salts, hydrates, solvates and N-oxides thereof, wherein R², R⁵ and R⁶ are as previously defined for structural formula (Ia); LG is a leaving group, such as, for example, - S(O)₂Me, -SMe or halo (*e.g*., F, Cl, Br, I) and R^{2c} is hydrogen or a progroup.

The intermediates also include 4-amino- or 4-hydroxy-2-pyrimidineamines according to structural formula (V): 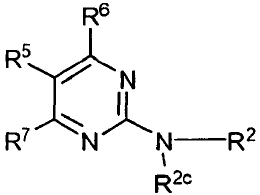 including salts, hydrates, solvates and N-oxides thereof, wherein R², R⁵ and R⁶ are as previously defined for structural formula (Ia), R⁷ is an amino or hydroxyl group and R^{2c} is hydrogen or a progroup.

The intermediates also include N4-substituted cytosines according to structural formula (VI): 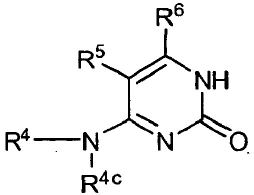 including salts, hydrates, solvates and N-oxides thereof, wherein R⁴, R⁵ and R⁶ are as previously defined for structural formula (Ia) and R^{4c} is hydrogen or a progroup.

Also disclosed herein are methods of synthesizing the compounds of formula (Ia) and prodrugs thereof. The method may involve reacting a 4-pyrimidineamine according to structural formula (III) with an amine of the formula HR^{2c}N-R², where R² and R^{2c} are as previously defined for structural formula (IV) to yield a 2,4-pyrimidinediamine according to structural formula (Ia) or a prodrug according to structural formula (II).

The method may involve reacting a 2-pyrimidineamine according to structural formula (IV) with an amine of the formula R⁴-NHR^{4c} where R⁴ and R^{4c} are as previously defined for structural formula (III) to yield a 2,4-pyrimidinediamine according to structural formula (Ia) or a prodrug according to structural formula (II).

The method may involve reacting a 4-amino-2-pyrimidineamine according to structural formula (V) (in which R⁷ is an amino group) with an amine of the formula R⁴-NHR^{4c}, where R⁴ and R^{4c} are as defined for structural formula (III), to yield a 2,4-pyrimidinediamine according to structural formula (Ia) or a prodrug according to structural formula (II). Alternatively, the 4-amino-2-pyrimidineamine may be reacted with a compound of the formula R⁴-LG, where R⁴ is as previously defined for structural formula (Ia) and LG is a leaving group.

The method may involve halogenating a 4-hydroxy-2-pyrimidineamine according to structural formula (V) (R⁷ is a hydroxyl group) to yield a 2-pyrimidineamine according to structural formula (IV) and reacting this pyrimidineamine with an appropriate amine, as described above.

The method may involve halogenating an N4-substituted cytosine according to structural formula (VI) to yield a 4-pyrimidineamine according to structural formula (III) and reacting this pyrimidineamine with an appropriate amine, as described above.

The compounds of formula (Ia) are potent inhibitors of degranulation of immune cells, such as mast, basophil, neutrophil and/or eosinophil cells. Thus, also disclosed herein are the compounds of the invention for use in methods of regulating, and in particular inhibiting, degranulation of such cells. The method generally involves contacting a cell that degranulates with an amount of a compound of formula (Ia) or prodrug thereof, or an acceptable salt, hydrate, solvate, N-oxide and/or composition thereof, effective to regulate or inhibit degranulation of the cell. The method may be practiced in *in vitro* contexts or in *in vivo* contexts as a therapeutic approach towards the treatment or prevention of diseases characterized by, caused by or associated with cellular degranulation.

While not intending to be bound by any theory of operation, biochemical data confirm that the 2,4-pyrimidinediamine compounds exert their degranulation inhibitory effect, at least in part, by blocking or inhibiting the signal transduction cascade(s) initiated by crosslinking of the high affinity Fc receptors for IgE ("FcεRI") and/or IgG ("FcγRI"). Indeed, the 2,4-pyrimidinediamine compounds are potent inhibitors of both FcεRI-mediated and FcγRI-mediated degranulation. As a consequence, the compounds of formula (Ia) may be used to inhibit these Fc receptor signalling cascades in any cell type expressing such FcεRI and/or FcγRI receptors including but not limited to macrophages, mast, basophil, neutrophil and/or eosinophil cells.

The methods disclosed herein also permit the regulation of, and in particular the inhibition of, downstream processes that result as a consequence of activating such Fc receptor signaling cascade(s). Such downstream processes include, but are not limited to, FcεRI-mediated and/or FcγRI-mediated degranulation, cytokine production and/or the production and/or release of lipid mediators such as leukotrienes and prostaglandins. The method generally involves contacting a cell expressing an Fc receptor, such as one of the cell types discussed above, with an amount of a compound of formula (Ia) or prodrug thereof, or an acceptable salt, hydrate, solvent, N-oxide and/or composition thereof, effective to regulate or inhibit the Fc receptor signaling cascade and/or a downstream process effected by the activation of this signaling cascade. The method may be practiced in *in vitro* contexts or in *in vivo* contexts as a therapeutic approach towards the treatment or prevention of diseases characterized by, caused by or associated with the Fc receptor signaling cascade, such as diseases effected by the release of granule specific chemical mediators upon degranulation, the release and/or synthesis of cytokines and/or the release and/or synthesis of lipid mediators such as leukotrienes and prostaglandins.

Also disclosed herein are the compounds of the invention for use in methods of treating and/or preventing diseases characterized by, caused by or associated with the release of chemical mediators as a consequence of activating Fc receptor signaling cascades, such as FcεRI and/or FcγRI-signaling cascades. The methods may be practiced in animals in veterinary contexts or in humans. The methods generally involve administering to an animal subject or human an amount of a compound of formula (Ia), or prodrug thereof, or an acceptable salt, hydrate, solvate, N-oxide and/or composition thereof, effective to treat or prevent the disease. As discussed previously, activation of the FcεRI or FcγRI receptor signaling cascade in certain immune cells leads to the release and/or synthesis of a variety of chemical substances that are pharmacological mediators of a wide variety of diseases.

Any of these diseases may be treated or prevented according to the methods disclosed herein.

For example, in mast cells and basophil cells, activation of the FcεRI or FcγRI signaling cascade leads to the immediate (*i.e*., within 1-3 min. of receptor activation) release of preformed mediators of atopic and/or Type I hypersensitivity reactions (*e.g*., histamine, proteases such as tryptase, etc.) *via* the degranulation process. Such atopic or Type I hypersensitivity reactions include, but are not limited to, anaphylactic reactions to environmental and other allergens (*e.g*., pollens, insect and/or animal venoms, foods, drugs, contrast dyes, etc.), anaphylactoid reactions, hay fever, allergic conjunctivitis, allergic rhinitis, allergic asthma, atopic dermatitis, eczema, urticaria, mucosal disorders, tissue disorders and certain gastrointestinal disorders.

The immediate release of the preformed mediators *via* degranulation is followed by the release and/or synthesis of a variety of other chemical mediators, including, among other things, platelet activating factor (PAF), prostaglandins and leukotrienes (e.g., LTC4) and the *de novo* synthesis and release of cytokines such as TNFα, IL-4, IL-5, IL-6, IL-13, etc. The first of these two processes occurs approximately 3-30 min. following receptor activation; the latter approximately 30 min. - 7 hrs. following receptor activation. These "late stage" mediators are thought to be in part responsible for the chronic symptoms of the above-listed atopic and Type I hypersensitivity reactions, and in addition are chemical mediators of inflammation and inflammatory diseases (e.g., osteoarthritis, inflammatory bowel disease, ulcerative colitis, Crohn's disease, idiopathic inflammatory bowel disease, irritable bowel syndrome, spastic colon, etc.), low grade scarring (e.g., scleroderma, increased fibrosis, keloids, post-surgical scars, pulmonary fibrosis, vascular spasms, migraine, reperfusion injury and post myocardial infarction), and sicca complex or syndrome. All of these diseases may be treated or prevented according to the methods disclosed herein.

Additional diseases which can be treated or prevented according to the methods disclosed herein include diseases associated with basophil cell and/or mast cell pathology. Examples of such diseases include, but are not limited to, diseases of the skin such as scleroderma, cardiac diseases such as post myocardial infarction, pulmonary diseases such as pulmonary muscle changes or remodeling and chronic obstructive pulmonary disease (COPD) and diseases of the gut such as inflammatory bowel syndrome (spastic colon).

The compounds of formula (Ia) are also potent inhibitors of the tyrosine kinase Syk kinase. Thus, also described herein are methods of regulating, and in particular inhibiting, Syk kinase activity. The method generally involves contacting a Syk kinase or a cell comprising a Syk kinase with an amount of a compound of formula (Ia) or prodrug thereof, or an acceptable salt, hydrate, solvate, N-oxide and/or composition thereof, effective to regulate or inhibit Syk kinase activity. The Syk kinase may be an isolated or recombinant Syk kinase. Alternatively, the Syk kinase may be an endogenous or recombinant Syk kinase expressed by a cell, for example a mast cell or a basophil cell. The method may be practiced in *in vitro* contexts or in *in vivo* contexts as a therapeutic approach towards the treatment or prevention of diseases characterized by, caused by or associated with Syk kinase activity.

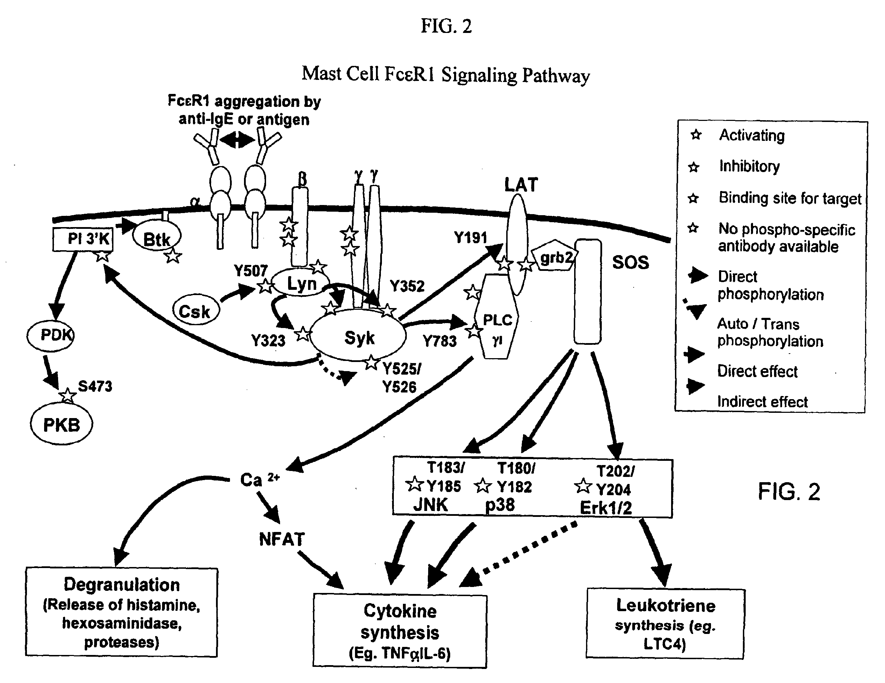

While not intending to be bound by any particular theory of operation, it is believed that the compounds of formula (Ia) inhibit cellular degranulation and/or the release of other chemical mediators primarily by inhibiting Syk kinase that gets activated through the gamma chain homodimer of FcεRI (*see, e.g.,* FIG. 2). This gamma chain homodimer is shared by other Fc receptors, including FcγRI, FcγRIII and FcαRI. For all of these receptors, intracellular signal transduction is mediated by the common gamma chain homodimer. Binding and aggregation of those receptors results in the recruitment and activation of tyrosine kinases such as Syk kinase. As a consequence of these common signaling activities, the 2,4-pyrimidinediamine compounds described herein may be used to regulate, and in particular inhibit, the signaling cascades of Fc receptors having this gamma chain homodimer, such as FcεRI, FcγRI, FcγRIII and FcαRI, as well as the cellular responses elicited through these receptors.

Syk kinase is known to play a critical role in other signaling cascades. For example, Syk kinase is an effector of B-cell receptor (BCR) signaling (Turner et al., 2000, Immunology Today 21:148-154) and is an essential component of integrin beta(1), beta(2) and beta(3) signaling in neutrophils (Mocsai et al., 2002, Immunity 16:547-558). As the 2,4-pyrimidinediamine compounds described herein are potent inhibitors of Syk kinase, they can be used to regulate, and in particular inhibit, any signaling cascade where Syk plays a role, such as, fore example, the Fc receptor, BCR and integrin signaling cascades, as well as the cellular responses elicited through these signaling cascades. The particular cellular response regulated or inhibited will depend, in part, on the specific cell type and receptor signaling cascade, as is well known in the art. Non-limiting examples of cellular responses that may be regulated or inhibited with the 2,4-pyrimidinediamine compounds include a respiratory burst, cellular adhesion, cellular degranulation, cell spreading, cell migration, phagocytosis (e.g., in macrophages), calcium ion flux (*e.g*., in mast, basophil, neutrophil, eosinophil and B-cells), platelet aggregation, and cell maturation (*e.g*., in B-cells).

Thus, also disclosed herein are the compounds of the invention for use in methods of regulating, and in particular inhibiting, signal transduction cascades in which Syk plays a role. The method generally involves contacting a Syk-dependent receptor or a cell expressing a Syk-dependent receptor with an amount of a compound of formula (Ia) or prodrug thereof, or an acceptable salt, hydrate, solvate, N-oxide and/or composition thereof, effective to regulate or inhibit the signal transduction cascade. The methods may also be used to regulate, and in particular inhibit, downstream processes or cellular responses elicited by activation of the particular Syk-dependent signal transduction cascade. The methods may be practiced to regulate any signal transduction cascade where Syk is not known or later discovered to play a role. The methods may be practiced in *in vitro* contexts or in *in vivo* contexts as a therapeutic approach towards the treatment or prevention of diseases characterized by, caused by or associated with activation of the Syk-dependent signal transduction cascade. Non-limited examples of such diseases include those previously discussed.

Cellular and animal data also confirm that the compounds of formula (Ia) may also be used to treat or prevent autoimmune diseases and/or symptoms of such diseases. Thus, in another aspect the present invention provides compounds of formula (Ia) for use in a method of treating or preventing an autoimmune disease and/or one or more symptoms associated therewith in a mammal. The methods generally involve administering to a subject suffering from an autoimmune disease or at risk of developing an autoimmune disease an amount of a compound of formula (Ia) or composition thereof, effective to treat or prevent the autoimmune disease and/or its associated symptoms. Autoimmune diseases that can be treated or prevented with the compounds of formula (Ia) include those diseases that are commonly associated with nonanaphylactic hypersensitivity reactions (Type II, Type III and/or Type IV hypersensitivity reactions) and/or those diseases that are mediated, at least in part, by activation of the FcγR signaling cascade in monocyte cells. Such autoimmune disease include, but are not limited to, those autoimmune diseases that are frequently designated as single organ or single cell-type autoimmune disorders and those autoimmune disease that are frequently designated as involving systemic autoimmune disorder. Non-limiting examples of diseases frequently designated as single organ or single cell-type autoimmune disorders include: Hashimoto's thyroiditis, autoimmune hemolytic anemia, autoimmune atrophic gastritis of pernicious anemia, autoimmune encephalomyelitis, autoimmune orchitis, Goodpasture's disease, autoimmune thrombocytopenia, sympathetic ophthalmia, myasthenia gravis, Graves' disease, primary biliary cirrhosis, chronic aggressive hepatitis, ulcerative colitis and membranous glomerulopathy. Non-limiting examples of diseases often designated as involving systemic autoimmune disorder include: systemic lupus erythematosis, rheumatoid arthritis, Sjogren's syndrome, Reiter's syndrome, polymyositis-dermatomyositis, systemic sclerosis, polyarteritis nodosa, multiple sclerosis and bullous pemphigoid.

### 5. BRIEF DESCRIPTION OF THE FIGURES

FIG. 1 provides a cartoon illustrating allergen-induced production of IgE and consequent release of preformed and other chemical mediators from mast cells;
FIG. 2 provides a cartoon illustrating the FcεRI signal transduction cascade leading to degranulation of mast and/or basophil cells;
FIG. 3 provides a cartoon illustrating the putative points of action of compounds that selectively inhibit upstream FcFRI-mediated degranulation and compounds that inhibit both FcεRI-mediated and ionomycin-induced degranulation;
FIG. 4 provides graphs illustrating the effects of certain 2,4-pyrimidinediamine compounds, DMSO (control) and ionomycin on Ca²⁺ flux in CHMC cells;

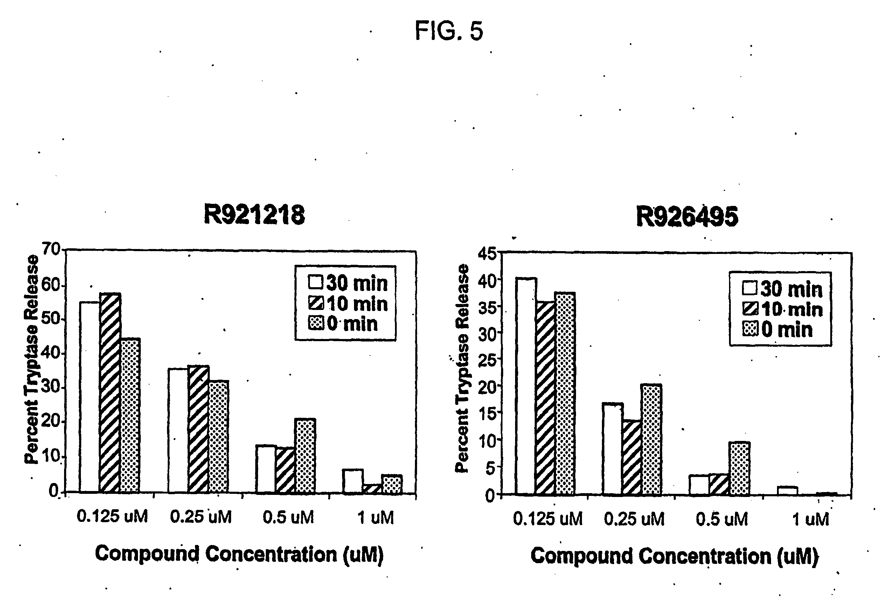

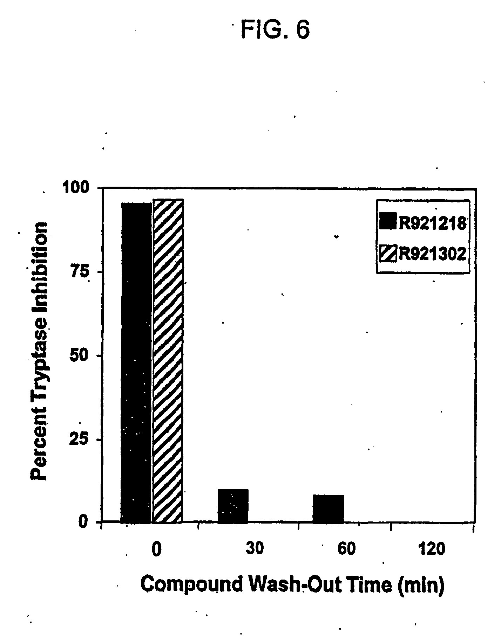

FIG. 5 provides data showing dose responsive inhibition of Syk kinase phosphorylation of an endogenous substrate (LAT) and a peptide substrate in the presence of increasing concentrations of compounds R921218 (X), R921219 (Y) and R921304 (Z);

### 6. DETAILED DESCRIPTION OF THE PREFERRED EMBODIMENTS

### 6.1 Definitions

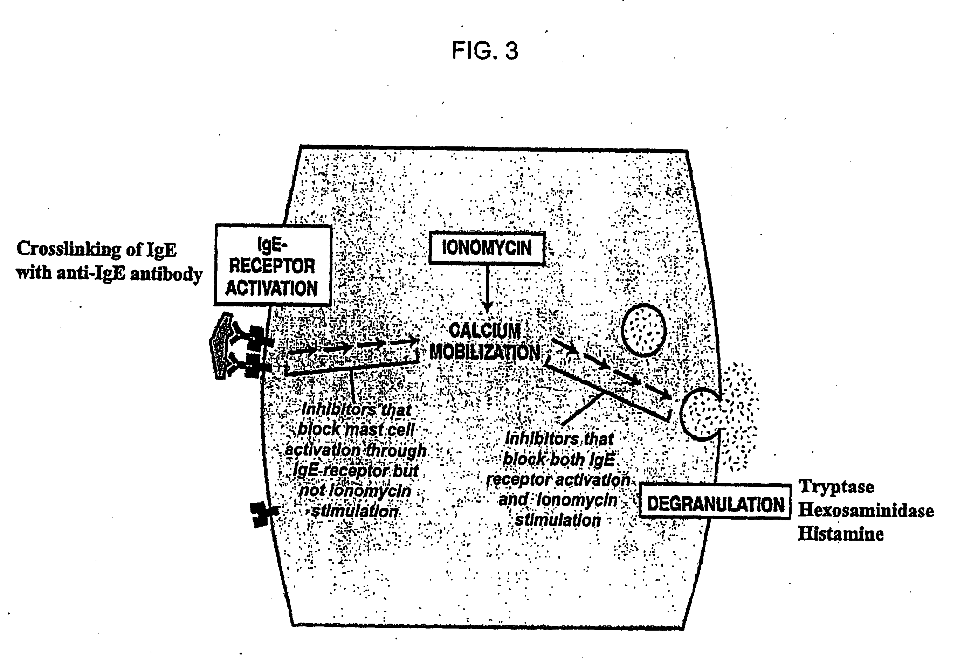

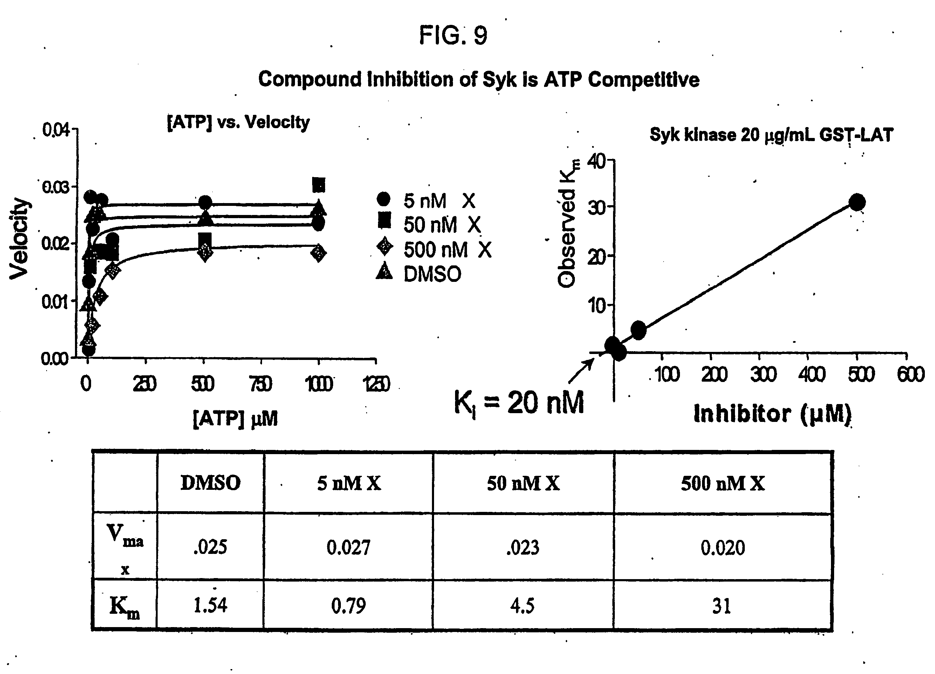

As used herein, the following terms are intended to have the following meanings:
"Alkyl" by itself or as part of another substituent refers to a saturated or unsaturated branched, straight-chain or cyclic monovalent hydrocarbon radical having the stated number of carbon atoms (*i.e.,* C1-C6 means one to six carbon atoms) that is derived by the removal of one hydrogen atom from a single carbon atom of a parent alkane, alkene or alkyne. Typical alkyl groups include, but are not limited to, methyl; ethyls such as ethanyl, ethenyl, ethynyl; propyls such as propan-1-yl, propan-2-yl, cyclopropan-1-yl, prop-1-en-1-yl, prop-1-en-2-yl, prop-2-en-1-yl, cycloprop-1-en-1-yl; cycloprop-2-en-1-yl, prop-1-yn-1-yl, prop-2-yn-1-yl, etc.; butyls such as butan-1-yl, butan-2-yl, 2-methyl-propan-1-yl, 2-methyl-propan-2-yl, cyclobutan-1-yl, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, but-1-yn-1-yl, but-1-yn-3-yl, but-3-yn-1-yl, etc.; and the like. Where specific levels of saturation are intended, the nomenclature "alkanyl," "alkenyl" and/or "alkynyl" is used, as defined below. In preferred embodiments, the alkyl groups are (C1-C6) alkyl.
"Alkanyl" by itself or as part of another substituent refers to a saturated branched, straight-chain or cyclic alkyl derived by the removal of one hydrogen atom from a single carbon atom of a parent alkane. Typical alkanyl groups include, but are not limited to, methanyl; ethanyl; propanyls such as propan-1-yl, propan-2-yl (isopropyl), cyclopropan-1-yl, etc.; butanyls such as butan-1-yl, butan-2-yl (*sec*-butyl), 2-methyl-propan-1-yl (isobutyl), 2-methyl-propan-2-yl (*t*-butyl), cyclobutan-1-yl, etc.; and the like. In preferred embodiments, the alkanyl groups are (C1-C6) alkanyl.
"Alkenyl" by itself or as part of another substituent refers to an unsaturated branched, straight-chain or cyclic alkyl having at least one carbon-carbon double bond derived by the removal of one hydrogen atom from a single carbon atom of a parent alkene. The group may be in either the *cis* or *trans* conformation about the double bond(s). Typical alkenyl groups include, but are not limited to, ethenyl; propenyls such as prop-1-en-1-yl, prop-1-en-2-yl, prop-2-en-1-yl, prop-2-en-2-yl, cycloprop-1-en-1-yl; cycloprop-2-en-1-yl ; butenyls such as but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, etc.; and the like. In preferred embodiments, the alkenyl group is (C2-C6) alkenyl.
"Alkynyl" by itself or as part of another substituent refers to an unsaturated branched, straight-chain or cyclic alkyl having at least one carbon-carbon triple bond derived by the removal of one hydrogen atom from a single carbon atom of a parent alkyne. Typical alkynyl groups include, but are not limited to, ethynyl; propynyls such as prop-1-yn-1-yl , prop-2-yn-1-yl, etc.; butynyls such as but-1-yn-1-yl, but-1-yn-3-yl, but-3-yn-1-yl, etc.; and the like. In preferred embodiments, the alkynyl group is (C2-C6) alkynyl.
"Alkyldiyl" by itself or as part of another substituent refers to a saturated or unsaturated, branched, straight-chain or cyclic divalent hydrocarbon group having the stated number of carbon atoms (*i.e*., C1-C6 means from one to six carbon atoms) derived by the removal of one hydrogen atom from each of two different carbon atoms of a parent alkane, alkene or alkyne, or by the removal of two hydrogen atoms from a single carbon atom of a parent alkane, alkene or alkyne. The two monovalent radical centers or each valency of the divalent radical center can form bonds with the same or different atoms. Typical alkyldiyl groups include, but are not limited to, methandiyl; ethyldiyls such as ethan-1,1-diyl, ethan-1,2-diyl, ethen-1,1-diyl, ethen-1,2-diyl; propyldiyls such as propan-1,1-diyl, propan-1,2-diyl, propan-2,2-diyl, propan-1,3-diyl, cyclopropan-1,1-diyl, cyclopropan-1,2-diyl, prop-1-en-1,1-diyl, prop-1-en-1,2-diyl, prop-2-en-1,2-diyl, prop-1-en-1,3-diyl, cycloprop-1-en-1,2-diyl, cycloprop-2-en-1,2-diyl, cycloprop-2-en-1,1-diyl, prop-1yn-1,3-diyl, etc.; butyldiyls such as, butan-1,1-diyl, butan-1,2-diyl, butan-1,3-diyl, butan-1,4-diyl, butan-2,2-diyl, 2-methyl-propan-1,1-diyl, 2-methyl-propan-1,2-diyl, cyclobutan-1,1-diyl; cyclobutan-1,2-diyl, cyclobutan-1,3-diyl, but-1-en-1,1-diyl, but-1-en-1,2-diyl, but-1-en-1,3-diyl, but-1-en-1,4-diyl, 2-methyl-prop-1-en-1,1-diyl, 2-methanylidene-propan-1,1-diyl, buta-1,3-dien-1,1-diyl, buta-1,3-dien-1,2-diyl, buta-1,3-dien-1,3-diyl, buta-1,3-dien-1,4-diyl, cyclobut-1-en-1,2-diyl, cyclobut-1-en-1,3-diyl, cyclobut-2-en-1,2-diyl, cyclobuta-1,3-dien-1,2-diyl, cyclobuta-1,3-dien-1,3-diyl, but-1-yn-1,3-diyl, but-1-yn-1,4-diyl, buta-1,3-diyn-1,4-diyl, etc.; and the like. Where specific levels of saturation are intended, the nomenclature alkanyldiyl, alkenyldiyl and/or alkynyldiyl is used. Where it is specifically intended that the two valencies are on the same carbon atom, the nomenclature "alkylidene" is used. In preferred embodiments, the alkyldiyl group is (C1-C6) alkyldiyl. Also preferred are saturated acyclic alkanyldiyl groups in which the radical centers are at the terminal carbons, *e.g*., methandiyl (methano); ethan-1,2-diyl (ethano); propan-1,3-diyl (propano); butan-1,4-diyl (butano); and the like (also referred to as alkylenos, defined *infra*).
"Alkyleno" by itself or as part of another substituent refers to a straight-chain saturated or unsaturated alkyldiyl group having two terminal monovalent radical centers derived by the removal of one hydrogen atom from each of the two terminal carbon atoms of straight-chain parent alkane, alkene or alkyne. The locant of a double bond or triple bond, if present, in a particular alkyleno is indicated in square brackets. Typical alkyleno groups include, but are not limited to, methano; ethylenos such as ethano, etheno, ethyno; propylenos such as propano, prop[1]eno, propa[1,2]dieno, prop[1]yno, etc.; butylenos such as butano, but[1]eno, but[2]eno, buta[1,3]dieno, but[1]yno, but[2]yno, buta[1,3]diyno, etc.; and the like. Where specific levels of saturation are intended, the nomenclature alkano, alkeno and/or alkyno is used. In preferred embodiments, the alkyleno group is (C1-C6) or (C1-C3) alkyleno. Also preferred are straight-chain saturated alkano groups, *e.g*., methano, ethano, propano, butano, and the like.
"Heteroalkyl," Heteroalkanyl," Heteraalkenyl," Heteroalkynyl," Heteroalkyldiyl" and "Heteroalkyleno" by themselves or as part of another substituent refer to alkyl, alkanyl, alkenyl, alkynyl, alkyldiyl and alkyleno groups, respectively, in which one or more of the carbon atoms are each independently replaced with the same or different heteratoms or heteroatomic groups. Typical heteroatoms and/or heteroatomic groups which can replace the carbon atoms include, but are not limited to, -O-, -S-, -S-O-, -NR'-, -PH-, -S(O)-, -S(O)₂-, -S(O) NR'-, -S(O)₂NR'-, and the like, including combinations thereof, where each R' is independently hydrogen or (C1-C6) alkyl.
"Cycloalkyl" and "Heterocycloalkyl" by themselves or as part of another substituent refer to cyclic versions of "alkyl," and "heteroalkyl" groups, respectively. For heteroalkyl groups, a heteroatom can occupy the position that is attached to the remainder of the molecule. Typical cycloalkyl groups include, but are not limited to, cyclopropyl; cyclobutyls such as cyclobutanyl and cyclobutenyl; cyclopentyls such as cyclopentanyl and cyclopentenyl; cyclohexyls such as cyclohexanyl and cyclohexenyl; and the like. Typical heterocycloalkyl groups include, but are not limited to, tetrahydrofuranyl (*e.g*., tetrahydrofuran-2-yl, tetrahydrofuran-3-yl, etc.), piperidinyl (*e.g*., piperidin-1-yl, piperidin-2-yl, etc.), morpholinyl (*e.g*., morpholin-3-yl, morpholin-4-yl, etc.), piperazinyl (*e.g*., piperazin-1-yl, piperazin-2-yl, etc.), and the like.
"Acyclic Heteroatomic Bridge" refers to a divalent bridge in which the backbone atoms are exclusively heteroatoms and/or heteroatomic groups. Typical acyclic heteroatomic bridges include, but are not limited to, -O-, -S-, -S-O-, -NR'-, -PH-, -S(O)-, -S(O)₂-, -S(O) NR'-, -S(O)₂NR'-, and the like, including combinations thereof, where each R' is independently hydrogen or (C1-C6) alkyl.
"Parent Aromatic Ring System" refers to an unsaturated cyclic or polycyclic ring system having a conjugated π electron system. Specifically included within the definition of "parent aromatic ring system" are fused ring systems in which one or more of the rings are aromatic and one or more of the rings are saturated or unsaturated, such as, for example, fluorene, indane, indene, phenalene, tetrahydronaphthalene, etc. Typical parent aromatic ring systems include, but are not limited to, aceanthrylene, acenaphthylene, acephenanthrylene, anthracene, azulene, benzene, chrysene, coronene, fluoranthene, fluorene, hexacene, hexaphene, hexalene, indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, tetrahydronaphthalene, triphenylene, trinaphthalene, and the like, as well as the various hydro isomers thereof.
"Aryl" by itself or as part of another substituent refers to a monovalent aromatic hydrocarbon group having the stated number of carbon atoms (*i.e*., C5-C15 means from 5 to 15 carbon atoms) derived by the removal of one hydrogen atom from a single carbon atom of a parent aromatic ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene, and the like, as well as the various hydro isomers thereof. In preferred embodiments, the aryl group is (C5-C15) aryl, with (C5-C10) being even more referred Particularly preferred aryls are cyclopentadienyl, phenyl and naphthyl.
"Arylaryl" by itself or as part of another substituent refers to a monovalent hydrocarbon group derived by the removal of one hydrogen atom from a single carbon atom of a ring system in which two or more identical or non-identical parent aromatic ring systems are joined directly together by a single bond, where the number of such direct ring junctions is one less than the number of parent aromatic ring systems involved. Typical arylaryl groups include, but are not limited to, biphenyl, triphenyl, phenyl-naphthyl, binaphthyl, biphenyl-naphthyl, and the like. Where the number of carbon atoms in an arylaryl group are specified, the numbers refer to the carbon atoms comprising each parent aromatic ring. For example, (C5-C15) arylaryl is an arylaryl group in which each aromatic ring comprises from 5 to 15 carbons, e.g., biphenyl, triphenyl, binaphthyl, phenylnaphthyl, etc. Preferably, each parent aromatic ring system of an arylaryl group is independently a (C5-C15) aromatic, more preferably a (C5-C10) aromatic. Also preferred are arylaryl groups in which all of the parent aromatic ring systems are identical, e.g., biphenyl, triphenyl, binaphthyl, trinaphthyl, etc.
"Biaryl" by itself or as part of another substituent refers to an arylaryl group having two identical parent aromatic systems joined directly together by a single bond. Typical biaryl groups include, but are not limited to, biphenyl, binaphthyl, bianthracyl, and the like. Preferably, the aromatic ring systems are (C5-C15) aromatic rings, more preferably (C5-C10) aromatic rings. A particularly preferred biaryl group is biphenyl.
"Arylalkyl" by itself or as part of another substituent refers to an acyclic alkyl group in which one of the hydrogen atoms bonded to a carbon atom, typically a terminal or *sp³* carbon atom, is replaced with an aryl group. Typical arylalkyl groups include, but are not limited to, benzyl, 2-phenylethan-1-yl, 2-phenylethen-1-yl, naphthyhmethyl, 2-naphthylethan-1-yl, 2-naphthylethen-1-yl, naphthobenzyl, 2-naphthophenylethan-1-yl and the like. Where specific alkyl moieties are intended, the nomenclature arylalkanyl, arylakenyl and/or arylalkynyl is used. In preferred embodiments, the arylalkyl group is (C6-C21) arylalkyl, *e.g*., the alkanyl, alkenyl or alkynyl moiety of the arylalkyl group is (C1-C6) and the aryl moiety is (C5-C15). In particularly preferred embodiments the arylalkyl group is (C6-C13), *e.g*., the alkanyl, alkanyl or alkynyl moiety of the arylalkyl group is (C1-C3) and the aryl moiety is (C5-C10).
"Parent Heteroaromatic Ring System" refers to a parent aromatic ring system in which one or more carbon atoms are each independently replaced with the same or different heteroatoms or heteroatomic groups. Typical heteroatoms or heteroatomic groups to replace the carbon atoms include, but are not limited to, N, NH, P, O, S, S(O), S(O)₂, Si, etc. Specifically included within the definition of "parent heteroaromatic ring systems" are fused ring systems in which one or more of the rings are aromatic and one or more of the rings are saturated or unsaturated, such as, for example, benzodioxan, benzofuran, chromane, chromene, indole, indoline, xanthene, etc. Also included in the definition of "parent heteroaromatic ring system" are those recognized rings that include common substituents, such as, for example, benzopyrone and 1-methyl-1,2,3,4-tetrazole. Specifically excluded from the definition of "parent heteroaromatic ring system" are benzene rings fused to cyclic polyalkylene glycols such as cyclic polyethylene glycols. Typical parent heteroaromatic ring systems include, but are not limited to, acridine, benzimidazole, benzisoxazole, benzodioxan, benzodioxole, benzofuran, benzopyrone, benzothiadiazole, benzothiazole, benzotriazole, benzoxaxine, benzoxazole, benzoxazoline, carbazole, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like.
"Heteroaryl" by itself or as part of another substituent refers to a monovalent heteroaromatic group having the stated number of ring atoms (*e.g*., "5-14 membered" means from 5 to 14 ring atoms) derived by the removal of one hydrogen atom from a single atom of a parent heteroaromatic ring system. Typical heteroaryl groups include, but are not limited to, groups derived from acridine, benzimidazole, benzisoxazole, benzodioxan, benzodiaxole, benzofuran, benzopyrone, benzothiadiazole, benzothiazole, benzotriazole, benzoxazine, benzoxazole, benzoxazoline, carbazole, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like, as well as the various hydro isomers thereof. In preferred embodiments, the heteroaryl group is a 5-14 membered heteroaryl, with 5-10 membered heteroaryl being particularly preferred.
"Heteroaryl-Heteroaryl" by itself or as part of another substituent refers to a monovalent heteroaromatic group derived by the removal of one hydrogen atom from a single atom of a ring system in which two or more identical or non-identical parent heteroaromatic ring systems are joined directly together by a single bond, where the number of such direct ring junctions is one less than the number of parent heteroaromatic ring systems involved. Typical heteroaryl-heteroaryl groups include, but are not limited to, bipyridyl, tripyridyl, pyridylpurinyl, bipurinyl, etc. Where the number of atoms are specified, the numbers refer to the number of atoms comprising each parent heteroaromatic ring systems. For example, 5-15 membered heteroaryl-heteroaryl is a heteroaryl-heteroaryl group in which each parent heteroaromatic ring system comprises from 5 to 15 atoms, e.g., bipyridyl, tripuridyl, etc. Preferably, each parent heteroaromatic ring system is independently a 5-15 membered heteroaromatic, more preferably a 5-10 membered heteroaromatic. Also preferred are heteroaryl-heteroaryl groups in which all of the parent heteroaromatic ring systems are identical.
"Biheteroaryl" by itself or as part of another substituent refers to a heteroaryl-heteroaryl group having two identical parent heteroaromatic ring systems joined directly together by a single bond. Typical biheteroaryl groups include, but are not limited to, bipyridyl, bipurinyl, biquinolinyl, and the like. Preferably, the heteroaromatic ring systems are 5-15 membered heteroaromatic rings, more preferably 5-10 membered heteroaromatic rings.
"Heteroarylalkyl" by itself or as part of another substituent refers to an acyclic alkyl group in which one of the hydrogen atoms bonded to a carbon atom, typically a terminal or *sp³* carbon atom, is replaced with a heteroaryl group. Where specific alkyl moieties are intended, the nomenclature heteroarylalkanyl, heteroarylakenyl and/or heteroarylalkynyl is used. In preferred embodiments, the heteroarylalkyl group is a 6-21 membered heteroarylalkyl, e.g., the alkanyl, alkenyl or alkynyl moiety of the heteroarylalkyl is (C1-C6) alkyl and the heteroaryl moiety is a 5-15-membered heteroaryl. In particularly preferred embodiments, the heteroarylalkyl is a 6-13 membered heteroarylalkyl, e.g., the alkanyl, alkenyl or alkynyl moiety is (C1-C3) alkyl and the heteroaryl moiety is a 5-10 membered heteroaryl.
"Halogen" or "Halo" by themselves or as part of another substituent, unless otherwise stated, refer to fluoro, chloro, bromo and iodo.
"Haloalkyl" by itself or as part of another substituent refers to an alkyl group in which one or more of the hydrogen atoms is replaced with a halogen. Thus, the term "haloalkyl" is meant to include monohaloalkyls, dihaloalkyls, trihaloalkyls, etc. up to perhaloalkyls. For example, the expression "(C1-C2) haloalkyl" includes fluoromethyl, difluoromethyl, trifluommethyl, 1-fluoroethyl, 1,1-difluoroethyl, 1,2-difluoroethyl, 1,1,1-trifluoroethyl, perfluoroethyl, etc.
   The above-defined groups may include prefixes and/or suffixes that are commonly used in the art to create additional well-recognized substituent groups. As examples, "alkyloxy" or "alkoxy" refers to a group of the formula -OR", "alkylamine" refers to a group of the formula -NHR" and "dialkylamine" refers to a group of the formula -NR"R", where each R" is independently an alkyl. As another example, "haloalkoxy" or "haloalkyloxy" refers to a group of the formula -OR''', where R''' is a haloalkyl.
"Protecting group" refers to a group of atoms that, when attached to a reactive functional group in a molecule, mask, reduce or prevent the reactivity of the functional group. Typically, a protecting group may be selectively removed as desired during the course of a synthesis. Examples of protecting groups can be found in Greene and Wuts, Protective Groups in Organic Chemistry, 3rd Ed., 1999, John Wiley & Sons, NY and Harrison et al., Compendium of Synthetic Organic Methods, Vols.1-8, 1971-1996, John Wiley & Sons, NY. Representative amino protecting groups include, but are not limited to, formyl, acetyl, trifluoroacetyl, benzyl, benzyloxycarbonyl ("CBZ"), tert-butoxycarbonyl ("Boc"), trimethylsilyl ("TMS"), 2-trimethylsilyl-ethanesulfonyl ("TES"), trityl and substituted trityl groups, allyloxycarbonyl, 9-fluorenylmethyloxycarbonyl ("FMOC"), nitroveratryloxycarbonyl ("NVOC") and the like. Representative hydroxyl protecting groups include, but are not limited to, those where the hydroxyl group is either acylated or alkylated such as benzyl and trityl ethers, as well as alkyl ethers, tetrahydropyranyl ethers, trialkylsilyl ethers (*e.g*., TMS or TIPPS groups) and allyl ethers.
"Prodrug" refers to a derivative of an active 2,4-pyrimidinediamine compound (drug) that requires a transformation under the conditions of use, such as within the body, to release the active 2,4-pyrimidinediamine drug. Prodrugs are frequently, but not necessarily, pharmacologically inactive until converted into the active drug. Prodrugs are typically obtained by masking a functional group in the 2,4-pyrimidinediamine drug believed to be in part required for activity with a progroup (defined below) to form a promoiety which undergoes a transformation, such as cleavage, under the specified conditions of use to release the functional group, and hence the active 2,4-pyrimidinediamine drug. The cleavage of the promoiety may proceed spontaneously, such as by way of a hydrolysis reaction, or it may be catalyzed or induced by another agent, such as by an enzyme, by light, by acid or base, or by a change of or exposure to a physical or environmental parameter, such as a change of temperature. The agent may be endogenous to the conditions of use, such as an enzyme present in the cells to which the prodrug is administered or the acidic conditions of the stomach, or it may be supplied exogenously.
   A wide variety of progroups, as well as the resultant promoieties, suitable for masking functional groups in the active 2,4-pyrimidinediamines compounds to yield prodrugs are well-known in the art. For example, a hydroxyl functional group may be masked as a sulfonate, ester or carbonate promoiety, which may be hydrolyzed *in vivo* to provide the hydroxyl group. An amino functional group may be masked as an amide, carbamate, imine, urea, phosphenyl, phosphoryl or sulfenyl promoiety, which may be hydrolyzed *in vivo* to provide the amino group. A carboxyl group may be masked as an ester (including silyl esters and thioesters), amide or hydrazide promoiety, which may be hydrolyzed *in vivo* to provide the carboxyl group. Other specific examples of suitable progroups and their respective promoieties will be apparent to those of skill in the art.
"Progroup" refers to a type of protecting group that, when used to mask a functional group within an active 2,4-pyrimidinediamine drug to form a promoiety, converts the drug into a prodrug. Progroups are typically attached to the functional group of the drug via bonds that are cleavable under specified conditions of use. Thus, a progroup is that portion of a promoiety that cleaves to release the functional group under the specified conditions of use. As a specific example, an amide promoiety of the formula NH-C(O)CH₃ comprises the progroup -C(O)CH₃.
"Fc Receptor" refers to a member of the family of cell surface molecules that binds the Fc portion (containing the specific constant region) of an immunoglobulin. Each Fc receptor binds immunoglobulins of a specific type. For example the Fcα receptor ("FcαR") binds IgA, the FcεR binds IgE and the FcγR binds IgG.
   The FcαR family includes the polymeric Ig receptor involved in epithelial transport of IgA/IgM, the mycloid specific receptor RcαRI (also called CD89), the Fcα/µR and at least two alternative IgA receptors (for a recent review see Monteiro & van de Winkel, 2003, Annu. Rev. Immunol, advanced e-publication. The FcαRI is expressed on neutrophils, eosinophils, moncytes/macrophages, dendritic cells and kupfer cells. The FcαRI inclues one alpha chain and the FcR gamma homodimer that bears an activation motif (ITAM) in the cytoplasmic domain and phosphorylates Syk kinase.
   The FcεR family includes two types, designated FcεRI and FcεRII (also known as CD23). FcεRI is a high affinity receptor (binds IgE with an affinity of about 10¹⁰M⁻¹) found on mast, basophil and eosinophil cells that anchors monomeric IgE to the cell surface. The FcεRI possesses one alpha chain, one beta chain and the gamma chain homodimer discussed above. The FcεRII is a low affinity receptor expressed on mononuclear phagocytes, B lymphocytes, eosinophils and platelets. The FcεRII comprises a single polypeptide chain and does not include the gamma chain homodimer.
   The FcγR family includes three types, designated FcγRI (also known as CD64), FcγRII (also known as CD32) and FcγRIII (also known as CD16). FcγRI is a high affinity receptor (binds IgG1 with an affinity of 10⁸M⁻¹) found on mast, basophil, mononuclear, neutrophil, eosinophil, deudritic and phagocyte cells that anchors nomomeric IgG to the cell surface. The FcγRI includes one alpha chain and the gamma chain dimer shared by FcαRI and FcεRI.
   The FcγRII is a low affinity receptor expressed on neutrophils, monocytes, eosinophils, platelets and B lymphocytes. The FcγRII includes one alpha chain, and does not include the gamma chain homodimer discussed above.
   The FcγRIII is a low affinity (bindes IgG1 with an affinity of 5x10⁵M⁻¹) expressed on NK, eosinophil, macrophage, neutrophil and mast cells. It comprises one alpha chain and the gamma homodimer shared by FcαRI, FcεRI and FcγRI.
   Skilled artisans will recognize that the subunit structure and binding properties of these various Fc receptors, cell types expressing them, are not completely characterized. The above discussion merely reflects the current state-of-the-art regarding these receptors (*see, e.g.,* Immunobiology: The Immune System in Health & Disease, 5th Edition, Janeway et al., Eds, 2001, ISBN 0-8153-3642-x, Figure 9.30 at pp. 371), and is not intended to be limiting with respect to the myriad receptor signaling cascades that can be regulated with the compounds described herein.
"Fc Receptor-Mediated Degranulation" or "Fc Receptor-Induced Degranulation" refers to degranulation that proceeds *via* an Fc receptor signal transduction cascade initiated by crosslinking of an Fc receptor.
"IgE-Induced Degranulation" or "FcεRI-Mediated Degranulation" refers to degranulation that proceeds *via* the IgE receptor signal transduction cascade initiated by crosslinking of FcεR1-bound IgE. The crosslinking may be induced by an IgE-specific allergen or other multivalent binding agent, such as an anti-IgE antibody. Referring to FIG. 2, in mast and/or basophil cells, the FcεRI signaling cascade leading to degranulation may be broken into two stages: upstream and downstream. The upstream stage includes all of the processes that occur prior to calcium ion mobilization (illustrated as "Ca²⁺" in FIG. 2; see also FIG. 3). The downstream stage includes calcium ion mobilization and all processes downstream thereof. Compounds that inhibit FcεRI-mediated degranulation may act at any point along the FcεRI-mediated signal transduction cascade. Compounds that selectively inhibit upstream FcεRI-mediated degranulation act to inhibit that portion of the FcεRI signaling cascade upstream of the point at which calcium ion mobilization is induced. In cell-based assays, compounds that selectively inhibit upstream FcεRI-mediated degranulation inhibit degranulation of cells such as mast or basophil cells that are activated or stimulated with an IgE-specific allergen or binding agent (such as an anti-IgE antibody) but do not appreciably inhibit degranulation of cells that are activated or stimulated with degranulating agents that bypass the FcεRI signaling pathway, such as, for example the calcium ionophores ionomycin and A23187.
"IgG-Induced Degranulation" or "FcγRI-Mediated Degranulation" refers to degranulation that proceeds *via* the FcγRI signal transduction cascade initiated by crosslinking of FcγRI-bound IgG. The crosslinking may be induced by an IgG-specific allergen or another multivalent binding agent, such as an anti-IgG or fragment antibody. Like the FcεRI signaling cascade, in mast and basophil cells the FcγRI signaling cascade also leads to degranulation which may be broken into the same two stages: upstream and downstream. Similar to FcεRI-mediated degranulation, compounds that selectively inhibit upstream FcγRI-mediated degranulation act upstream of the point at which calcium ion mobilization is induced. In cell-based assays, compounds that selectively inhibit upstream FcγRI-mediated degranulation inhibit degranulation of cells such as mast or basophil cells that are activated or stimulated with an IgG-specific allergen or binding agent (such as an anti-IgG antibody or fragment) but do not appreciably inhibit degranulation of cells that are activated or stimulated with degranulating agents that bypass the FcγRI signaling pathway, such as, for example the calcium ionophores ionomycin and A23187.
"Ionophore-Induced Degranulation" or "Ionophore-Mediated Degranulation" refers to degranulation of a cell, such as a mast or basophil cell, that occurs upon exposure to a calcium ionophore such as, for example, ionomycin or A23187.
"Syk Kinsase" refers to the well-known 72kDa non-receptor (cytoplasmic) spleen protein tyrosine kinase expressed in B-cells and other hematopoetic cells. Syk kinase includes two consensus Src-homology 2 (SH2) domains in tandem that bind to phosphorylated immunoreceptor tyrosine-based activation motifs ("ITAMs"), a "linker" domain and a catalytic domain (for a review of the structure and function of Syk kinase see Sada et al., 2001, J. Biochem. (Tokyo) 130:177-186); see also Turner et al., 2000, Immunology Today 21:148-154). Syk kinase has been extensively studied as an effector of B-cell receptor (BCR) signaling (Turner *et al*., 2000, *supra*). Syk kinase is also critical for tyrosine phosphorylation of multiple proteins which regulate important pathways leading from immunoreceptors, such as Ca²⁺ mobilization and mitogen-activated protein kinase (MAPK) cascades (see, e.g., FIG. 2) and degranulation. Syk kinase also plays a critical role in integrin signaling in neutrophils (*see, e.g.,* Mocsai et al. 2002, Immunity 16:547-558).
   As used herein, Syk kinase includes kinases from any species of animal, including but not limited to, homosapiens, simian, bovine, porcine, rodent, etc., recognized as belonging to the Syk family. Specifically included are isoforms, splice variants, allelic variants, mutants, both naturally occuring and man-made. The amino acid sequences of such Syk kinases are well known and available from GENBANK. Specific examples of mRNAs encoding different isoforms of human Syk kinase can be found at GENBANK accession no. gi|21361552|ref|NM_003177.2|, gi|496899|emb|Z29630.1|HSSYKPTK[496899] and gi|15030258|gb|BC011399.1|BC011399[15030258],
   Skilled artisans will appreciate that tyrosine kinases belonging to other families may have active sites or binding pockets that are similar in three-dimensional structure to that of Syk. As a consequence of this structural similarity, such kinases, referred to herein as "Syk mimics," are expected to catalyze phosphorylation of substrates phosphorylated by Syk. Thus, it will be appreciated that such Syk mimics, signal transduction cascades in which such Syk mimics play a role and biological responses effected by such Syk mimics and Syk mimic-dependent signaling cascades may be regulated, and in particular inhibited, with the 2,4-pyrimidinediamine compounds described herein.
"Syk-Dependent Signaling Cascade" refers to a signal transduction cascade in which Syk kinase plays a role. Non-limiting examples of such Syk-dependent signaling cascades include the FcαRI, FcεRI, FcγRI, FcγRIII, BCR and integrin signaling cascades.
"Autoimmune Disease" refers to those diseases which are commonly associated with the nonanaphylactic hypersensitivity reactions (Type II, Type III and/or Type IV hypersensitivity reactions) that generally result as a consequence of the subject's own humoral and/or cell-mediated immune response to one or more immunogenic substances of endogenous and/or exogenous origin. Such autoimmune diseases are distinguished from diseases associated with the anaphylactic (Type I or IgE-mediated) hypersensitivity reactions.

### 6.2 The 2,4-Pyrimidinediamine Compounds

The compounds of the invention are compounds according to structural formula (Ia): 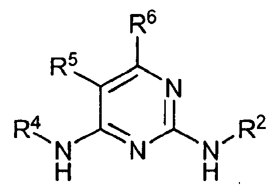 including salts, hydrates, solvates and N-oxides thereof, wherein R², R⁴, R⁵ and R⁶ are as previously defined. Additional specific embodiments of the 2,4-pyrimidinediamine compounds of the invention are described below.

In a first embodiment of the compounds of structural formula (Ia), R², R⁴, R⁵, and R⁶ are as previously defined for (Ia), with the proviso that R² is not 3,4,5-trimethoxyphenyl, 3,4,5-tri (C1-C6) alkoxyphenyl or 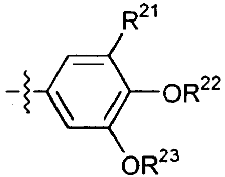 where R²¹, R²² and R²³ are as defined for R¹, R² and R³, respectively of U.S. Patent No. 6,235,746. In a specific embodiment of this first embodiment, R²¹ is hydrogen, halo, straight-chain or branched (C1-C6) alkyl optionally substituted with one or more of the same or different R²⁵ groups, hydroxyl, (C1-C6) alkoxy optionally substituted with one or more of the same or different phenyl or R²⁵ groups, thiol (-SH), (C1-C6) alkylthio optionally substituted with one or more of the same or different phenyl or R²⁵ groups, amino (-NH₂), -NHR²⁶ or -NR²⁶R²⁶; R²² and R²³ are each, independently of one another, a (C1-C6) straight-chain or branched alkyl optionally substituted with one or more of the same or different R²⁵ groups; R²⁵ is selected from the group consisting of halo, hydroxyl, (C1-C6) alkoxy, thiol, (C1-C6) alkylthio, (C1-C6) alkylamino and (C1-C6) dialkylamino; and each R²⁶ is independently a (C1-C6) alkyl optionally substituted with one or more of the same or different phenyl or R²⁵ groups or a -C(O)R²⁷, where R²⁷ is a (C1-C6) alkyl optionally substituted with one or more of the same or different phenyl or R²⁵ groups.

In another specific embodiment of this first embodiment, R²¹ is methoxy optionally substituted with one or more of the same or different halo groups and/or R²² and R²³ are each, independently of one another, a methyl or ethyl optionally substituted with one or more of the same or different halo groups.

In a second embodiment of the compounds of structural formula (Ia), R², R¹, and R⁵ are as previously described for (Ia), and R⁶ is hydrogen, with the proviso that R² is not 3,4,5-trimethoxyphenyl, 3,4,5-tri (C1-C6) alkoxyphenyl or 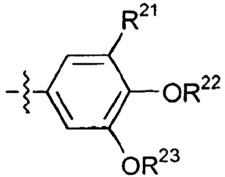 where R²¹, R²² and R²³ are as defined above, in connection with the first embodiment.

In a third embodiment, the compounds of structural formula (Ia) exclude the compounds described in Examples 1-141 of U.S. Patent No. 6,235,746.

In a fourth embodiment, the compounds of structural formula (Ia) exclude compounds defined by formula (1) or formula 1(a) of this U.S. Patent No. 6,235,746 (see, e.g., the disclosure at Col. 1, line 48 through Col. 7, line 49 and Col. 8, lines 9-36).

In a fifth embodiment, the compounds of structural formula (Ia) exclude the compounds defined by formulae (I) and (X) of WO 02/04429 or any compound disclosed in WO 02/04429.

In a sixth embodiment of the compounds of structural formula (Ia), when R⁵ is cyano or -C(O)NHR, where R is hydrogen or (C1-C6) alkyl; and R⁶ is hydrogen, then R² is other than a substituted phenyl group.

In a seventh embodiment, the compounds of structural formula (Ia) exclude compounds defined by formulae (I) and (IV) of U.S. Patent No. 4,983,608 or any compound disclosed in U.S. Patent No. 4,983,608.

Those of skill in the art will appreciate that in the compounds of formula (Ia), R² and R⁴ may be the same or different, and may vary broadly. In R⁴ (and/or R² when R² is an optionally substituted ring, such as optionally substituted cycloalkyls, cycloheteroalkyls, aryls and heteroaryls) the ring may be attached to the remainder of the molecule through any available carbon or heteroatom. The optional substituents may be attached to any available carbon atoms and/or heteroatoms.

In an eighth embodiment of the compounds of structural formula (Ia), R² is an optionally substituted phenyl or an optionally substituted (C5-C15) aryl, subject to the provisos that (I) when R⁶ is hydrogen, then R² is not 3,4,5-trimethoxyphenyl or 3,4,5-tri (C1-C6) alkoxyphenyl; (2) when R² is a 3,4,5-trisubstituted phenyl, then the substituents at the 3- and 4-positions are not simultaneously methoxy or (C1-C6) alkoxy; or (3) when R⁶ is hydrogen then R⁵ is other than cyano. Alternatively, R² is subject to the provisos described in connection with the first or second embodiments. The optionally substituted aryl or phenyl group may be attached to the remainder of the molecule through any available carbon atom. Specific examples of optionally substituted phenyls include phenyls that are optionally mono-, di- or tri-substituted with the same or different R⁸ groups, where R⁸ is as previously defined for structural formula (I) and subject to the above provisos. When the phenyl is mono-substituted, the R⁸ substituent may be positioned at either the *ortho, meta or para* position. When positioned at the *ortho, meta* or *para* position, R⁸ is preferably selected from the group consisting of (C1-C10) alkyl, (C1-C10) branched alkyl, -OR^{a} optionally substituted with one or more of the same or different R^{b} groups, -O-C(O)OR^{a}, -O-(CH₂)*ₘ*-C(O)OR^{a}, -C(O)OR³, -O-(CH₂)*ₘ*-NR^{c}R^{c}, -O-C(O)NR^{c}R^{c}, -O-(CH₂)*ₘ*-C(O)NR^{c}R^{c}, -O-C(NH)NR^{c}R^{c}, -O-(CH₂)*ₘ*-C(NH)NR^{c}R^{c} and -NH-(CH2)*ₘ*-NR^{c}R^{c}, where *m*, R^{a} and R^{c} are as previously defined for structural formula (I). In one embodiment of these compounds, -NR^{c}R^{c} is a 5-6 membered heteroaryl which optionally includes one or more of the same or different additional heteroatoms. Specific examples of such 5-6 membered heteroaryls include, but are not limited to, oxadiazolyl, triazolyl, thiazolyl, oxazolyl, tetrazolyl and isoxazolyl.

In another embodiment of these compounds, -NR^{c}R^{c} is a 5-6 membered saturated cycloheteroalkyl ring which optionally includes one or more of the same or different heteroatoms. Specific examples of such cycloheteroalkyls include, but are not limited to, pyrrolidinyl, pyrazolidinyl, imidazolidinyl, piperidinyl, piperazinyl and morpholinyl.

In still another embodiment of these compounds, each R^{a} is independently a (C1-C6) alkyl and/or each -NR^{c}R^{c} is -NHR^{a}, where R^{a} is a (C1-C6) alkyl. In one specific embodiment, R⁸ is -O-CH₂-C(O)NHCH₃. In another specific embodiment R⁸ is -OH.

When the phenyl is di-substituted or tri-substituted, the R⁸ substituents may be positioned at any combination of positions. For example, the R⁸ substituents may be positioned at the 2,3-, 2,4-, 2,5-, 2,6-, 3,4-, 3,5-, 2,3,4-, 2,3,5-, 2,3,6-, 2,4,5-, 2,4,6-, 2,5,6- or 3,4,5-positions. In one embodiment of compounds including a disubstituted phenyl, the substituents are positioned other than 3,4. In another embodiment they are positioned 3,4. In one embodiment of compounds including a trisubstituted phenyl, the substituents are positioned other than 3,4,5 or, alternatively, no two of the substituents are positioned 3,4. In another embodiment, the substituents are positioned 3,4,5.

Specific examples of R⁸ substituents in such di- and trisubstituted phenyls include the various R⁸ substituents described above in connection with the *ortho, meta* and *para* substituted phenyls.

In another specific embodiment, R⁸ substituents useful for substitituting such di-and trisubstituted phenyls include (C1-C6) alkyl, (C1-C6) alkoxy, methoxy, halo, chloro, (C1-C6) perhaloalkyl, -CF₃, (C1-C6) perhaloalkoxy and -OCF₃. In a preferred embodiment, such R⁸ substituents are positioned 3,4 or 3,5. Specific examples of preferred di-substituted phenyl rings include 3-chloro-4-methoxy-phenyl, 3-methoxy-4-chlorophenyl, 3-chloro-4-trifluoromethoxy-phenyl, 3-trifluoromethoxy-4-chloro-phenyl, 3,4-dichloro-phenyl, 3,4-dimethoxyphenyl and 3,5-dimethoxyphenyl.

In another embodiment of compounds including a trisubstituted phenyl, the trisubstituted phenyl has the formula: 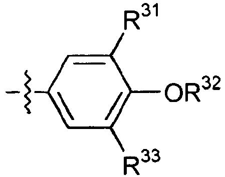 wherein: R³¹ is methyl or (C1-C6) alkyl; R³² is hydrogen, methyl or (C1-C6) alkyl; and R³³ is a halo group.

In a ninth embodiment of the compounds of structural formula (Ia), R² is an optionally substituted heteroaryl. Typical heteroaryl groups according to this thirteenth embodiment comprise from 5 to 15, and more typically from 5 to 11 ring atoms, and include one, two, three or four of the same or different heteratoms or heteroatomic groups selected from the group consisting ofN, NH, O, S, S(O) and S(O)₂. The optionally substituted heteroaryl may be attached to its C2 nitrogen atom through any available carbon atom or heteroatom, but is typically attached *via* a carbon atom. The optional substituents may be the same or different, and may be attached to any available carbon atom or heteroatom. In one embodiment of these compounds, R⁵ is other than bromo, nitro, trifluoromethyl, cyano or -C(O)NHR, where R is hydrogen or (C1-C6) alkyl. In still another embodiment of compounds including an optionally substituted heteroaryl group as the R² substituent, the heteroaryl is unsubstituted or substituted with from one to four of the same or different R⁸ groups, where R⁸ is as previously defined for structural formula (Ia). Specific examples of such optionally substituted heteroaryls include, but are not limited to, the following heteroaryl groups: 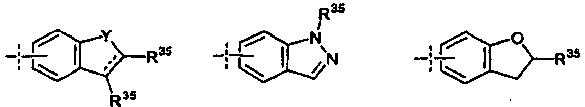 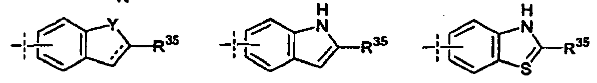 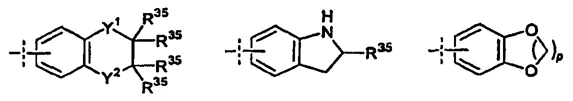 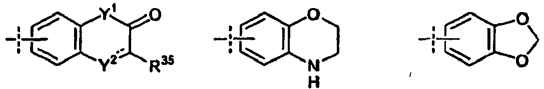 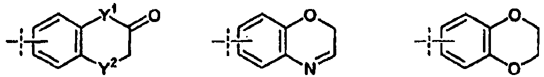 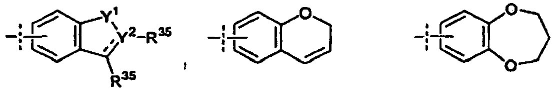 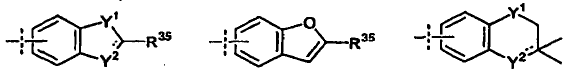 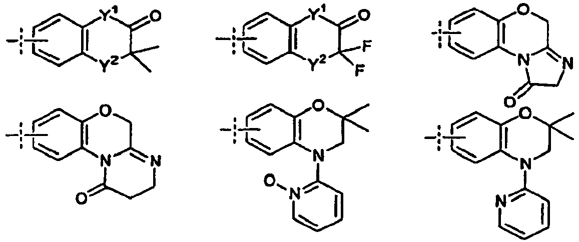 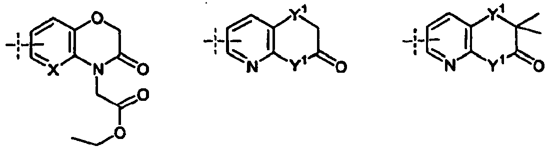 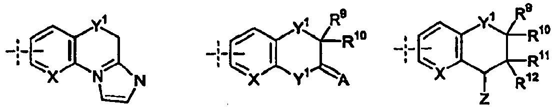 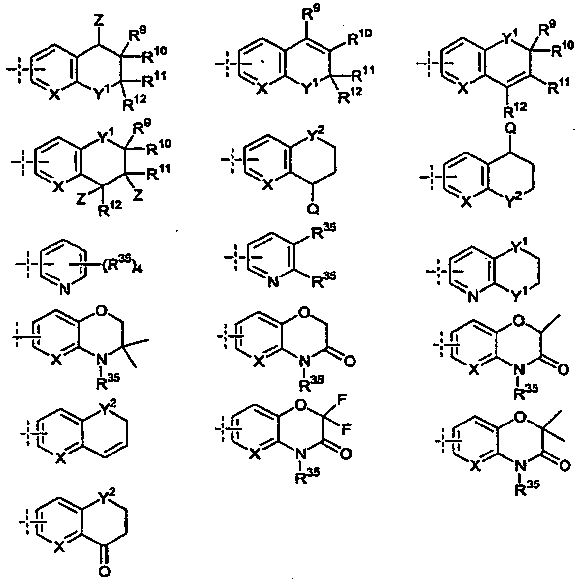 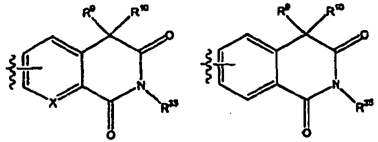 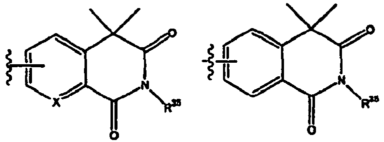 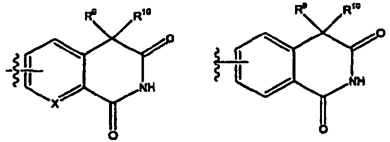 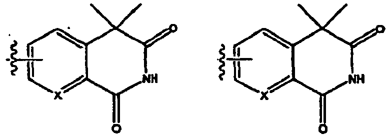 wherein:
p is an integer from one to three;
each --- independently represents a single bond or a double bond;
R³⁵ is hydrogen or R⁸, where R⁸ is as previously defined for structural formula (I);
X is selected from the group consisting of CH, N and N-O;
each Y is independently selected from the group consisting of O, S and NH;
each Y¹ is independently selected from the group consisting of O, S, SO, SO₂, SONR³⁶, NH and NR³⁷;
each Y² is independently selected from the group consisting of CH, CH₂, O, S, N, NH and NR³⁷;
R³⁶ is hydrogen or alkyl;
R³⁷ is selected from the group consisting of hydrogen and a progroup, preferably hydrogen or a progroup selected from the group consisting of aryl, arylalkyl, heteroaryl, R^{a}, R^{b}-CR^{a}R^{b}-O-C(O)R⁸, -CR^{a}R^{b}-O-PO(OR⁸)₂, -CH₂-O-PO(OR⁸)₂, -CH₂-PO(OR⁸)₂, -C(O)-CR^{a}R^{b}-N(CH₃)₂, -CR^{a}R^{b}-O-C(O)-CR⁸R^{b}-N(CH₃)₂, -C(O)R⁸, -C(O)CF₃ and -C(O)-NR⁸-C(O)R⁸;
A is selected from the group consisting of O, NH and NR³⁸;
R³⁸ is selected from the group consisting of alkyl and aryl;
R⁹, R¹⁰, R¹¹ and R¹² are each, independently of one another, selected from the group consisting of alkyl, alkoxy, halogen, haloalkoxy, aminoalkyl and hydroxyalkyl, or, alternatively, R⁹ and R¹⁰ and/or R¹¹ and R¹² are taken together form a ketal;
each Z is selected from the group consisting of hydroxyl, alkoxy, aryloxy, ester, carbamate and sulfonyl;
Q is selected from the group consisting of -OH, OR⁸, NR^{c}R^{c}, -NHR³⁹-C(O)R⁸, -NHR³⁹-C(O)OR⁸, -NR³⁹-CHR⁴⁰-R^{b}, -NR³⁹-(CH₂)*ₘ*-R^{b} and -NR³⁹-C(O)-CHR⁴⁰-NR^{c}R^{c};
R³⁹ and R⁴⁰ are each, independently of one another, selected from the group consisting of hydrogen, alkyl, aryl, alkylaryl;arylalkyl and NHR⁸; and
R^{a}, R^{b} and R^{c} are as previously defined for structural formula (I). Preferred R^{b} substitutents for Q are selected from -C(O)OR⁸, -O-C(O)R⁸, -O-P(O)(OR⁸)₂ and -P(O)(OR⁸)₂.

In one embodiment of the above-depicted heteroaryls, as well as other 5-15 membered heteroaryls according to this embodiment of the invention, each R⁸ is independently selected from the group consisting of R^{d}, -NR^{c}R^{c}, -(CH₂)*ₘ*-NR^{c}R^{c}, -C(O)NR^{c}R^{c}, -(CH₂)*ₘ*-C(O)NR^{c}R^{c}, -C(O)OR^{d}, -(CH₂)*ₘ*-C(O)OR^{d} and -(CH₂)*ₘ*-OR^{d}, where m, R^{c} and R^{d} are as previously defined for structural formula (I).

In a specific embodiment, R^{d} and/or R^{c} is selected from the group consisting of R^{a} and (C3-C8) cycloalkyl optionally substituted with one or more of the same or different hydroxyl, amino or carboxyl groups.

In another embodiment of the above-depicted heteroaryls, each R³⁵ is a hydrogen atom, a (C1-C6) carbon chain, including methyl, ethyl, isopropyl, a cycloalkyl group, including cyclopropyl, cyclobutyl, cyclopentyl, cyclohexyl, cycloheptyl, cyclooctyl, a 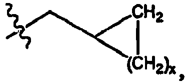 wherein x = 1-8, -CH₂CONHMe, -CH₂CH₂NHMe, - CH₂CH₂CONHMe, -CH₂CH₂CH₂NHMe or -CH₂CH₂CH₂OCH₃.

In still another embodiment of the above-depicted heteroaryls, the aromatic ring connectivity is either at the 5 or 6 position.

In a tenth embodiment of the compounds of structural formula (Ia), R² is an optionally substituted phenyl, aryl or heteroaryl, with the provisos that: (1) when R⁶ and optionally R⁵ is hydrogen, then R² is other than 3,4,5-trimethoxyphenyl or 3,4,5-tri(C1-C6) alkoxyphenyl; (3) when R⁴ is 3-methoxyphenyl or 3-(C1-C6) alkoxyphenyl and R² is a 3,4,5-trisubstituted phenyl, the substituents positioned at the 3 and 4 positions are not both simultaneously methoxy or (C1-C6) alkoxy; (4) when R² is a substituted phenyl and R⁶ is hydrogen, then R⁵ is other than cyano or -C(O)NHR, where R is hydrogen or (C1-C6) alkyl; and/or (5) when R² and R⁴ are each independently a substituted or unsubstituted pyrrole or indole, then the pyrrole or indole is attached to the remainder of the molecule *via* a ring carbon atom. Alternatively, R² is subject to the provisos described in connection with the first or second embodiment.

In this tenth embodiment of the invention, the R² and R⁴ substituents may be the same or different. Specific optionally substituted phenyl, aryl and/or heteroaryls include those illustrated above in connection with the eighth and ninth embodiments.

In an eleventh embodiment of the compounds of structural formulae (I) and (Ia), including the above-described first through fourteenth embodiments thereof, R⁶ is hydrogen and R⁵ represents a group as defined for formula (Ia) above which is an electronegative group. As will be recognized by skilled artisans, electronegative groups are atoms or groups of atoms that have a relatively great tendency to attract electrons to themselves. Specific examples of electronegative groups according to this eleventh embodiment include, but are not limited to, -CN, -NC, -NO₂, halo, bromo, chloro, fluoro, (C1-C3) haloalkyl, (C1-C3) perhaloalkyl, (C1-C3) fluoroalkyl, (C1-C3) perfluoroalkyl, -CF₃, (C1-C3) haloalkoxy, (C1-C3) perhaloalkoxy, (C1-C3) fluoroalkoxy, (C1-C3) perfluoroalkoxy, -OCF₃, -C(O)R^{a}, -C(O)OR^{a}, -C(O)CF₃ and -C(O)OCF₃. In a specific embodiment, the electronegative group is a halogen-containing electronegative group, such as -OCF₃, -CF₃, bromo, chloro or fluoro. In another specific embodiment, R⁵ is fluoro.

In a twelfth embodiment, the compounds of structural formula (Ia) are compounds according to structural formula (Ic): 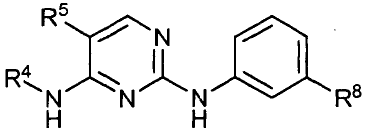 and salts, hydrates, solvates and N-oxides thereof, wherein:
R⁴ is as defined for formula (Ia) above;
R⁵ is F or -CF₃; and
R⁸ is -O(CH₂)*ₘ*-R^{b}, where *m* and R^{b} are as previously defined for structural formula (I). In a specific embodiment, R⁸ is -O-CH₂-C(O)NH-CH₃ and/or R⁴ is a heteroaryl according to the thirteenth embodiment.

In a thirteenth embodiment, the compounds of structural formula (Ia) include any compound selected from TABLE 1 that inhibits an Fc receptor signal transduction cascade, a Syk kinase activity, a Syk-kinase dependent receptor signal transduction cascade orcell degranulation as measured in an *in vitro* assay, optionally subject to the proviso that the compound is not a compound excluded by the above-described embodiments. In a specific embodiment, such compounds have an IC₅₀ of about 20 µM or less as measured in an *in vitro* degranulation assay, such as one of the degranulation assays described in the Examples section.

In a fourteenth embodiment, the compounds of structural formula (Ia) include any compound selected from TABLE 1 that inhibits the FcγR1 or FcεR1 receptor cascade with an IC₅₀ of about 20 µM or less as measured in an *in vitro* assay, such as one of the *in vitro* assays provided in the Examples section, optionally subject to the proviso that the compound is not a compound excluded by the above-described embodiments.

In a fifthteenth embodiment, the compounds of structural formulae (Ia) are those wherein R² is selected from the group consisting of

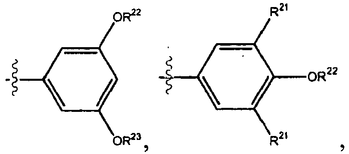

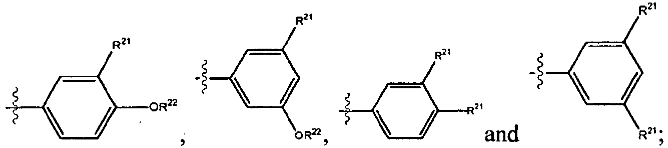

R⁴, R⁸, R^{a}, R^{b}, R^{c}, R^{d} are as described above, R⁵ is a fluorine atom; R⁶ is a hydrogen atom and each R²¹ is independently a halogen atoms or an alkyl optionally substituted with one or more of the same or different halo groups, R²² and R²³ are each, independently of one another, a hydrogen atom, methyl or ethyl optionally substituted with one or more of the same or different halo groups, each *m* is independently an integer from 1 to 3, and each *n* is independently an integer from 0 to 3.

In a sixteenth embodiment, applicable to the first through fifteenth embodiments, R⁵ is a halogen atom, such as fluorine, and R⁶ is a hydrogen atom.

Also specifically described are combinations of the above first through sixteenth embodiments.

Those of skill in the art will appreciate that the 2,4-pyrimidinediamine compounds described herein may include functional groups that can be masked with progroups to create prodrugs. Produgs of the 2,4-pyrimidinediamine compounds defined in the appended claims are not part of the present invention. Such prodrugs are usually, but need not be, pharmacologically inactive until converted into their active drug form. Indeed, active 2,4-pyrimidinediamine compounds of formula (Ia) described in TABLE 1, *infra*, may include promoieties that are hydrolyzable or otherwise cleavable under conditions of use. For example, ester groups commonly undergo acid-catalyzed hydrolysis to yield the parent carboxylic acid when exposed to the acidic conditions of the stomach, or base-catalyzed hydrolysis when exposed to the basic conditions of the intestine or blood. Thus, when administered to a subject orally, 2,4-pyrimidinediamines that include ester moieties may be considered prodrugs of their corresponding carboxylic acid, regardless of whether the ester form is pharmacologically active. Referring to TABLE 1, numerous ester-containing 2,4-pyrimidinediamines of the invention are active in their ester, "prodrug" form.

In the prodrugs described herein, any available functional moiety may be masked with a progroup to yield a prodrug. Functional groups within the 2,4-pyrimidinediamine compounds that may be masked with progroups for inclusion in a promoiety include, but are not limited to, amines (primary and secondary), hydroxyls, sulfanyls (thiols), carboxyls, etc. Myriad progroups suitable for masking such functional groups to yield promoieties that are cleavable under the desired conditions of use are known in the art. All of these progroups, alone or in combinations, may be included in the prodrugs described herein.

In one illustrative embodiment, the prodrugs described herein are compounds according to structural formula (Ia) in which R^{c} and R^{d} may be, in addition to their previously-defined alternatives, a progroup.

Replacing the hydrogens attached to N2 and N4 in the 2,4-pyrimidinediamines of structural formula (Ia) with substituents adversely effects the activity of the compounds. However, as will be appreciated by skilled artisans, these nitrogens may be included in promoieties that, under conditions of use, cleave to yield 2,4-pyrimidinediamines according to structural formula (Ia). Thus, the prodrugs include compounds according to structural formula (II): 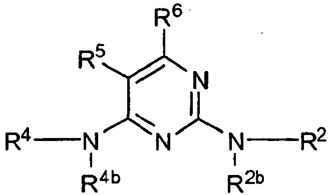 including salts, hydrates, solvates and N-oxides thereof, wherein:
R², R⁴, R⁵ and R⁶ are as previously defined for structural formula (Ia); and
R^{2b} and R^{4b} are each, independently of one another, a progroup. Specific examples of such progroups include, but are not limited to, (C1-C6) alkyl, -C(O)CH₃, -C(O)NHR³⁶ and -S(O)₂R³⁶, where R³⁶ is (C1-C6) alkyl; (C5-C15) aryl and (C3-C8) cycloalkyl.

In the prodrugs of structural formula (11), the various substituents may be as described for the various first through fifteenth embodiments previously described for the compounds of structural formula (Ia), or combinations of such embodiments.

Those of skill in the art will appreciate that many of the compounds of the invention and prodrugs thereof, as well as the various compound species specifically described and/or illustrated herein, may exhibit the phenomena of tautomerism, conformational isomerism, geometric isomerism and/or optical isomerism. For example, the compounds of the invention and prodrugs thereof may include one or more chiral centers and/or double bonds and as a consequence may exist as stereoisomers, such as double-bond isomers (i.e., geometric isomers), enantiomers and diasteromers and mixtures thereof, such as racemic mixtures. As another example, the compounds and prodrugs of the invention may exist in several tautomeric forms, including the enol form, the keto form and mixtures thereof. As the various compound names, formulae and compound drawings within the specification and claims can represent only one of the possible tautomeric, conformational isomeric, optical isomeric or geometric isomeric forms, it should be understood that the invention encompasses any tautomeric, conformational isomeric, optical isomeric and/or geometric isomeric forms of the compounds or prodrugs having one or more of the utilities described herein, as well as mixtures of these various different isomeric forms. In cases of limited rotation around the 2,4-pryimidinediamine core structure, atrop isomers are also possible and are also specifically included in the compounds of the invention.

Moreover, skilled artisans will appreciate that when lists of alternative substituents include members which, owing to valency requirements or other reasons, cannot be used to substitute a particular group, the list is intended to be read in context to include those members of the list that are suitable for substituting the particular group. For example, skilled artisans will appreciate that while all of the listed alternatives for R^{b} can be used to substitute an alkyl group, certain of the alternatives, such as =O, cannot be used to substitute a phenyl group. It is to be understood that only possible combinations of substituent-group pairs are intended.

The compounds of the invention and/or prodrugs thereof may be identified by either their chemical structure or their chemical name. When the chemical structure and the chemical name conflict, the chemical structure is determinative of the identity of the specific compound.

Depending upon the nature of the various substituents, the 2,4-pyrimidinediamine compounds of the invention and prodrugs thereof may be in the form of salts. Such salts include salts suitable for pharmaceutical uses ("pharmaceutically-acceptable salts"), salts suitable for veterinary uses, etc. Such salts may be derived from acids or bases, as is well-known in the art.

In one embodiment, the salt is a pharmaceutically acceptable salt. Generally, pharmaceutically acceptable salts are those salts that retain substantially one or more of the desired pharmacological activities of the parent compound and which are suitable for administration to humans. Pharmaceutically acceptable salts include acid addition salts formed with inorganic acids or organic acids. Inorganic acids suitable for forming pharmaceutically acceptable acid addition salts include, by way of example and not limitation, hydrohalide acids (*e.g*., hydrochloric acid, hydrobromic acid, hydriodic, etc.), sulfuric acid, nitric acid, phosphoric acid, and the like. Organic acids suitable for forming pharmaceutically acceptable acid addition salts include, by way of example and not limitation, acetic acid, trifluoroacetic acid, propionic acid, hexanoic acid, cyclopentanepropionic acid, glycolic acid, oxalic acid, pyruvic acid, lactic acid, malonic acid, succinic acid, malic acid, maleic acid, fumaric acid, tartaric acid, citric acid, palmitic acid, benzoic acid, 3-(4-hydroxybenzoyl) benzoic acid, cinnamic acid, mandelic acid, alkylsulfonic acids (*e.g*., methanesulfonic acid, ethanesulfonic acid, 1,2-ethane-disulfonic acid, 2-hydroxyethanesulfonic acid, etc.), arylsulfonic acids (*e.g*., benzenesulfonic acid, 4-chlorobenzenesulfonic acid, 2-naphthalenesulfonic acid, 4-toluenesulfonic acid, cycloalkylsulfonic acids (*e.g*., camphorsulfonic acid), 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like.

Pharmaceutically acceptable salts also include salts formed when an acidic proton present in the parent compound is either replaced by a metal ion (*e.g*., an alkali metal ion, an alkaline earth metal ion or an aluminum ion), an ammonium ion or coordinates with an organic base (*e.g.*, ethanolamine, diethanolamine, triethanolamine, N-methylglucamine, morpholine, piperidine, dimethylamine, diethylamine, etc.).

The 2,4-pyrimidinediamine compounds and of the invention, as well as the salts thereof, may also be in the form of hydrates, solvates and N-oxides, as are well-known in the art.

### 6.3 Methods of Synthesis

The compounds of the invention and prodrugs thereof may be synthesized via a variety of different synthetic routes using commercially available starting materials and/or starting materials prepared by conventional synthetic methods. Suitable exemplary methods that may be routinely adapted to synthesize the 2,4-pyrimidinediamine compounds of the invention and prodrugs thereof are found in U.S. Patent No. 5,958,935. Specific examples describing the synthesis of numerous compounds of the invention and prodrugs thereof, as well as intermediates therefor, are provided in the Examples section. All of the compounds of structural formula (Ia) and (II) may be prepared by routine adaptation of these methods.

A variety of exemplary synthetic routes that can be used to synthesize the 2,4-pyrimidinediamine compounds of the invention are described in Schemes (I)-(XI), below. In Schemes (I)-(XI), like-numbered compounds have similar structures. These methods may be routinely adapted to synthesize the prodrugs according to structural formula (II).

In one exemplary embodiment, the compounds can be synthesized from substituted or unsubstituted uracils or thiouracils as illustrated in Scheme (I), below:

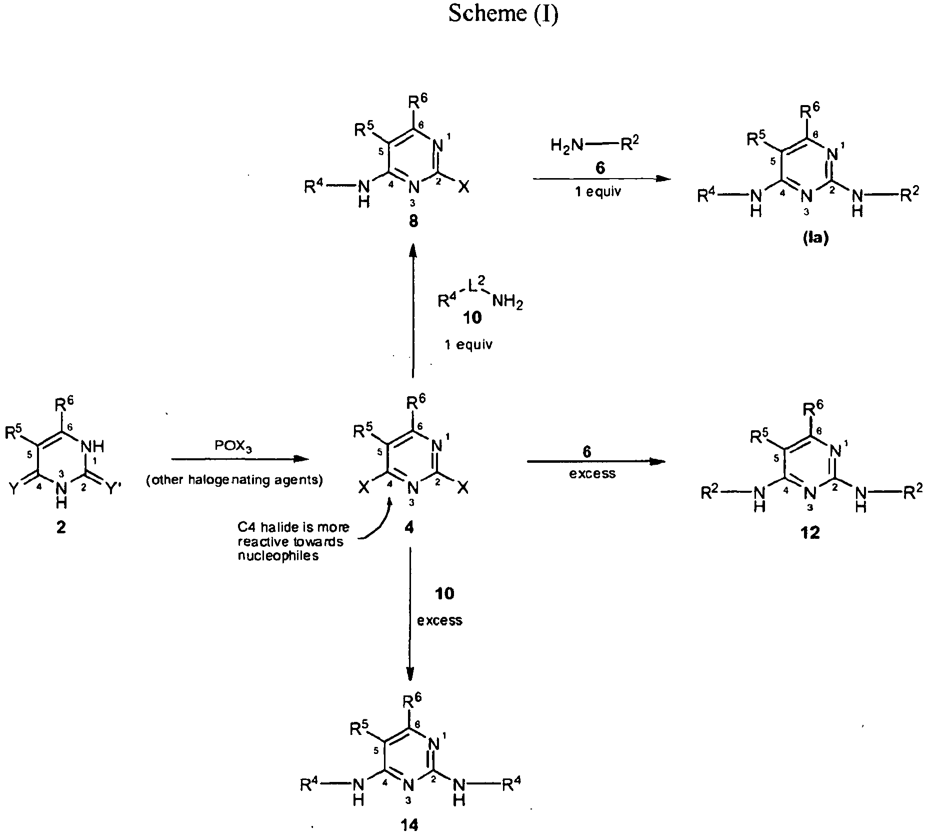

In Scheme (I), R², R⁴, R⁵, R⁶ are as previously defined for structural formula (Ia), X is a halogen (*e.g*., F, Cl, Br or I) and Y and Y' are each, independently of one another, selected from the group consisting of O and S. Referring to Scheme (I), uracil or thiouracil 2 is dihalogenated at the 2- and 4-positions using standard halogenating agent POX₃ (or other standard halogenating agent) under standard conditions to yield 2,4-bishalo pyrimidine **4**. Depending upon the R⁵ substituent, in pyrimidine **4**, the halide at the C4 position is more reactive towards nucleophiles than the halide at the C2 position. This differential reactivity can be exploited to synthesize 2,4-pyrimidinediamines according structural formula (Ia) by first reacting 2,4-bishalopyrimidine **4** with one equivalent of amine **10**, yielding 4N-substituted-2-halo-4-pyrimidineamine **8**, followed by amine **6** to yield a 2,4-pyrimidinediamine according structural formula (I). 2N,4N-bis(substituted)-2,4-pyrimidinediamines **12** and **14** can be obtained by reacting 2,4-bishalopyrimidine **4** with excess **6** or **10**, respectively.

In most situations, the C4 halide is more reactive towards nucleophiles, as illustrated in the Scheme. However, as will be recognized by skilled artisans, the identity of the R⁵ substituent may alter this reactivity. For example, when R⁵ is trifluoromethyl, a 50:50 mixture of 4N-substituted-4-pyrimidineamine **8** and the corresponding 2N-substituted-2-pyrimidineamine is obtained. Regardless of the identity of the R⁵ substituent, the regioselectivity of the reaction can be controlled by adjusting the solvent and other synthetic conditions (such as temperature), as is well-known in the art.

The reactions depicted in Scheme (I) may proceed more quickly when the reaction mixtures are heated via microwave. When heating in this fashion, the following conditions may be used: heat to 175°C in ethanol for 5-20 min. in a Smith Reactor (Personal Chemistry) in a sealed tube (at 20 bar pressure).

The uracil or thiouracil **2** starting materials may be purchased from commercial sources or prepared using standard techniques of organic chemistry. Commercially available uracils and thiouracils that can be used as starting materials in Scheme (I) include, by way of example and not limitation, uracil (Aldrich #13,078-8; CAS Registry 66-22-8); 2-thio-uracil (Aldrich #11,558-4; CAS Registry 141-90-2); 2,4-dithiouracil (Aldrich #15,846-1; CAS Registry 2001-93-6); 5-acetouracil (Chem. Sources Int'I 2000; CAS Registry 6214-65-9); 5-azidouracil; 5-aminouracil (Aldrich #85,528-6; CAS Registry 932-52-5); 5-bromouracil (Aldrich #85,247-3; CAS Registry 51-20-7); 5-(trans-2-bromovinyl)-uracil (Aldrich #45,744-2; CAS Registry 69304-49-0); 5-(trans-2-chlorovinyl)-uracil (CAS Registry 81751-48-2); 5-(trans-2-carboxyvinyl)-uracil; uracil-5-carboxylic acid (2,4-dihydroxypyrimidine-5-carboxylic acid hydrate; Aldrich #27,770-3; CAS Registry 23945-44-0); 5-chlorouracil (Aldrich #22,458-8; CAS Registry 1820-81-1); 5-cyanouracil (Chem. Sources Int'1 2000; CAS Registry 4425-56-3); 5-ethyluracil (Aldrich #23,044-8; CAS Registry 4212-49-1); 5-ethenyluracil (CAS Registry 37107-81-6); 5-fluorouracil (Aldrich #85,847-1; CAS Registry 51-21-8); 5-iodouracil (Aldrich #85,785-8; CAS Registry 696-07-1); 5-methyluracil (thymine; Aldrich #13,199-7; CAS Registry 65-71-4); 5-nitrouracil (Aldrich #85,276-7; CAS Registry 611-08-5); uracil-5-sulfamic acid (Chem. Sources Int'1 2000; CAS Registry 5435-16-5); 5-(trifluoromethyl)-uracil (Aldrich #22,327-1; CAS Registry 54-20-6); 5-(2,2,2-trifluoroethyl)-uracil (CAS Registry 155143-31-6); 5-(pentafluoroethyl)-uracil (CAS Registry 60007-38-3); 6-aminouracil (Aldrich #A5060-6; CAS Registry 873-83-6) uracil-6-carboxylic acid (orotic acid; Aldrich #0-840-2; CAS Registry 50887-69-9); 6-methyluracil (Aldrich #D11,520-7; CAS Registry 626-48-2); uracil-5-amino-6-carboxylic acid (5-aminoorotic acid; Aldrich #19,121-3; CAS Registry #7164-43-4); 6-amino-5-nitrosouracil (6-amino-2,4-dihydroxy-5-nitrosopyrimidine; Aldrich #27,689-8; CAS Registry 5442-24-0); uracil-5-fluoro-6-carboxylic acid (5-fluoroorotic acid; Aldrich #42,513-3; CAS Registry 00000-00-0); and uracil-5-nitro-6-carboxylic acid (5-nitroorotic acid; Aldrich #18,528-0; CAS Registry 600779-49-9). Additional 5-, 6- and 5,6-substituted uracils and/or thiouracils are available from General Intermediates of Canada, Inc., Edmonton, Alberta, CA (www.generalintermediates.com) and/or Interchim, France (www.interchim.com), or may be prepared using standard techniques. Myriad textbook references teaching suitable synthetic methods are provided *infra.*

Amines **6** and **10** may be purchased from commercial sources or, alternatively, may be synthesized utilizing standard techniques. For example, suitable amines may be synthesized from nitro precursors using standard chemistry. Specific exemplary reactions are provided in the Examples section. See also Vogel, 1989, Practical Organic Chemistry, Addison Wesley Longman, Ltd. and John Wiley & Sons, Inc.

Skilled artisans will recognize that in some instances, amines **6** and **10** and/or substituents R⁵ and/or R⁶ on uracil or thiouracil **2** may include functional groups that require protection during synthesis. The exact identity of any protecting group(s) used will depend upon the identity of the functional group being protected, and will be apparent to these of skill in the art. Guidance for selecting appropriate protecting groups, as well as synthetic strategies for their attachment and removal, may be found, for example, in Greene & Wuts, Protective Groups in Organic Synthesis, 3d Edition, John Wiley & Sons, Inc., New York (1999) and the references cited therein (hereinafter "Greene & Wuts").

A specific embodiment of Scheme (I) utilizing 5-fluorouracil (Aldrich #32,937-1) as a starting material is illustrated in Scheme (Ia), below:

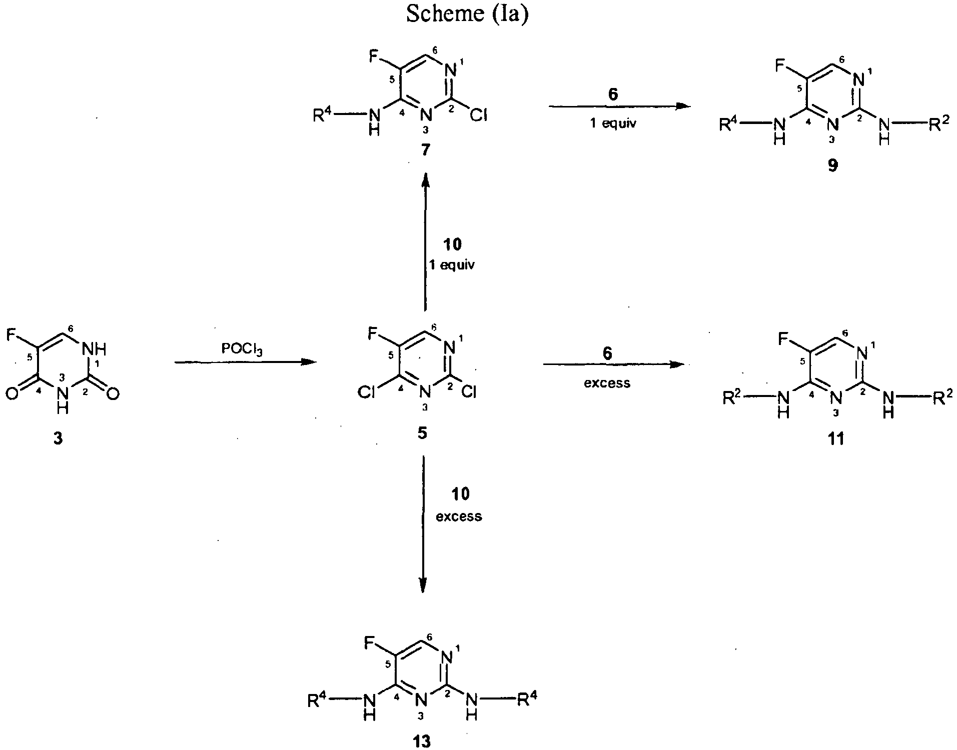

In Scheme (Ia), R², R⁴ are as previously defined for Scheme (I). According to Scheme (Ia), 5-fluorouracil **3** is halogenated with POCl₃ to yield 2,4-dichloro-5-fluoropyrimidine **5**, which is then reacted with excess amine **6** or **10** to yield N2,N4-bis substituted 5-fluoro-2,4-pyrimidinediamine **11** or **13**, respectively. Alternatively, asymmetric 2N,4N-disubstituted-5-fluoro-2,4-pyrimidinediamine **9** may be obtained by reacting 2,4-dichloro-5-fluoropyrimidine **5** with one equivalent of amine **10** (to yield 2-chloro-N4-substituted-5-fluoro-4-pyrimidineamine 7) followed by one or more equivalents of amine **6**.

In another exemplary embodiment, the 2,4-pyrimidinediamine compounds of the invention may be synthesized from substituted or unsubstituted cytosines as illustrated in Schemes (IIa) and (IIb), below:

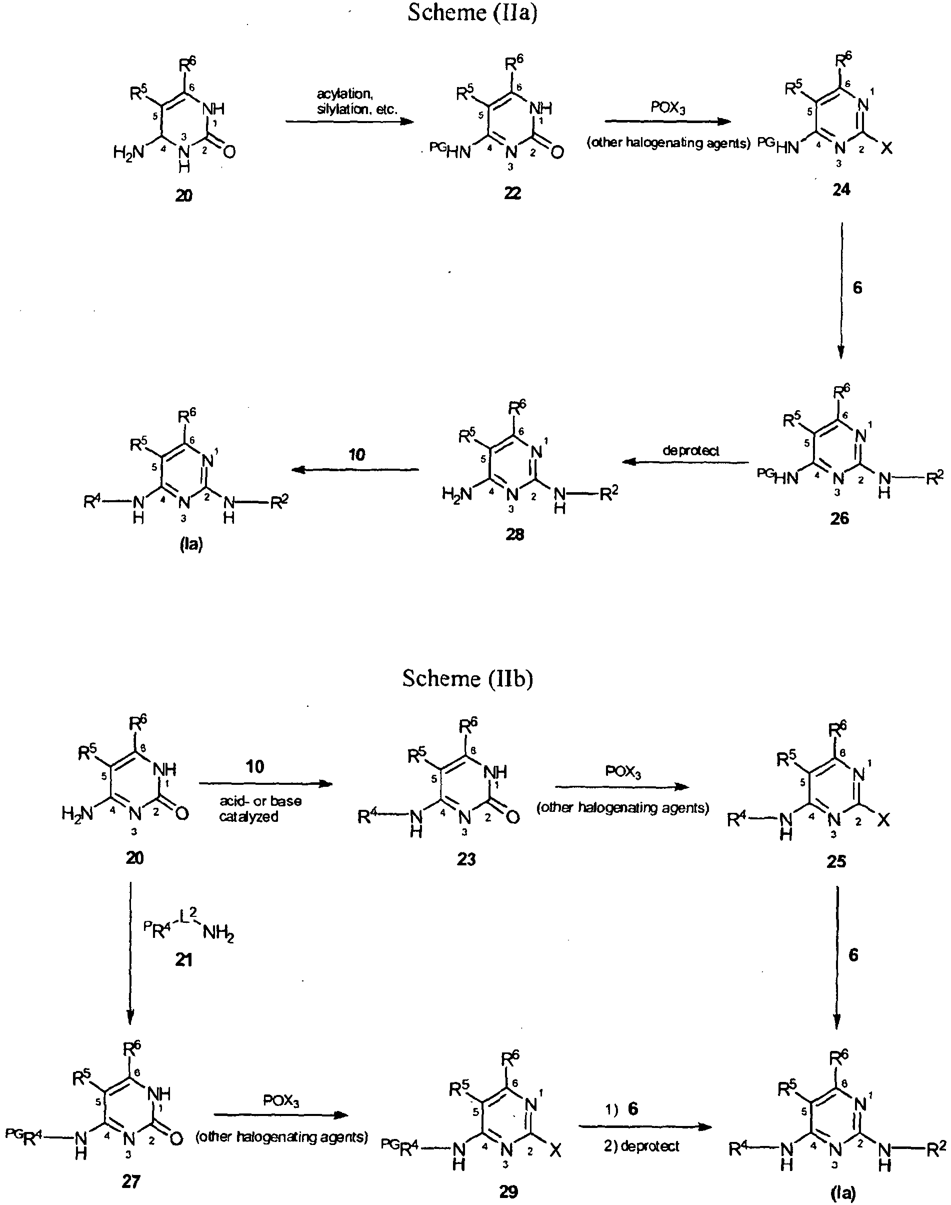

In Schemes (IIa) and (IIb), R², R⁴, R⁵, R⁶ and X are as previously defined for Scheme (I) and PG represents a protecting group. Referring to Scheme (IIa), the C4 exocyclic amine of cytosine **20** is first protected with a suitable protecting group PG to yield N4-protected cytosine **22**. For specific guidance regarding protecting groups useful in this context, *see* Vorbrüggen and Ruh-Pohlenz, 2001, Handbook of Nucleoside Synthesis, John Wiley & Sons, NY, pp. 1-631 ("Vorbruggen"). Protected cytosine **22** is halogenated at the C2 position using a standard halogenation reagent under standard conditions to yield 2-chloro-4N-protected-4-pyrimidineamine **24**. Reaction with amine **6** followed by deprotection of the C4 exocyclic amine and reaction with amine **10** yields a 2,4-pyrimidinediamine according to structural formula **(Ia)**.

Alternatively, referring to Scheme (IIb), cytosine **20** may be reacted with amine **10** or protected amine **21** to yield N4-substituted cytosine **23** or **27**, respectively. These substituted cytosines may then be halogenated as previously described, deprotected (in the case of N4-substituted cytosine **27**) and reacted with amine **6** to yield a 2,4-pyrimidinediamine according to structural formula **(Ia)**.

Commercially-available cytosines that may be used as starting materials in Schemes (IIa) and (IIb) include, but are not limited to, cytosine (Aldrich #14,201-8; CAS Registry 71-30-7); N⁴-acetylcytosine (Aldrich #37,791-0; CAS Registry 14631-20-0); 5-fluorocytosine (Aldrich #27,159-4; CAS Registry 2022-85-7); and 5-(trifluoromethyl)-cytosine. Other suitable cytosines useful as starting materials in Schemes (IIa) are available from General Intermediates of Canada, Inc., Edmonton, Alberta, CA (www.generalintermediates.com) and/or Interchim, France (www.interchim.com), or may be prepared using standard techniques. Myriad textbook references teaching suitable synthetic methods are provided *infra.*

In still another exemplary embodiment, the 2,4-pyrimidinediamine compounds of the invention may be synthesized from substituted or unsubstituted 2-amino-4-pyrimidinols as illustrated in Scheme (III), below:

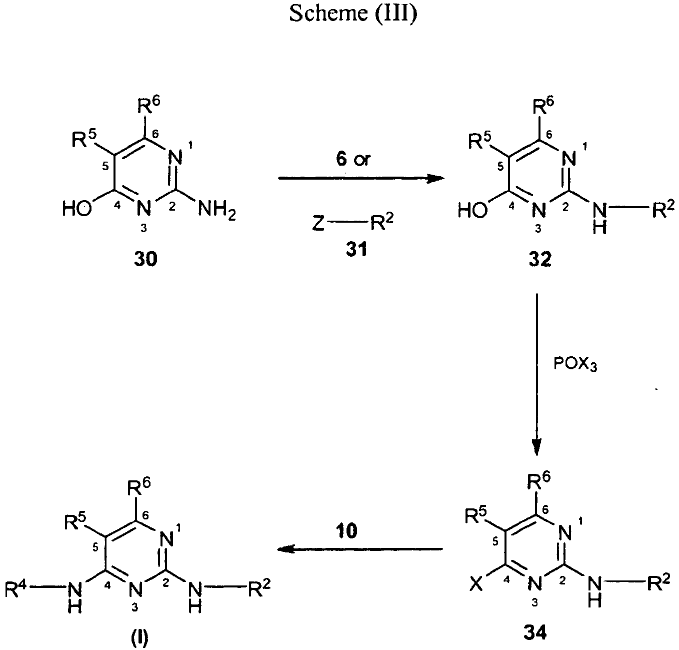

In Scheme (III), R², R⁴, R⁵, R⁶, and X are as previously defined for Scheme (I) and Z is a leaving group as discussed in more detail in connection with Scheme IV, *infra.* Referring to Scheme (III), 2-amino-4-pyrimidinol **30** is reacted with amine **6** (or optionally protected amine **21**) to yield N2-substituted-4-pyrimidinol **32**, which is then halogenated as previously described to yield N2-substituted-4-halo-2-pyrimidineamine **34**. Optional deprotection (for example if protected amine **21** was used in the first step) followed by reaction with amine **10** affords a 2,4-pyrimidinediamine according to structural formula (Ia). Alternatively, pyrimidinol **30** can be reacted with acylating agent **31**.

Suitable commercially-available 2-amino-4-pyrimidinols **30** that can be used as starting materials in Scheme (III) include, but are not limited to, 2-amino-6-chloro-4-pyrimidinol hydrate (Aldrich #A4702-8; CAS Registry 00000-00-0) and 2-amino-6-hydroxy-4-pyrimidinol (Aldrich #A5040-1; CAS Registry 56-09-7). Other 2-amino-4-pyrimidinols **30** useful as starting materials in Scheme (III) are available from General Intermediates of Canada, Inc., Edmonton, Alberta, CA (www.generalintermediates.com) and/or Interchim, France (www.interchim.com), or may be prepared using standard techniques. Myriad textbook references teaching suitable synthetic methods are provided *infra.*

Alternatively, the 2,4-pyrimidinediamine compounds of the invention may be prepared from substituted or unsubstituted 4-amino-2-pyrimidinols as illustrated in Scheme (IV), below:

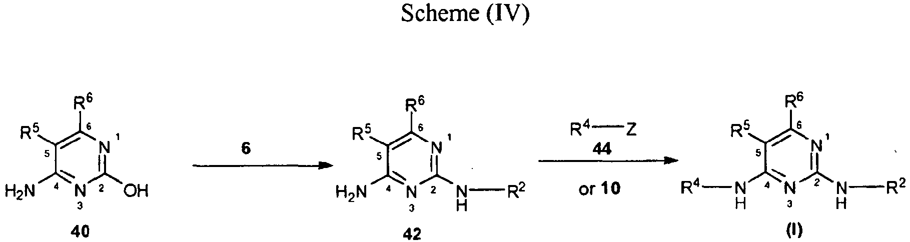

In Scheme (IV), R², R⁴, R⁵, R⁶ are as previously defined for Scheme (I) and Z represents a leaving group. Referring to Scheme (IV), the C2-hydroxyl of 4-amino-2-pyrimidinol **40** is more reactive towards nucleophiles than the C4-amino such that reaction with amine **6** yields N2-substituted-2,4-pyrimidinediamine **42**. Subsequent reaction with compound **44**, which includes a good leaving group Z, or amine **10** yields a 2,4-pyrimidinediamine according to structural formula **(Ia)**. Compound **44** may include virtually any leaving group that can be displaced by the C4-amino of N2-substituted-2,4-pyrimidinediamine **42**. Suitable leaving groups Z include, but are not limited to, halogens, methanesulfonyloxy (mesyloxy; "OMs"), trifluoromethanesulfonyloxy ("OTf") and *p*-toluenesulfonyloxy (tosyloxy; "OTs"), benzene sulfonyloxy ("besylate") and metanitro benzene sulfonyloxy ("nosylate"). Other suitable leaving groups will be apparent to those of skill in the art.

Substituted 4-amino-2-pyrimidinol starting materials may be obtained commercially or synthesized using standard techniques. Myriad textbook references teaching suitable synthetic methods are provided *infra.*

In still another exemplary embodiment, the 2,4-pyrimidinediamine compounds of the invention can be prepared from 2-chloro-4-aminopyrimidines or 2-amino-4-chloropyrimidines as illustrated in Scheme (V), below:

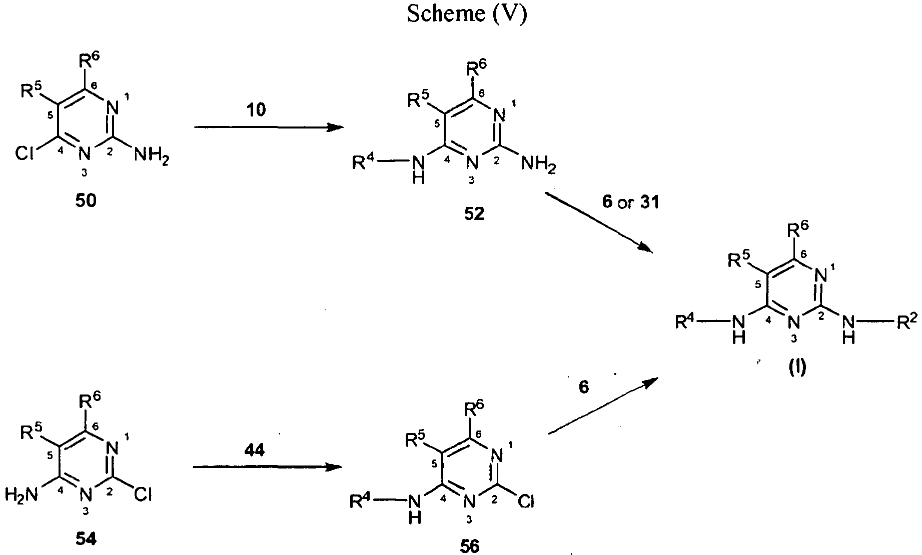

In Scheme (V), R², R⁴, R⁵, R⁶ and X are as defined for Scheme (I) and Z is as defined for Scheme (IV). Referring to Scheme (V), 2-amino-4-chloropyrimidine **50** is reacted with amino **10** to yield 4N-substituted-2-pyrimidineamine **52** which, following reaction with compound **31** or amine **6**, yields a 2,4-pyrimidinediamine according to structural formula **(I)**. Alternatively, 2-chloro-4-amino-pyrimidine **54** may be reacted with compound **44** followed by amine **6** to yield a compound according to structural formula **(Ia)**.

A variety of pyrimidines **50** and **54** suitable for use as starting materials in Scheme (V) are commercially available, including by way of example and not limitation, 2-amino-4,6-dichloropyrimidine (Aldrich #A4860-1; CAS Registry 56-05-3); 2-amino-4-chloro-6-methoxy-pyrimidine (Aldrich #51,864-6; CAS Registry 5734-64-5); 2-amino-4-chloro-6-methylpyrimidine (Aldrich #12,288-2; CAS Registry 5600-21-5); and 2-amino-4-chloro-6-methylthiopyrimidine (Aldrich #A4600-5; CAS Registry 1005-38-5). Additional pyrimidine starting materials are available from General Intermediates of Canada, Inc., Edmonton, Alberta, CA (www.generalintermediates.com) and/or Interchim, France (www.interchim.com), or may be prepared using standard techniques. Myriad textbook references teaching suitable synthetic methods are provided *infra*.

Alternatively, 4-chloro-2-pyrimidineamines **50** may be prepared as illustrated in Scheme (Va):

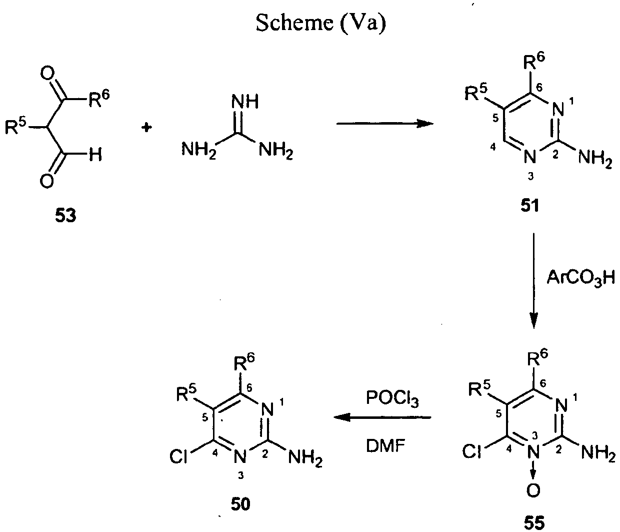

In Scheme (Va), R⁵ and R⁶ are as previously defined for structural formula (Ia). In Scheme (Va), dicarbonyl **53** is reacted with guanidine to yield 2-pyrimidineamine **51**. Reaction with peracids like m-chloroperbenzoic acid, trifluoroperacetic acid or urea hydrogen peroxide complex yields N-oxide **55**, which is then halogenated to give 4-chloro-2-pyrimidineamine **50**. The corresponding 4-halo-2-pyrimidineamines may be obtained by using suitable halogenation reagents.

In yet another exemplary embodiment, the 2,4-pyrimidinediamine compounds of the invention can be prepared from substituted or unsubstituted uridines as illustrated in Scheme (VI), below:

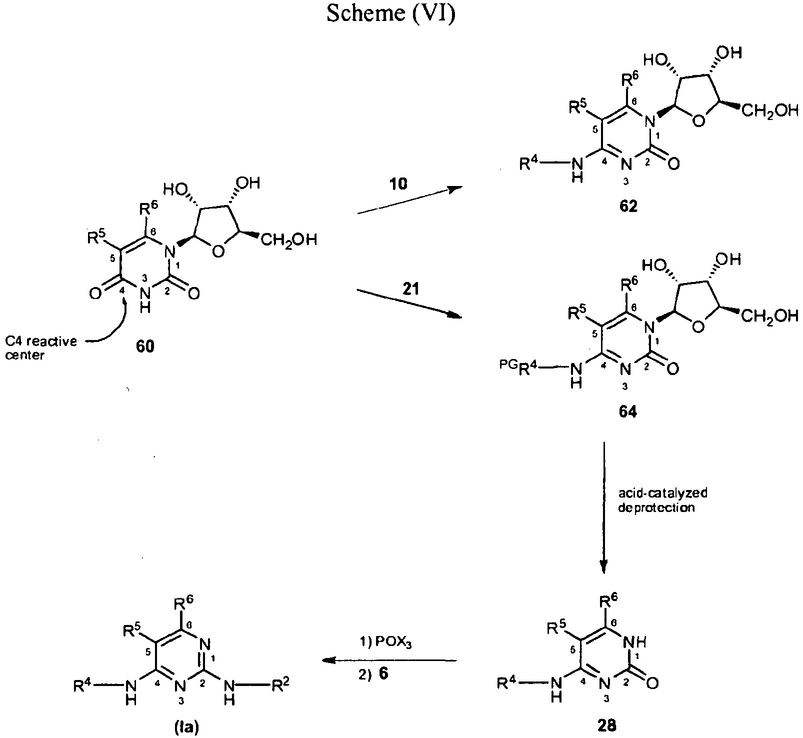

In Scheme (VI), R², R⁴, R⁵, R⁶ and X are as previously defined for Scheme (I) and the superscript PG represents a protecting group, as discussed in connection with Scheme (IIb). According to Scheme (VI), uridine **60** has a C4 reactive center such that reaction with amine **10** or protected amine **21** yields N4-substituted cytidine **62** or **64**, respectively. Acid-catalyzed deprotection of N4-substituted **62** or **64** (when "PG" represents an acid-labile protecting group) yields N4-substituted cytosine **28**, which may be subsequently halogenated at the C2-position and reacted with amine **6** to yield a 2,4-pyrimidinediamine according to structural formula **(Ia)**.

Cytidines may also be used as starting materials in an analogous manner, as illustrated in Scheme (VII), below:

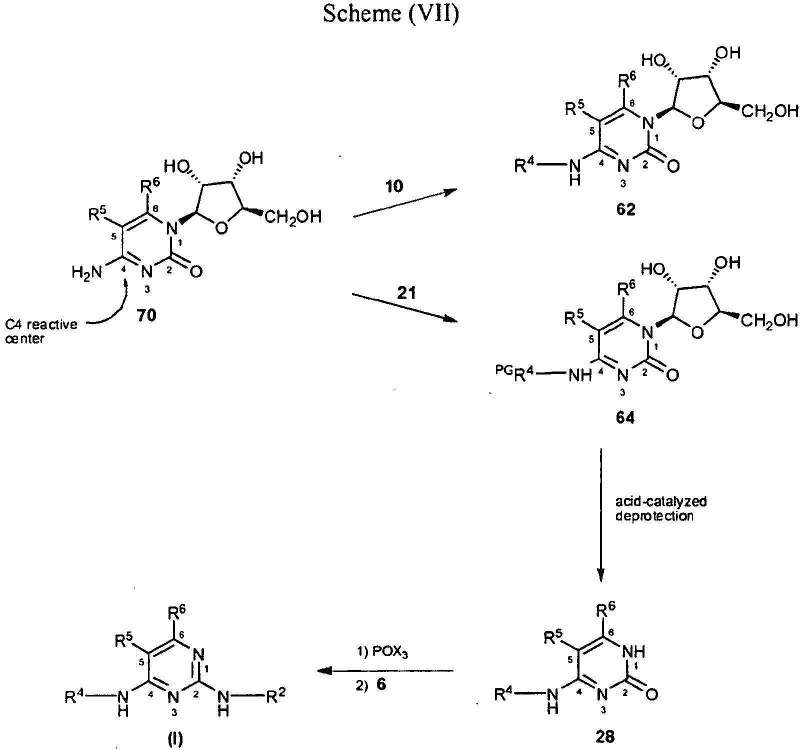

In Scheme (VII), R², R⁴, R⁵, R⁶ and X are as previously defined in Scheme (I) and the superscript PG represents a protecting group as discussed above. Referring to Scheme (VII), like uridine **60**, cytidine **70** has a C4 reactive center such that reaction with amine **10** or protected amine **21** yields N4-substituted cytidine **62** or **64**, respectively. These cytidines **62** and **64** are then treated as previously described for Scheme (VI) to yield a 2,4-pyrimidinediamine according to structural formula **(Ia)**.

Although Schemes (VI) and (VII) are exemplified with ribosylnucleosides, skilled artisans will appreciate that the corresponding 2'-deoxyribo and 2',3'-dideoxyribo nucleosides, as well as nucleosides including sugars or sugar analogs other than ribose, would also work.

Numerous uridines and cytidines useful as starting materials in Schemes (VI) and (VII) are known in the art, and include, by way of example and not limitation, 5-trifluoromethyl-2'-deoxycytidine (Chem. Sources #ABCR F07669; CAS Registry 66,384-66-5); 5-bromouridine (Chem. Sources Int'l 2000; CAS Registry 957-75-5); 5-iodo-2'-deoxyuridine (Aldrich #1-775-6; CAS Registry 54-42-2); 5-fluorouridine (Aldrich #32,937-1; CAS Registry 316-46-1); 5-iodouridine (Aldrich #85,259-7; CAS Registry 1024-99-3); 5-(trifluoromethyl)uridine (Chem. Sources Int'l 2000; CAS Registry 70-00-8); 5-trifluoromethyl-2'-deoxyuridine (Chem. Sources Int'l 2000; CAS Registry 70-00-8). Additional uridines and cytidines that can be used as starting materials in Schemes (VI) and (VII) are available from General Intermediates of Canada, Inc., Edmonton, Alberta, CA (www.generalintermediates.com) and/or Interchim, France (www.interchim.com). or may be prepared using standard techniques. Myriad textbook references teaching suitable synthetic methods are provided *infra.*

The 2,4-pyrimidinediamine compounds of the invention can also be synthesized from substituted pyrimidines, such as chloro-substituted pyrimidines, as illustrated in Schemes (VIII) and (IX), below:

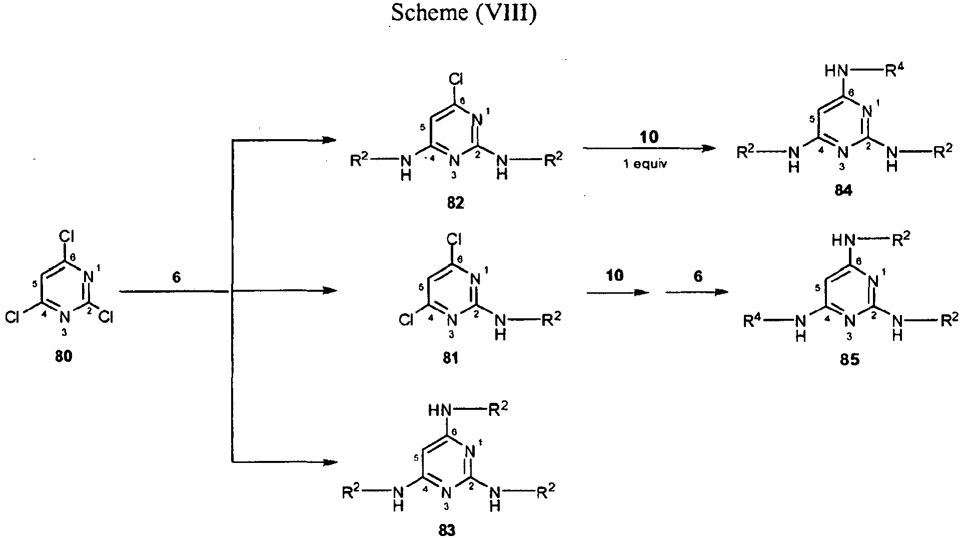

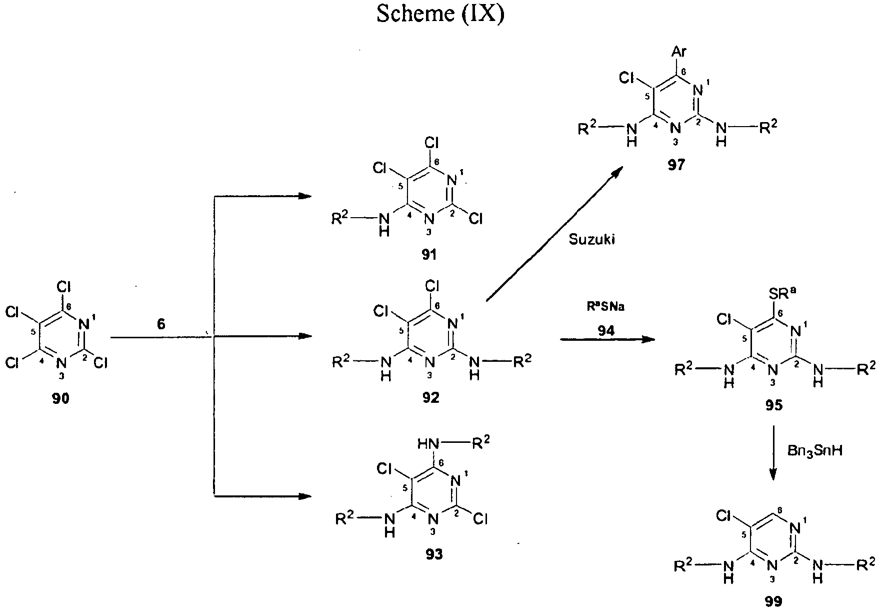

In Schemes (VIII) and (IX), R², R⁴ and R^{a} are as previously defined for structural formula (I) and "Ar" represents an aryl group. Referring to Scheme (VIII), reaction of 2,4,6-trichloropyrimidine **80** (Aldrich #T5,620-0; CAS#3764-01-0) with amine **6** yields a mixture of three compounds: substituted pyrimidine mono-, di- and triamines **81**, **82** and **83**, which can be separated and isolated using HPLC or other conventional techniques. Mono- and diamines **81** and **82** may be further reacted with amines **6** and/or **10** to yield N2,N4,N6-trisubstituted-2,4,6-pyrimidinetriamines **84** and **85**, respectively.

N2,N4-bis-substituted-2,4-pyrimidinediamines can be prepared in a manner analogous to Scheme (VIII) by employing 2,4-dichloro-5-methylpyrimidine or 2,4-dichloro-pyrimidine as starting materials. In this instance, the mono-substituted pyrimidineamine corresponding to compound **81** is not obtained. Instead, the reaction proceeds to yield the N2,N4-bis-substituted-2,4-pyrimidinediamine directly.

Referring to Scheme (IX), 2,4,5,6-tetrachloropyrimidine **90** (Aldrich #24,671-9; CAS#1780-40-1) is reacted with excess amine **6** to yield a mixture of three compounds: **91**, **92**, and **93**, which can be separated and isolated using HPLC or other conventional techniques. As illustrated, N2,N4-bis-substituted-5,6,-dichloro-2,4-pyrimidinediamine **92** may be further reacted at the C6 halide with, for example a nucleophilic agent **94** to yield compound **95**. Alternatively, compound **92** can be converted into N2,N4-bis-subsituted-5-chloro-6-aryl-2,4-pyrimidinediamine **97** *via* a Suzuki reaction. 2,4-Pyrimidinediamine **95** may be converted to 2,4-pyrimidinediamine **99** by reaction with Bn₃SnH.

As will be recognized by skilled artisans, 2,4-pyrimidinediamines according to the invention, synthesized *via* the exemplary methods described above or by other well-known means, may also be utilized as starting materials and/or intermediates to synthesize additional 2,4-pyrimidinediamine compounds of the invention. A specific example is illustrated in Scheme (X), below:

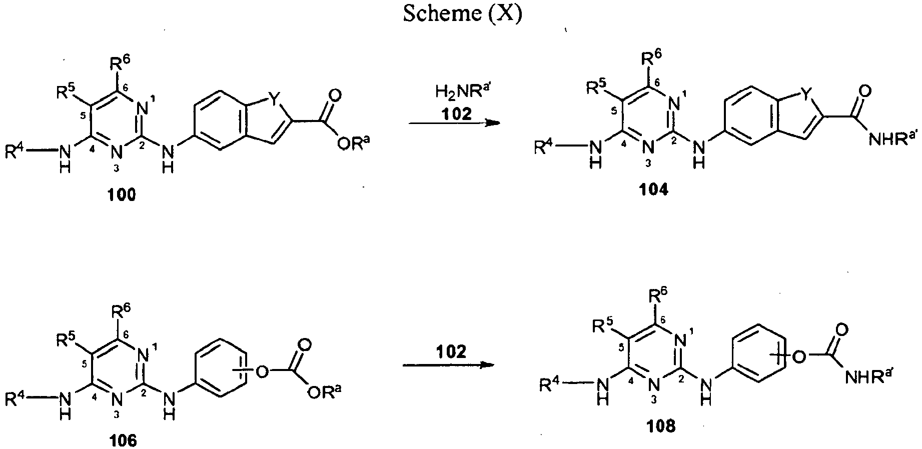

In Scheme (X), R⁴, R⁵, R⁶ and R^{a} are as previously defined for structural formula (Ia). Each R^{a} is independently an R^{a}, and may be the same or different from the illustrated R^{a}. Referring to Scheme (X), carboxylic acid or ester **100** may be converted to amide **104** by reaction with amine **102**. In amine **102**, R^{a'} may be the same or different than R^{a} of acid or ester **100**. Similarly, carbonate ester **106** may be converted to carbamate **108**.

A second specific example is illustrated in Scheme (XI), below:

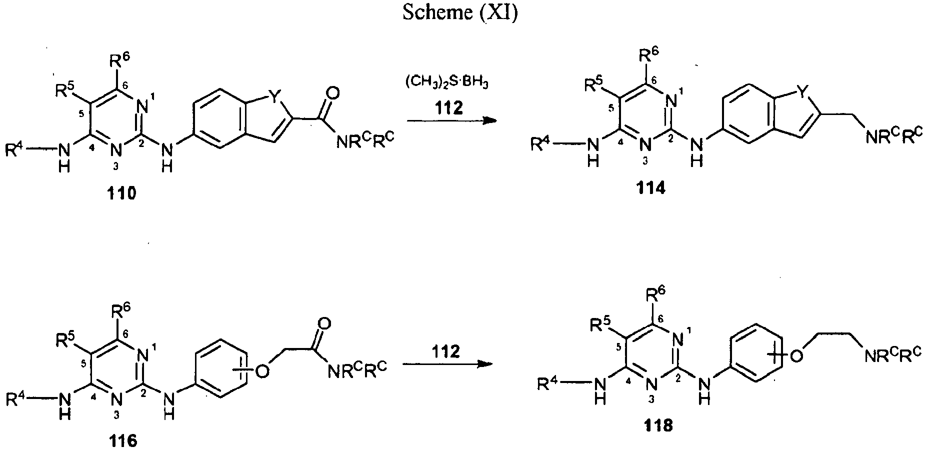

In Scheme (XI), R⁴, R⁵, R⁶ and R^{c} are as previously defined for structural formula (Ia). Referring to Scheme (XI), amide **110** or **116** may be converted to amine **114** or **118**, respectively, by borane reduction with borane methylsulfide complex **112**. Other suitable reactions for synthesizing 2,4-pyrimidinediamine compounds from 2,4-pyrimidinediamine starting materials will be apparent to those of skill in the art.

Although many of the synthetic schemes discussed above do not illustrate the use of protecting groups, skilled artisans will recognize that in some instances substituents R², R⁴, R⁵ and/or R⁶ may include functional groups requiring protection. The exact identity of the protecting group used will depend upon, among other things, the identity of the functional group being protected and the reaction conditions used in the particular synthetic scheme, and will be apparent to those of skill in the art. Guidance for selecting protecting groups and chemistries for their attachment and removal suitable for a particular application can be found, for example, in Greene & Wuts, *supra.*

Prodrugs according to structural formula (II) may be prepared by routine modification of the above-described methods. Alternatively, such prodrugs may be prepared by reacting a suitably protected 2,4-pyrimidinediamine of structural formula (Ia) with a suitable progroup. Conditions for carrying out such reactions and for deprotecting the product to yield a prodrug of formula (II) are well-known.

Myriad references teaching methods useful for synthesizing pyrimidines generally, as well as starting materials described in Schemes (I)-(IX), are known in the art. For specific guidance, the reader is referred to Brown, D. J., "The Pyrimidines", in The Chemistry of Heterocyclic Compounds, Volume 16 (Weissberger, A., Ed.), 1962, Interscience Publishers, (A Division of John Wiley & Sons), New York ("Brown I"), Brown, D. J., "The Pyrimidines", in The Chemistry of Heterocyclic Compounds, Volume 16, Supplement I (Weissberger, A. and Taylor, E. C., Ed.), 1970, Wiley-Interscience, (A Division of John Wiley & Sons), New York (Brown II"); Brown, D. J., "The Pyrimidines", in The Chemistry of Heterocyclic Compounds, Volume 16, Supplement II (Weissberger, A. and Taylor, E. C., Ed.), 1985, An Interscience Publication (John Wiley & Sons), New York ("Brown III"); Brown, D. J., "The Pyrimidines" in The Chemistry of Heterocyclic Compounds, Volume 52 (Weissberger, A. and Taylor, E. C., Ed.),1994, John Wiley & Sons, Inc., New York, pp. 1-1509 (Brown IV"); Kenner, G. W. and Todd, A., in Heterocyclic Compounds, Volume 6, (Eldemeld, R. C., Ed.), 1957, John Wiley, New York, Chapter 7 (pyrimidines); Paquette, L. A., Principles of Modern Heterocyclic Chemistry, 1968, W. A. Benjamin, Inc., New York, pp. 1 - 401 (uracil synthesis pp. 313, 315; pyrimidine synthesis pp. 313-316; amino pyrimidine synthesis pp. 315); Joule, J. A., Mills, K. and Smith, G. F., Heterocyclic Chemistry, 3rd Edition, 1995, Chapman and Hall, London, UK, pp. 1 - 516; Vorbrüggen, H. and Ruh-Pohlenz, C., Handbook ofNucleoside Synthesis, John Wiley & Sons, New York, 2001, pp. 1-631 (protection of pyrimidines by acylation pp. 90-91; silylation of pyrimidines pp. 91-93); Joule, J. A., Mills, K. and Smith, G. F., Heterocyclic Chemistry, 4th Edition, 2000, Blackwell Science, Ltd, Oxford, UK, pp. 1-589; and Comprehensive Organic Synthesis, Volumes 1-9 (Trost, B. M. and Fleming, I., Ed.), 1991, Pergamon Press, Oxford, UK.

### 6.4 Inhibition of Fc Receptor Signal Cascades

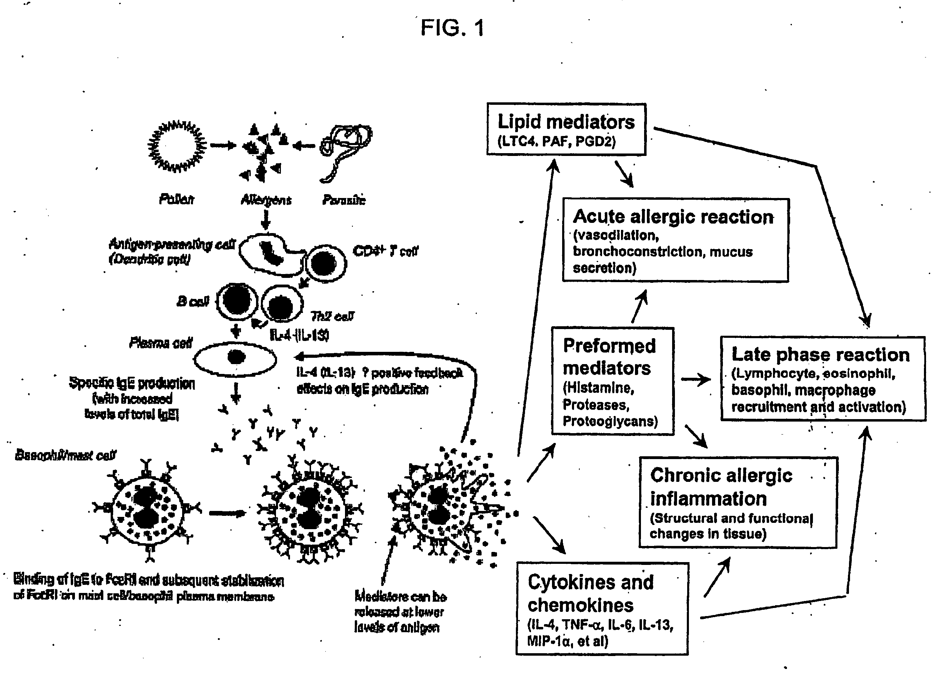

Active 2,4-pyrimidinediamine compounds of the invention inhibit Fc receptor signalling cascades that lead to, among other things, degranulation of cells. As a specific example, the compounds inhibit the FcεRI and/or FcγRI signal cascades that lead to degranulation of immune cells such as neutrophil, eosinophil, mast and/or basophil cells. Both mast and basophil cells play a central role in allergen-induced disorders, including, for example, allergic rhinitis and asthma. Referring to FIG. 1, upon exposure allergens, which may be, among other things, pollen or parasites, allergen-specific IgE antibodies are synthesized by B-cells activated by IL-4 (or IL-13) and other messengers to switch to IgE class specific antibody synthesis. These allergen-specific IgEs bind to the high affinity FcεRI. Upon binding of antigen, the FcεR1-bound IgEs are cross-linked and the IgE receptor signal transduction pathway is activated, which leads to degranulation of the cells and consequent release and/or synthesis of a host of chemical mediators, including histamine, proteases (e.g., tryptase and chymase), lipid mediators such as leukotrienes (e.g., LTC4), platelet-activating factor (PAF) and prostaglandins (e.g., PGD2) and a series of cytokine, including TNF-α, IL-4, IL-13, IL-5, IL-6, IL-8, GMCSF, VEGF and TGF-β. The release and/or synthesis of these mediators from mast and/or basophil cells accounts for the early and late stage responses induced by allergens, and is directly linked to downstream events that lead to a sustained inflammatory state.

The molecular events in the FcεRI signal transduction pathway that lead to release of preformed mediators *via* degranulation and release and/or synthesis of other chemical mediators are well-known and are illustrated in FIG. 2. Referring to FIG. 2, the FcεRI is a heterotetrameric receptor composed of an IgE-binding alpha-subunit, a beta subunit, and two gamma subunits (gamma homodimer). Cross-linking of FcεRI-bound IgE by multivalent binding agents (including, for example IgE-specific allergens or anti-IgE antibodies or fragments) induces the rapid association and activation of the Src-related kinase Lyn. Lyn phosphorylates immunoreceptor tyrosine-based activation motifs (ITAMS) on the intracellular beta and gamma subunits, which leads to the recruitment of additional Lyn to the beta subunit and Syk kinase to the gamma homodimer. These receptor-associated kinases, which are activated by intra- and intermolecular phosphorylation, phosphorylate other components of the pathway, such as the Btk kinase, LAT, and phospholipase C-gamma PLC-gamma). Activated PLC-gamma initiates pathways that lead to protein kinase C activation and Ca²⁺ mobilization, both of which are required for degranulation. FcεR1 cross-linking also activates the three major classes of mitogen activated protein (MAP) kinases, *i.e*. ERK1/2, JNK1/2, and p38. Activation of these pathways is important in the transcriptional regulation of proinflammatory mediators, such as TNF-α and IL-6, as well as the lipid mediator leukotriene CA (LTC4).

Although not illustrated, the FcγRI signaling cascade is believed to share some common elements with the FceRI signaling cascade. Importantly, like FcεRI, the FcγRI includes a gamma homodimer that is phosphorylated and recruits Syk, and like FcεRI, activation of the FcγRI signaling cascade leads to, among other things, degranulation. Other Fc receptors that share the gamma homodimer, and which can be regulated by the active 2,4-pyrimidinediamine compounds include, but are not limited to, FcαRI and FcγRIII.

The ability of the 2,4-pyrimidinediamine compounds of the invention to inhibit Fc receptor signaling cascades may be simply determined or confirmed in *in vitro* assays. Suitable assays for confirming inhition of FcεRI-mediated degranulation are provided in the Examples section. In one typical assay, cells capable of undergoing FcεRI mediated degranulation, such as mast or basophil cells, are first grown in the presence of IL-4, Stem Cell Factor (SCF), IL-6 and IgE to increase expression of the FcεRI, exposed to a 2,4-pyrimidinediamine test compound of the invention and stimulated with anti-IgE antibodies (or, alternatively, an IgE-specific allergen). Following incubation, the amount of a chemical mediator or other chemical agent released and/or synthesized as a consequence of activating the FcεRI signaling cascade may be quantified using standard techniques and compared to the amount of the mediator or agent released from control cells (*i.e.,* cells that are stimulated but that are not exposed to test compound). The concentration of test compound that yields a 50% reduction in the quantity of the mediator or agent measured as compared to control cells is the IC₅₀ of the test compound. The origin of the mast or basophil cells used in the assay will depend, in part, on the desired use for the compounds and will be apparent to those of skill in the art For example, if the compounds will be used to treat or prevent a particular disease in humans, a convenient source of mast or basophil cells is a human or other animal which constitutes an accepted or known clinical model for the particular disease. Thus, depending upon the particular application, the mast or basophil cells may be derived from a wide variety of animal sources, ranging from, for example, lower mammals such as mice and rats, to dogs, sheep and other mammals commonly employed in clinical testing, to higher mammals such as monkeys, chimpanzees and apes, to humans. Specific examples of cells suitable for carrying out the *in vitro* assays include, but are not limited to, rodent or human basophil cells, rat basophil leukemia cell lines, primary mouse mast cells (such as bone marrow-derived mouse mast cells "BMMC") and primary human mast cells isolated from cord blood ("CHMC") or other tissues such as lung. Methods for isolating and culturing these cell types are well-known or are provided in the Examples section (*see,* e.g., Demo et al., 1999, Cytometry 36(4):340-348 and copending application Serial No. 10/053,355, filed November 8, 2001). Of course, other types of immune cells that degranulate upon activation of the FcεRI signaling cascade may also be used, including, for example, eosinophils.

As will be recognized by skilled artisans, the mediator or agent quantified is not critical. The only requirement is that it be a mediator or agent released and/or synthesized as a consequence of initiating or activating the Fc receptor signaling cascade. For example, referring to FIG. 1, activation of the FcεRI signaling cascade in mast and/or basophil cells leads to numerous downstream events. For example, activation of the FcεRI signal cascade leads to the immediate release (i.e., within 1-3 min. following receptor activation) of a variety of preformed chemical mediators and agents *via* degranulation. Thus, in one embodiment, the mediator or agent quantified may be specific to granules (*i.e*., present in granules but not in the cell cytoplasm generally). Examples of granule-specific mediators or agents that can be quantified to determine and/or confirm the activity of a 2,4-pyrimidinediamine compound of the invention include, but are not limited to, granule-specific enzymes such as hexosaminidase and tryptase and granule-specific components such as histamine and serotonin. Assays for quantifying such factors are well-known, and in many instances are commercially available. For example, tryptase and/or hexosaminidase release may be quantified by incubating the cells with cleavable substrates that fluoresce upon cleavage and quantifying the amount of fluorescence produced using conventional techniques. Such cleavable fluorogenic substrates are commercially available. For example, the fluorogenic substrates Z-Gly-Pro-Arg-AMC (Z=benzyloxycarbonyl; AMC=7-amino-4-methylcoumarin, BIOMOL Research Laboratories, Inc., Plymouth Meeting, PA 19462, Catalog No. P-142) and Z-Ala-Lys-Arg-AMC (Enzyme Systems Products, a division of ICN Biomedicals, Inc., Livermore, CA 94550, Catalog No. AMC-246) can be used to quantify the amount of tryptase released. The fluorogenic substrate 4-methylumbelliferyl-N-acetyl-β-D-glucosaminide (Sigma, St. Louis, MO, Catalog #69585) can be used to quantify the amount of hexosaminidase released. Histamine release may be quantified using a commercially available enzyme-linked immunosorbent assay (ELISA) such as Immunotech histamine ELISA assay #IM2015 (Beckman-Coulter, Inc.). Specific methods of quantifying the release of tryptase, hexosaminidase and histamine are provided in the Examples section. Any of these assays may be used to determine or confirm the activity of the 2,4-pyrimidinediamine compounds of the invention.

Referring again to FIG. 1, degranulation is only one of several responses initiated by the FcεRI signaling cascade. In addition, activation of this signaling pathway leads to the *de novo* synthesis and release of cytokines and chemokines such as IL-4, IL-5, IL-6, TNF-α, IL-13 and MIP1-α), and release of lipid mediators such as leukotrienes (e.g., LTC4), platelet activating factor (PAF) and prostaglandins. Accordingly, the 2,4-pyrimidinediamine compounds of the invention may also be assessed for activity by quantifying the amount of one or more of these mediators released and/or synthesized by activated cells.

Unlike the granule-specific components discussed above, these "late stage" mediators are not released immediately following activation of the FcεRI signaling cascade. Accordingly, when quantifying these late stage mediators, care should be taken to insure that the activated cell culture is incubated for a time sufficient to result in the synthesis (if necessary) and release of the mediator being quantified. Generally, PAF and lipid mediators such as leukotriene C4 are released 3-30 min. following FcεRI activation. The cytokines and other late stage mediators are released approx. 4-8 hrs. following FcεRI activation. Incubation times suitable for a specific mediator will be apparent to those of skill in the art. Specific guidance and assays are provided in the Examples section.

The amount of a particular late stage mediator released may be quantified using any standard technique. In one embodiment, the amount(s) may be quantified using ELISA assays. ELISA assay kits suitable for quantifying the amount of TNFα, IL-4, IL-5, IL-6 and/or IL-13 released are available from, for example, Biosource International, Inc., Camarillo, CA 93012 (see, e.g., Catalog Nos. KHC3011, KHC0042, KHC0052, KHC0061 and KHC0132). ELISA assay kits suitable for quantifying the amount of leukotriene C4 (LTC4) released from cells are available from Cayman Chemical Co., Ann Arbor, MI 48108 (see, e.g., Catalog No. 520211).

Typically, active 2,4-pyrimidinediamine compounds of the invention will exhibit IC₅₀s with respect to FcεRI-mediated degranulation and/or mediator release or synthesis of about 20 µM or lower, as measured in an *in vitro* assay, such as one of the *in vitro* assays described above or in the Examples section. Of course, skilled artisans will appreciate that compounds which exhibit lower IC₅₀s, for example on the order of 10 µM, 1 µM, 100 nM, 10 nM, 1 nM, or even lower, are particularly useful.

Skilled artisans will also appreciate that the various mediators discussed above may induce different adverse effects or exhibit different potencies with respect to the same adverse effect. For example, the lipid mediator LTC4 is a potent vasoconstrictor - it is approximately 1000-fold more potent at inducing vasoconstriction than histamine. As another example, in addition to mediating atopic or Type I hypersensitivity reactions, cytokines can also cause tissue remodeling and cell proliferation. Thus, although compounds that inhibit release and/or synthesis of any one of the previously discussed chemical mediators are useful, skilled artisans will appreciate that compounds which inhibit the release and/or synthesis of a plurality, or even all, of the previously described mediators find particular use, as such compounds are useful for ameliorating or avoiding altogether a plurality, or even all, of the adverse effects induced by the particular mediators. For example, compounds which inhibit the release of all three types of mediators-granule-specific, lipid and cytokine-are useful for treating or preventing immediate Type I hypersensitivity reactions as well as the chronic symptoms associated therewith.

Compounds of the invention capable of inhibiting the release of more than one type of mediator (*e.g*., granule-specific or late stage) may be identified by determining the IC₅₀ with respect to a mediator representative of each class using the various *in vitro* assays described above (or other equivalent *in vitro* assays). Compounds of the invention which are capable of inhibiting the release of more than one mediator type will typically exhibit an IC₅₀ for each mediator type tested of less than about 20 µM. For example, a compound which exhibits an IC₅ₒ of 1 µM with respect to histamine release (IC₅₀^{histamine}) and an IC₅₀ of 1 nM with respect to leukotriene LTC4 synthesis and/or release (IC₅₀^{LTC4}) inhibits both immediate (granule-specific) and late stage mediator release. As another specific example, a compound that exhibits an IC₅₀^{tryptase} of 10 µM, an IC₅₀^{LTC4} of 1 µM and an IC₅₀^{IL-4} of 1 µM inhibits immediate (granule-specific), lipid and cytokine mediator release. Although the above specific examples utilize the IC₅₀s of one representative mediator of each class, skilled artisans will appreciate that the IC₅₀s of a plurality, or even all, mediators comprising one or more of the classes may be obtained. The quantity(ies) and identity(ies) of mediators for which IC₅₀ data should be ascertained for a particular compound and application will be apparent to those of skill in the art.

Similar assays may be utilized to confirm inhibition of signal transduction cascades initiated by other Fc receptors, such as FcαRI, FcγRI and/or FcγRIII signaling, with routine modification. For example, the ability of the compounds to inhibit FcγRI signal transduction may be confirmed in assays similar to those described above, with the exception that the FcγRI signaling cascade is activated, for example by incubating the cells with IgG and an IgG-specific allergen or antibody, instead of IgE and an IgE-specific allergen or antibody. Suitable cell types, activating agents and agents to quantify to confirm inhibition of other Fc receptors, such as Fc receptors that comprise a gamma homodimer, will be apparent to those of skill in the art.

One particularly useful class of compounds includes those 2,4-pyrimidinediamine compounds that inhibit the release of immediate granule-specific mediators and late stage mediators with approximately equivalent IC₅₀s. By approximately equivalent is meant that the IC₅₀s for each mediator type are within about a 10-fold range of one another. Another particularly useful class of compounds includes those 2,4-pyrimidinediamine compounds that inhibit the release of immediate granule-specific mediators, lipid mediators and cytokine mediators with approximately equivalent IC₅₀s. In a specific embodiment, such compounds inhibit the release of the following mediators with approximately equivalent IC₅₀s: histamine, tryptase, hexosaminidase, IL-4, IL-5, IL-6, IL-13, TNFα and LTC4. Such compounds are particularly useful for, among other things, ameliorating or avoiding altogether both the early and late stage responses associated with atopic or immediate Type I hypersensitivity reactions.

Ideally, the ability to inhibit the release of all desired types of mediators will reside in a single compound. However, mixtures of compounds can also be identified that achieve the same result. For example, a first compound which inhibits the release of granule specific mediators may be used in combination with a second compound which inhibits the release and/or synthesis of cytokine mediators.

In addition to the FcεRI or FcγRI degranulation pathways discussed above, degranulation of mast and/or basophil cells can be induced by other agents. For example, ionomycin, a calcium ionophore that bypasses the early FcεRI or FcγRI signal transduction machinery of the cell, directly induces a calcium flux that triggers degranulation. Referring again to FIG. 2, activated PLCγ initiates pathways that lead to, among other things, calcium ion mobilization and subsequent degranulation. As illustrated, this Ca²⁺ mobilization is triggered late in the FcεRI signal transduction pathway. As mentioned above, and as illustrated in FIG. 3, ionomycin directly induces Ca²⁺ mobilization and a Ca²⁺ flux that leads to degranulation. Other ionophores that induce degranulation in this manner include A23187. The ability of granulation-inducing ionophores such as ionomycin to bypass the early stages of the FcεRI and/or FcγRI signaling cascades may be used as a counter screen to identify active compounds of the invention that specifically exert their degranulation-inhibitory activity by blocking or inhibiting the early FcγRI or FcγRI signaling cascades, as discussed above. Compounds which specifically inhibit such early FcεRI or FcγRI-mediated degranulation inhibit not only degranulation and subsequent rapid release of histamine, tryptase and other granule contents, but also inhibit the pro-inflammatory activation pathways causing the release of TNFα, IL-4, IL-13 and the lipid mediators such as LTC4. Thus, compounds which specifically inhibit such early FcεRI and/or FcγRI-mediated degranulation block or inhibit not only acute atopic or Type I hypersensitivity reactions, but also late responses involving multiple inflammatory mediators.

Compounds of the invention that specifically inhibit early FcεRI and/or FcγRI-mediated degranulation are those compounds that inhibit FcεRI and/or FcγRI-mediated degranulation (for example, have an IC₅₀ of less than about 20 µM with respect to the release of a granule-specific mediator or component as measured in an *in vitro* assay with cells stimulated with an IgE or IgG binding agent) but that do not appreciably inhibit ionophore-induced degranulation. In one embodiment, compounds are considered to not appreciably inhibit ionophore-induced degranulation if they exhibit an IC₅₀ of ionophore-induced degranulation of greater than about 20 µM, as measured in an *in vitro* assay. Of course, active compounds that exhibit even higher IC₅₀s of ionophore-induced degranulation, or that do not inhibit ionophore-induced degranulation at all, are particularly useful. In another embodiment, compounds are considered to not appreciably inhibit ionophore-induced degranulation if they exhibit a greater than 10-fold difference in their IC₅₀s of FcεRT and/or FcγRI-mediated degranulation and ionophore-induced degranulation, as measured in an *in vitro* assay. Assays suitable for determining the IC₅₀ of ionophore-induced degranulation include any of the previously-described degranulation assays, with the modification that the cells are stimulated or activated with a degranulation-inducing calcium ionophore such as ionomycin or A23187 (A.G. Scientific, San Diego, CA) instead of anti-IgE antibodies or an IgE-specific allergen. Specific assays for assessing the ability of a particular 2,4-pyrimidinediamine compound of the invention to inhibit ionophore-induced degranulation are provided in the Examples section.

As will be recognized by skilled artisans, compounds which exhibit a high degree of selectivity of FcεRI-mediated degranulation find particular use, as such compounds selectively target the FcεRI cascade and do not interfere with other degranulation mechanisms. Similarly, compounds which exhibit a high degree of selectivity of FcγRI-mediated degranulation find particular use, as such compounds selectively target the FcγRI cascade and do not interfere with other degranulation mechanisms. Compounds which exhibit a high degree of selectivity are generally 10-fold or more selective for FcεRI- or FcγRI -mediated degranulation over ionophore-induced degranulation, such as ionomycin-induced degranulation.

Biochemical and other data confirm that the 2,4-pyrimidinediamine compounds described herein are potent inhibitors of Syk kinase activity. For example, in experiments with an isolated Syk kinase, of twenty four 2,4-pyrimidinediamine compounds tested, all but two inhibited the Syk kinase catalyzed phosphorylation of a peptide substrate with IC50s in the submicromolar range. The remaining compounds inhibited phosphorylation in the micromolar range. In addition, of sixteen compounds tested in an *in vitro* assay with mast cells, all inhibited phosphorylation of Syk kinase substrates (*e.g*., PLC-gammal, LAT) and proteins downstream of Syk kinase (*e.g*., JNK, p38, Erk1/2 and PKB, when tested), but not proteins upstream of Syk kinase in the cascade (*e.g*., Lyn). Phosphorylation of Lyn substrates was not inhibited by the 2,4-pyrimidinediamine compounds tested. Moreover, for the following compounds, a high correlation was observed between their inhibition of Syk kinase activity in biochemical assays (IC₅₀s in the range of 3 to 1850 nM) and their inhibition of FcεR1-mediated degranulation in mast cells (IC₅₀s in the range of 30 to 1650 nM): R921304, R940347.

Accordingly, the activity of the 2,4-pyrimidinediamine compounds of the invention may also be confirmed in biochemical or cellular assays of Syk kinase activity. Referring again to FIG. 2, in the FcεRI signaling cascade in mast and/or basophil cells, Syk kinase phosphorylates LAT and PLC-gammal, which leads to, among other things, degranulation. Any of these activities may be used to confirm the activity of the 2,4-pyrimidinediamine compounds of the invention. In one embodiment, the activity is confirmed by contacting an isolated Syk kinase, or an active fragment thereof with a 2,4-pyrimidinediamine compound in the presence of a Syk kinase substrate (*e.g*., a synthetic peptide or a protein that is known to be phophorylated by Syk in a signaling cascade) and assessing whether the Syk kinase phosphorylated the substrate. Alternatively, the assay may be carried out with cells that express a Syk kinase. The cells may express the Syk kinase endogenously or they may be engineered to express a recombinant Syk kinase. The cells may optionally also express the Syk kinase substrate. Cells suitable for performing such confirmation assays, as well as methods of engineering suitable cells will be apparent to those of skill in the art. Specific examples of biochemical and cellular assays suitable for confirming the activity of the 2,4-pyrimidinediamine compounds are provided in the Examples section.

Generally, compounds that are Syk kinase inhibitors will exhibit an IC₅₀ with respect to a Syk kinase activity, such as the ability of Syk kinase to phosphorylate a synthetic or endogenous substrate, in an *in vitro* or cellular assay in the range of about 20 µM or less. Skilled artisans will appreciate that compounds that exhibit lower IC50s, such as in the range of 10 µM, 1 µM, 100 nM, 10 nM, 1 nM, or even lower, are particularly useful.

### 6.4 Uses and Compositions

As previously discussed, the active compounds of the invention inhibit Fc receptor signaling cascades, especially those Fc receptors including a gamma homodimer, such as the FcεRI and/or FcγRI signaling cascades, that lead to, among other things, the release and/or synthesis of chemical mediators from cells, either *via* degranulation or other processes. As also discussed, the active compounds are also potent inhibitors of Syk kinase. As a consequence of these activities, the active compounds of the invention may be for use in a variety of *in vitro. in vivo* and *ex vivo* contexts to regulate or inhibit Syk kinase, signaling cascades in which Syk kinase plays a role, Fc receptor signaling cascades, and the biological responses effected by such signaling cascades. For example, in one embodiment, the compounds may be for use in inhibiting Syk kinase, either *in vitro* or *in vivo,* in virtually any cell type expressing Syk kinase. They may also be for use in regulating signal transduction cascades in which Syk kinase plays a role. Such Syk-dependent signal transduction cascades include, but are not limited to, the FcεRI, FcγRI, FcγRIII, BCR and integrin signal transduction cascades. The compounds may also be for use *in vitro* or *in vivo* to regulate, and in particular inhibit, cellular or biological responses effected by such Syk-dependent signal transduction cascades. Such cellular or biological responses include, but are not limited to, respiratory burst, cellular adhesion, cellular degranulation, cell spreading, cell migration, cell aggregation, phagcytosis, cytokine synthesis and release, cell maturation and Ca²⁺ flux. Importantly, the compounds may be for use in inhibiting Syk kinase *in vivo* as a therapeutic approach towards the treatment or prevention of diseases mediated, either wholly or in part, by a Syk kinase activity. Non-limiting examples of Syk kinase mediated diseases that may be treated or prevented with the compounds are those discussed in more detail, below.

In another embodiment, the active compounds may be for use in regulating or inhibiting the Fc receptor signaling cascades and/or FcεRI- and/or FcγRI-mediated degranulation as a therapeutic approach towards the treatment or prevention of diseases characterized by, caused by and/or associated with the release or synthesis of chemical mediators of such Fc receptor signaling cascades or degranulation. Such treatments may be administered to animals in veterinary contexts or to humans. Diseases that are characterized by, caused by or associated with such mediator release, synthesis or degranulation, and that can therefore be treated or prevented with the active compounds include, by way of example and not limitation, atopy or anaphylactic hypersensitivity or allergic reactions, allergies (e.g., allergic conjunctivitis, allergic rhinitis, atopic asthma, atopic dermatitis and food allergies), low grade scarring (*e.g*., of scleroderma, increased fibrosis, keloids, post-surgical scars, pulmonary fibrosis, vascular spasms, migraine, reperfusion injury and post myocardial infarction), diseases associated with tissue destruction (*e.g*., of COPD, cardiobronchitis and post myocardial infarction), diseases associated with tissue inflammation (*e.g*., irritable bowel syndrome, spastic colon and inflammatory bowel disease), inflammation and scarring.

In addition to the myriad diseases discussed above, cellular and animal empirical data confirm that the 2,4-pyrimidinediamine compounds described herein are also useful for the treatment or prevention of autoimmune diseases, as well as the various symptoms associated with such diseases. The types of autoimmune diseases that the 2,4-pyrimidinediamine compounds may be for use in treating or preventing generally include those disorders involving tissue injury that occurs as a result of a humoral and/or cell-mediated response to immunogens or antigens of endogenous and/or exogenous origin. Such diseases are frequently referred to as diseases involving the nonanaphylactic (*i.e*., Type II, Type III and/or Type IV) hypersensitivity reactions.

As discussed previously, Type I hypersensitivity reactions generally result from the release of pharmacologically active substances, such as histamine, from mast and/or basophil cells following contact with a specific exogenous antigen. As mentioned above, such Type I reactions play a role in numerous diseases, including allergic asthma, allergic rhinitis, etc.

Type II hypersensitivity reactions (also referred to as cytotoxic, cytolytic complement-dependent or cell-stimulating hypersensitivity reactions) result when immunoglobulins react with antigenic components of cells or tissue, or with an antigen or hapten that has become intimately coupled to cells or tissue. Diseases that are commonly associated with Type II hypersensitivity reactions include, but are not limited, to autoimmune hemolytic anemia, erythroblastosis fetalis and Goodpasture's disease.

Type III hypersensitivity reactions, (also referred to as toxic complex, soluble complex, or immune complex hypersensitivity reactions) result from the deposition of soluble circulating antigen-immunoglobulin complexes in vessels or in tissues, with accompanying acute inflammatory reactions at the site of immune complex deposition. Non-limiting examples of prototypical Type III reaction diseases include the Arthus reaction, rheumatoid arthritis, serum sickness, systemic lupus erythematosis, certain types of glomerulonephritis, multiple sclerosis and bullous pemphingoid.

Type IV hypersensitivity reactions (frequently called cellular, cell-mediated, delayed, or tuberculin-type hypersensitivity reactions) are caused by sensitized T-lymphocytes which result from contact with a specific antigen. Non-limiting examples of diseases cited as involving Type IV reactions are contact dermatitis and allograft rejection.

The 2,4-pyrimidine diamine compounds of the invention may be for use in treating or preventing autoimmune diseases associated with any of the above nonanaphylactic hypersensitivity reactions. In particular, the 2,4-pyrimidine diamine compounds of the invention may be for use in treating or preventing those autoimmune diseases frequently characterized as single organ or single cell-type autoimmune disorders including, but not limited to: Hashimoto's thyroiditis, autoimmune hemolytic anemia, autoimmune atrophic gastritis of pernicious anemia, autoimmune encephalomyelitis, autoimmune orchitis, Goodpasture's disease, autoimmune thrombocytopenia, sympathetic ophthalmia, myasthenia gravis, Graves' disease, primary biliary cirrhosis, chronic aggressive hepatitis, ulcerative colitis and membranous glomerulopathy, as well as those autoimmune diseases frequently characterized as involving systemic autoimmune disorder, which include but are not limited to: systemic lupus erythematosis, rheumatoid arthritis, Sjogren's syndrome, Reiter's syndrome, polymyositis-dermatomyositis, systemic sclerosis, polyarteritis nodosa, multiple sclerosis and bullous pemphigoid.

It will be appreciated by skilled artisans that many of the above-listed autoimmune diseases are associated with severe symptoms, the amelioration of which provides significant therapeutic benefit even in instances where the underlying autoimmune disease may not be ameliorated. Many of these symptoms, as well as their underlying disease states, result as a consequence of activating the FcγR signaling cascade in monocyte cells. As the 2,4-pyrimidinediamine compounds described herein are potent inhibitors of such FcγR signaling in monocytes and other cells, they may be for use in the treatment and/or prevention of myriad adverse symptoms associated with the above-listed autoimmune diseases.

As a specific example, rheumatoid arthritis (RA) typically results in swelling, pain, loss of motion and tenderness of target joints throughout the body. RA is characterized by chronically inflamed synovium that is densely crowded with lymphocytes. The synovial membrane, which is typically one cell layer thick, becomes intensely cellular and assumes a form similar to lymphoid tissue, including dentritic cells, T-, B- and NK cells, macrophages and clusters of plasma cells. This process, as well as a plethora of immunopathological mechanisms including the formation of antigen-immunoglobulin complexes, eventually result in destruction of the integrity of the joint, resulting in deformity, permanent loss of function and/or bone erosion at or near the joint. The compounds of the invention may be for use in the treatment or amelioration of any one, several or all of these symptoms of RA. Thus, in the context of RA, therapeutic benefit (discussed more generally, *infra*) is provided when a reduction or amelioration of any of the symptoms commonly associated with RA is achieved, regardless of whether the treatment results in a concomitant treatment of the underlying RA and/or a reduction in the amount of circulating rheumatoid factor ("RF").

As another specific example, systemic lupus erythematosis ("SLE") is typically associated with symptoms such as fever, joint pain (arthralgias), arthritis, and serositis (pleurisy or pericarditis). In the context of SLE, therapeutic benefit is provided when a reduction or amelioration of any of the symptoms commonly associated with SLE are achieved, regardless of whether the treatment results in a concomitant treatment of the underlying SLE.

As another specific example, multiple sclerosis ("MS") cripples the patient by disturbing visual acuity; stimulating double vision; disturbing motor functions affecting walking and use of the hands; producing bowel and bladder incontinence; spasticity; and sensory deficits (touch, pain and temperature sensitivity). In the context of MS, the therapeutic benefit is provided when an improvement or a reduction in the progression of any one or more of the crippling effects commonly associated with MS

is achieved, regardless of whether the treatment results in a concomitant treatment of the underlying MS.

When for use in the treatment or prevention of such diseases, the active compounds may be for administration singly, as mixtures of one or more active compounds or in mixture or combination with other agents useful for treating such diseases and/or the symptoms associated with such diseases. The active compounds may also be for administration in mixture or in combination with agents useful to treat other disorders or maladies, such as steroids, membrane stablizers, 5LO inhibitors, leukotriene synthesis and receptor inhibitors, inhibitors of IgE isotype switching or IgE synthesis, IgG isotype switching or IgG synthesis, β-agonists, tryptase inhibitors, aspirin, COX inhibitors, methotrexate, anti-TNF drugs, retuxin, PD4 inhibitors, p38 inhibitors, PDE4 inhibitors, and antihistamines, to name a few. The active compounds may be for administration *per se* in the form of prodrugs or as pharmaceutical compositions, comprising an active compound or prodrug.

Pharmaceutical compositions comprising the active compounds of the invention (or prodrugs thereof) may be manufactured by means of conventional mixing, dissolving, granulating, dragee-making levigating, emulsifying, encapsulating, entrapping or lyophilization processes. The compositions may be formulated in conventional manner using one or more physiologically acceptable carriers, diluents, excipients or auxiliaries which facilitate processing of the active compounds into preparations which can be used pharmaceutically.

The active compound or prodrug may be formulated in the pharmaceutical compositions *per se,* or in the form of a hydrate, solvate, N-oxide or pharmaceutically acceptable salt, as previously described. Typically, such salts are more soluble in aqueous solutions than the corresponding free acids and bases, but salts having lower solubility than the corresponding free acids and bases may also be formed.

Pharmaceutical compositions of the invention may take a form suitable for virtually any mode of administration, including, for example, topical, ocular, oral, buccal, systemic, nasal, injection, transdermal, rectal, vaginal, etc., or a form suitable for administration by inhalation or insufflation.

For topical administration, the active compound(s) or prodrug(s) may be formulated as solutions, gels, ointments, creams, suspensions, etc. as are well-known in the art.

Systemic formulations include those designed for administration by injection, *e.g*., subcutaneous, intravenous, intramuscular, intrathecal or intraperitoneal injection, as well as those designed for transdermal, transmucosal oral or pulmonary administration.

Useful injectable preparations include sterile suspensions, solutions or emulsions of the active compound(s) in aqueous or oily vehicles. The compositions may also contain formulating agents, such as suspending, stabilizing and/or dispersing agent. The formulations for injection may be presented in unit dosage form, *e.g*., in ampules or in multidose containers, and may contain added preservatives.

Alternatively, the injectable formulation may be provided in powder form for reconstitution with a suitable vehicle, including but not limited to sterile pyrogen free water, buffer, dextrose solution, etc., before use. To this end, the active compound(s) may be dried by any art-known technique, such as lyophilization, and reconstituted prior to use.

For transmucosal administration, penetrants appropriate to the barrier to be permeated are used in the formulation. Such penetrants are known in the art.

For oral administration, the pharmaceutical compositions may take the form of, for example, lozenges, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulfate). The tablets may be coated by methods well known in the art with, for example, sugars, films or enteric coatings.

Liquid preparations for oral administration may take the form of, for example, elixirs, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol, cremophore^{™} or fractionated vegetable oils); and preservatives (e.g., methyl or propyl-p-hydroxybenzoates or sorbic acid). The preparations may also contain buffer salts, preservatives, flavoring, coloring and sweetening agents as appropriate.

Preparations for oral administration may be suitably formulated to give controlled release of the active compound or prodrug, as is well known.

For buccal administration, the compositions may take the form of tablets or lozenges formulated in conventional manner.

For rectal and vaginal routes of administration, the active compound(s) may be formulated as solutions (for retention enemas) suppositories or ointments containing conventional suppository bases such as cocoa butter or other glycerides.

For nasal administration or administration by inhalation or insufflation, the active compound(s) or prodrug(s) can be conveniently delivered in the form of an aerosol spray from pressurized packs or a nebulizer with the use of a suitable propellant, e.g., dichlorodifluoromethane, trichlorofluoromethane, dichlorotetrafluoroethane, fluorocarbons, carbon dioxide or other suitable gas. In the case of a pressurized aerosol, the dosage unit may be determined by providing a valve to deliver a metered amount. Capsules and cartridges for use in an inhaler or insufflator (for example capsules and cartridges comprised of gelatin) may be formulated containing a powder mix of the compound and a suitable powder base such as lactose or starch.

A specific example of an aqueous suspension formulation suitable for nasal administration using commercially-available nasal spray devices includes the following ingredients: active compound or prodrug (0.5-20 mg/ml); benzalkonium chloride (0.1-0.2 mg/mL); polysorbate 80 (TWEEN® 80; 0.5-5 mg/ml); carboxymethylcellulose sodium or microcrystalline cellulose (1-15 mg/ml); phenylethanol (1-4 mg/ml); and dextrose (20-50 mg/ml). The pH of the final suspension can be adjusted to range from about pH5 to pH7, with a pH of about pH 5.5 being typical.

Another specific example of an aqueous suspension suitable for administration of the compounds *via* inhalation, contains 1-20 mg/mL Compound or prodrug, 0.1-1% (v/v) Polysorbate 80 (TWEEN®80), 50 mM citrate and/or 0.9% sodium chloride.

For ocular administration, the active compound(s) or prodrug(s) may be formulated as a solution, emulsion, suspension, etc. suitable for administration to the eye. A variety of vehicles suitable for administering compounds to the eye are known in the art. Specific non-limiting examples are described in U.S. Patent No. 6,261,547; U.S. Patent No. 6,197,934; U.S. Patent No. 6,056,950; U.S. Patent No. 5,800,807; U.S. Patent No. 5,776,445; U.S. Patent No. 5,698,219; U.S. Patent No. 5,521,222; U.S. Patent No. 5,403,841; U.S. Patent No. 5,077,033; U.S. Patent No. 4,882,150; and U.S. Patent No. 4,738,851.

For prolonged delivery, the active compound(s) or prodrug(s) can be formulated as a depot preparation for administration by implantation or intramuscular injection. The active ingredient may be formulated with suitable polymeric or hydrophobic materials (e.g., as an emulsion in an acceptable oil) or ion exchange resins, or as sparingly soluble derivatives, e.g., as a sparingly soluble salt. Alternatively, transdermal delivery systems manufactured as an adhesive disc or patch which slowly releases the active compound(s) for percutaneous absorption may be used. To this end, permeation enhancers may be used to facilitate transdermal penetration of the active compound(s). Suitable transdermal patches are described in for example, U.S. Patent No. 5,407,713.; U.S. Patent No. 5,352,456; U.S. Patent No. 5,332,213; U.S. Patent No. 5,336,168; U.S. Patent No. 5,290,561; U.S. Patent No. 5,254,346; U.S. Patent No. 5,164,189; U.S. Patent No. 5,163,899; U.S. Patent No. 5,088,977; U.S. Patent No. 5,087,240; U.S. Patent No. 5,008,110; and U.S. Patent No. 4,921,475.

Alternatively, other pharmaceutical delivery systems may be employed. Liposomes and emulsions are well-known examples of delivery vehicles that may be used to deliver active compound(s) or prodrug(s). Certain organic solvents such as dimethylsulfoxide (DMSO) may also be employed, although usually at the cost of greater toxicity.

The pharmaceutical compositions may, if desired, be presented in a pack or dispenser device which may contain one or more unit dosage forms containing the active compound(s). The pack may, for example, comprise metal or plastic foil, such as a blister pack. The pack or dispenser device may be accompanied by instructions for administration.

### 6.5 Effective Dosages

The active compound(s) of the invention or compositions thereof, will generally be used in an amount effective to achieve the intended result, for example in an amount effective to treat or prevent the particular disease being treated. The compound(s) may be for administration therapeutically to achieve therapeutic benefit or prophylactically to achieve prophylactic benefit. By therapeutic benefit is meant eradication or amelioration of the underlying disorder being treated and/or eradication or amelioration of one or more of the symptoms associated with the underlying disorder such that the patient reports an improvement in feeling or condition, notwithstanding that the patient may still be afflicted

with the underlying disorder. For example, administration of a compound to a patient suffering from an allergy provides therapeutic benefit not only when the underlying allergic response is eradicated or ameliorated, but also when the patient reports a decrease in the severity or duration of the symptoms associated with the allergy following exposure to the allergen. As another example, therapeutic benefit in the context of asthma includes an improvement in respiration following the onset of an asthmatic attack, or a reduction in the frequency or severity of asthmatic episodes. Therapeutic benefit also includes halting or slowing the progression of the disease, regardless of whether improvement is realized.

For prophylactic administration, the compound may be for administration to a patient at risk of developing one of the previously described diseases. For example, if it is unknown whether a patient is allergic to a particular drug, the compound may be for administration prior to administration of the drug to avoid or ameliorate an allergic response to the drug. Alternatively, prophylactic administration may be applied to avoid the onset of symptoms in a patient diagnosed with the underlying disorder. For example, a compound may be for administration to an allergy sufferer prior to expected exposure to the allergen. Compounds may also be for administration prophylactically to healthy individuals who are repeatedly exposed to agents known to one of the above-described maladies to prevent the onset of the disorder. For example, a compound may be for administration to a healthy individual who is repeatedly exposed to an allergen known to induce allergies, such as latex, in an effort to prevent the individual from developing an allergy. Alternatively, a compound may be for administration to a patient suffering from asthma prior to partaking in activities which trigger asthma attacks to lessen the severity of, or avoid altogether, an asthmatic episode.

The amount of compound for administration will depend upon a variety of factors, including, for example, the particular indication being treated, the mode of administration, whether the desired benefit is prophylactic or therapeutic, the severity of the indication being treated and the age and weight of the patient, the bioavailability of the particular active compound, etc. Determination of an effective dosage is well within the capabilities of those skilled in the art.

Effective dosages may be estimated initially from *in vitro* assays. For example, an initial dosage for use in animals may be formulated to achieve a circulating blood or serum concentration of active compound that is at or above an IC₅₀ of the particular compound as measured in as *in vitro* assay, such as the *in vitro* CHMC or BMMC and other *in vitro* assays described in the Examples section. Calculating dosages to achieve such circulating blood or serum concentrations taking into account the bioavailability of the particular compound is well within the capabilities of skilled artisans. For guidance, the reader is referred to Fingl & Woodbury, "General Principles," In: Goodman and Gilman's The Pharmaceutical Basis of Therapeutics, Chapter 1, pp. 1-46, latest edition, Pagamonon Press, and the references cited therein.

Initial dosages can also be estimated from *in vivo* data, such as animal models. Animal models useful for testing the efficacy of compounds to treat or prevent the various diseases described above are well-known in the art. Suitable animal models of hypersensitivity or allergic reactions are described in Foster, 1995, Allergy 50(21 Suppl):6-9, discussion 34-38 and Tumas et al., 2001, J. Allergy Clin. Immunol. 107(6):1025-1033. Suitable animal models of allergic rhinitis are described in Szelenyi et al., 2000, Armeimittelforschung 50(11):1037-42; Kawaguchi et al.,1994, Clin. Exp. Allergy 24(3):238-244 and Sugimoto et al., 2000, Immunopharmacology 48(1):1-7. Suitable animal models of allergic conjunctivitis are described in Carreras et al., 1993, Br. J. Ophthalmol. 77(8):509-514; Saiga et al., 1992, Ophthalmic Res. 24(1):45-50; and Kunert et al., 2001, Invest Ophthalmol. Vis. Sci. 42(11):2483-2489. Suitable animal models of systemic mastocytosis are described in O'Keefe et al., 1987, J. Vet. Intern. Med. 1(2):75-80 and Bean-Knudsen et al., 1989, Vet. Pathol. 26(1):90-92. Suitable animal models of hyper IgE syndrome are described in Claman et al., 1990, Clin. Immunol. Immunopathol. 56(1):46-53. Suitable animal models of B-cell lymphoma are described in Hough et al., 1998, Proc. Natl. Acad. Sci. USA 95:13853-13858 and Hakim et al., 1996, J. Immunol. 157(12):5503-5511. Suitable animal models of atopic disorders such as atopic dermatitis, atopic eczema and atopic asthma are described in Chan et al:, 2001, J. Invest. Dermatol. 117(4):977-983 and Suto et al., 1999, Int. Arch. Allergy Immunol. 120(Suppl 1):70-75. Ordinarily skilled artisans can routinely adapt such information to determine dosages suitable for human administration. Additional suitable animal models are described in the Examples section.

Dosage amounts will typically be in the range of from about 0.0001 or 0.001 or 0.01 mg/kg/day to about 100 mg/kg/day, but may be higher or lower, depending upon, among other factors, the activity of the compound, its bioavailability, the mode of administration and various factors discussed above. Dosage amount and interval may be adjusted individually to provide plasma levels of the compound(s) which are sufficient to maintain therapeutic or prophylactic effect. For example, the compounds may be administered once per week, several times per week (e.g., every other day), once per day or multiple times per day, depending upon, among other things, the mode of administration, the specific indication being treated and the judgment of the prescribing physician. In cases of local administration or selective uptake, such as local topical administration, the effective local concentration of active compound(s) may not be related to plasma concentration. Skilled artisans will be able to optimize effective local dosages without undue experimentation.

Preferably, the compound(s) will provide therapeutic or prophylactic benefit without causing substantial toxicity. Toxicity of the compound(s) may be determined using standard pharmaceutical procedures. The dose ratio between toxic and therapeutic (or prophylactic) effect is the therapeutic index. Compounds(s) that exhibit high therapeutic indices are preferred.

The invention having been described, the following examples are offered by way of illustration and not limitation.

### 7. EXAMPLES

### 7.1 Synthesis of Starting Materials and Intermediates Useful for Synthesizing The 2,4-Pyrimidinediamine Compounds According to Schemes (I)-(V)

A variety of starting materials and N4-monosubstituted-2-pyrimidineamines and N2-monosubstituted-4-pyrimidinediamines [mono Substitution Nucleophilic Aromatic Reaction (SNAR) products] useful for synthesizing the 2,4-pyrimidinediamine compounds of the invention according to Schemes (I)-(V) were prepared as described below. Conditions suitable for synthesizing the mono SNAR products are exemplified with 2-chloro-N4-(3,4-ethylenedioxyphenyl)-5-fluoro-4-pyrimidineamine (**R926087**).

| Section Number | Name of compound and reference number | Experimental |
|---|---|---|
| 7.1 | Synthesis of Starting Materials and Intermediates Useful for Synthesizing The 2,4-Pyrimidinediamine Compounds According to Schemes (I)-(V) | A variety of starting materials and N4-monosubstituted-2-pyrimidineamines and N2-monosubstituted-4-pyrimidinediamines [mono Substitution Nucleophilic Aromatic Reaction (SNAR) prfoducts] useful for synthesizing the 2,4-pyrimidinediamine compounds of the invention according to Schemes (I)-(V) were prepared as described below. Conditions suitable for synthesizing the mono SNAR products are exemplified with 2-chloro-N4-(3,4-ethylenedioxyphenyl)-5-fluoro-4-pyrimidineamine (R926087) |
| 7.1.1 | 2,4-Dichloro-5-fluoropyrimidine | To a dry reaction flask equipped with a stir bar and a reflux condenser was placed 5-fluorouracil (0.65g, 5mmol) followed by phosphorus oxychloride (POCl₃) (1.53g, 10mmol). The resultant mixture was heated at 110 °C for 8 hours under a nitrogen atmosphere. The reaction was cooled to room temperature, phosphorus pentachlonde (PCl₅) (3.12g, 15mmol) was added and heated to 110 °C for a period of 12 hours. After cooling to room temperature, the mixture was poured into ice-water, saturated with sodium chloride and left for 1 hour at 0 °C to complete the decomposition of POCl₃ and PCl₅. The solid of 2,4-dichloro-5-fluoropyrimidine was collected by rapid filtration, dried using blotting paper and stored at low temperature. ¹HNMR (CDCl₃): δ 8 47 (s, 1H); ¹³C NMR (CDCl₃): δ 155.42, 151.87, 147.43 and 147.13; ¹⁹F NMR (CDCl₃): - 38149. |
| 7.12 | 2,4-Dichloro-5-nitropyrimidme (Aldrich D6, 930-0) | A suspension of 5-nitrouracil (10g, 63 mmol) in POCl₃ (100 mL) was refluxed for 5h in the presence of N,N-dimethylaniline (10 mL), cooled to room temperature and poured on to crushed ice with vigorous stirring. The aqueous layer was extracted with ethyl acetate. The combined organic layers were dried over MgSO₄ and the solvent was evaporated under reduce pressure. The residue was purified by chromatography on silica gel (hexane/ethyl acetate; 1/1; v/v) to give the desired 2,4-dichloro-5-nitropyrimidine. LCMS: ret. time: 23.26 mm.; purity: 95%; ¹H NMR (CDCl₃): δ 9.16 (1H, s). |
| 7.1.3 | 2,4-Dichloro-5-cyanopyrimidine | In like manner to the preparation of 2,4-dichloro-5-nitropyrimidine, the reaction of 5-cyanouracil with POCl₃, and N,N-dimethylamine gave 2,4-dichloro-5-cyanopyrimidine. LCMS: ret. time: 13.75 min.; purity: 95%. |
| 7.1.4 | 2,4-Dichloro-5-trifluoromethylpyrimidine | In like manner to the preparation of 2,4-dichloro-5-nitropyrimidine, the reaction of 5-cyanouracil with POCl₃ and N,N-dimethylaniline gave 2,4-dichloro-5-cyanopyrimidine. ¹H NMR (CD₃OD): δ 9.07; LCMS: ret. time: 16.98 min. (fast method); purity: 70%. |
| 7.1.114 | 2-Chloro-N4-[(2,2-dimethyl-4H-5-pyrido[1,4]oxazin-3-one)-7-yl]-5-fluoro-4-pyrimidineamine (R940344) | In like manner to the preparation of 2-chloro-5-fluoro-N4-(3-methyloxycarbonyl-4-methoxyphenyl)-4-pyrimidineamine, 2,4-dichloro-5-fluoropyrimidine and 7-amino-2,2-dimethyl-4H-5-pyrido[1,4]oxazin-3-one were reacted to produce 2-chloro-N4-[(2,2-dimethyl-4H-5-pyrido[1,4]oxazin-3-one)- 7-yl]-5-fluoro-4-pyrimidineamine R940344. 1H NMR (DMSO-d6): □11.32 (1H, s), 10.20 (1H, s), 8.45 (1H, d, J= 3.6 Hz), 8.33 (1H, d, J= 2.1 Hz), 7.84 (1H, d, J= 2.1 Hz), 1.54 (6H, s) ; purity 90.8%; MS (m/e): 324 (MH+). |
| 7.1.116 | 2-Chloro-5-fluoro-N4-[2H-pyrido[3,2-b]-1,4-oxazin-3(4H)-one-6-yl]-4-pyrimidineamine (R945298) | In a manner similar to the preparation of 2-chloro-N4-(3,4-ethylenedioxyphenyl)-5-fluoro-4-pyrimidineamine, 2,4-dichloro-5-fluoropyrimidine and 6-amino-2H-pyrido[3,2-b]-1,4-oxazin-3(4H)-one were reacted to yield 2-chloro-5-fluoro-N4-[2H-pyrido[3,2-b]-1,4-oxazin-3(4H)-one-6-yl]-4-pyrimidineamine. 1H NMR (DMSO-d6): δ 4.63 (s, 2H), 7.34 (d, J= 8.7 Hz, 1H), 7.44 (d, J= 8.4 Hz, 1H), 8.33 (d, J= 3.3 Hz, 1H), 10.14 (s, 1H, NH), 11.19 (s, 1H, NH); 19F NMR (282 MHz, DMSO-d6): δ - 152.35; LCMS: ret. time: 26.74 min.; purity: 85.90%; MS (m/e): 296.13 (MH+). |
| 7.2 | Synthesis of Amines and Amine Precursors | |
| 7.2.1 | 5-Amino-2-(2-hydroxyethyleneoxy)pyri dine | A methanolic solution (50 mL) of 2-(2-hydroxyethyleneoxy)-5-nitropyridine (0.5 g) was hydrogenated in the presence of Pd/C (10%; 0.05 g) using a balloon filled with hydrogen for 2h. After the filtration through a pad of celite and washing with methanol the solution was concentrated to give the 5-amino-2-(2-hydroxyethyloxy)pyridine. 1H NMR (CDCl3): □ 7.58 (d, 1H, J= 3 Hz), 7.05 (dd, 1H, J= 2.7 and 8.1 Hz), 6.64 (d, 1H, J= 8.7 Hz), 4.36 (m, 2H), 3.89 (m, 2H). |
| 7.2.2 | 4-Chloro-3-methoxyaniline | In like manner to the preparation of 5-amino-2-(2-hydroxyethyleneoxy)pyridine, the hydrogenation of 4-chloro-3-methoxynitrobenzene gave 4-chloro-3-methoxyaniline. LCMS: purity: 98%; MS: 199 (M+ acetonitrile). |
| 7.2.3 | 2-[5-Amino-2-oxo-1,3-benzoxazol-3(2H)-yl]acetamide | In like manner to the preparation of 5-amino-2-(2-hydroxyethyleneoxy)pyridine, the hydrogenation of 2-[1,3-benzoxazol-2-oxo-5-nitro-3(2H)-yl)acetamide gave 2-[5-amino-2-oxo-1,3-bcnzoxazol-3(2H)-yl]acetamidc. LCMS: purity: 96%; MS: 208 (MH+). |
| 7.2.4 | 7-nitro-1,2,3,4-tetrahydroisoquinoline | 7-nitro-1,2,3,4-tetrahydroisoquinoline was prepared by nitration of 1,2,3,4-tetrahydroisoquinoline according to the following reference: Grunewald, Gary L.; Dahanukar, Vilas H.; Caldwell, Timothy M.; Criscione, Kevin R.; Journal of Medicinal Chemistry (1997), 40(25), 3997-4005. |
| 7.2.5 | 2-(t-Butoxycarbonyl)-7-nitro-1,2,3,4-tetrahydroisoquinoline | A mixture of 7-nitro-1,2,3,4-tetrahydroisoquinoline (0.55g, 3.1 mmole), di-t-butyldicarbonate (0.70g, 3.2 mmole), triethylamine (1.0 mL, 7.7 mmole) in dichloromethane (8 mL) was stirred at rt for 8h. The reaction mixture was diluted with water (50 mL) and stirred for 1h. The organic phase was separated and washed with brine. Concentration of the organic phase gave 2-(t-butoxycarbonyl)-7-nitro-1,2,3,4-tetrahydroisoquinoline. 1H NMR (CDCl3): δ 8.03-7.95 (m, 2H), 7.28 (d, 1H, J= 8.4 Hz), 4.66 (s, 2H), 3.68 (t, 2H, J= 6.0 Hz), 2.92 (t, 2H, J= 6.0 Hz),1.49 (s, 9H). |
| 7.2.6 | 2,3-Dihydro-6-nitro-4-benzypyranon | 3-(p-Nitrophenyl)-propionic acid is dissolved in concentrated sulfuric acid and treated with P2O5. The mixture is stirred for 1 hr at room temperature and poured onto ice. Filtration gave 2,3-dihydro-6-nitro-4-benzypyranon as a white solid. 1H NMR (DMSO): □ 8.47 (d, J = 3.0 Hz, 1H), 8.35 (dd, J = 3.0, 9.0 Hz, 1H), 7.29 (d, J = 9.0 Hz, 1H), 4.70 (t, J = 7.2 Hz, 1H), 2.90 (t, J = 7.2 Hz, 1H). |
| 7.2.7 | 3,4-Dihydro-4-hydroxy-6-amino-2H-1-benzopyran | A mixture 2,3-dihydro-6-nitro-4-benzypyranon and Pd/C (10%) in MeOH was hydrogenated at 22oC for 3 hours (40psi). The mixture was filtered and concentrated to dryness to give 3,4-dihydro-4-hydroxy-6-amino-2H-1-benzopyran as a brown oil. 1H NMR (DMSO): □ 6.40-6.56 (m, 3H), 5.05 (bs, 1H), 4.45 (bs, 1H), 3.94-4.09 (m, 2H), 1.76-1.98 (m, 2H). |
| 7.2.9 | 3-(N-morpholinocarbonyl)anili ne | To a 0°C solution of 3-nitrobenzoylchloride (0.50g, 2.7 mmole) and pyridine (0.27 mL, 3.2 mmole) in anhydrous dichloromethane (15 mL) was added morpholine (0.28 mL, 3.2 mmole). The reaction mixture was allowed to warm to rt and was stirred for 20h. The solvents were removed under vacuum and the residue suspended in ethyl acetate and washed with 1N HCl. The organic layer was washed with a saturated solution of NaHCO3 and brine. Removal of the solvents under vacuum provided 1-(N-morpholinocarbonyl)-3-nitrobenzene which was used without further purification. |
| | | A mixture of 1-(N-morpholinocarbonyl)-3-nitrobenzene (0.64 g) and 10% Pd on activated carbon (60 mg) in degassed methanol (65 mL) was stirred under a balloon of H2 for 2h. The reaction mixture was filtered through Celite@ filter aid and then concentrated under reduced pressure to provide 3-(N-morpholinocarbonyl)aniline in quantitative yield. |
| | | 1H NMR (CDCl3 δ 7.19-7.14 (m, 1H), 6.75-6.69 (m, 3H), 3.58-3.71 (m, 10H). |
| 7.2.10 | 3-(N-propylcarbonyl)aniline | In like manner to the preparation of 3-(N-morpholinocarbonyl)aniline, 3-nitrobenzoylchloride and n-propylamine were reacted to prepare 1-[(N-propylamino)carbonyl]-3-nitrobenzene which underwent hydrogenation to provide 3-(N-propylcarbonyl)aniline. 1H NMR (CDC13): δ 7.18 (t, 1H, J= 7.5 Hz), 7.13 (t, 1H, J= 1.8 Hz), 7.05-7.01 (m, 1H), 6.78 (ddd, 1H, J= 1.2, 2.4, and 7.5 Hz), 6.10 (bs, 1H), 3.58-3.53 (bs, 2H), 3.43-3.34 (m, 2H), 1.68-1.57 (m, 2H), 0.97 (t, 3H, J= 7.2 Hz). |
| 7.2.11 | 3-[4-(Ethoxycarbonyl)piperidi nocarbonyl]aniline | In like manner to the preparation of 3-(N-morpholinocarbonyl)aniline, 3-nitrobenzoylchloride and ethyl isonipecotate were reacted to prepare 1-[4-(ethoxycarbonyl)piperidinocarbonyl]-3-nitrobenzene which underwent hydrogenation to provide 3-[4-(ethoxycarbonyl)piperidinocarbonyl]aniline. |
| 7.2.12 | 3-(N-methylcarbonyl)aniline | In like manner to the preparation of 3-(N-morpholinocarbonyl)aniline, 3-nitrobenzoylchloride and methylamine hydrochloride were reacted to prepare 1-[(N-methylamino)carbonyl]-3-nitrobenzene which underwent hydrogenation to provide 3-(N-methylcarbonyl)aniline. 1H NMR (CDCl3): δ 7.18 (t, 1H, J= 7.5 Hz), 7.13 (t, 1H, J= 1.8 Hz), 7.04-6.99 (m, 1H), 6.81-6.75 (m, 1H), 6.05 (bs, 1H), 3.84 (bs, 2H), 2.99 (d, 3H, J= 4.8 Hz). |
| 7.2.13 | 7-Amino-1-tetralone | In like manner to the preparation of ethyl 4-aminophenoxyacetate, hydrogenation of 7-nitro-1-tetralone was carried out to prepare 7-amino-1-tetralotie. 1H NMR (CDCl3): δ 7.32 (d, 1H, J= 2.4 Hz), 7.05 (d, 1H, J= 8.1 Hz), 6.82 (dd, 1H, J= 2.4 and 8.1 Hz), 2.85 (t, 2H, J= 6.6 Hz), 2.61 (t, 2H, J= 6.6 Hz), 2.14-2.04 (m, 2H). |
| 7.2.14 | 7-Amino-2-(t-butoxycarbonyl)-1,2,3,4-tetrahydroisoquinoline | In like manner to the preparation of ethyl 4-aminophenoxyacetate, hydrogenation of 2-(t-butoxycarbonyl)-7-nitro-1,2,3,4-tetrahydroisoquinoline was carried out to prepare 7-amino-2-(t-butoxycarbonyl)-1,2,3,4-tetrahydroisoquinoline. 1H NMR (CDCl3): δ 6.92 (d, 1H, J= 8.4 Hz), 6.52 (dd, 1H, J= 2.4 and 8.4 Hz), 6.44 (bs, 1H), 4.47 (s, 2H), 3.63-3.48 (m, 2H), 2.71 (t, 2H, J= 5.1 Hz), 1.45 (s, 9H). |
| 7.2.15 | 7-Amino-1,2,3,4-tetrahydroisoquinoline | In like manner to the preparation of ethyl 4-aminophenoxyacetate, hydrogenation of 7-nitro-1,2,3,4-tetrahydroisoquinoline was carried out to prepare 7-amino-1,2,3,4-tetrahydroisoquinoline. 1H NMR (DMSO-d6): δ 9.35 (bs, 1H), 6.82 (d, 1H, J= 8.1 Hz), 6.45 (dd, 1H, J= 2.4 and 8.4 Hz), 6.30 (d, 1H, J= 2.4 Hz), 5.05 (s, 2H), 4.05 (s, 2H), 3.24 (t, 2H, J= 6.6 Hz), 2.78 (t, 2H, J= 6.6 Hz). |
| 7.2.16 | 2-(3-aminophenoxy)-N,2-dimethylpropanamide | In like manner to the preparation of ethyl 4-aminophenoxyacetate, hydrogenation of N,2-dimethyl-2-(3-nitrophenoxy)propanamide was carried out to prepare 2-(3-aminophenoxy)-N,2-dimethylpropanamide. 1H NMR (CDCl3): δ 7.03 (t, 1H, J= 7.8 Hz), 6.71 (bs, 1H), 6.39 (dd, 1H, J= 1.2 and 6.9 Hz), 6.29 (dd, 1H, J= 2.4 and 9.6 Hz), 6.25-6.22 (m, 1H), 2.86 (d, 3H, J= 4.2 Hz), 2.86 (d, 3H, J= 4.2 Hz), 1.50 (s, 6H). |
| 7.2.17 | Ethyl 2-(3-aminophenoxy)-2-methylpropanate | In like manner to the preparation of ethyl 4-aminophenoxyacetate, hydrogenation of ethyl 2-methyl-2-(3-nitrophenoxy)propanate was carried out to prepare ethyl 2-(3-aminophenoxy)-2-methylpropanate. 1H NMR (CDCl3): δ 6.99 (t, 2H, J= 8.7 Hz), 6.32 (dt, 1H, J= 1.2 and 7.2 Hz), 6.24-6.18 (m, 2H), 4.23 (q, 2H, J= 7.2 Hz), 1.58 (s, 6H), 1.24 (t, 3H, J= 6.9 Hz). |
| 7.2.18 | N-methyl-2-(5-amino-2-methylphenoxy)acetamide | In like manner to the preparation of ethyl 4-aminophenoxyacetate, hydrogenation of N-methyl-2-(2-methyl-5-nitrophenoxy)acetamide was carried out to prepare N-methyl-2-(5-amino-2-methylphenoxy)acetamide. 1H NMR (CD3OD): δ 6.86 (d, 1H, J= 7.5 Hz), 6.32-6.25 (m, 2H), 4.43 (s, 2H), 2.82 (s, 3H), 2.14 (s, 3H). |
| 7.2.19 | 6-Amino-2-(methoxycarbonyl)-(1H)-indole | 6-Amino-2-(methoxycarbonyl)-(1H)-indole was prepared according to the following references: |
| | | 1. Adams, Richard E.; Press, Jeffery B.; Deegan, Edward G.; Synthetic Communications (1991), 12 (5), 675-681. |
| | | 2. Boger, Dale L.; Yun, Weiya; Han, Nianhe; Johnson, Douglas S.; Biiorganic & Medicinal Chemistry (1995), 3(6), 611-621 |
| 7.2.20 | Preparation of 3-hydroxy-5-(methoxycarbonylmethyle neoxy)aniline and 3,5-bis(methoxycarbonylmeth yleneoxy)aniline | Benzyl N-(3,5-dihydroxyphenyl)carbamate |
| | | To a mixture of 5-aminobenzene-1,3-diol (0.60 g, 3.7 mmole) and sodium hydrogencarbonate (1.4 g, 16 mmole) in THF/water (15 mL, 1:1 v/v) was added dropwise benzyl chloroformate 1.6 mL, 11 mmole). After 3h at rt, THF was removed under vacuum and the remaining aqueous layer was extracted with ethyl acetate. Purification by column chromatograpy over silica gel provided benzyl N-(3,5-dihydroxyphenyl)carbamate. 1H NMR (CD3OD): δ 7.42-7.25 (m, 5H), 6.46 (d, 2H, J= 2.4 Hz), 5.97-5.94 (m, 1H), 5.14 (s, 2H). |
| | | Benzyl N-[3-hydroxy-5-(methoxycarbonylmethyleneoxy) phenyl]carbamate and Benzyl N-[3,5-bis(methoxycarbonyl methyleneoxy)phenyl]carbamate |
| | | In like manner to the preparation of ethyl 4-nitrophenoxyacetate, benzyl N-(3,5-dihydroxyphenyl)carbamate and methyl bromoacetate were reacted to give a mixture of benzyl N-[3-hydroxy-5-(methoxycarbonylmethyleneoxy)phenyl]carbamate 1H NMR (DMSO-d6): δ 9.62 (s, 1H), 9.44 (s, 1H), 7.42-7.31 (m, 5H), 6.63 (s, 1H), 6.50 (t, 1H, J= 2.4 Hz), 5.93 (t, 1H, J= 2.4 Hz), 5.10 (s, 2H), 4.63 (s, 2H), 3.67 (s, 3H), and benzyl N-[3,5-bis(methoxycarbonylmethyleneoxy)phenyl]carbamate |
| | | 1H NMR (CDCl3): δ 7.38-7.3 (m, 5H), 6.86 (s, 1H), 6.67 (d, 2H, J= 1.8 Hz), 6.19 (t, 1H, J= 2.4 Hz), 5.16 (s, 2H), 4.57 (s, 4H), 3.78 (s, 6H)which were separated by column chromatograpy over silica gel. |
| | | 3-Hydroxy-5-(methoxycarbonylmethyleneoxy)aniline |
| | | In like manner to the preparation of ethyl 4-aminophenoxyacetate, hydrogenation of benzyl N-[3-hydroxy-5-(methoxycarbonylmethyleneoxy)phenyl]carbamate was carried out to prepare 3-hydroxy-5-(methoxycarbonylmethyleneoxy)aniline. 1H NMR (CD3OD): δ 5.87-5.80 (m, 2H), 5.78-5.72 (m, 1H), 4.56 (s, 2H), 3.76 (s, 3H). |
| | | 3,5-Bis(methoxycarbonylmethyleneoxy)aniline |
| | | In like manner to the preparation of ethyl 4-aminophenoxyacetate, hydrogenation of benzyl N-[3,5-bis(methoxycarbonylmethyleneoxy)phenyl]carbamate was carried out to prepare 3,5-bis(methoxycarbonylmethyleneoxy)aniline. 1H NMR (CD3OD): δ 5.92 (d, 2H, J= 2.4 Hz), 5.83 (t, 1H, J= 2.4 Hz), 4.58 (s, 4H), 3.78 (s, 6H). |
| 7.2.22 | Ethyl 6-Nitro-3-carboxy-4H-imidazo[5,1-c]-1,4-benzoxazine | Was prepared according to J. of Heterocyclic Chemistry, 26, 205, (1989) |
| 7.2.23 | Ethyl 6-Amino-3-carboxy-4H-imidazo[5,1-c]-1,4-benzoxazine | Ethyl 6-Nitro-3-carboxy-4H-imidazo[5,1-c]-1,4-benzoxazine was reduced shaken in MeOH under 40 p.s.i. H2 with 20 weight percent of 10% Pd/C (Degussa) for 1 h then filtered and the solvent evaporated. The compound was purified directly by column chromatograph (EtOActhexane) to yield Ethyl 6-Amino-3-carboxy-4H-imidazo[5,1-c]-1,4-benzoxazine 1H (DMSO-d6) 8.41 (s, 1H), 6.98 (m, 1H), 6.82 (m, 1H), 6.43 (m, 1H), 5.28 ((s. 2H), 4.23 (q, 2H, J=6.2 Hz), 1.27 (t, 2H, J=6.2 Hz) purity 92 % MS (m/e): 232 (MH+). |
| 7.2.24 | 6-Amino-3,3-dimethyl-1,4-benzoxazine | A mixture of 15 g 2-Amino-4-nitrophenol and 40 g Boc2O in 300 mL CHCl3 was refluxed overnight filtered and the filtrate was evaporated to near dryness. The residue was triturated with hexanes, collected by suction filtration, and dried to yield 2-N-Boc-amino-4-nitrophenol. The 2-N-Boc-amino-4-nitrophenol was refluxed in acetone with 15.6 mL of 1-Chloro-2-methylpropene and 25 g potassium carbonate overnight. The reaction mixture was poured into ice-slush, the solid was collected by suction filtration and washed with water. The solid was dissolved in EtOAc and the organic was washed with 10% NaOH solution, water, then brine and dried over MgSO4. The organic was filtered to remove the drying agent and evaporated to yield 18 g 1-(2-N-Boc-amino-4-nitrophenoxy)-2-methyl-2-propene. 7.8 g of 1-(2-N-Boc-amino-4-nitrophenoxy)-2-methyl-2-propene was stirred overnight in methanolic HCl in a round-bottom flask with a septum wired on, and then heated with a reflux condenser attached at 80° C for 10 minutes. The reaction was cooled and the methanol was removed by rotary-evaporation. The residue was dissolved in 30 mL of 4N HCl, transferred to a new vessel to leave behind any undisolved solids and cooled to 0° C. 1.83 g of NaNO2 in 5 mL water was added drop wise and the solution was neutralized with solid sodium bicarbonate. A solution of 1.64 g NaN3 in 17 mL water was added slowly drop wise and the reaction was stirred 30 minutes. The precipitate was collected by suction filtration, washed well with water and dried on the funnel to yield 5.7 g 1-(2-Azido-4-nitrophenoxy)-2-methyl-2-propene. 7 g of 1-(2-Azido-4-nitrophenoxy)-2-methyl-2-propene was refluxed in 300 mL benzene overnight, cooled then evaporated. The crude product was recrystalized from EtOAc/Hexanes to yield 3-Methyl-6-nitro-azirino[2,1-c]-1,4-benzoxazine in two crops with a combined mass of 5.1 1 g of 3-Methyl-6-nitro-azirino[2,1-c]-1,4-benzoxazine was dissolved in 500 mL of MeOH/5% THF, 200 mg of 10% Pd/C (Degussa) was added and the resulting mixture was shaken under 30 p.s.i. H2 atmosphere for 8 hours. The reaction mixture was filtered through a pad of celite and the solvent evaporated. The residue was dissolved in a minimum amount of DCM/THF/MeOH and loaded onto a 5 cm by 20 cm 3% MeOH/DCM SiO2 column and the compound was eluted isocratically with a small amount of positive pressure. The appropriate fractions were combined and evaporated to yield 590 mg of 6-Amino-3,3-dimethyl-1,4-benzoxazine. 1H (DMSO-d6) 6.30 (d, 1H), 5.75 (d, 1H), 5.65 (dd, 1H), 3.58 (s, 2H), 1.08 (s, 6H) purity 99 % MS (m/e): 179 (MH+). |
| 7.2.25 | Ethyl 4-Aminophenoxyacetate | Ethyl4-Nitrophenoxyacetate |
| | | A dry reaction flask equipped with a reflux condenser, N2 inlet and a magnetic stirring bar was charged with 3-nitrophenol (76.45 g, 550 mmol), K2CO3 (76.45 g, 550 mmol) and dry acetone (500 mL) under N2 atmosphere. To this at room temperature was added ethyl bromoacetate (55.44 mL, 500 mmol) over a period of 15 min. The reaction mixture was refluxed for 16h, cooled and poured over ice-water (4 Kg). The resulting aqueous solution was extracted with CH2Cl2 (3 x 500 mL), dried over anhydrous Na2SO4 and solvent was removed to obtain 103g (92%) of the desired ethyl 4-nitrophenoxyacetate. 1H NMR (CDCl3): □ 8.20 (d, 2H, J= 8.2 Hz), 6.95 (d, 2H, J= 8.1 Hz), 4.72 (s, 2H), 4.25 (q, 2H), 1.23 (t, 3H); LCMS: ret. time: 27.07 min.; purity: 100%; MS: 267 (M+ acetonitrile). |
| | | Ethyl 4-Aminophenoxyacetate |
| | | A solution of ethyl 4-nitrophenoxyacetate (15 g) in EtOH (400 mL) was hydrogenated at 40 PSI for 40 minutes in the presence of 10% Pd/C (1.5 g, 10% by weight). After the filtration through a celite the solvent was removed under a reduced pressure to obtain ethyl 4-aminophenoxyacetate. 1H NMR (CDCl3): □ 6.77 (d, 2H, 8.1 Hz), 6.60 (d, 2H, J= 8.0 Hz), 4.50 (s, 2H), 4.24 (q, 2H), 1.24 (t, 3H); LCMS: ret. time: 12.00 min.; purity: 100%; MS (m/e): 196 (MH+). |
| 7.2.26 | tert-Butyl 4-Aminophenoxyacetate | tert-Butyl 4-Nitrophenoxyacetate |
| | | In like manner to the preparation of ethyl 4-nitrophenoxyacetate, 4-nitrophenol and tert-butyl bromoacetate were reacted to prepare tert-butyl 4-nitrophenoxyacetate. 1H NMR (CDCl3): □ 8.2 (d, 2H, J= 8.1 Hz), 6.95 (d, 2H, J= 8.2 Hz), 4.60 (s, 2H), 1.42 (s, 9H). |
| | | tert-Butyl 4-Aminophenoxyacetate |
| | | In like manner to the preparation of ethyl 4-aminophenoxyacetate, hydrogenation of tert-butyl 4-nitrophenoxyacetate was carried out to prepare tert-butyl 4-aminophenoxyacetate. 1H NMR (CDCl3): □ 6.74 (d, 2H, J= 9 Hz), 6.62 (d, 2H, J= 9 Hz), 4.42 (s, 2H), 1.42 (s, 9H); LCMS: ret. time: 16.35 min.; purity: 94%; MS (m/e): 224 (MH+). |
| 7.2.27 | Ethyl 3-Aininophenoxyacetate | Ethyl 3-Nitrophenoxyacetate |
| | | In like manner to the preparation of ethyl 4-nitrophenoxyacetate, 3-nitrophenol and ethyl bromoacetate were reacted to prepare ethyl 3-nitrophenoxyacetate. 1H NMR (CDCl3): □ 7.88 (dt, 1H, J= 1.2 and 8.7 Hz), 7.71 (t, 1H, J= 2.4 Hz), 7.45 (t, 1H, J= 8.4 Hz), 7.27 (dt, 1H, J= 2.4 and 8.4 Hz), 4.70 (s, 2H), 4.29 (q, 2H, J= 6.9 Hz), 1.30 (t, 3H, J= 6.9 Hz); LCMS: ret. time: 27.28 min.; purity: 96%. |
| | | Ethyl 3-Aminophenoxyacetate |
| | | In like manner to the preparation of ethyl 4-aminophenoxyacetate, hydrogenation of ethyl 3-nitrophenoxyacetate was carried out to prepare ethyl 3-aminophenoxyacetate. 1H NMR (CDCl3): □ 7.05 (t, 1H, J= 7.2 Hz), 6.30 (m, 3H), 4.56 (s, 2H), 4.25 (q, 2H, J= 7.2 Hz), 1.29 (t, 3H, J= 6.9 Hz); LCMS: ret. time: 10.69 min.; purity: 96%; MS (m/e): 196 (MH+). |
| 7.2.28 | (+)-Ethyl 2-(4-Aminophenoxy)propionat e | In like manner to the preparation of ethyl 4-aminophenoxyacetate, hydrogenation of ethyl (+)-2-(4-nitrophenoxy)propionate was carried out to prepare (+) ethyl 2-(4-aminophenoxy)propionate. 1H NMR (CDCl3): □ 6.70 (d, 2H), 6.58 (d, 2H), 4.60 (m, 1H), 4.20 (q, 2H), 3.2 (bs, 2H), 1.45 (d, 3H), 1.22 (t, 3H). |
| 7.2.29 | N-Methyl 3-Aminophenoxyacetamide | N-Methyl 3-Nitrophenoxyacetamide |
| | | A mixture of ethyl 3-nitrophenoxyacetate (9.12g, 40 mmol), methylamine hydrochloride (26.8g, 400 mmol) and diisopropylethylamine (35.5 mL, 200 mL) in MeOH (100 mL) was stirred in a pressure vial at 90 oC for 6h. The reaction was cooled to room temperature, diluted with water (1 liter), the solid formed was filtered, washed with water and dried to get the desired N-methyl 3-nitrophenoxyacetamide (8g, 95%). 1H NMR CDCl3): □ 7.91 (dd, 1H, J= 1.8 and 8.1 Hz), 7.78 (t, 1H, J= 2.4 Hz), 7.50 (t, 1H, J= 8.7 Hz), 7.29 (dd, 1H, J= 1.8 and 8.4 Hz), 6.50 (bs, 2H), 4.57 (s, 2H), 2.95 and 2.93 (2s, 3H); LCMS: ret. time: 17.54 min.; purity: 100%; MS (m/e): 211 (MH+). |
| | | N-Methyl 3-Aminophenoxyacetamide |
| | | In like manner to the preparation of ethyl 4-aminophenoxyacetate, the hydrogenation of N-methyl 3-nitrophenoxyacetamide (8 g, 39 mmol) was conducted to give the desired N-methyl 3-aminophenoxyacetamide (6g, 86%). 1H NMR (CD3OD): □ 6.99 (t, 1H, J= 8.1 Hz), 6.37-6.25 (m, 3H), 4.41 (s, 2H), 2.80 (s, 3H); LCMS: ret. time: 19.80 min.; purity: 100%. |
| 7.2.30. | 2-Methoxycarbonyl-5-aminobenzofuran (R926610) | 2-Methoxycarbonyl-5-nitrobenzofuran (R926609) |
| | | To a suspension of 5-nitro-2-benzofurancarboxylic acid (5 g, 24.15 mmol) in CH2Cl2 (250 mL) at 0 oC was added DMF (0.100 mL) followed by (COCl)2 (2M in CH2Cl2, 36.23 mL, 72.46 mL) over a period of 10 min. The reaction was stirred at 0 oC for 1h and then at room temperature for 30 min. The reaction solvent was removed under a reduced pressure, dried under high vacuum and again suspended in CH2Cl2 (250 mL). The solution was cooled to 0 oC, were added pyridine (4.8 mL, 48.03 mmol) followed by MeOH (10 mL, excess) and stirred overnight. The extractive work-up with CH2Cl2 gave the expected 2-methoxycarbonyl-5-nitrobenzofuran (R926609). 1H NMR (CDCl3): □ 8.66 (d, 1H, J= 2.4 Hz), 8.36 (dd, 1H, J= 2.4 and 9.6 Hz), 7.71 (d, 1H, J= 9.3 Hz), 7.65 (s, 1H), 4.01 (s, 3H); LCMS: ret. time: 26.94 min. |
| | | 2-Methoxycarbonyl-5-aminobenzofuran (R926610) |
| | | In like manner to the preparation of ethyl 4-aminophenoxyacetate, the hydrogenation of 2-methoxycarbonyl-5-nitrobenzofuran (2 g) in MeOH gave 2-methoxycarbonyl-5-aminobenzofuran. 1H NMR (CDCl3): □7.38 (bt, 2H), 6.90 (bd, 1H), 6.85 (bdd, 1H), 3.98 (s, 3H). |
| 7.2.31 | Methyl 2-(2-methyl-5-nitrophenoxy)acetate | In like manner to the preparation of ethyl 4-nitrophenoxyacetate, 2-methyl-5-nitrophenol and methyl bromoacetate were reacted to prepare methyl 2-(2-methyl-5-nitrophenoxy)acetate. 1H NMR (CD3OD): δ 7.80 (dd, 1H, J= 2.4 and 8.1 Hz), 7.65 (d, 1H, J= 2.4 Hz), 7.38 (d, 1H, J= 8.1 Hz), 4.90 (s, 2H), 3.80 (s, 3H), 2.36 (s, 3H). |
| 7.2.32 | Ethyl 2-methyl-2-(3-nitrophenoxy)propanate | A mixture of 3-nitrophenol (0.50g, 3.6 mmole), ethyl bromodimethylacetate (0.64g, 3.3 mmole), K2CO3 (1.3 g, 9.4 mmole), potassium iodide (catalytic) in absolute ethanol (8 mL) was heated at 70°C for 18h. The reaction mixture was cooled, poured into a saturated solution of NaHCO3, and extracted with dichloromethane. The product, ethyl 2-methyl-2-(3-nitrophenoxy)propanate, was obtained after purification by column chromatography over silica gel. 1H NMR (CDCl3): δ 7.85 (dt, 1H, J= 1.2 and 8.1 Hz), 7.68 (t, 1H, J= 2.4 Hz), 7.40 (t, 1H, J= 8.4 Hz), 7.19-7.13 (m, 1H), 4.26 (q, 2H, J= 7.2 Hz), 1.64 (s, 6H), 1.26 (t, 3H, J= 7.21), |
| 7.2.33 | N-Methyl-2-(2-methyl-5-nitrophenoxy)acetamide | In like manner to the preparation of N-methyl 3-nitrophenoxyacetamide, methyl 2-methyl-5-nitrophenoxyacetate and methylamine hydrochloride were reacted to prepare N-methyl-2-(2-methyl-5-nitrophenoxy)acetamide. 1H NMR (CD3OD): δ 7.82 (dd, 1H, J= 2.4 and 8.1 Hz), 7.69 (d, 1H, J= 2.4 Hz), 7.40 (d, 1H, J= 8.1 Hz), 4.66 (s, 2H), 2.83 (s, 3H), 2.40 (s, 3H). |
| 7.2.34 | N,2-Dimethyl-2-(3-nitrophenoxy)propanamid e | In like manner to the preparation of ethyl 2-methyl-2-(3-nitrophenoxy)propanate, 3-nitrophenol and N,2-dimethyl-2-bromopropanamide (prepared according to the following reference: Guziec, Frank S., Jr.; Torres, Felix F. Journal of Organic Chemistry (1993), 58(6), 1604-6) were reacted to prepare N,2-dimethyl-2-(3-nitrophenoxy)propanamide. 1H NMR (CDCl3): δ 7.94 (dt, 1H, J= 1.2 and 8.1 Hz), 7.78 (t, 1H, J= 2.4 Hz), 7.45 (t, 1H, J= 8.4 Hz), 7.22 (ddd, 1H, J= 1.2, 2.4, and 8.1 Hz), 6.61 (bs, 1H), 2.89 (d, 3H, J= 5.1 Hz), 1.55 (s, 6H). |
| 7.2.35 | 4-Amino-[(1H, 1,2,3,4-tetrazolyl)methyleneoxy]b enzene | 4-Nitro-[(1H,1,2,3,4-tetrazol-5-yl)methyleneoxy]benzene |
| | | A mixture of 2-cyanomethoxy-4-nitrophenyl (5.8 g, 32.6 mmol), sodium azide (6.3 g, 98.0 mmol) and ammonium chloride (8.5 g, 163.3 mmol) was suspended in DMF (100 mL) containing acetic acid (1 mL) and the mixture heated at 70 °C. After 17 h, the reaction was cooled to room temperature and 2 N aqueous hydrochloric acid (100 mL) was added. The solid which precipitated out of the reaction mixture was collected by filtration, washed with water (2 x 20 mL) then hexane (30 mL), affording compound 4-nitro-[(1H,1,2,3,4-tetrazol-5-yl)methyleneoxy]benzene (6.7 g, 99%) as an orange solid: 1H NMR (300 MHz, DMSO-d6) δ 8.25 (d, J = 9.2 Hz, 2H), 7.29 (d, J = 9.1 Hz, 2H), 5.68 (s, 2H); ESI MS m/z 220 [C8H7N5O3 - H]-. 4-Amino-[(1H,1,2,3,4-tetrazolyl)methyleneoxy]benzene |
| | | A mixture of 4-nitro-[(1,2,3,4-tetrazol-5-yl)methyleneoxy]benzene (6.7 g, 30.4 mmol) and 5 wt % palladium on carbon (700 mg) suspended in ethanol/concentrated hydrochloric acid (14:1, 150 mL) was hydrogenated in a sealed vessel at 50 psi. The mixture was shaken until no further hydrogen uptake was observed, after which the reaction was filtered through diatomaccous earth with chloroform and the filtrate concentrated to afford crude product. Purification by flash chromatography (7:2.5:0.5 CHCl3/CH3OH/NH4OH) afforded 4-amino-[(1H,1,2,3,4-tetrazolyl)methyleneoxy)]benzene as a brown solid: 1H NMR (300 MHz, DMSO-d6) δ 6.76 (d, J = 8.7 Hz, 2H), 6.52 (d, J = 8.7 Hz, 2H), 5.07 (s, 2H); ESI MS m/z 190 [C8H9N5O - H]-. |
| 7.2.36 | 4-Amino-[(1-methyl-1,2,3,4-tetrazol-5-yl)methyleneoxy]-benzene | 4-Nitro-[(1-methyl-1,2,3,4-tetrazol-5-yl)methyleneoxy]-benzene and 4-Nitro-[(2-methyl-1,2,3,4-tetrazol-5-yl)methyleneoxy]benzene |
| | | A mixture of 4-nitro-[(1H,1,2,3,4-tetrazolyl)methyleneoxy]benzene (10.00 g, 45.2 mmol), cesium carbonate (22.09 g, 67.8 mmol) and methyl iodide (7.70 g, 54.3 mmol) in DMF (200 mL) was stirred at room temperature for 24 h. The reaction mixture was concentrated to remove most of the DMF and the crude residue was partitioned between chloroform (100 mL) and water (50 mL). The organic phase was separated, washed with brine, dried (Na2SO4) and concentrated to afford crude product as a orange solid. Purification by flash chromatography (chloroform) afforded 4-nitro-[(1-methyl-1,2,3,4-tetrazol-5-yl)methyleneoxy]-benzene: 1H NMR (300 MHz, DMSO-d6) δ 8.26 (d, J = 9.2 Hz, 2H), 7.31 (d, J = 9.2 Hz, 2H), 5.72 (s, 2H), 4.15 (s, 3H); and 4-nitro-(2-methyl-1,2,3,4-tetrazol-5-yl)methyleneoxy]benzene: 1H NMR (300 MHz, DMSO-d6) δ 8.24 (d, J = 9.3 Hz, 2H), 7.29 (d, J = 9.3 Hz, 2H), 5.58 (s, 2H), 4.41 (s, 3H). |
| | | 4-Amino-[(1-methyl-1,2,3,4-tetrazol-5-yl)methyleneoxy]-benzene |
| | | A mixture of 4-nitro-[(1-methyl-1,2,3,4-tetrazol-5-yl)methyleneoxy]-benzene (3.60 g, 15.3 mmol) and 5% Pd/C (0.40 g) in 14:1 ethanol/concentrated hydrochloric acid (75 mL) was shaken at room temperature in a atmosphere of hydrogen at 50 psi. After 4 h no further hydrogen uptake was observed. The reaction mixture was filtered through diatomaceous earth, the solids washed with a 6:3:1 chloroform/methanol/concentrated ammonium hydroxide solution and the filtrate concentrated to afford crude 4-amino-[(1-methyl-1,2,3,4-tetrazol-5-yl)methyleneoxy]-benzene, which was purified by flash chromatography (95:5 chloroform/ methanol): 1H NMR (300 MHz, DMSO-d6) δ 7.48 (br s, 2H), 6.79 (d, J = 6.9 Hz, 2H), 6.55 (d, J = 6.9 Hz, 2H), 5.36 (s, 2H), 4.10 (s, 3H). |
| 7.2.37 | 4-Amino-[(2-methyl-1,2,3,4-tetrazol-5-yl)methyleneoxy]benzene | A mixture of 4-nitro-[(2-methyl-1,2,3,4-tetrazol-5-yl)methyleneoxy]benzene (3.60 g, 15.3 mmol) and 5% Pd/C (0.40 g) in 14:1 ethanol/concentrated hydrochloric acid (75 mL) was shaken at room temperature in a hydrogen atmosphere at 50 psi. After 3 h no further hydrogen uptake was observed. The reaction mixture was filtered through diatomaceous earth, the solids washed with a 6:3:1 chloroform/methanol/concentrated ammonium hydroxide solution and the filtrate concentrated to afford crude 4-amino-[(2-methyl-1,2,3,4-tetrazol-5-yl)methyleneoxy]benzene, which was purified by flash chromatography (95:5 chloroform/ methanol): 1H NMR (300 MHz, DMSO-d6) δ 6.80 (br s, 2H), 6.75 (d, J = 9.0 Hz, 2H), 6.50 (d, J = 9.0 Hz, 2H), 5.17 (s, 2H), 4.37 (s, 3H). |
| 7.2.38 | 2-Ethoxycarbonyl-5-aminoindole (R926611) | In like manner to the preparation of ethyl 4-aminophenoxyacetate, the hydrogenation of 2-ethoxycarbonyl-5-nitroindole gave the 2-ethoxycarbonyl-5-aminoindol. LCMS: ret. time: 13.44 min.; purity: 93%; MS (m/e): 205 (MH+). |
| 7.2.39 | 5-[(4-Aminophenoxy)methyl]-3-phenyl-1,2,4-oaxadiazole | Preparation of 5-[4-(Nitrophenoxy)methyl]-3-phenyl-1,2,4-oxadiazole 4-Nitrophenol (0.36 g, 2.56 mmole), 5-(chloromethyl)-3-phenyl-1,2,4-oxadiazole (0.5 g, 2.56 mmole) and anhydrous K2CO3 (0.39 g, 2.82 mmole) were dissolved in anhydrous acetone (20 mL) and heated to reflux for 12 h. Reaction mixture was cooled and the solvent removed under vacuum. The crude solid formed was collected by filtration, washed with water and dried under vacuum to provide 5-[(4-nitrophenoxy)methyl]-3-phenyl-1,2,4-oxadiazole (0.70 g, 92%). 1H NMR (CDCl3): □8.25 (d, 2H, J= 8.8 Hz), 8.08 (dd, 2H, J= 8.2 Hz), 7.52-7.49 (m, 3H), 7.13 (d, 2H, J= 8.8 Hz), 5.45 (s, 2H). |
| | | Preparation of 5-[(4-Aminophenoxy)methyl]-3-phenyl-1,2,4-oxadiazole |
| | | The 5-[(4-nitrophenoxy)methyl]-3-phenyl-1,2,4-oxadiazole (0.5 g, 1.68 mmole) was dissolved in methanol:methylenechloride (1:1) (120 mL). Aqueous solution of (15 mL) sodium hydrosulfite (0.88g, 5.05 mmole) and K2CO3 (0.70g, 5.06 mmole) was added dropwise under nitrogen for 10 min. The contents were allowed to stir at room temperature. After consumption of starting material, reaction mixture was concentrated, diluted with water till the homogeneous layer formed. The aqueous layer was extracted with several times with ethylacetate and methylene chloride. The turbid organic layers were combined, dried with anhydrous Na2SO4 and concentrated. Purification of the solid concentrate by silica gel chromatography provided 5-[(4-aminophenoxy)methyl]-3-phenyl-1,2,4-oxadiazole (0.23g, 51%). 1H NMR (CDCl3): □8.11 (m, 2H), 7.52-7.46 (m, 3H), 6.87 (d, 2H, J= 8.8 Hz), 6.64 (d, 2H, J= 8.8 Hz), 5.26 (s, 2H), 3.49 (br s, 2H). |
| | | Preparation of 5-[(4-Nitrophenoxy)methyl]-3-methyl-1,2,4-oxadiazole |
| | | A mixture of 4-nitrophenoxy acetic acid (2.25 g, 11.4 mmole), acetamideoxime, triethylamine hydrochloride (3.85g, 27.62 mmole), EDCI.HCl (4.37g, 22.79 mmole) and diisopropylethylamine (7.42g, 57.40 mmole) in anhydrous THF (250 ml) was refluxed for 18h. The unhomogenous brown colored reaction mixture was quenched with water and extracted with EtOAc (3 x 300 mL). The combined organic layers washed successively with aqueous NaHCO3, brine and dried over anhydrous Na2SO4. Removal of solvent and purified by chromatographic purification provided 5-[(4-nitrophenoxy)methyl]-3-methyl-1,2,4-oxadiazole (1.62 g, 60 %). 1H NMR (CDCl3): □8.24 (d, 2H, J= 8.8 Hz), 7.08 (d, 2H, J= 8.8 Hz), 5.36 (s, 2H), 2.44 (s, 3H). |
| | | Preparation of 5-[(4-Aminophenoxy)methyl]-3-methyl-1,2,4-oxadiazole |
| | | In like manner to the preparation of 5-[(4-aminophenoxy)methyl]-3-phenyl-1,2,4-oxadiazole, 5-(4-nitrophenoxymethyl)-3-methyl-1,2,4-oxadiazole was reacted with aqueous solution of sodium hydrosulfite and K2CO3 to prepare 5-[(4-aminophenox)ymethyl]-3-methyl-1,2,4-oxadiazole. 1H NMR (CDCl3): □6.82 (d, 2H, J= 8.8 Hz), 6.63 (d, 2H, J= 8.8 Hz), 5.15 (s, 2H), 3.38 (br s, 2H), 2.41 (s, 3H). |
| 7.2.40 | Ethyl 2-(4-aminophenyl)-2-methylpropionate | Ethyl 2-methyl-2-(4-nitrophenyl)propionate |
| | | A dry reaction flask charged with ethyl 4-nitrophenylacetate (5.0 g, 23.89 mmole), iodomethane (8.48 g, 3.72 mL, 59.74 mmole), 18-crown-6 (1.57 g, 5.93 mmole) in dry THF (200 mL) was cooled to -78 OC under nitrogen atmosphere. While stirring the contents, t-BuOK (5.90 g, 52.57 mmole) was added portionwise. The resulting violet precipitate was stirred at -78 OC for 2h and allowed the contents to warm to room temperature. The reaction was stirred at room temperature for 6h. At this time, once again the contents were cooled to -78 OC another portion of iodomethane, t-BuOK, and 18-crown-6 were added successively and stirred at the same temperature for 2h. The reaction was allowed to warm to room temperature and stirred overnight. The reaction was quenched with saturated aq. NH4Cl (75 mL), the resulting homogenous mixture extracted with ether (4 x 200 mL), dried over anhydrous Na2SO4, and concentrated. The concentrate was purified by silica gel column chromatography with 1%EtOAc/hexanes to provide ethyl 2-methyl-2-(4-nitrophenyl)propionate as a pale yellow oil (2.38, 42%). 1H NMR (CDCl3): □8.17 (d, 2H, J= 8.8 Hz), 7.49 (d, 2H, J= 8.8 Hz), 4.12 (qt, 2H, J= 7.0 Hz), 1.60 (s, 6H), 1.17 (t, 3H, J= 7.0 Hz). |
| | | Ethyl-2-(4-aminophenyl)-2-methylpropionate |
| | | In like manner to the preparation of ethyl 4-aminophenoxyacetate, the hydrogenation of ethyl 2-methyl-2-(4-nitrophenyl)propionate provided ethyl-2-(4-aminophenyl)-2-methylpropionate. 1H NMR (CDCl3): □7.16 (d, 2H, J= 8.8 Hz), 6.63 (d, 2H, J= 8.8 Hz), 4.09 (qt, 2H, J= 7.0 Hz), 3.62 (br s, 2H), 1.52 (s, 6H), 1.17 (t, 3H, J= 7.0 Hz). |
| 7.2.41 | Anilines substituted with 1,3,4-oxadiazole moieties | N'1-(3-Chlorobenzoyl)-3-nitrobenzene-1-carbohydrazide |
| | | To a solution of 3-chlorobenzohydrazide (1 equivalent) and pyridine (2 equivalents) in CH2Cl2 at 0 oC was added a CH2Cl2 solution of 3-nitrobenzoyl chloride (1 equivalents) and stirred at 0 oC for 1 h and then at room temperature for overnight. The resulting solution was concentrated and diluted with water, basified with NaHCO3, the solid was filtered, washed with water, dried and analyzed to obtain N'1-(3-chlorobenzoyl)-3-nitrobenzene-1-carbohydrazide. 1H NMR (DMSO-d6): □ 10.99 (s, 1H), 10.79 (s, 1H), 8.73 (bs, 1H), 8.43 (bdd, 1H, J= 1.2 and 8.1 Hz), 8.33 (bdd, 1J, J= 8.4 Hz), 7.95 (s, 1H), 7.87 (m, 2H), 7.67 (bdd, 1H, J= 1.2 and 8.1 Hz), 7.57 (t, 1H, J= 7.8 Hz); LCMS: purity: 85%; MS (m/e): 320 (MH+). |
| | | [2-(3-Chlorophenyl)-1,3,4-oxadiazol-5-yl]-3-nitrobenzene |
| | | A suspension of N'1-(3-chlorobenzoyl)-3-nitrobenzene-1-carbohydrazide (0.321 g) in POCl3 (3 mL) was stirred at 90 oC for 24 h. The resulting clear solution was quenched with ice-water, solid obtained was filtered washed with water, dried and analyzed to give [2-(3-chlorophenyl)-1,3,4-oxadiazol-5-yl]-3-nitrobenzene. 1H NMR (DMSO-d6): □ 8.86 (t, 1H, J= 1.8 Hz), 8.59 (dt, 1H, J= 1.8 and 8.4 Hz), 8.48 (m, 1H), 8.25 (t, 1H, J= 1.8 Hz), 8.16 (dt, 1H, J= 1.2 and 7.5 Hz), 7.93 (t, 1H, J= 8.1 Hz), 7.75 (m, 1H), 7.66 (t, 1H, J= 7.5 Hz), LCMS: purity: |
| | | 86%; MS (m/e): 302 (MH+). |
| | | Reduction of [2-(3-chlorophenyl)-1,3,4-oxadiazol-5-yl]-3-nitrobenzene The hydrogenation of [2-(3-chlorophenyl)-1,3,4-oxadiazol-5-yl]-3-nitrobenzene (0.2 g) using 10% Pd/C (0.04 g) in MeOH (200 mL) at 15 PSI for 1h gave a mixture of two products viz. 3-amino-[2-(3-chlorophenyl)-1,3,4-oxadiazol-5-yl]benzene and 3-amino-(2-phenyl-1,3,4-oxadiazol-5-yl)benzene which were separated by silica gel column chromatography using n-hexanes then n-hexanes: 5-10% EtOAc as a solvent system. 3-Amino-[2-(3-chlorophenyl)-1,3,4-oxadiazol-5-yl]benzene: 1H NMR (DMSO-d6): □ 8.08 (m, 2H), 7.64 (m, 4H), 7.42 (m, 1H), 7.10 (m, 1H); LCMS: purity: 82%; MS (m/e): 272 (MH+). 3-Amino-(2-phenyl-1,3,4-oxadiazol-5-yl)benzene: 1H NMR (DMSO-d6): □ 8.13 (m, 1H), 7.54 (m, 5H), 7.30 (m, 1H), 6.86 (dd, 1H, J= 1.5 and 8.1 Hz); LCMS: purity: 93%; MS (m/e): 238 (MH+). |
| | | N' 1-(Ethoxycarbonylmethylenecabonyl)-3-nitrobenzene-1-carbohydrazide In like manner to the preparation of N'1-(3-chlorobenzoyl)-3-nitrobenzene-1-carbohydrazide, the reaction of 3-nitrobenzoyl chloride with ethoxycarbonylmethlenecarbohydrazide gave N'1-(ethoxycarbonylmethylenecabonyl)-3-nitrobenzene-1-carbohydrazide.1H NMR (CD3OD): □8.74 (m, 1H), 8.44 (dd, 1H, 1.8 and 8.1 Hz), 8.25 (bd, 1H, J= 8.4 Hz), 7.76 (t, 1H, J= 8.4 Hz), 4.22 (q, 2H, J= 6.9 Hz), 3.44 (bs, 2H), 1.29 (t, 3H, J= 6.8 Hz); LCMS: purity: 93%; MS (m/e): 296 (MH+). |
| | | [2-(Ethoxycarbonylmethylene)-1,3,4-oxadiazol-5-yl]-3-nitrobenzene |
| | | In like manner to the preparation of [2-(3-chlorophenyl)-1,3,4-oxadiazol-5-yl]-3-nitrobenzene the reaction of POCl3 with N'1-(ethoxycarbonylmethylenecabonyl)-3-nitrobenzene-1-carbohydrazide gave [2-(ethoxycarbonylmethylene)-1,3,4-oxadiazol-5-yl]-3-nitrobenzene. 1H NMR (CDCl3): □ 8.88 (t, 1H, J= 1.8 Hz), 8.42 (m, 2H), 7.74 (t, 1H, J= 7.5 Hz), 4.27 (q, 2H, J= 7.2 Hz), 4.08 (s, 2H), 1.31 (t, 3H, J= 7.2 Hz); LCMS: purity: 95%; MS (m/e): 278 (MH+). |
| 7.2.42 | Synthesis of (+)-5-Amino-(2,3-dihydro-2-methoxycarbonyl)benzofu ran | 2-Methoxycarbonyl-5-nitrobenzofuran |
| | | A mixture of 2-carboxy-5-nitrobenzofuran (2.0 g), MeOH (10 mL) and Concentrated H2SO4 (2.1 mL) was heated in a sealed tube at 60 oC for 3 h. Upon cooling to the room temperature it was quenched with ice-water and carefully basified with addition of NaHC03. The solid obtained was filtered, washed with water, dried and analyzed to give 2-methoxycarbonyl-5-nitrobenzofuran. 1H NMR (CDCl3): □ 8.66 (d, 1H, J= 2.4 Hz), 8.36 (dd, 1H, J= 2.4 and 9.6 Hz), 7.71 (d, 1H, J= 9.3 Hz), 7.65 (s, 1H), 4.01 (s, 3H); LCMS: purity: 97%; MS (m/e): 222 (MH+). |
| | | (+)-5-Amino-(2,3-dihydro-2-methoxycarbonyl)benzofuran |
| | | A suspension of 2-methoxycarbonyl-5-nitrobenzofuran (2.0 g), 10% Pd/C (2.0 g), Na2SO4 (2.0 g) in MeOH (500 mL) was hydrogenated at 55 PSI for 3 days. The resulting solution was filtered through a pad of celite, concentrated and chromatographed using n-hexanes then 10%, 20% EtOAc/n-hexanes to give (+)-5-amino-(2,3-dihydro-2-methoxycarbonyl)benzofuran. 1H NMR (CDCl3): □ 6.69 (d, 1H, J= 8.1 Hz), 6.56 (d, 1H, J= 1.2 Hz), 6.48 (dd, 1H, J= 1.8 and 7.5 Hz), 5.14 (dd, 1H, J= 6.6 and 7.2 Hz), 3.79 (s, 3H), 3.47 (dd, 1H, J= 10.5 and 10.8 Hz), 3.26 (dd, 1H, J= 7.2 and 6.6 Hz); LCMS: purity: 100%; MS (m/e): 194 (MH+). |
| 7.2.43 | 3-[1-Bis(ethoxycarbonyl)ethox y]aniline | Preparation of Diethyl 2-methyl-2-(3-nitrophenoxy)malonate |
| | | Diethyl 2-bromo-2-methylmalonate (1.0 g, 3.95 mmole) was added to a stirred suspension of potassium fluoride (0.57 g, 9.8 mmole) in dry DMF (5 mL). After stirring for 20 min at room temperature, 3-nitrophenol (0.55 g, 3.95 mmole) was added. The resulting mixture was stirred at 60 OC for 6 h, cooled to room temperature, diluted with water (30 mL) and extracted with ethyl acetate (3 X 200 mL). The organic layer was washed with aq. 1N NaOH (2 X 75 mL), dried over anhydrous Na2SO4, filtered and evaporated in vacuo to provide diethyl 2-methyl-2-(3-nitrophenoxy)malonate (0.89 g, 80%). 1H NMR (CDCl3): □ 7.92 (dd, 1H, J = 2.3 and 8.2 Hz), 7.82 (t, 1H, J = 2.3 Hz), 7.41 (t, 1H, J = 8.2 Hz), 7.30 (dd, 1H, J = 2.3 and 8.2 Hz), 4.28 (qt, 4H, J = 7.0 Hz), 1.81 (s, 3H), 1.26 (t, 6H, J = 7.0 Hz). |
| | | Preparation of 3-[1-Bis(ethoxycarbonyl)ethoxy]aniline |
| | | Diethyl 2-methyl-2-(3-nitrophenoxy)malonate (0.75 g, 2.40 mmole) was dissolved in toluene: ethanol (1:1, 100 mL), transferred to par shaker bottle containing Pd/C (0.15 g) and anhydrous Na2SO4 (5.0 g) in the presence of nitrogen atmosphere. The resulting mixture was treated with hydrogen (30 PSI) till the disappearance of diethyl 2-methyl-2-(3-nitrophenoxy)malonate (2 h). The mixture was filtered through celite covered with anhydrous Na2SO4 followed by washing the celite pad with EtOAc. The filtrated was concentrated and dried under vacuo to furnish 3-[1-bis(ethoxycarbonyl)ethoxy]aniline in quantitative yield. 1H NMR (CDCl3): □ 6.98 (t, 1H, J = 8.2 Hz), 6.37-6.28 (m, 3H), 4.26 (qt, 4H, J = 7.0 Hz), 3.65 (br s, 2H), 1.72 (s, 3H), 1.24 (t, 6H, J = 7.0 Hz). |
| 7.2.44 | Preparation of 4-(4-aminophenoxymethyl)-2-methoxycarbonyl-furan | Preparation of 4-(4-nitrophenoxymethyl)-2-methoxycarbonyl-furan |
| | | 3-Nitrophenol (1.0 g, 7.19 mmole), methyl 5-(chloromethyl)-2-furoate (1.38 g, 7.90 mmole) and anhydrous K2CO3 (1.19 g, 8.60 mmole) in acetone (30 mL) were refluxed for 8 h. The reaction mixture was cooled and diluted with water. The resultant white solid was filtered, washed with water and air dried overnight to give 1.81 g (90%) of the desired product. 1H NMR (CDCl3): □ 7.86 (dd, 1H, J = 2.3 and 8.2 Hz), 7.80 (t, 1H, J = 2.3 Hz), 7.45 (t, 1H, J = 8.2 Hz), 7.27 (dd, 1H, J = 2.3 and 8.2 Hz), 7.17 (d, 1H, J = 3.5 Hz), 6.58 (d, 1H, J = 3.5 Hz), 5.13 (s, 2H), 3.90 (s, 3H). |
| | | Preparation of 4-(4-aminophenoxymethyl)-2-methoxycarbonyl-furan |
| | | In like manner to the reduction of diethyl 2-methyl-2-(3-nitrophenoxy)malonate, 4-(4-nitrophenoxymethyl)-2-methoxycarbonyl-furan was reduced to provide 4-(4-aminophenoxymethyl)-2-methoxycarbonyl-furan. 1H NMR (CDCl3): □ 7.15 (d, 1H, J = 3.5 Hz), 7.05 (t, 1H, J = 8.2 Hz), 6.50 (d, 1H, J = 3.5 Hz), 6.37-6.27 (m, 3H), 5.01 (s, 2H), 3.89 (s, 3H). |
| 7.2.45 | Preparation of 6-amino-1-(methoxycarbonyl)methyl indazoline | Preparation of 1-(methoxycarbonyl)methyl-6-nitroindazoline |
| | | To a solution of 6-nitroindazoline (2.0 g, 12.25 mmole) in dry DMF was added anhydrous K2CO3 (1.84 g, 13.31 mmole) and methyl 2-bromoacetate (2.04 g, 13.33 mmole). The resulting mixture was stirred at room temperature for 12 h. The reaction mixture was quenched with water and the resulting solid was collected by filtration, washed with excessive water, and air dried. The yellow solid collected was purified by silica gel column chromatography using gradient solvent system to furnish two products. The desired product (1.12 g, 41%) with high Rf value on the TLC in 30% EtOAc : hexanes was collected. |
| | | In like manner to the reduction of diethyl 2-methyl-2-(3-nitrophenoxy)malonate, 1-(Methoxycarbonyl)methyl-6-nitro-indazoline was reduced to provide 6-amino-1-(methoxycarbonyl)methylindazoline. 1H NMR (CDCl3): □ 7.73 (d, 1H, J = 1.1 Hz), 7.35 (d, 1H, J = 8.2 Hz), 6.49 (dd, 1H, J= 1.8 and 8.8 Hz), 6.39 (s, 1H), 5.34 (br s, 2H), 5.10 (s, 2H), 3.64 (s, 3H). |
| | | Preparation of 1-(methoxycarbonyl)methyl-5-nitroindazoline |
| | | In like manner to the preparation of 1-(methoxycarbonyl)methyl-6-nitroindazoline, 1-(methoxycarbonyl)methyl-5-nitroindazoline was prepared by alkylation of 5-nitroindazoline with methyl 2-bromoacetate in presence of K2CO3. The desired product (1.34 g, 46%) with high Rf value on the TLC in 30% EtOAc : hexanes was collected by silica gel column chromatographic purification. 1H NMR (CDCl3): □ 8.75 (d, 1H, J = 1.8 Hz), 8.30 (dd, 1H, J = 2.3 and 8.2 Hz), 8.26 (s, 1H), 7.40 (d, 1H, J = 8.2 Hz), 5.22 (s, 2H), 3.78 (s, 3H). Preparation of 5-amino-1-(methoxycarbonyl)methylindazoline |
| | | In like manner to the reduction of diethyl 2-methyl-2-(3-nitrophenoxy)malonate, 1-(Methoxycarbonyl)methyl-5-nitro-indazoline was reduced to provide 5-amino-1-(methoxycarbonyl)methylindazoline. 1H NMR (CDCl3): □ 7.84 (d, 1H, J = 2.3 Hz), 7.15 (d, 1H, J = 8.8 Hz), 6.95 (d, 1H, J= 2.3 Hz), 6.88 (dd, 1H, J = 2.3 and 8.8 Hz), 5.09 (s, 2H), 3.73 (s, 3H). |
| | | Preparation of 1-(2-ethoxycarbonylethyl)-6-nitroindazoline |
| | | In like manner to the preparation of 1-(methoxycarbonyl)methyl-6-nitroindazoline, 1-(ethoxycarbonyl)ethyl-6-nitroindazoline was prepared by alkylation of 6-nitroindazoline with ethyl 3-bromopropionate in presence of K2CO3. The desired product (58%) with high Rf value on the TLC in 30% EtOAc : Hexanes was collected by silica gel column chromatographic purification. 1H NMR (CDCl3): □ 8.49 (s, 1H), 8.12 (s, 1H), 8.01 (dd, 1H, J = 1.7 and 8.8 Hz), 7.82 (d, 1H, J = 8.8 Hz), 4.74 (t, 2H, J = 6.4 Hz), 4.09 (qt, 2H, J = 7.0 Hz), 3.03 (t, 2H, J = 6.4 Hz), 1.18 (t, 3H, J = 7.0 Hz). |
| | | Preparation of 6-amino-1-(2-ethoxycarbonylethyl)indazoline |
| | | In like manner to the reduction of diethyl 2-methyl-2-(3-nitrophenoxy)malonate, 1-(2-ethoxycarbonylethyl)-6-nitroindazoline was reduced to provide 6-amino-1-(2-ethoxycarbonylethyl)indazoline. 1H NMR (CDCl3): □ 7.81 (s, 1H), 7.46 (d, 1H, J = 8.8 Hz), 6.60 (app s, 1H), 6.55 (dd, 1H, J= 2.3 and 8.8 Hz), 4.51 (t, 2H, J = 7.0 Hz), 4.11 (qt, 2H, J = 7.0 Hz), 3.52 (br s, 2H), 2.91 (t, 2H, J = 7.0 Hz), 1.18 (t, 3H, J = 7.0 Hz). |
| | | Preparation of 1-(2-ethoxycarbonylethyl)-5-nitroindazoline |
| | | In like manner to the preparation of 1-(methoxycarbonyl)methyl-5-nitroindazoline, 1-(ethoxycarbonyl)ethyl-5-nitroindazoline was prepared by alkylation of 5-nitroindazoline with ethyl 3-bromopropionate in presence of K2CO3. The desired product (43%) with high Rf value on the TLC in 30% EtOAc : Hexanes was collected by silica gel column chromatographic purification. 1H NMR (CDCl3): □ 8.70 (d, 1H, J = 1.7 Hz), 8.27 (dd, 1H, J = 2.3 and 8.8 Hz), 8.20 (d, 1H, J =1.7 Hz), 7.59 (d, 1H, J = 8.8 Hz), 4.70 (t, 2H, J = 6.4 Hz), 4.07 (qt, 2H, J = 7.0 Hz), 3.01 (t, 2H, J = 6.4 Hz), 1.16 (t, 3H, J = 7.0 Hz). |
| | | Preparation of 5-amino-1-(2-ethoxycarbonylethyl)indazoline |
| | | In like manner to the reduction of diethyl 2-methyl-2-(3-nitrophenoxy)malonate, 1-(2-ethoxycarbonylethyl)-5-nitroindazoline was reduced to provide 5-amino-1-(2-ethoxycarbonylethyl)indazoline. 1H NMR (CDCl3): □ 7.78 (s, 1H), 7.30 (d, 1H, J = 8.8 Hz), 6.91 (d, 1H, J = 2.3 Hz), 6.87 (dd, 1H, J = 2.3 and 8.8 Hz), 4.59 (t, 2H, J = 6.4 Hz), 4.08 (qt, 2H, J = 7.0 Hz), 3.02 (br s, 2H), 2.92 (t, 2H, J = 7.0 Hz), 1.16 (t, 3H, J = 7.0 Hz). Preparation of 5-amino-2-methylindazoline |
| | | In like manner to the reduction of diethyl 2-methyl-2-(3-nitrophenoxy)malonate, commercially available 2-methyl-5-nitroindazoline was reduced to provide 5-amino-2-methylindazoline. 1H NMR (CDCl3): □ 7.61 (s, 1H), 7.53 (d, 1H, J = 8.8 Hz), 6.81 (dd, 1H, J = 2.3 and 8. 8 Hz), 6.75 (d, 1H, J = 2.3 Hz), 4.13 (s, 3H), 3.85 (br s, 2H). |
| 7.2.46 | Preparation of methyl 3-methoxy-4-[(6-nitroindazol-1-yl)methyl]benzoate | In like manner to the reduction of diethyl 2-methyl-2-(3-nitrophenoxy)malonate, methyl 3-methoxy-4-[(6-nitroindazol-1-yl)methyl]benzoate was reduced to provide methyl 4-[(6-aminoindazol-1-yl)methyl]benzoate. 1H NMR (CDCl3): □ 7.88 (s, 1H), 7.53 (d, 1H, J = 8.8 Hz), 7.51 (d, 1H, J = 8.8 Hz), 7.50 (d, 1H, J = 1. 7 Hz), 6.67 (d, 1H, J = 8.8 Hz), 6.56 (dd, 1H, J = 1.7 and 8.8 Hz), 6.45 (d, 1H, J = 1.2 Hz), 5.50 (s, 2H), 3.94 (s, 3H), 3.87 (s, 3H), 3.79 (br s, 2H). |
| | | Preparation of Methyl 4-[(6-aminoindazol-2-yl)methyl]benzoate |
| | | In like manner to the reduction of diethyl 2-methyl-2-(3-nitrophenoxy)malonate, methyl 3-methoxy-4-[(6-nitroindazol-2-yl)methyl]benzoate was reduced to provide methyl 4-[(6-aminoindazol-2-yl)methyl]benzoate. 1H NMR (CDCl3): □ 7.78 (s, 1H), 7.56-7.53 (m, 2H), 7.43 (d, 1H, J = 8.8 Hz), 6.98 (d, 1H, J = 8.2 Hz), 6.81 (app s, 1H), 6.58 (dd, 1H, J = 1.8 and 8.8 Hz), 5.53 (s, 2H), 3.91 (s, 3H), 3.89 (s, 3H). |
| 7.2.47 | Preparation of 6-amino-1-[2-methoxy-4-(o-toluylsulfonamidocarboxy )benzyl]indazoline | Preparation of 6-nitro-1-[2-methoxy-4-(o-toluylsulfonamidocarboxy) benzyl]indazoline |
| | | Ester hydrolysis of methyl 3-methoxy-4-[(6-nitroindazol-1-yl)methyl]benzoate in presence of LiOH:H2O produced the corresponding acid. The acid (1.65 g, 5.04 mmole) thus formed was converted to the acid chloride by reacting with SOCl2 (3.68 mL, 50.45 mmole) at reflux temperature for 5 h. The reaction mixture was cooled to room temperature and concentrated under vacuo. To acid chloride concentrate dissolved in dry CH2Cl2 (75 mL), o-toluylbenzenesulfonamide (0.95 g, 5.54 mmole) and 4-(dimethylamino)-pyridine (0.67 g, 5.54 mmole) were added successively at room temperature and stirred for 12 h. The reaction mixture was concentrated, dissolved in EtOAc (700 mL) and successively treated with 2 N HCl (2X100 mL), water (150 mL) and brine (100 mL). Usual workup and purification by silica gel column chromatography provided the product (1.57 g, 64%). 1H NMR (DMSO-d6): □8.75 (s, 1H), 8.31 (s, 1H), 8.00 (d, 1H, J = 8.8 Hz), 7.95-7.91 (m, 2H), 7.50 (d, 1H, J = 1.2 Hz), 7.46-7.27 (m, 4H), 6.92 (d, 1H, J = 7.6 Hz), 5.76 (s, 2H), 3.81 (s, 3H), 2.54 (s, 3H). |
| | | Preparation of 6-amino-1-[2-methoxy-4-(o-toluylsulfonamidocarboxy)benzylJindazoline |
| | | In like manner to the reduction of diethyl 2-methyl-2-(3-nitrophenoxy)malonate, 6-nitro-1-[2-methoxy-4-(o-toluylsulfonamidocarboxy)benzyl]indazoline was reduced to provide 6-amino-1-[2-methoxy-4-(o-toluylsulfonamidocarboxy)benzyl]indazoline. 1H NMR (CDCl3): □ 7.96 (dd, 1H, J = 1.2 and 8.2 Hz), 7.76 (s, 1H), 7.51 (d, 1H, J = 1.2 Hz), 7.49-7.44 (m, 1H), 7.37 (d, 2H, J = 8.8 Hz), 7.34-7.32 (m, 1H), 7.30 (d, 1H, J = 8.8 Hz), 6.51-6.47 (m, 2H), 6.35 (s, 1H), 5.35 (s, 2H), 3.89 (s, 3H), 2.54 (s, 3H). |
| | | Preparation of methyl 3-methoxy-4-[(5-nitroindazol-1-yl)methyl]benzoate |
| | | In like manner to the preparation of methyl 3-methoxy-4-[(6-nitroindazol-1-yl)methyl]benzoate, methyl 3-methoxy-4-[(5-nitroindazol-1-yl)methyl]benzoate was prepared by alkylation of 5-nitroindazoline with methyl (4-bromomethyl)-3-methoxybenzoate in presence of K2CO3. The desired product (47%) with high Rf value on the |
| | | TLC in 30% EtOAc : Hexanes as eluent was collected by silica gel column chromatographic purification. 1H NMR (CDCl3): 0 8.73 (d, 1H, J= 1.8 Hz),8.26-8.22 (m, 2H), 7.56 (s, 1H), 7.54 (dd, 1H, J= 1.8 and 8.2 Hz), 7.49 (d, 1H, J = 9.4 Hz), 6.98 (d, 1H, J = 8.2 Hz), 5.66 (s, 2H), 3.91 (s, 3H), 3.89 (s, 3H). Low Rf : Methyl 3-methoxy-4-[(5-nitroindazol-2-yl)methyl]benzoate. |
| | | Preparation of 5-nitro-1-[2-methoxy-4-(o-toluylsulfonamidocarboxy)benzyl]indazoline |
| | | In like manner to the preparation of 6-nitro-1-[2-methoxy-4-(o-toluylsulfonamidocarboxy)benzyl]indazoline, 5-nitro-1-[2-methoxy-4-(o-toluylsulfonamidocarboxy)benzyl]indazoline was prepared from methyl 3-methoxy-4-[(5-nitroindazol-1-yl)methyl]benzoate. 1H NMR (DMSO-d6): □8.81 (d, 1H, J=2.3 Hz), 8.39 (s, 1H), 8.21 (dd, 1H, J = 1.8 and 8.8 Hz), 7.87 (dd, 2H, J = 3.6 and 8.8 Hz), 7.48 (d, 1H, J = 1.2 Hz), 7.39 (dd, 1H, J = 1.2 and 8.2 Hz), 7.33-7.15 (m, 3H), 6.85 (d, 1H, J = 8.2 Hz), 5.65 (s, 2H), 3.76 (s, 3H), 2.49 (s, 3H). |
| | | Preparation of 5-amino-1-[2-methoxy-4-(0-toluyl-sulfonamidocarboxy)benzyl]indazoline |
| | | In like manner to the preparation of 6-amino-1-[2-methoxy-4-(o-toluylsulfonamidocarboxy)benzyl]indazoline, 5-amino-1-[2-methoxy-4-(o-toluylsulfonamidocarboxy)benzyl]indazoline was prepared by reduction of 5-nitro-1-[2-methoxy-4-(o-toluylsulfonamidocarboxy)benzyl]indazoline . 1H NMR (DMSO-d6): □7.87 (dd, 1H, J = 1.2 and 7.7 Hz), 7.73 (s, 1H), 7.50 (s, 1H), 7.35-7.14 (m, 5H), 6.78 (d, 1H, J = 1.8 Hz), 6.75 (s, 1H), 6.53 (d, 1H, J = 8.2 Hz), 5.44 (s, 2H), 3.82 (s, 3H), 2.50 (s, 3H). |
| 7.2.48 | Preparation of 8-amino-4H-imidazo[2,1-c][1,4]-benzoxazine | 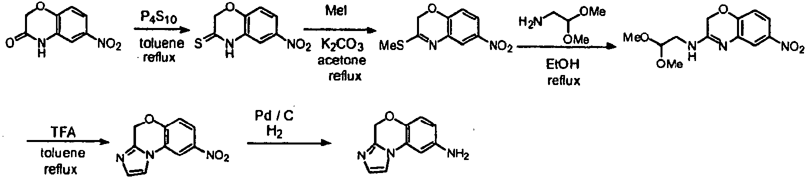 |
| 7.3 | Synthesis of 2,4-Pyrimidinediamines | A variety of 2,4-pyrimidinediamines of the invention were synthesized from the above starting materials and intermediates and other commercially available reagents. Conditions suitable for synthesizing N2,N4-bis-substituted-2,4-pyrimidinediamine compounds ("general SNAr" reaction conditions; Substitution Nucleophilic Aromatic Reaction) are exemplified with N2,N4-bis(4-ethoxyphenyl)-2,4-pyrimdinediamine (R926069) and N2,N4-bis(3-hydroxyphenyl)-5-fluoro-2,4-pyrimidinediamine (R921218). Conditions suitable for synthesizing asymmetric N2,N4-disubstituted-2,4-pyrimdinediamines are exemplified by N4-(3,4-ethylenedioxyphenyl)-5-fluoro-N2-(3-hydroxyphenyl)-2,4-pyrimidinediame (R926210). |
| 7.3.908 | N4-[(2,2-Dimethyl-4H-5-pyrido[1,4]oxazin-3-one)-7-yl)-5-fluoro-N2-[3-(methylaminocarbonylmet hyleneoxy)phenyl]-2,4-pyrimidinediamine (R940345) | In like manner to the preparation of 5-fluoro-N2-[3-(methylaminocarbonylmethyleneoxy)phenyl]-N4-(3-aminocarbonylphenyl)-2,4-pyrimidinediamine, 2-chloro-N4-[(2,2-dimethyl-4H-5-pyrido[1,4)oxazin-3-one)-7-yl)-5-fluoro-4-pyrimidineamine and 3-(methylaminocarbonylmethyleneoxy)aniline were reacted to produce N4-[(2,2-dimethyl-4H-5-pyrido[1,4]oxazin-3-one)-7-yl)-5-fluoro-N2-[3-(methylaminocarbonylmethyleneoxy)phenyl]-2,4-pyrimidinediamine R940345. 1H NMR (DMSO-d6): □11.23 (1H, s), 9.69 (1H, s), 9.54 (1H, s), 8.50 (1H, s), 8.25 (1H, d, J= 3.3 Hz), 8.06 (1H, m), 7.96 (1H, t, J= 2.5 Hz), 7.41-7.36 (2H, m), 7.24 (1H, t, J= 8.25 Hz), 6.34 (1H, d, J= 8.7 Hz), 4.47 (2H, s), 2.74 (3H, d, J= 3.3 Hz), 1.53 (6H, s) ; purity: 98.4% ; MS (m/e): 468 (MH+). |
| 7.3.910 | N4-[(2,2-Dimethyl-4H-5-pyrido[1,4]oxazin-3-one)-6-yl]-5-fluoro-N2-[3-(methylaminocarbonylmet hyleneoxy)phenyl]-2,4-pyrimidinediamine (R940347) | In like manner to the preparation of 5-fluoro-N2-[3-(methylaminocarbonylmethyleneoxy)phenyl)-N4-(3-aminocarbonylphenyl)-2,4-pyrimidinediamine, 2-chloro-N4-[(2,2-dimethyl-4H-5-pyrido[1,4]oxazin-3-one)-6-yl]-5-fluoro-4-pyrimidineamine and 3-(methylaminocarbonylmethyleneoxy)aniline were reacted to produce N4-[(2,2-dimethyl-4H-5-pyrido[1,4]oxazin-3-one)-6-yl]-5-fluoro-N2-[3-(methylaminocarbonylmethyleneoxy)phenyl]-2,4-pyrimidinediamine R940347. 1H NMR (DMSO-d6): □11.20 (1H, s), 9.46 (1H, s), 8.26 (1H, d, J= 3.6 Hz), 8.06 (1H, s), 7.71 (1H, m), 7.49 (1H, d, J= 8.4 Hz), 7.45 (1H, s), 7.38 (1H, d, J= 9 Hz), 7.21 (1H, t, J= 8.1 Hz), 6.61 (1H, d, J= 8.7 Hz), 4.47 (2H, s), 2.74 (3H, s), 1.52 (6H, s); purity: 100% ; MS (m/e): 468 (MH+). |
| 7.3.964 | 5-Fluoro-N2-(3-methylaminocarbonylmet hyleneoxyphenyl)-N4-[2H-pyrido[3,2-b]-1,4-oxazin-3(4H)-one-7-yl]-2,4-pyrimidinediamine (R945242) | 2H-Pyrido[3,2-b]-1,4-oxazin-3(4H)-one (500 mg) was treated with nitric acid (5 mL) and sulfuric acid (5 mL). The reaction mixture was heated to 70 °C for 30 min and then poured into ice-water. The solution was neutralized with sodium bicarbonate to pH 6. The yellow precipitation was collected by filtration, washed with water and dried to give a mixture of nitrated products (regio-isomers). |
| | | The mixture of nitrated compounds was reduced under hydrogenolysis conditions using 10% Pd-C in methanol at 40 psi for 30 min. The catalyst was filtered off. The filtrate was evaporated and treated with 2,4-dichloro-5-fluoropyrimidine (200 mg) in methanol (5 mL), water (5 mL). The reaction mixture was heated at 70°C overnight, then evaporated. The residue was reacted with 3-methylaminocarbonylmethyleneoxyaniline (300 mg) in methanol (5 mL) and water (1 mL) at 100 °C overnight. The reaction mixture was diluted with 1N HCl solution (60 mL). The brown precipitation was collected by filtration, washed with water and dried to give 5-fluoro-N2-(3-methylaminocarbonylmethyleneoxyphenyl)-N4-[2H-pyrido[3,2-b]-1,4-oxazin-3(4H)-one-7-yl]-2,4-pyrimidinediamine. 1H NMR (DMSO-d6): δ 2.6 (d, J= 4.8 Hz, 3H), 4.33 (s, 2H), 4.63 (s, 2H), 6.48 (dd, J= 2.4 and 7.5 Hz, 1H), 7.11 (t, J= 8.1 Hz, 1H), 7.27 (d, J= 7.8 Hz, 1H), 7.36 (s, 1H), 7.86 (d, J= 2.1 Hz, 1H), 7.97 (m, 1H), 8.12 (d, J= 3.6 Hz, 1H), 8.38 (d, J= 2.1 Hz, 1H), 9.33 (s, 1H), 9.46 (s, 1H), 11.18 (s, 1H); 19F NMR (282 MHz, DMSO-d6): δ - 164.49; LCMS: ret. time: 13.16 min.; purity: 79.30%; MS (m/e): 440.16 (MH+). |
| 7.3.965 | 5-Fluoro-N2-(3-methylaminocarbonylmet hyleneoxyphenyl)-N4-[2H-pyrido[3,2-b]-1,4-oxazin-7-yl]-2,4-pyrimidinediamine (R945263) | 2H-Pyrido[3,2-b]-1,4-oxazin-3(4H)-one (1g, 6.66 mmol) was refluxed with boron hydride methyl sulfide complex (2 mL) in THF (10 mL) for 30 min to give 2H-pyrido[3,2-b]-1,4-oxazine. In a manner analogous to the preparation of 5-fluoro-N2-(3-methylaminocarbonylmethyleneoxyphenyl)-N4-[2H-pyrido[3,2-b]-1,4-oxazin-3(4H)-one-7-yl]-2,4-pyrimidinediamine, 2H-pyrido[3,2-b]-1,4-oxazine was nitrated, reduced and reacted with 2,4-dichloro-5-fluoropyrimidine (400 mg) and 3-methylaminocarbonylmethyleneoxyaniline (500 mg) to give 5-fluoro-N2-(3-methylaminocarbonylmethyleneoxyphenyl)-N4-[2H-pyrido[3,2-b ]-1,4-oxazin-7-yl]-2,4-pyrimidinediamine as a gray solid. 1H NMR (CDCl3): δ 2.91 (d, J= 4.8 Hz, 3H), 3.55 (t, J= 4.2 Hz, 2H), 4.24 (t, J= 4.5 Hz, 2H), 4.49 (s, 2H), 4.90 (br, 1H), 6.51 (dd, J= 2.7 and 8.1 Hz, 1H), 6.64 (s, 1H), 6.90 (dd, J= 2.1 and 8.1 Hz, 1H), 7.08 (s, 1H), 7.14 (br, 1H), 7.18 (t, J= 8.1 Hz, 1H), 7.28 (d, J= 2.1 Hz, 1H), 7.51 (t, J= 2.1 Hz, 1H), 7.93 (d, J= 3.0 Hz, 1H), 7.95 (d, J= 2.4 Hz, 1H); LCMS: ret. time: 11.91 min.; purity: 100%; MS (m/e): 426.12 (MH+). |
| 7.3.966 | 5-Fluoro-N2-(3-methylaminocarbonylmet hyleneoxyphenyl)-N4-[2H-pyrido[3,2-b]-1,4-oxazin-3(4H)-one-6-yl]-2,4-pyrimidinediamine (R921304) | 2H-Pyrido[3,2-b]-1,4-oxazin-3(4H)-one (2.5 g) was dissolved in acetic acid (6 mL) and acetic anhydride (30 mL). Fuming nitric acid (3 mL) was added dropwise to the reaction solution in ice-bath. The reaction solution was stirred in ice-bath overnight. Solution was poured into crashed ice. The light yellow precipitation was collected by filtration, washed with water and dried to give a mixture of nitrated products (regio-isomers). The mixture was crystallized from dichloromethane to give 6-nitro-2H-pyrido[3,2-b]-1,4-oxazin-3(4H)-one (1 g) as a light yellow solid. |
| | | 6-Nitro-2H-pyrido[3,2-b]-1,4-oxazin-3(4H)-one (1g) was reduced under hydrogenolysis conditions using 10% Pd-C in methanol (50 mL) and 1N HCl solution (10 mL) at 50 psi for 2 h. The catalyst was filtered off and washed with methanol and 1N HCl solution. The filtrate was evaporated to give 6-amino-2H-pyrido[3,2-b]-1,4-oxazin-3(4H)-one. |
| | | In a manner analogous to the preparation of 5-fluoro-N2-(3-methylaminocarbonylmethyleneoxyphenyl)-N4-[2H-pyrido[3,2-b]-1,4-oxazin-3(4H)-one-7-yl]-2,4-pyrimidinediamine, 6-amino-2H-pyrido[3,2-b]-1,4-oxazin-3(4H)-one was reacted with 2,4-dichloro-5-fluoropyrimidine (500 mg) and 3-methylaminocarbonylmethyleneoxyaniline (500 mg) to give 5-fluoro-N2-(3-methylaminocarbonylmethyleneoxyphenyl)-N4-[2H-pyrido[3,2-b]-1,4-oxazin-3(4H)-one-6-yl]-2,4-pyrimidinediamine as a beige solid. 1H NMR (DMSO-d6): δ 2.63 (d, J= 4.5 Hz, 3H), 4.35 (s, 2H), 4.62 (s, 2H), 6.47 (dd, J= 1.8 and 8.1 Hz, 1H), 7.10 (t, J= 8.1 Hz, 1H), 7.25 (d, J= 8.1 Hz, 1H), 7.37 (m, 2H), 7.59 (d, J= 8.4 Hz, 1H), 7.96 (d, J= 5.1 Hz, 1H), 8.13 (d, J= 3.6 Hz, 1H), 9.26 (s, 1H), 9.29 (s, 1H), 11.13 (s, 1H); 19F NMR (282 MHz, DMSO-d6): 8 - 163.20; LCMS: ret. time: 25.22 min.; purity: 97.55%; MS (m/e): 440.25 (MH+). |
| 7.3.967 | 5-Fluoro-N2-(3-methylaminocarbonylmet hyleneoxyphenyl)-N4-[2H-pyrido[3,2-b]-1,4-oxazin-6-yl]-2,4-pyrimidinediamine (R945299) | 6-Nitro-2H-pyrido[3,2-b]-1,4-oxazin-3(4H)-one (500 mg) was refluxed with boron hydride methyl sulfide complex (1 mL) in THF (10 mL) for 30 min to give 6-nitro-2H-pyrido[3,2-b]-1,4-oxazine. In a manner analogous to the preparation of 5-fluoro-N2-(3-methylaminocarbonylmethyleneoxyphenyl)-N4-[2H-pyrido[3,2-b]-1,4-oxazin-3(4H)-one-7-yl]-2,4-pyrimidinediamine, 6-nitro-2H-pyrido[3,2-b]-1,4-oxazine was reduced and reacted with 2,4-dichloro-5-fluoropyrimidine (500 mg) and 3-methylaminocarbonylmethyleneoxyaniline (500 mg) to give 5-fluoro-N2-(3-methylaminocarbonylmethyleneoxyphenyl)-N4-[2H-pyrido[3,2-b]-1,4-oxazin-6-yl]-2,4-pyrimidinediamine as a gray solid. 1H NMR (CD3OD): δ 2.81 (s, 3H), 3.48 (t, J= 4.5 Hz, 2H), 4.14 (t, J= 4.5 Hz, 2H), 4.44 (s, 2H), 6.60 (ddd, J= 1.5 and 2.7 and 7.5 Hz, 1H), 6.94 (d, J= 8.1 Hz, 1H), 7.14 (d, J= 3.0 Hz, 1H), 7.17 (t, J= 7.8 Hz, 1H), 7.40 (d, J= 8.9 Hz, 1H), 7.42 (t, J= 2.1 Hz, 1H), 7.92 (d, J= 3.3 Hz, 1H); 19F NMR (282 MHz, CD3OD): δ - 168.20; LCMS: ret. time: 25.49 min.; purity: 97.56%; MS (m/e): 426.23 (MH+). |
| 7.3.1100 | Synthesis of Intermediates, 2,4-Pyrimidinediamines and 2,4,6-Pyrimidinetriamines According to Schemes VIII and IX | A variety of intermediates and 2,4-pyrimidinediamine compounds were synthesized according to Schemes VIII and IX. Scheme VIII is exemplified by the reaction of 2,4,6-trichloropyrimidine with 3-hydroxyaniline to form a mixture of three compounds, which were separated and purified by chromotography. Scheme IX is exemplified by the reaction of 2,4,5,6-tetrachloridepyrimidine with 3,4-ethylenedioxyaniline to form a mixture of three compounds, which were separated and purified by chromotography. |
| 7.3.1101 | Reaction of 2,4,6-trichloropyrimidine with 3-hydroxyaniline 4,6-Dichloro-N2-(3-hydroxyphenyl)-2-pyrimidineamine (R926407) N2,N4-Bis(3-hydroxyphenyl)-6-chloro-2,4-pyrimidinediamine (R926408) and N2,N4,N6-Tris(3-hydroxyphenyl)-2,4,6-pyrimidinetriamine (R926409) | A mixture of 2,4,6-trichloroaniline (0.183g, 1 mmol) and 3-hydroxyaniline (0.327g, 3 mmol) in 5 mL MeOH was heated at 100 oC in a sealed vial for 24h. The reaction mixture was diluted with H2O, acidified with 2N HCl and extracted with EtOAc (3 x 50 mL). Upon removal of solvent the residue was purified by chromatography (as well as preparative TLC) to afford three products, mainly the mono-SNAr, 4,6-dichloro-N2-(3-hydroxyphenyl)-2-pyrimidineamine (R926407),: 1H NMR (CDCl3): □7.16 (t, 1H, J= 8.1 Hz), 6.78 (m, 2H), 6.64 (dd, 1H, J= 1.2 and 8.1 Hz), 6.58 (s, 1H); LCMS: ret. time: 25.08 min.; purity: 99%; MS (m/e): 256 (M+); bis-SNAr product, N2,N4-bis(3-hydroxyphenyl)-6-chloro-2,4-pyrimidinediamine (R926408), 1H NMR (CD3OD): □ 7.21 (m, 1H), 7.14-7.03 (m, 5H), 6.50 (m, 1H), 6.44 (m, 1H), 6.16 (s, 1H); LCMS: ret. time: 25.14 min.; purity: 99%; MS (m/e): 329 (M+); and tris-SNAr product, N2,N4,N6-tris(3-hydroxyphenyl)-2,4,6-pyrimidinetriamine (R926409), 1H NMR (CD3OD): □ 7.29 (m, 1H), 7.12-7.05 (m, 5H), 7.02 (m, 2H), 6.88 (dd, 2H, J= 1.2 and 8.1 Hz), 6.46 (dd, 1H, J= 1.5 and 8.1 Hz), 6.41 (dt, 1H); LCMS: ret. time: 20.49 min.; purity: 94%; MS (m/e): 402 (MH+). |
| 7.3.1102 | N2,N4-Bis(4-methoxycarbonylmethyle neoxyphenyl)-6-chloro-2,4-pyrimidinediamine (R926411) | In like manner to the reaction of 2,4,6-trichloropyrimidine with 3-hydroxyaniline, the reaction of 2,4,6-trichloropyrimidine with methyl 4-aminophenoxyacetate gave N2,N4-bis(4-methoxycarbonylmethyleneoxyphenyl)-6-chloro-2,4-pyrimidinediamine. 1H NMR (CD3OD): □ 7.65 (bs, 1H), 7.40 (bd, 4H), 6.82 (bd, 4H), 6.00 (s, 1H), 6.62 (bs, 4H), 3.78 (bs, 6H); LCMS: ret. time: 29.87 min.; purity: 98%; MS (m/e): 473 (MH+). |
| 7.3.1103 | Reaction of 2,4,6-trichloropyrimidine with 3,4-ethylenedioxyaniline 4,6-Dichloro-N2-(3,4-ethylenedioxyphenyl)-2-pyrimidineamine (R926515) N2,N4-Bis(3,4-ethylenedioxyphenyl)-6-chloro-2,4-pyrimidinediamine (R926245) N2,N4,N6 -Tris(3,4-ethylenedioxyphenyl)-2,4,6-pyrimidinetriamine (R926516) | A mixture of 2,4,6-trichloroaniline (1 mmol) and 3,4-ethylenedioxyaniline (3 mmol) in 5 mL MeOH was heated at 100 oC in a sealed vial for 24h. The reaction mixture was diluted with H2O, acidified with 2N HCl and extracted with EtOAc (3 x 50 mL). Upon removal of solvent the residue was purified by chromatography (as well as preparative TLC) to afford three products, mainly the Mono-SNAr product, 4,6-dichloro-N2-(3,4-ethylenedioxyphenyl)-2-pyrimidineamine (R926515). 1H NMR (CD3OD): □ 7.05 (s, 1H), 6.83 (m, 2H), 6.45 (bs, 1H), 4.20 (bs, 4H); LCMS: ret. time: 29.75 min.; purity: 96%; MS (m/e): 298 (M+); Bis-SNAr product, N2,N4-bis(3,4-ethylenedioxyphenyl)-6-chloro-2,4-pyrimidinediamine (R926245): 1H NMR (CDCl3): □7.23 (d, 1H, J= 3 Hz), 6.90-6.70 (m, 6H), 6.02 (s, 1H), 4.26 (bs, 4H), 4.23 (m, 4H); LCMS: ret. time: 31.34 min.; purity: 95%; MS (m/e): 413 (MH+) and Tris-SNAr product, N2,N4,N6 -tris(3,4-ethylenedioxyphenyl)-2,4,6-pyrimidinetriamine (R926516) 1H NMR (CD3OD): □ 7.16 (d, 1H, J= 3Hz), 7.05 (bd, 1H), 6.99-6.90 (m, 3H), 6.80-6.70 (m, 4H), 6.03 (s, 1H), 4.22 (s, 4H), 4.20 (s, 8H); LCMS: ret. time: 27.72 min.; purity: 61%; MS (m/e): 528 (M+). |
| 7.3.1104 | Reaction of 2,4,6-trichloropyrimidine with ethyl-4-aminophenoxyacetate 4,6-Dichloro-N2-(4-ethoxycarbonylmethyl)-4,6-dichloro-2-pyrimidineamine (R926549) 2,6-Dichloro-N4-(ethoxycarbonylmethyl)-4-pyrimidineamine (R926550) | A mixture of 2,4,6-trichloroaniline (1 mmol) and ethyl 2-aminoacetate (3 mmol) in 5 mL MeOH was heated at 100 oC in a sealed vial for 24h. The reaction mixture was diluted with H2O, acidified with 2N HCl and extracted with EtOAc (3 x 50 mL). Upon removal of solvent the residue was purified by chromatography (as well as preparative TLC) to afford three products, mainly the mono-SNAr product, 4,6-dichloro-N2-(4-ethoxycarbonylmethyl)-4,6-dichloro-2-pyrimidineamine (R926549). 1H NMR (CDCl3): □ 6.67 (s, 1H), 5.85 (bs, 1H), 4.23 (q, 2H, J= 7.2 Hz), 4.19 (s, 2H), 1.29 (t, 3H, J= 7.2 Hz); LCMS: ret. time: 26.18 min.; purity: 100%; MS (m/e): 250 (MH+); and Mono-SNAr product, 2,6-dichloro-N4-(ethoxycarbonylmethyl)-4-pyrimidineamine (R926550): 1H NMR (CDCl3): d 6.37 (bs, 1H), 4.28 (q, 2H, J= 6.9 Hz), 4.19 (bs, 2H), 1.31 (t, 3H, J= 7.2 Hz) |
| 7.3.1105 | 6-Chloro-N2-(4-ethoxycarbonylmethylene oxyphenyl)-N4-(methoxycarbonylmethyl) -2,4-pyrimidinediamine (R926555) | In like manner to the preparation of 4-[N-(L)-phenylalanine ethyl ester]-N2-(3-hydroxyphenyl)-5-ethoxycarbonyl-2-pyrimidineamine, the reaction of ethyl 4-aminophenoxyacetate with methyl 2-aminoacetate gave 6-chloro-N2-(4-ethoxycarbonylmethyleneoxyphenyl)-N4-(methoxycarbonylmethyl)-2,4-pyrimidinediaminc. 1H NMR (CD3OD): □ 7.40 (d, 2H, J= 8.7 Hz), 6.86 (d, 2H, J= 9.3 Hz), 5.97 (s, 1H), 4.64 (s, 2H), 4.26 (q, 2H, J= 7.2 Hz), 4.14 (q, 2H, J= 6.9 Hz), 4.05 9s, 2H), 1.25 (m, 6H); LCMS: ret. time: 26.21 min.; purity: 93%; MS (m/e): 409 (MH+). |
| 7.3.1106 | Reaction of 3,4-ethylenedioxyaniline with 2,4,5,6-tetrachloropyrimidine. N4-(3,4-Ethylenedioxyphenyl)-2,5,6-trichloro-4-pyrimidineamine (R926466) N2,N4-Bis(3,4-ethylenedioxyphenyl)-5,6-dichloro-2,4-pyrimidinediamine (R926467)and N4,N6-Bis(3,4-ethylenedioxyphenyl)-2,5-dichloro-4,6-pyrimidinediamine (R926468) | A mixture of 3,4-ethylenedioxyaniline (0.775 g, 5 mmol) and 2,4,5,6-tetrachloropyrimidine (0.434 g, 2 mmol) in the presence of DIPEA (1.043 mL, 6 mmol) in EtOAc (10 mL) was heated at 80 oC for 3 days. The reaction was diluted with water (50 mL), acidified (2N HCl) and extracted with EtOAc (3 x 50 mL). The residue obtained after removal of solvent was chromatographed using 5-30% EtOAc/hexanes to obtain three products viz. N4-(3,4-Ethylenedioxyphenyl)-2,5,6-trichloro-4-pyrimidineamine (R926466): 1H NMR (CDCl3): □ 7.18 (d, 1H, J= 2.7 Hz), 6.92 (dd, 1H, J= 2.1 and 8.7 Hz), 6.87 (d, 1H, J= 9 Hz); LCMS: ret. time: 33.53 min.; purity: 100%; MS(m/e): 292 (MH+); N2,N4-Bis(3,4-ethylenedioxyphenyl)-5,6-dichloro-2,4-pyrimidinediamine (R926467): 1H NMR (CDCl3): □ 7.11 (d, 1H, J= 2.4 Hz), 7.06 (d, 1H, J= 2.1 Hz), 7.04 (s, 1H0, 6.94 (m, 2H), 6.84 (d, 1H, J= 8.1 Hz), 6.76 (bd, 2H, J= 8.7 Hz), 4.27 (bs, 4H), 4.24 (bs, 1H); LCMS: ret. time: 26.54 min.; purity: 87%; MS(m/e): 364 (MH+); and N4,N6-Bis(3,4-ethylenedioxyphenyl)-2,5-dichloro-4,6-pyrimidinediamine (R926468): 1H NMR (CDCl3): □ 7.07 (t, 1H, J= 2.4 Hz), 6.99 (s, 2H), 6.83 (dd, 2H, J= 2.4 and 8.7 Hz), 6.75 (dd, 2H, J= 1.8 and 9 Hz), 4.19 (bs, 4H); LCMS: ret. time: 34.70 min.; purity: 99%; MS(m/e): 365 (MH+). |
| 7.3.1107 | Reaction of 2,4,5,6-tetrachloropyrimidine with ethyl-4-aminophenoxyacetate N4-(4-Ethoxycarbonylmethylene oxyphenyl)-2,5,6-trichloro-4-pyrimidineamine (R926568) N2,N4-Bis(4-ethoxycarbonylmethylene oxyphenyl)-5,6-dichloro-2,4-pyrimidinediamine (R926569) N2,N5-Bis(4-ethoxycarbonylmethylene oxyphenyl)-2,5-pyrimidinediamine (R926570) | In like manner to the reaction of 2,4,6-trichloropyrimidine with 3,4-ethylenedioxyaniline, the reaction of 2,4,5,6-tetrachloropyrimidine with ethyl 4-aminophenoxyacetate gave a mixture of mono-SNAr product, N4-(4-ethoxycarbonyl methyleneoxyphenyl)-2,5,6-trichloro-4-pyrimidineamine (R926568): 1H NMR (CDCl3): □ 7.46 (dd, 2H, J= 2.4 and 6.9 Hz), 7.3 (s, 1H), 6.95 (dd, 2H, J= 2.4 and 6.9 Hz), 4.63 (s, 2H), 4.28 (q, 2H, J= 7.2 Hz), 1.30 (t, 3H, J= 7.2 Hz); LCMS: ret. time: 30.62 min.; purity: 99%; MS (m/e): 378 (MH+); Bis-SNAr product, N2,N4-bis((4-ethoxycarbonylmethylene oxyphenyl)-5,6-dichloro-2,4-pyrimidinediamine (R926569): 1H NMR (CDCl3): □ 7.42 (d, 2H, J= 9 Hz), 7.35 (d, 2H, J= 8.7 Hz), 6.90 (d, 2H, J= 9Hz), 6.83 (d, 2H, J= 8.7 Hz), 4.67 (s, 2H), 4.60 (s, 2H), 4.28 (2q, 4H, J= 4.8 Hz), 1.31 (2t, 6H, J= 6.3 Hz); LCMS: ret. time: 33.09 min.; purity: 85%; MS (m/e): 537 (MH+) and Bis-SNAr product, N2,N5-bis((4-ethoxycarbonylmethyleneoxyphenyl)-2,5-pyrinidinediamine (R926570): 1H NMR (CDCl3): □ 7.45 (d, 4H, J= 8.7 Hz), 6.92 (d, 4H, J= 9Hz), 6.85 (s, 1H), 4.61 (s, 4H), 4.26 (q, 4H, J= 6.9 Hz), 1.30 (t, 6H, J= 7.2 Hz); LCMS: ret. time: 31.66 min.; purity: 97%; MS (m/e): 535 (MH+). |
| 7.3.1108 | Reaction of 2,4,5,6-tetrachloropyrimidine with tert-Butyl-4-aminophenoxyacetate, N4-(4-tert-Butoxyoxycarbonylmethy leneoxyphenyl)-2,5,6-trichloro-4-pyrimidineamine (R926575), N2,N4-Bis(4-tert-butoxyoxycarbonylmethyl eneoxyphenyl)-5,6-dichloro-2,4-pyrimidinediamine (R926576) and N4,N6-Bis(4-tert-butoxyoxycarbonylmethyl eneoxyphenyl)-2,5-dichloro-4,6-pyrimidinediamine (R926577) | In like manner to the reaction of 2,4,6-trichloropyrimidine with 3,4-ethylenedioxyaniline, the reaction of 2,4,5,6-tetrachloropyrimidine with tert-butyl-4-aminophenoxyacetate gave a mixture of mono-SNAr product, N4-(4-tert-butoxyoxycarbonyl methyleneoxyphenyl)-2,5,6-trichloro-4-pyrimidineamine (R926575): 1H NMR (CDCl3): □ 7.45 (dd, 2H, J= 2.4 and 7.2 Hz), 6.93 (dd, 2H, J= 2.4 and 7.2 Hz), 4.52 (s, 2H); LCMS: ret. time: 32.56 min.; purity: 100%; MS (m/e): 402 (MH+); Bis-SNAr product, N2,N4-bis(4-tert-butoxyoxycarbonylmethyleneoxyphenyl)-5,6-dichloro-2,4-pyrimidinediamine (R926576): 1H NMR (CDCl3): □ 7.42 (d, 2H, J= 9 Hz), 7.35 (d, 2H, 9.3 Hz), 7.08 (s, 1H), 6.90 (d, 2H, J= 9.3 Hz), 6.82 (d, 2H, J= 8.7 Hz), 4.53 (s, 2H), 4.49 (s, 2H), 1.50 (s, 9H), 1.49 (s, 9H); LCMS: ret. time: 36.04 min.; purity: 92%; MS (m/e): 591 (MH+) and Bis-SNAr product, N4,N6-bis(4-tert-butoxyoxycarbonylmethyleneoxyphenyl)-2,5-dichloro-4,6-pyrimidinediamine (R926577): 1H NMR (CDCl3): □ 7.43 (d, 4H, J= 8.7 Hz), 6.90 (dd, 4H, J= 9.3 Hz), 4.50 (s, 2H), 1.49 (s, 18H); LCMS: ret. time: 35.31 min.; purity: 100%; MS (m/e): 591 (MH+). |
| 7.3.1109 | Reaction of 2,4,5,6-tetrachloropyrimidine with 3-hydroxyaniline, N4-(3-Hydroxyphenyl)-2,5,6-trichloro-4-pyrimidineamine (R926590), N2,N4-Bis(3-hydroxyphenyl)-5,6-dichloro-2,4-pyrimidinediamine (R926591) and N4,N6-Bis(3 -hydroxyphenyl)-2,5-dichloro-4,6-pyrimidinediamine (R926592) | In like manner to the reaction of 2,4,6-trichloropyrimidine with 3,4-ethylenedioxyaniline, the reaction of 2,4,5,6-tetrachloropyrimidine with tert-butyl-4-aminophenoxyacetate gave a mixture of mono-SNAr product, N4-(3-hydroxyphenyl)-2,5,6-trichloro-4-pyrimidineamine (R926590): 1H NMR (CDCl3): □ 7.38 (bs, 1H), 7.32 (t, 1H, J= 2.4 Hz), 7.22 (s, 1H), 7.01 (dd, 1H, J= 1.2 and 8.1 Hz), 6.68 (dd, 1H, J= 1.8 and 8.1 Hz); LCMS: ret. time: 26.09 min.; purity: 99%; MS (m/e): 292 (MH+); Bis-SNAr product, N2,N4-bis(3-hydroxyphenyl)-5,6-dichloro-2,4-pyrimidinediamine (R926591): 1H NMR (CDCl3): □7.45 (s, 1H), 7.30 (t, 1H, J= 2.4 Hz), 7.18 (t, 1H, J= 2.4 Hz), 7.07 (t, 1H, j= 6.6 Hz), 6.98 (t, 1H, J= 8.1 Hz), 6.75 (m, 2H), 6.54 (dd, 1H, J= 2.4 and 8.1 Hz); LCMS: ret. time: 26.54 min.; purity: 87%; MS (m/e): 364 (MH+); and Bis-SNAr product, N4,N6-bis(3-hydroxyphenyl)-2,5-dichloro-4,6-pyrimidinediamine (R926592): 1H NMR (CDCl3): □7.34 (t, 2H, j= 2.4 Hz), 7.21 (t, 2H, J= 7.5 Hz), 6.98 (m, 4H), 6.60 (m, 2H); LCMS: ret. time: 25.38 min.; purity: 73%; MS (m/e): 364 (MH+). |
| | Synthesis of Anilines | |
| 7.4.6 | 3-Chloro-4-(methoxycarbonylmethyle neoxy)nitrobenzene | A dry reaction flask equipped with a magnetic stirring bar, reflux condenser and N2 inlet was charged with a commercially available 2-chloro-4-nitrophenol (3.48 g, 20 mmol), K2CO3 (3.03 g, 21.81 mmol) and dry acetone (100 mL) under N2 atmosphere. To this was added methyl bromoacetate (1.72 mL, 18.18 mmol) and refluxed for 6 hours. Upon cooling, the reaction mixture was diluted with ice-water (1 liter), solid obtained was filtered, washed with water (2 x 50 mL), and dried to give 3-chloro-4-(methoxycarbonylmethyleneoxy)nitrobenzene. 1H NMR (CDCl3): □ 8.33 (d, 1H, J= 3 Hz), 8.13 (dd, 1H, J= 2.7 and 9.3 Hz), 6.87 (d, 1H, J= 9.3 Hz), 4.84 (s, 2H), 3.83 (s, 3H); LCMS: purity: 87%; MS (m/e): 287 (M+ acetonitrile). |
| 7.4.7 | 3-Chloro-4-(methoxycarbonylmethyle neoxy)aniline | To a solution of3-chloro-4-(methoxycarbonylmethyleneoxy)nitrobenzene (1.00 g) in MeOH (50 mL) was added 0.050 g of 10% Pd/C, degassed and hydrogenated with a balloon filled with hydrogen (ca. 1 atmosphere) for 2 hours. The reaction mixture was filtered through a pad of celite, concentrated and the resulting residue was then sonicated with ethyl acetate and filtered. The filtrate upon concentration and drying under a high vacuum gave the 3-chloro-4-(methoxycarbonylmethyleneoxy)aniline. 1H NMR (CDCl3): □ 6.79 (d, 1H, J= 9 Hz), 6.73 (d, 1H, J= 2.1 Hz), 6.50 (dd, 1H, J= 2.7 and 9.3 Hz), 4.60 (s, 2H), 3.80 (s, 3H); LCMS: purity: 87%; MS (m/e): 216 (MH+). |
| 7.4.8 | 3-Chloro-4-(2-hydroxyethyleneoxy)nitro benzene | A dry reaction flask equipped with a magnetic stirring bar, N2 inlet and a rubber septum was charged with 3-chloro-4-(methoxycarbonylmethyleneoxy)nitrobenzene (1.23 g, 5 mmol) and CH2Cl2 (50 mL) under N2 atmosphere. The reaction solution was cooled to -78 oC and to it was added diisobutyllithiumaluminum hydride diisobutyl lithiumaluminum hydride (1.0 M in toluene, 15 mL, 15 mmol) over a period of 15 minutes. The reaction mixture was stirred at -78 oC for 2 hours and at room temperature for 1 hour, quenched with saturated solution of Rochelle's salt and again stirred for 2 hours. Upon extraction with CH2Cl2, drying over anhydrous Na2SO4 and evaporation of solvent gave 3-chloro-4-(2-hydroxyethyleneoxy)nitrobenzene. 1H NMR (CDCl3): □ 8.30 (d, 1H, J= 3 Hz). 8.15 (dd, 1H, J= 2.4 and 9 Hz), 7.02 (d, 1H, J= 8.7 Hz), 4.25 (t, 2H, J= 4.8 Hz), 4.07 (m, 2H); LCMS: purity: 92%. |
| 7.4.9 | 3-Chloro-4-(2-hydroxyethyleneoxy)anili ne | In like manner to the preparation of 3-chloro-4-(methoxycarbonylmethyleneoxy)aniline, the hydrogenation of 3-chloro-4(2-hydroxyethyleneoxy)nitrobenzene with balloon filled with hydrogen (ca. 1 atmosphere) in the presence of 10% Pd/C as a catalyst gave 3-chloro-4-[2-hydroxyethyleneoxy)aniline. LCMS: MS (m/e): 187 (M+) |
| 7.4.10 | 2-(N-Methylaminocarbonyl)-5-nitrobenzofuran | A dry reaction flask equipped with a magnetic stirring bar, a rubber septum and N2 inlet was charged with 2-carboxy-5-nitrobenzofuran (2.07 g, 10 mmol), N,N-dimethylformamide (DMF) (0.100 mL) and CH2Cl2 (50 mL) under N2 atmosphere. The reaction mixture was cooled to 0 oC and to it was added oxalyl chloride [(COCI)2] (2.65 mL, 30 mmol) over a period of 10 minutes. The resulting mixture was stirred for 2 hours by the time the 0 oC became room temperature and also the reaction became as a clear solution. It was concentrated and dried under high vacuum to yield the intermediate acid chloride. The resulting acid chloride was cooled to 0 oC and to it were added CH2Cl2 (50 mL), pyridine (2.96 mL, 30 mmol) followed by methylamine hydrogen chloride salt (1.34 g, 20 mmol). Upon stirring for 24 hours at room temperature, the solvent was removed under a reduced pressure and residue was suspended in water (200 mL). The solid formed was filtered, washed well with water and dried to give 2-(N-methylaminocarbonyl)-5-nitrobenzofuran. 1H NMR (CDCl3): □8.63 (d, 1H, J= 2.4 Hz), 8.33 (dd, 1H, J= 2.4 and 9.3 Hz), 7.60 (d, 1H, J= 7.8 Hz), 7.59 (s, 1H), 3.07 (d, 3H, J= 4.8 Hz); LCMS: purity: 98%; MS (m/e): 221 (MH+). |
| 7.4.11 | (+)-5-Amino-[2-(N-methylaminocarbonyl)-2,3-dihydro]benzofuran | A suspension of 2-(N-methylaminocarbonyl)-5-nitrobenzofuran (1.5 g), 10% Pd/C (1.5 g), Na2SO4 (1.5 g) in MeOH (200 mL) was hydrogenated at 55 PSI for 3 days. The resulting solution was filtered through a pad of celite, concentrated to give (+)-5-amino-[2-(N-methylaminocarbonyl)-2,3-dihydro]benzofuran. 1H NMR (CDCl3): □ 6.65 (m, 2H), 6.53 (m, 1H), 5.01 (dd, 1H, J= 6.0 and 6.6 Hz), 3.46 (dd, 1H, J= 9.9 and 10.2 Hz), 3.18 (dd, 1H, J= 6.0 and 4.2 Hz), 2.75 (d, 3H). |
| 7.4.12 | 2-(N,N-Dimethylaminocarbonyl)-5-nitrobenzofuran | In like manner to the preparation of 2-(N-methylaminocarbonyl)-5-nitrobenzofuran, the reaction of 2-carboxy-5-nitrobenzofuran with oxalyl chloride followed by dimethylamine hydrogen chloride salt afforded 2-(N,N-dimethylaminocarbonyl)-5-nitrobenzofuran. 1H NMR (CDCl3): □ 8.61 (d, 1H, J= 2.4 Hz), 8.31 (dd, 1H, J= 2.4 and 9.3 Hz), 7.63 (d, 1H, J= 9.3 Hz), 7.40 9s, 1H), 3.35 (s, 3H), 3.17 (s, 3H); LCMS: purity: 97%; MS (m/e): 235 (MH+). |
| 7.4.13 | (+)-5-Amino-[2-(N,N-dimethylaminocarbonyl)-2,3-dihydro]benzofuran | In like manner to the preparation of (+)-5-amino-[2-(N-methylaminocarbonyl)-2,3-dihydro]benzofuran, the hydrogenation of 2-(N,N-dimethylaminocarbonyl)-5-nitrobenzofuran yielded (+)-5-amino-[2-(N,N-dimethylaminocarbonyl)-2,3-dihydro]benzofuran. 1H NMR (DMSO-d6): □ 6.44 (m, 2H), 6.27 (dd, 1H, J= 2.1 and 8.7 Hz), 5.42 (dd, 1H, J= 6.5 and 7.5 Hz), 4.54 (bd, J= 5.4 Hz), 3.23 (m, 2H), 2.83 (s, 3H), 2.82 (s, 3H); LCMS: purity: 70%; MS (m/e): 207 (MH+), |
| 7.4.14 | 2-[(1R, 2S, 5R)-Menthyloxycarbonyl)-5-nitrobenzofuran | In like manner to the preparation of 2-(N-methylaminocarbonyl)-5-nitrobenzofuran, the reaction of 2-carboxy-5-nitrobenzofuran with oxalyl chloride followed by treatment with (1R, 2S, 5R)-(-)-menthol afforded 2-[(1R, 2S, 5R)-menthyloxycarbonyl)-5-nitrobenzofuran. 1H NMR (CDCl3): □ 8.63 (d, 1H, J= 2.4 Hz), 8.35 (dd, J= 2.4 and 8.7 Hz), 7.69 (d, 1H, J= 9.3 Hz), 7.62 (s, 1H), 5.00 (dt, 1H, J= 4.8 and 10.5 Hz), 2.14 (bd, 1H, J= 9.3 Hz), 1.95 (m, 1H), 1.76 (m, 2H), 1.56 (m, 3H), 1.11 (m, 2H), 0.94 (d, 3H), 0.93 (d, 3H), 0.82 (d, 3H, J= 7.2 Hz); LCMS: purity: 99.67%. |
| 7.4.15 | 5-Amino-[2(R)-(1R, 2S, 5R)-menthyloxycarbonyl-2,3-dihydro]benzofuran | In like manner to the preparation of (+)-5-amino-[2-(N-methylamino)carbonyl]-2,3-dihydrobenzofuran, the hydrogenation of 2-[(1R, 2S, 5R)-menthyloxycarbonyl)-5-nitrobenzofuran yielded a diastereomeric mixture of 5-amino-[2-(1R, 2S, 5R)-menthyloxycarbonyl-2,3-dihydro]benzofuran, from which the 5-amino-[2(R)-(1R, 2S, 5R)-menthyloxycarbonyl-2,3-dihydro]benzofuran was isolated as a crystalline diastereoisomer using solvent diffusion method* (CH2Cl2:n-n-hexanes) of crystallization. 1H NMR (CDCl3): □ 6.77 (bd, (1H), 6.73 (bs, 1H), 6.68 (dd, 1H, J= 2.4 and 8.7 Hz), 5.11 (dd, 1H, J= 6.9 and 7.5 Hz), 4.76 (dt, 4.5 and 11.1 Hz), 3.49 (dd, 1H, J= 9.9 and 10.5 Hz), 3.25 (dd, 1H, J= 7.2 and 7.8 Hz), 1.99 (bd, 1H), 1.86 (dpent, 1H, J= 3.0 and 6.9 Hz), 1.70 (m, 1H), 1.66 (m, 1H), 1.46 (m, 2H), 1.02 (m, 1H). 0.90 (d, 3H, 7.2 Hz), 0.89 (d, 3H, J= 6.6 Hz), 0.75 (d, 3H, J= 6.9 Hz); MS (m/e): 318 (MH+). |
| | | * Solvent Diffusion Method: The organic molecule was dissolved in a minimum amount of CH2Cl2 and the container was placed in a jar containing anti-solvent (n-hexanes), the lid was placed to avoid a loss of solvent and allowed to equilibrate them till the crysatalliztion was seen. The resulting crystals were isolated by decantation of the solvent. |
| 7.4.16 | 3,5-Dichloro-4-methoxyaniline | To a solution of commercially available 3,5-dichloro-4-methoxynitrobenzene (1.00 g, 4.5 mmol) in MeOH (100 mL) was added 10% Pd/C (0.100 g), degassed and hydrogenated using balloon filled with hydrogen (ca. 1 atmosphere) for 2 hours. Upon filtration through celite and concentration afforded 3,5-dichloro-4-methoxyaniline, which was isolated as 3,5-dichloro-4-methoxyaniline hydrogen chloride salt by acidification with equivalent amount of HCl (4M, dioxane). Alternatively, this transformation was also achieved by stirring 3,5-dichloro-4-methoxynitrobenzene (1.00 g, 4.5 mmol) with Na2S2O4 (3.91 g, 22.5 mmol) and K2CO3 (3.12 g, 22.5 mmol) in MeOH:H2O (50 mL, each) at room temperature for 24 hours. The extraction with ethyl acetate followed by removal of solvent gave 3,5-dichloro-4-methoxyaniline. LCMS: purity: 87%; MS (m/e): 233 (M+ acetonitrile). |
| 7.4.17 | 4-Chloro-3-methoxyaniline | To a solution of commercially available 3,5-dichloro-4-methoxynitrobenzene (1.00 g, 4.5 mmol) in MeOH (100 mL) was added 10% Pd/C (0.100 g), degassed and hydrogenated using balloon filled with hydrogen (ca. 1 atmosphere) for 2 hours. Upon filtration through celite and concentration afforded 3,5-dichloro-4-methoxyaniline, which was isolated as 3,5-dichloro-4-methoxyaniline hydrogen chloride salt by acidification with equivalent amount of HCl (4M, dioxane). Alternatively, this transformation was also achieved by stirring 3,5-dichloro-4-methoxynitrobenzene (1.00 g, 4.5 mmol) with Na2S2O4 (3.91 g, 22.5 mmol) and K2CO3 (3.12 g, 22.5 mmol) in MeOH:H2O (50 mL, each) at room temperature for 24 hours. The extraction with ethyl acetate followed by removal of solvent gave 3,5-dichloro-4-methoxyaniline. LCMS: purity: 87%; MS (m/e): 233 (M+ acetonitrile). |
| 7.4.18 | 4-Chloro-3,5-dimethylaniline | To a suspension of commercially available 4-chloro-3,5-dimethylnitrobenzene (0.185 g, 1 mmol) in EtOH: H2O (5 mL, each) at room temperature was added ammonium chloride (0.265 g, 5 mmol) and iron powder (0.280 g, 5 mmol), stirred for 5 minutes at room temperature followed by 10 minutes at 60 oC. Upon cooling to room temperature, the reaction mixture was filtered through a pad of celite, washed with ethanol and the filtrate was concentrated. The resulting residue was diluted with water, saturated with sodium chloride and extracted with ethyl acetate. The organic solvent was removed under a reduced pressure to afford the desired 4-chloro-3,5-dimethylaniline. 1H NMR (CDCl3): □ 6.34 (s, 2H), 3.42 (bs, 2H), 2.20 (s, 6H); LCMS: purity: 82%; MS: 156 (MH+). |
| 7.4.19 | 3,4,5-Trimethylaniline | In like manner to the hydrogenation of 3-(methoxycarbonylmethyleneoxy)aniline, the hydrogenation of commercially available 3,4,5-trimethylnitrobenzene gave 3,4,5-trimethylaniline. LCMS: purity: 91%; MS (m/e): 136 (MH+). |
| | Synthesis of Anilines and mono SNAr Products | |
| 7.4.91 | 2-Isopropoxy-5-nitropyridine | A solution of 2-bromo-5-nitropyridine (1.0g, 4.9 mmol), potassium t-butoxide (6.9 ml, 6.9 mmol, 1N solution in THF), and isopropyl alcohol (75 mL) was heated at 75°C for 2 days. The reaction mixture was concentrated in vacuo and the residue suspended in water and sonicated at room temperature for several minutes. The product was collected as a tan solid by filtration. 1H NMR (CDCl3): δ 9.06 (d, J= 3.0 Hz, 1H), 8.31 (dd, J= 3.0 and 9.3 Hz, 1H), 6.73 (d, J= 9.3 Hz, 1H), 5.43 (quintet, J= 5.7 Hz, 1H), and 1.37 (d, J= 6.3 Hz, 1H). |
| 7.4.92 | 5-Amino-2-isopropoxypyridine | In like manner to the preparation of ethyl 4-aminophenoxyacetate, hydrogenation of 2-isopropoxy-5-nitropyridine was carried out to prepare 5-amino-2-isopropoxypyridine. 1H NMR (CDCl3): δ 7.65 (d, J= 2.7 Hz, 1H), 7.01 (dd, J= 3.0 and 8.7 Hz, 1H), 6.54 (d, J= 9.0 Hz, 1H), 5.13 (quintet, J= 6.6 Hz, 1H), 3.20 (bs, 2H), and 1.32 (d, J= 6.6 Hz, 6H). |
| 7.4.94 | 3-Chloro-4-(N-morpholino)nitrobenzene | A mixture of 2-chloro-4-fluoronitrobenzene (1.36g, 7.72 mmol) and morpholine (8.0 mL, 90 mmol) was heated at 80°C for 3 hours. The reaction mixture was poured into water (150 mL) and the product collected as a yellow solid after filtration. 1H NMR (CDCl3): δ 8.26 (d, J= 3.0 Hz, 1H), 8.11 (dd, J= 3.0 ad 9.3 Hz, 1H), 7.05 (d, J= 8.7 Hz, 1H), 3.91-3.87 (m, 4H), and 3.23-3.19 (m, 4H). |
| 7.4.95 | 3-Chloro-4-(N-morpholino)aniline | To a solution of 3-chloro-4-(N-morpholino)nitrobenzene (1.0g, 4.1 mmol) in ethanol/water (70 mL, 2:1) was added iron powder (1.4g, 25 mmol) followed by NH4Cl (0.46g, 8.6 mmol). The reaction mixture was stirred at room temperature for 10 minutes and then heated at 70°C for 1.5h. After cooling to room temperature, the reaction mixture was filtered through celite and the filter cake was washed with methanol. Concentration of the filtrate in vacuo gave a white solid, which was dissolved in ethyl acetate and washed with NaHCO3 (aq) and brine. The organic layer was then dried (MgSO4), filtered, and concentrated in vacuo to give the product as a white solid. 1H NMR (CDCl3): δ 6.98-6.91 (m, H), 6.82 (bs, 1H), 6.67-6.61 (m, 1H), 3.90-3.82 (m, 4H), and 3.02-2.90 (m, 4H). |
| 7.4.97 | 3-Chloro-4-isopropoxynitrobenzene | In a like manner to the preparation of 2-isopropoxy-5-nitropyridine, 3-chloro-4-fluoronitrobenzene was reacted with isopropanol and potassium t-butoxide to provide 3-chloro-4-isopropoxynitrobenzene. 1H NMR (CDCl3): δ 8.26 (d, J= 3.0 Hz, 1H), 8.11 (dd, J= 3.0 and 8.7 Hz, 1H), 6.97 (d, J= 8.7 Hz, 1H), 4.71 (quintet, J= 6.0 Hz, 1H), and 1.43 (d, J= 6.0 Hz, 6H). |
| 7.4.98 | 3-Chloro-4-isopropoxyaniline | In a like manner to the preparation of 3-chloro-4-(N-morpholino)aniline, 3-chloro-4-isopropoxynitrobenzene was reduced to provide 3-chloro-4-isopropoxyaniline. 1H NMR (DMSO-d6): δ 6.80 (d, J= 8.7 Hz, 1H), 6.59 (d, J= 2.4 Hz, 1H), 6.43 (dd, J= 3.0, 8.7 Hz, 1H), 4.92 (bs, 2H), 4.24 (quintet, J= 5.7 Hz, 1H), and 1.18 (d, J= 5.7 Hz, 6H). |
| 7.4.100 | 5-Amino-2-(N,N-dimethylaminomethyl)ben zofuran | Borane-methyl sulfide complex (4.0 mL, 43 mmole) was added to a suspension of 2-[(N,N-dimethylamino)carbonyl]-5-nitrobenzofuran (1.0 g, 43 mmole) in anhydrous THF (10 mL). The reaction mixture was heated at reflux for 3h. Upon cooling, the solvent was removed in vacuo to give a gel-like solid. Cold (0 °C) methanol (50 mL) was cautiously added dropwise and the resulting mixture was heated at 80 °C for 30 min providing a clear yellow solution. The solvent was removed in vacuo and the resulting solid was suspended in methanol (50 mL) and HCl (1.5 mL, 4N in dioxane) was added. After heating at 80 °C for 30 min the solvent was removed under reduced pressure to give an amorphous solid. The solid was dissolved in methanol (20 mL) and ammonia (2N in methanol) was added until basic. A precipitate formed after dilution with dichloromethane (50 mL). Filtration and concentration gave crude 2-(N,N-dimethylaminomethyl)-5-nitrobenzofuran as a yellow oil (1.0g) which was used without further purification. To a suspension of crude 2-(N,N-dimethylaminomethyl)-5-nitrobenzofuran (1.0 g) in methanol (degassed, 60 mL) was added Na2S2O4 (0.50 g) and 10% Pd/C (100 mg). The reaction mixture was stirred under an atmosphere of H2 for 10h. Solids were removed by filtration through Celite@ filter aid, and the filter cake was washed several times with methanol. Concentration gave dark yellow oil. The product, 5-amino-2-(N,N-dimethylaminomethyl)benzofuran, was obtained after purification by column chromatography over silica gel (mobile phase: 0% to 5% Methanol (containing 2N NH3)/dichloromethane). 1H NMR (CD3OD): δ 7.24 (d, 1H, J= 8.7 Hz), 6.91 (d, 1H, J= 2.4 Hz), 6.76 (dd, 1H, J= 2.4 and 8.7 Hz), 6.65 (s, 1H), 3.87 (s. 2H), 2.48 (s, 6H). |
| | Bis-SNAr and Subsequent Reactions | |
| | Snthesis of Anilines | |
| 7.4.139 | 2-Chloro-6-methyl-4-nitrophenol | To a suspenssion of commercially available 6-methyl-4-nitrophenol (5g, 32.6 mmol) in water (300 mL) at room temperature was added N-chlorosuccinimide (8.7 g, 32.6 mmol) followed by an aqueous solution of potassium hydroxyde 5N (13 mL, 65.2 mmol). After stirred at room temperature for 2 hours, the resulting reaction was acidified with 2N HCl (pH >2) and extracted with ethyl acetate (3 x 200 mL). The organic phase was separated, washed with brine, dried (Na2SO4), concentrated and the resulting residue was purified by flash chromatography (EtOAc:n-hexanes 15 : 85; v/v) to afford 2-chloro-6-methyl-4-nitrophenol (3.7 g, 60%). 1H NMR (DMSO-d6):□10.84 (1H, s), 8.20 (1H, d, J= 3.3 Hz), 8.13 (1H, dt, J= 2.7 Hz, J= 0.6 Hz), 2.39 (3H, s); LCMS: purity: 96.69%. |
| 7.4.140 | 4-Amino-2-chloro-6-methylphenol | 2-Chloro-6-methyl-4-nitrophenol (2.5 g, 13.32 mmol) was dissolved in glacial AcOH (22 mL), and iron powder (2.23 g, 40 mmol) was added. The mixture was heated at 90 oC with mechanical stirring for 2 hours, then was cooled to room temperature and diluted with EtOAc (200 mL). The mixture was filtered through a pad of Celite. The filtrate was washed with brine, dried (Na2SO4) and concentrated in vacuo. The resulting residue was purified by chromatography on silica gel with CH2Cl2 to give 4-amino-2-chloro-6-methylphenol (1.03 g, 50%). 1H NMR (DMSO-d6): 08.02 (1H, d, J= 0.9 Hz), 6.47 (1H, d, J= 2.1 Hz), 6.38 (1H, d, J= 2.4 Hz), 4.74 (2H, s), 2.16 (3H, s). |
| 7.4.141 | 3-Chloro-4-methoxy-5-methylnitrobenzene | To a solution of 2-chloro-6-methyl-4-nitrophenol (1.2 g, 6.5 mmol) in acetone (10 mL), was added potassium carbonate (1.34 g, 9.75 mmol) followed by dimethyl sulfate (1.33 mL, 7.8 mmol). The mixture was stirred under reflux for 2 hours. Ammonium hydroxyde (1 mL) was added and the mixture was heated under reflux for 30 minutes. The mixture was cooled to room temperature and the solvent was removed in vacuo. The residue was poured into water, saturated with sodium chloride and the resulting solid was filtered to give the desired 3-chloro-4-methoxy-5-methylnitrobenzene (1.1 g, 84%). 1H NMR (DMSO-d6): □8.28 (1H, d, J= 2.7 Hz), 8.24 (1H, dt, J= 2.7 Hz, J= 0.75 Hz), 3.95 (3H, d, J= 0.9 Hz), 2.48 (3H, d, J= 0.9 Hz); LCMS: purity: 98%. |
| 7.4.142 | 3-Chloro-4-methoxy-5-methylaniline | 3-Chloro-4-methoxy-5-methylnitrobenzene (1.1g, 5.4 mmol) was dissolved in glacial AcOH (9 mL), and iron powder (0.917 g, 16.4 mmol) was added. The mixture was heated at 90 oC under a mechanical stirring for 2 hours, then was cooled to room temperature and diluted with EtOAc (200 mL). The mixture was filtered through a pad of Celite. The filtrate was washed with brine, dried (Na2SO4) and concentrated in vacuo. The residue was purified by chromatography on silica gel with CH2Cl2 to give 3-chloro-4-methoxy-5-methylaniline. 1H NMR (DMSO-d6): □6.51 (1H, d, J= 2.7 Hz), 6.41 (1H, dd, J= 1.8 Hz, J= 0.9 Hz), 5.11 (2H, s), 3.68 (3H, d, J= 0.9 Hz), 2.21 (3H, s); LCMS: purity: 95%. |
| 7.4.143 | (+) 2-Ethoxycarbonyl-2-methyl-6-nitro-3-oxo-4H-benz[1,4]oxazine | A mixture of potassium fluoride (KF) (1.8 g, 32.4 mmol), DMF (10 mL), diethyl-2-bromo-2-methylmalonate (3.2 g, 12.9 mmol), and 4-nitro-2-aminophenol (2 g, 12.9 mmol) was stirred for 16 hours, then poured into water, and extracted with EtOAc. The extract was washed with brine, dried, and concentrated to give the desired (+) 2-ethoxycarbonyl-2-methyl-6-nitro-3-oxo-4H-benz[1,4]oxazine, which was recrystallized from EtOH (2.2 g, 62%). 1H NMR (DMSO-d6): □11.47 (1H, s), 7.99 (1H, dd, J= 9 Hz, J= 2.7 Hz), 7.85 (1H, d, J= 2.7 Hz), 7.36 (1H, d, J= 8.7 Hz), 4.23 (2H, q, J= 7 Hz), 1.85 (3H, s), 1.17 (3H, t, J= 7.2 Hz); LCMS: purity: 98 %; MS (m/e): 281 (MH+). |
| 7.4.144 | (+) 6-Amino-2-ethoxycarbonyl-2-methyl-3-oxo-4H-benz[1,4]oxazine | A solution of (+) 2-ethoxycarbonyl-2-methyl-6-nitro-3-oxo-4H-benz[1,4]oxazine (0.5 g, 1.78 mmol) in methanol was hydrogenated at 30 PSI for 1 hour in the presence of 10% Pd/C (0.05 g, 10% by weight). After the filtration through a Celite pad, the solvent was removed under reduced pressure to obtain (+) 6-amino-2-ethoxycarbonyl-2-methyl-3-oxo-4H-benz[1,4]oxazine. 1H NMR (DMSO-d6). □10.73 (1H, s), 6.76 (1H, d, J= 12 Hz), 6.23-6.20 (2H, m), 5.0 (2H, s), 4.16 (2H, q, J= 6.9 Hz), 1.69 (3H, s), 1.15 (3H, t, J= 6.9 Hz); LCMS: purity: 99 %; MS (m/e): 251 (MH+). |
| 7.4.145 | 3,5-Dimethy-4-methoxynitrobenzene | To a solution of 2,6-dimethyl-4-nitrophenol (1 g, 5.9 mmol) in acetone (9 mL), was added potassium carbonate (1.22 g, 8.85 mmol) followed by dimethyl sulfate (0.68 mL, 7.1 mmol). The mixture was stirred under reflux for 2 hours. Ammonium hydroxyde (1 mL) was added and the mixture was heated under reflux for an extra 30 minutes. The mixture was cooled to room temperature and the solvent was removed in vacuo. The residue was poured into water, saturated with sodium chloride and the resulting solid was filtered to give the desired 3,5-dimethyl-4-methoxynitrobenzene. 1H NMR (DMSO-d6): □8.06 (2H, s), 3.84 (3H, s), 2.42 (6H, s); LCMS: purity: 91%. |
| 7.4.146 | 3,5-Dimethyl-4-methoxyaniline | A solution of 3,5-dimethyl-4-methoxynitrobenzene (0.83 g, 4.5 mmol) in methanol was hydrogenated at 30 PSI for 1 hour in the presence of 10% Pd/C (0.1 g, 10% by weight). After the filtration through a Celite pad, the solvent was removed under reduced pressure to obtain 3,5-dimethyl-4-methoxyaniline. 1H NMR (DMSO-d6): □6.28 (2H, s), 4.69 (2H, brads), 3.60 (3H, d, J= 0.9 Hz), 2.16 (6H, s); LCMS: purity: 100 %. |
| 7.4.157 | N4-(2,2-Dimethyl-3-oxo-4H-5-pyrid[1,4]oxazin-6-yl)-N2-(3-chloro-4-methoxyphenyl)-5-fluoro-2,4-pyrimidinediamine R940371 | In like manner to the preparation of N2-(3-chloro-4-hydroxy-5-methylphenyl)-N4-(2,2-dimethyl-3-oxo-4H-benz[1,4]-oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3-chloro-4-methoxyaniline were reacted to yield N4-(2,2-dimethyl-3-oxo-4H-5-pyrid[1,4]oxazin-6-yl)-N2-(3-chloro-4-methoxyphenyl)-5-fluoro-2,4-pyrimidinediamine. 1H NMR (DMSO-d6): □11.20 (1H, s), 9.39 (1H, s), 9.34 (1H, s), 8.22 (1H, d, J= 3.3 Hz), 7.90 (1H, s), 7.62-7.54 (2H, m), 7.47 (1H, d, J= 8.4 Hz), 7.08 (1H, d, J= 9 Hz), 3.87 (3H, s), 1.53 (6H, s); LCMS: purity: 97.92 %; MS (m/e): 445 (MH+). |
| 7.4.158 | N4-(2,2-Dimethyl-3-oxo-4H-5-pyrid[1,4]oxazin-6-yl)-N2-(3,5-dimethoxyphenyl)-5-fluoro-2,4-pyrimidinediamine R940372 | In like manner to the preparation of N2-(3-chloro-4-hydroxy-5-methylphenyl)-N4-(2,2-dimethyl-3-oxo-4H-benz[1,4]-oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3,5-dimethoxyaniline were reacted to yield N4-(2,2-dimethyl-3-oxo-4H-5-pyrid[1,4]oxazin-6-yl)-N2-(3,5-dimethoxyphenyl)-5-fluoro-2,4-pyrimidinediamine. 1H NMR (DMSO-d6): □11.15 (1H, s), 9.34 (1H, s), 9.30 (1H, s), 8.23 (1H, d, J= 3.3 Hz), 7.74 (1H, dd, J= 8.7 Hz, J= 3.9 Hz), 7.43 (1H, d, J= 8.7 Hz), 7.02 (2H, s), 6.16 (1H, s), 3.75 (6H, s), 1.53 (6H, s); LCMS: purity: 100 %; MS (m/e): 441 (MH+). |
| 7.4.159 | N2-(3,4-Dichlorophenyl)-N4-(2,2-dimethyl-3-oxo-4H-5-pyrid[1,4]oxazin-6-yl)-5-fluoro-2,4-pyrimidinediamine R940373 | In like manner to the preparation of N2-(3-chloro-4-hydroxy-5-methylphenyl)-N4-(2,2-dimethyl-3-oxo-4H-benz[1,4]-oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3,4-dichloroaniline were reacted to yield N2-(3,4-dichlorophenyl)-N4-(2,2-dimethyl-3-oxo-4H-5-pyrid[1,4]oxazin-6-yl)-5-fluoro-2,4-pyrimidinediamine. 1H NMR (DMSO-d6): □ 11.20 (1H, s), 9.68 (1H, s), 9.54 (1H, s), 8.27 (1H, d, J= 3.6 Hz), 8.14 (1H, d, J= 2.1 Hz), 7.64 (1H, dd, J= 8.7 Hz, J=2.1 Hz), 7.53-7.47 (3H, m), 1.53 (6H, s); LCMS: purity: 100 %; MS (m/e): 449 (M+). |
| 7.4.160 | N4-[(2,2-Dimethyl-3-oxo-4H-5-pyrid[1,4]oxazin-6-yl)-5-fluoro-N2-(indazolin-6-yl)-2,4-pyrimidinediamine R940380 | In like manner to the preparation of N2-(3-chloro-4-hydroxy-5-methylphenyl)-N4-(2,2-dimethyl-3-oxo-4H-benz[1,4]oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 6-aminoindazole were reacted to yield N4-(2,2-dimethyl-3-oxo-4H-5-pyrid[1,4]oxazin-6-yl)-5-fluoro-N2-(indazolin-6-yl)-2,4-pyrimidinediamine. 1H NMR (DMSO-d6): □11.16 (1H, s), 9.48 (1H, s), 9.28 (1H, s), 8.27 (1H, d, J= 3.3 Hz), 8.17 (1H, s), 7.98 (1H, s), 7.84 (1H, m), 7.65 (1H, d, J= 9 Hz), 7.47 (1H, d, J= 8.7 Hz), 7.36 (1H, d, J= 8.7 Hz), 1.53 (6H, s); LCMS: purity: 100 %; MS (m/e): 421 (MH+). |
| 7.4.161 | N2-(3-tert-Butylphenyl)-N4-(2,2-dimethyl-3-oxo-4H-5-pyrid[1,4]oxazin-6-yl)-5-fluoro-2,4-pyrimidinediamine R940381 | In like manner to the preparation of N2-(3-chloro-4-hydroxy-5-methylphenyl)-N4-(2,2-dimethyl-3-oxo-4H-benz[1,4]oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3-tert-butylaniline were reacted to yield N2-(3-tert-butylphenyl)-N4-(2,2-dimethyl-3-oxo-4H-5-pyrid[1,4]oxazin-6-yl)-5-fluoro-2,4-pyrimidinediamine. 1H NMR (DMSO-d6): □11.16 (1H, s), 9.27 (1H, s), 9.23 (1H, s), 8.22 (1H, d, J= 3.6 Hz), 7.74 (1H, m), 7.70 (1H, d, J= 8.4 Hz), 7.57 (1H, s), 7.44 (1H, d, J= 8.7 Hz), 7.205 (1H, t, J= 7.9 Hz), 7.01 (1H, d, J= 7.8 Hz), 1.53 (6H, s), 1.33 (9H, s); LCMS: purity: 100 %; MS (m/e): 437 (MH+). |
| 7.4.162 | N4-[(2,2-Dimethyl-3-oxo-4H-5-pyrid[1,4]oxazin-6-yl)-5-fluoro-N2-(3-hydroxyphenyl)-2,4-pyrimidinediamine R940382 | In like manner to the preparation of N2-(3-chloro-4-hydroxy-5-methylphenyl)-N4-(2,2-dimethyl-3-oxo-4H-benz[1,4]oxazm-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3-aminophenol were reacted to yield N4-(2,2-dimethyl-3-oxo-4H-5-pyrid[1,4]oxazin-6-yl)-5-fluoro-N2-(3-hydroxyphenyl)-2,4-pyrimidinediamine. 1H NMR (DMSO-d6): □11.16 (1H, s), 9.29 (1H, s), 9.25 (1H, s), 9.23 (1H, s), 8.20 (1H, d, J= 3.6 Hz), 7.74 (1H, d, J= 8.4 Hz), 7.44 (1H, d, J= 8.7 Hz), 7.23 (1H, t, J= 1.25 Hz), 7.16 (1H, d, J= 8.1 Hz), 7.04 (1H, t, J= 7.9 Hz), 6.40 (1H, dd, J= 6.9 Hz, J= 1.2 Hz), 1.53 (6H, s); LCMS: purity: 100 %; MS (m/e): 397 (MH+). |
| 7.4.163 | N4-(2,2-dimethyl-3-oxo-4H-5-pyrid[1,4]oxazin-6-yl)-N2-(3-fluoro-4-methoxyphenyl)-5-fluoro-2,4-pyrimidinediamine R940384 | In like manner to the preparation of N2-(3-chloro-4-hydroxy-5-methylphenyl)-N4-(2,2-dimethyl-3-oxo-4H-benz[1,4]oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3-fluoro-4-methoxyaniline were reacted to yield N4-(2,2-dimethyl-3-oxo-4H-5-pyrid[1,4]oxazin-6-yl)-N2-(3-fluoro-4-methoxyphenyl)-5-fluoro-2,4-pyrimidinediamine. 1H NMR (DMSO-d6): □ 11.20 (1H, s), 9.42 (1H, s), 9.35 (1H, s), 8.21 (1H, d, J= 3.6 Hz), 7.75 (1H, dd, J= 14.4 Hz, J= 2.4 Hz), 7.56 (1H, d, J= 8.1 Hz), 7.46 (1H, d, J= 8.7 Hz), 7.37 (1H, d, J= 9.6 Hz), 7.08 (1H, t, J= 9.3 Hz), 3.85 (3H, s), 1.53 (6H, s); LCMS: purity: 97 %; MS (m/e): 429 (MH+). |
| 7.4.164 | N2-(3-Chlorophenyl)-N4-(2,2-dimethyl-3-oxo-4H-5-pyrid[1,4]oxazin-6-yl)-5-fluoro-2,4-pyrimidinediamine R940386 | In like manner to the preparation of N2-(3-chloro-4-hydroxy-5-methylphenyl)-N4-(2,2-dimethyl-3-oxo-4H-benz[1,4]oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3-chloroaniline were reacted to yield N2-(3-chlorophenyl)-N4-(2,2-dimethyl-3-oxo-4H-5-pyrid[1,4]oxazin-6-yl)-5-fluoro-2,4-pyrimidinediamine. I H NMR (DMSO-d6): □11.10 (1H, s), 9.50 (1H, s), 9.43 (1H, s), 8.16 (1H, d, J= 3.3 Hz), 7.85 (1H, t, J= 1.95 Hz), 7.47 (2H, d, J =8.7 Hz), 7.38 (1 H, d, J= 8.7 Hz), 7.18 (1H, t, J= 8.1 Hz), 6.89 (1H, ddd, J= 7.8 Hz, J= 2.1 Hz, J= 1.2 Hz), 1.43 (6H, s); LCMS: purity: 100 %; MS (m/e): 415 (MH+). |
| 7.4.165 | N2-(3,5-Dichlorophenyl)-N4-(2,2-dimethyl-3-oxo-4H-5-pyrid[1,4]oxazin-6-yl)-5-fluoro-2,4-pyrimidinediamine R940387 | In like manner to the preparation of N2-(3-chloro-4-hydroxy-5-methylphenyl)-N4-(2,2-dimethyl-3-oxo-4H-benz[1,4]oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3,5-dichloroaniline were reacted to yield N2-(3,5-dichlorophenyl)-N4-(2,2-dimethyl-3-oxo-4H-5-pyrid[1,4]oxazin-6-yl)-5-fluoro-2,4-pyrimidinediamine. 1H NMR (DMSO-d6): □11.10 (1H, s), 9.66 (1H, s), 9.49 (1H, s), 8.19 (1H, m), 7.70 (2H, m), 7.39 (2H, m), 6.99 (1H, t. J= 1.95 Hz), 1.42 (6H, s); LCMS: purity: 96 %; MS (m/e): 450 (MH+). |
| 7.4.166 | N4-(2,2-Dimethyl-3-oxo-4H-5-pyrid[1,4]oxazin-6-yl)-5-fluoro-N2-(1-methylindazolin-6-yl)-2,4-pyrimidinediamine R940389 | In like manner to the preparation of N2-(3-chloro-4-hydroxy-5-methylphenyl)-N4-(2,2-dimethyl-3-oxo-4H-benz[1,4]oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 6-amino-1-methylindazole were reacted to yield N4-(2,2-dimethyl-3-oxo-4H-5-pyrid[1,4]oxazin-6-yl)-5-fluoro-N2-(1-methylindazolin-6-yl)-2,4-pyrimidinediamine. 1H NMR (DMSO-d6): □11.22 (1H, s), 9.60 (1H, s), 9.43 (1H, s), 8.29 (1H, d, J= 3.6 Hz), 8.13 (1H, s), 7.95 (1H, s), 7.72 (1H, d, J= 8.4 Hz), 7.64 (1H, d, J= 9 Hz), 7.47 (1H, d, J= 8.1 Hz), 7.34 (1H, dd, J= 8.7 Hz, J= 1.8 Hz), 3.19 (3H, s), 1.53 (6H, s); LCMS: purity: 100 %; MS (m/e): 435 (MH+). |
| 7.4.167 | N2-(3-Chloro-4-trifluoromethoxyphenyl)-N4-(2,2-dimethyl-3-oxo-4H-5-pyrid[1,4]oxazin-6-yl)-5-fluoro-2,4-pyrimidinediamine R940390 | In like manner to the preparation of N2-(3-chloro-4-hydroxy-5-methylphenyl)-N4-(2,2-dimethyl-3-oxo-4H-benz[1,4]oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3-chloro-4-trifluoromethoxyaniline were reacted to yield N2-[3-chloro-4-trifluoromethoxyphenyl]-N4-(2,2-dimethyl-3-oxo-4H-5-pyrid[1,4]oxazin-6-yl)-5-fluoro-2,4-pyrimidinediamine. 1H NMR (DMSO-d6): □11.24 (1H, s), 9.76 (1H, s), 9.60 (1H, s), 8.28 (1H, d, J= 3.6 Hz), 8.14 (1H, d, J= 2.4 Hz), 7.70 (1H, dd, J= 9 Hz, J= 2.7 Hz), 7.54-7.43 (3H, m), 1.53 (6H, s); LCMS: purity: 94.6 %; MS (m/e): 499 (MH+). |
| 7.4.168 | N2-(3-Chloro-4-methoxy-5-methylphenyl)-N4-(2,2-dimethyl-3-oxo-4H-5-pyrid[1,4]oxazin-6-yl)-5-fluoro-2,4-pyrimidinediamine R940391 | In like manner to the preparation of N2-(3-chloro-4-hydroxy-5-methylphenyl)-N4-(2,2-dimethyl-3-oxo-4H-benz[1,4]oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3-chloro-4-methoxy-5-methylaniline were reacted to yield N2-(3-chloro-4-methoxy-5-methylphenyl)-N4-(2,2-dimethyl-3-oxo-4H-5-pyrid[1,4]oxazin-6-yl)-5-fluoro-2,4-pyrimidinediamine. 1H NMR (DMSO-d6): □11.20 (1H, s), 9.24 (1H, s), 9.40 (1H, s), 8.23 (1H, dd, J= 3.3 Hz, J= 0.9 Hz), 7.76 (1H, d, J= 2.7 Hz), 7.61 (1H, d, J= 8.4 Hz), 7.47 (1H, d, J= 8.1 Hz), 7.42 (1H, d, J= 2.7 Hz), 3.76 (3H, d, J= 1.2 Hz), 2.27 (3H, s), 1.53 (6H, s); LCMS: purity: 100 %; MS (m/e): 459 (MH+). |
| 7.4.169 | N4-(2,2-Dimethyl-3-oxo-4H-5-pyrid[1,4]oxazm-6-yl)-5-fluoro-N2-(3-methoxycarbonylmethyle neoxyphenyl)-2,4-pyrimidinediamine R940392 | In like manner to the preparation of N2-(3-chloro-4-hydroxy-5-methylphenyl)-N4-(2,2-dimethyl-3-oxo-4H-benz[1,4]oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3-(methoxycarbonylmethyleneoxy)aniline were reacted to yield N4-(2,2-dimethyl-3-oxo-4H-5-pyrid[1,4]oxazin-6-yl)5-fluoro-N2-[3-methoxycarbonylmethyleneoxyphenyl]-2,4-pyrimidinediamine. 1H NMR (DMSO-d6): □11.16 (1H, s), 9.36 (1H, s), 9.34 (1H, s), 8.23 (1H, d, J=3.3 Hz), 7.69 (1H, d, J= 8.7 Hz), 7.47 (1H, d, J= 8.7 Hz), 7.42 (1H, s), 7.3 (1H, d, J= 8.1 Hz), 7.17 (1H, t, J= 8.1 Hz), 6.53 (1H, dd, J= 8.4 Hz, J= 2.4 Hz), 4.78 (2H, s), 3.79 (3H, s), 1.53 (6H, s); LCMS: purity: 94.69 %; MS (m/e): 469 (MH+). |
| 7.4.171 | N2-(3-Chloro-4-hydroxy-5-methylphenyl)-N4-(2,2-dimethyl-3-oxo-4H-5-pyrid[1,4]oxazin-6-yl)-5-fluoro-2,4-pyrimidinediamine R940394 | In like manner to the preparation of N2-(3-chloro-4-hydroxy-5-methylphenyl)-N4-(2,2-dimethyl-3-oxo-4H-benz[1,4]oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 4-amino-2-chloro-6-methylphenol were reacted to yield N2-(3-chloro-4-hydroxy-5-methylphenyl)-N4-(2,2-dimethyl-3-oxo-4H-5-pyrid[1,4]oxazin-6-yl)-5-fluoro-2,4-pyrimidinediamine. 1H NMR (DMSO-d6): □11.16 (1H, s), 9.27 (1H, s), 9.15 (1H, s), 8.67 (1H, s), 8.19 (1H, d, J= 3.6 Hz), 7.64 (2H, m), 7.42 (1H, d, J= 8.4 Hz), 7.29 (1H, d, J= 2.7 Hz), 2.22 (3H, s), 1.53 (6H, s); LCMS: purity: 97.69%; MS (m/e): 444 (M+). |
| 7.4.172 | N2-(3,5-Dimethyl-4-methoxyphenyl)-N4-(2,2-dimethyl-3-oxo-4H-5-pyrid[1,4]oxazin-6-yl)-5-fluoro-2,4-pyrimidinediamine R940395 | In like manner to the preparation of N2-(3-chloro-4-hydroxy-5-methylphenyl)-N4-(2,2-dimethyl-3-oxo-4H-benz[1,4]oxazin-6-yl)-5-fluoro-2,4-pyrimidinediamine, 2-chloro-N4-(2,2-dimethyl-3-oxo-4H-5-pyrid[1,4]oxazin-6-yl)-5-fluoro-4-pyrimidineamine and 3,5-dimethyl-4-methoxyaniline were reacted to yield N2-(3,5-dimethyl-4-methoxyphenyl)-N4-(2,2-dimcthyl-3-oxo-4H-5-pyrid[1,4]oxazin-6-yl)-5-fluoro-2,4-pyrimidinediamine, 1H NMR (DMSO-d6):□11.16 (1H, s), 9.23 (1H, s), 9.11 (1H, s), 8.19 (1H, d, J= 3.6 Hz), 7.69 (1 H, d, J= 8.1 Hz), 7.44 (1H, d, J= 8.4 Hz), 7.33 (2H, s), 3.68 (3H, s), 2.23 (6H, s), 1.53 (6H, s); LCMS: purity: 99%; MS (m/e): 439 (MH+). |
| 7.4.181 | (+) 4-(N-tert-Butoxycarbonyl)amino-6-nitro-1-benzopyran | A solution of (+) 4-amino-6-nitro-1-benzopyran in dioxane-water was treated with di-tert-butyl carbonate and sodium bicarbonate. The mixture was stirred for 2 hours at 0 oC and diluted with hexane. The mixture was filtered, and the remaining solids were carefully washed with hexane and dried under reduced pressure to give (+) 4-(N-tert-butoxycarbonyl)amino-6-nitro-1-benzopyran as a pale yellow solid. 1H NMR (CDCl3): □ 8.23 (d, 1H, J= 2.7 Hz), 8.04 (dd, 1H, J= 2.7, 9.6 Hz), 6.88 (d, 1H, J= 9.6 Hz), 4.89 (bs, 1H), 4.81 (bs, 1H), 4.26-4.38 (m, 2H), 2.03-2.26 (m, 2H). |
| 7.4.184 | (+) 4-(N-tert-Butoxycarbonyl-N-methyl)amino-6-nitro-1-benzopyran | A solution of 4-amino-6-nitro-1-benzopyran in THF was treated with sodium hydride followed by methyl iodide. The mixture was stirred for 24 hours at 0 oC. The mixture was diluted with water and extracted with dichloromethane. The organic phase was dried over magnesium sulfate and concentrated under reduced pressure to give (+) 4-(N-tert-butoxycarbonyl-N-methyl)amino-6-nitro-1-benzopyran as a pale yellow solid. 1H NMR (CDCl3): □ 7.98 (dd, 1H, J= 3.4, 9.3 Hz), 7.90 (bs, 1H), 6.82 (d, 1H, J= 9.3 Hz), 5.65 (bs, 1H), 4.18-4.44 (m, 2H), 1.98-2.06 (m, 2H), 2.55 (s, 3H). |
| 7.4.188 | (+) 4-(N-Acetyl)amino-6-nitro-1-benzopyran | A solution of 4-hydroxy-6-nitro-1-benzopyran in dry acetonitrile was treated with concentrated sulfuric acid. The mixture was stirred for 1 hour at 22 oC to give (+) 4-(N-acetyl)amino-6-nitro-1-benzopyran as a pale brownish precipitate, which was filtered off and dried. 1H NMR (CDCl3): □ 8.13 (d, 1H, J= 2.8 Hz), 8.04 (dd, 1H, J= 2.8, 8.7 Hz), 6.88 (d, 1H, J= 8.7 Hz), 5.87 (bs, 1H), 5.17-5.24 (m, 1H), 4.25-4.39 (m, 2H), 2.04-2.26 (m, 2H), 2.08 (s, 3H). |
| 7.4.189 | (+) 4-Amino-6-nitro-1-benzopyran | A solution of (+) 4-(N-acetyl)amino-6-nitro-1-benzopyran in concentrated HCl was refluxed for 16 hours. The reaction mixture was concentrated to dryness under reduced pressure and basified by addition of potassium carbonate. Water was added and the aqueous phase was extracted with methylene chloride and dried over magnesium sulfated. Removal of the volatiles under reduced pressure gave (+) 4-amino-6-nitro-1-benzopyran as a yellow solid, which used in the next step without further purification. 1H NMR (CDCl3): □ 8.23 (d, 1H, J= 3.0 Hz), 7.96 (dd, 1H, J= 3.0, 9.0 Hz), 6.80 (d, 1H, J= 9.0 Hz), 4.04-4.41 (m, 3H), 1.78-2.14 (m, 2H). |
| 7.4.190 | (S)-4-Amino-6-nitro-1-benzopyran (L)-(+)-Tartaric Acid Salt | A solution of (+)-4-amino-6-nitro-1-benzopyran in ethanol-water was treated with L-(+)-tartaric acid and heated to give a clear solution. The mixture was kept for 3 days at 22 oC and the resulting precipitate was filtered and washed carefully with ethanol to give enantiomerically pure (S)-4-amino-6-nitrobenzo-1-pyran (L)-(+)-tartaric acid salt. 1H NMR (DMSO-d6): □ 8.44 (d, 1H, J= 2.7 Hz), 8.08 (dd, 1H, J= 2.7, 9.3 Hz), 7.01 (d, 1H, J= 9.3 Hz), 4.32-4.39 (m, 3H), 3.97 (s, 2H), 1.90-2.26 (m, 2H). |
| 7.4.191 | (R)-4-Amino-6-nitro-1-benzopyran (D)-(-)-Tartaric Acid Salt | A solution of (+)-4-amino-6-nitrobenzopyran in ethanol-water was treated with D-(-)-tartaric acid and heated to give a clear solution. The mixture was kept for 3 days at 22 oC and the resulting precipitate was filtered and washed carefully with ethanol to give enantiomerically pure (R)-4-amino-6-nitro-1-benzopyran (D)-(-)-tartaric acid salt. 1H NMR (DMSO-d6): □ 8.44 (d, 1H, J= 2.7 Hz), 8.08 (dd, 1H, J= 2.7, 9.3 Hz), 7.01 (d, 1H, J= 9.3 Hz), 4.32-4.39 (m, 3H), 3.97 (s, 2H), 1.90-2.26 (m, 2H). |
| 7.4.281 | N2-[3-(N-Cyclobutylamino)carbony Imethyleneoxyphenyl]-5-fluoro-N4-(2H-3-oxo-4H-5-pyrid[1,4]oxazin-6-yl)-2,4-pyrimidinediamine (R945389) | In a manner similar to the preparation of N4-(3,4-ethylenedioxyphenyl)-S-fluoro-N2-(3-hydroxyphenyl)-2,4-pyrimidinediamine, 3-(N-cyclobutylaminocarbonylmethyleneoxy)aniline (100 mg) and 2-chloro-5-fluoro-N4-(2H-3-oxo-4H-5-pyrid[1,4]oxazin-6-yl)-4-pyrimidineamine (50 mg) were reacted to give N2-[3-(N-cyclobutylamino)carbonylmethyleneoxyphenyl]-5-fluoro-N4-(2H-3-oxo-4H-5-pyrid[1,4]oxazin-6-yl)-2,4-pyrimidinediamine. 1H NMR (DMSO-d6): δ 1.53-1.65 (m, 2H), 1.90-2.03 (m, 2H), 2.07-2.17 (m, 2H), 4.25 (q, J= 8.1 Hz, 1H), 4.32 (s, 2H), 4.61 (s, 2H), 6.46 (dd, J= 1.8 and 8.1 Hz, 1H), 7.10 (t, J= 8.1 Hz, 1H), 7.23 (dd, J= 0.9 and 8.4 Hz, 1H), 7.38 (m, 2H), 7.60 (d, J= 8.7 Hz, 1H), 8.13 (d, J= 3.6 Hz, 1H), 8.22 (d, J= 8.1 Hz, 1H), 9.23 (s, 1H), 9.26 (s, 1H), 11.12 (s, 1H); 19F NMR (282 MHz, DMSO-d6): δ - 163.26; LCMS: ret. time: 9.98 min.; purity: 92.21%; MS (m/e): 480.25 (MH+). |
| 7.4.282 | N2-[3-(N-Cyclopropylamino)carbon ylmethyleneoxyphenyl]-5-fluoro-N4-(2H-3-oxo-4H-5-pyrid[1,4]oxazin-6-yl)-2,4-pyrimidinediamine (R945390) | In a manner similar to the preparation of N4-(3,4-ethylenedioxyphenyl)-5-fluoro-N2-(3-hydroxyphenyl)-2,4-pyrimidinediamine, 3-(N-cyclopropylaminocarbonylmethyleneoxy)aniline (100 mg) and 2-chloro-5-fluoro-N4-(2H-3-oxo-4H-5-pyrid[1,4]oxazin-6-yl)-4-pyrimidineamine (50 mg) were reacted to give N2-[3-(N-cyclopropylamino)carbonylmethyleneoxyphenyl]-5-fluoro-N4-(2H-3-oxo-4H-5-pyrid[1,4]oxazin-6-yl)-2,4-pyrimidinediamine. 1H NMR (DMSO-d6): δ 0.47 (m, 2H), 0.61 (m, 2H), 2.66 (m, J= 3.6 Hz, 1H), 4.32 (s, 2H), 4.62 (s, 2H), 6.44 (dd, J= 2.4 and 7.5 Hz, 1H), 7.09 (t, J= 8.1 Hz, 1H), 7.22 (dd, J= 0.9 and 8.1 Hz, 1H), 7.37 (m, 2H), 7.59 (d, J= 8.4 Hz, 1H), 8.05 (d, J= 4.5 Hz, 1H), 8.13 (d, J= 3.6 Hz, 1H), 9.22 (s, 1H), 9.26 (s, 1H), 11.12 (s, 1H); 19F NMR (282 MHz, DMSO-d6): δ - 163.27; LCMS: ret. time: 8.89 min.; purity: 83.29%; MS (m/e): 466.24 (MH+). |
| 7.4.283 | N2-[4-(4-Acetylpiperazino)phenyl]-5-fluoro-N4-(2H-3-oxo-4H-5-pyrid[1,4]oxazin-6-yl)-2,4-pyrimidinediamine (R945391) | In a manner similar to the preparation of N4-(3,4-ethylenedioxyphenyl)-5-fluoro-N2-(3-hydroxyphenyl)-2,4-pyrimidinediamine, 4-(4-acetylpiperazino)aniline (100 mg) and 2-chloro-5-fluoro-N4-(2H-3-oxo-4H-5-pyrid[1,4]oxazin-6-yl)-4-pyrimidineamine (50 mg) were reacted to give N2-[4-(4-acetylpiperazino)phenyl]-5-fluoro-N4-(2H-3-oxo-4H-5-pyrid[1,4]oxazin-6-yl)-2,4-pyrimidinediamine. 1H NMR (DMSO-d6): δ 2.02 (s, 3H), 2.96 (t, J= 5.1 Hz, 2H), 3.02 (t, J= 5.1 Hz, 2H), 3.55 (br, 4H), 4.62 (s, 2H), 6.84 (d, J= 9.0 Hz, 2H), 7.36 (d, J= 8.4 Hz, 1H), 7.48 (d, J= 9.0 Hz, 2H), 7.57 (d, J= 8.7 Hz, 1H), 8.07 (d, J= 3.6 Hz, 1H), 9.01 (s, 1H), 9.13 (s, 1H), 11.14 (s, 1H); 19F NMR (282 MHz, DMSO-d6): δ - 164.84; LCMS: ret. time: 7.29 min.; purity: 88.46%; MS (m/e): 479.27 (MH+). |
| 7.4.284 | 5-Fluoro-N2-[4-(4-methoxycarbonylpiperazi no)phenyl]-N4-(2H-3-oxo-4H-5-pyrid[1,4]oxazin-6-yl)-2,4-pyrimidinediamine (R945392) | In a manner similar to the preparation of N4-(3,4-ethylenedioxyphenyl)-5-fluoro-N2-(3-hydroxyphenyl)-2,4-pyrimidinediamine, 4-(4-methoxycarbonylpiperazino)aniline (100 mg) and 2-chloro-5-fluoro-N4-(2H-3-oxo-4H-5-pyrid[1,4]oxazin-6-yl)-4-pyrimidineamine (50 mg) were reacted to give 5-fluoro-N2-[4-(4-methoxycarbonylpiperazino)phenyl]-N4-(2H-3-oxo-4H-5-pyrid[1,4]-oxazin-6-yl)-2,4-pyrimidinediamine. 1H NMR (DMSO-d6): δ 2.98 (t, J= 5.1 Hz, 4H), 3.48 (t, J= 5.1 Hz, 4H), 3.60 (s, 3H), 4.62 (s, 2H), 6.83 (d, J= 9.3 Hz, 2H), 7.36 (d, J= 8.4 Hz, 1H), 7.47 (d, J= 9.0 Hz, 2H), 7.56 (d, J= 8.4 Hz, 1H), 8.06 (d, J= 3.6 Hz, 1H), 9.01 (s, 1H), 9.13 (s, 1H); 19F NMR (282 MHz, DMSO-d6): δ - 164.84; LCMS: ret. time: 8.61 min.; purity: 83.00%; MS (m/e): 495.25 (MH+). |
| 7.4.290 | N2-(3,4-Dichlorophenyl)-5-fluoro-N4-(2H-3-oxo-4H-5-pyrid[1,4] oxazin-6-yl)-2,4-pyrimidinediamine (R945398) | In a manner similar to the preparation of N4-(3,4-ethylenedioxyphenyl)-5-fluoro-N2-(3-hydroxyphenyl)-2,4-pyrimidinediamine, 3,4-dichloroaniline (100 mg) and 2-chloro-5-fluoro-N4-(2H-3-oxo-4H-5-pyrid[1,4]oxazin-6-yl)-4-pyrimidineamine (50 mg) were reacted to give N2-(3,4-dichlorophenyl)-5-fluoro-N4-(2H-3-oxo-4H-5-pyrid[1,4]oxazin-6-yl)-2,4-pyrimidinediamine. 1H NMR (DMSO-d6): δ 4.63 (s, 2H), 7.39-7.42 (m, 3H), 7.52 (dd, J= 2.4 and 8.7 Hz, 1H), 8.06 (d, J= 2.1 Hz, 1H), 8.18 (d, J= 3.6 Hz, 1H), 9.46 (s, 1H), 9.59 (s, 1H), 11.17 (s, 1H); 19F NMR (282 MHz, DMSO-d6): δ - 162.48; LCMS: ret. time: 13.30 min.; purity: 90.24%; MS (m/e): 421.07 (MH+). |
| 7.4.291 | N2-(3-Chloro-4-methoxyphenyl)-5-fluoro-N4-(2H-3-oxo-4H-5-pyrid[1,4]oxazin-6-yl)-2,4-pyrimidinediamine (R945399) | In a manner similar to the preparation of N4-(3,4-ethylenedioxyphenyl)-5-fluoro-N2-(3-hydroxyphenyl)-2,4-pyrimidinediamine, 3-chloro-4-methoxyaniline (100 mg) and 2-chloro-5-fluoro-N4-(2H-3-oxo-4H-5-pyrid[1,4]oxazin-6-yl)-4-pyrimidineamine (50 mg) were reacted to give N2-(3-chloro-4-methoxyphenyl)-5-fluoro-N4-(2H-3-oxo-4H-5-pyrid[1,4]oxazin-6-yl)-2,4-pyrimidinediamine. 1H NMR (DMSO-d6): δ 3.76 (s, 3H), 4.62 (s, 2H), 7.00 (d, J= 9.0 Hz, 1H), 7.40 (d, J= 8.7 Hz, 1H), 7.47 (m, 2H), 7.80 (d, J= 2.4 Hz, 1H), 8.12 (d, J= 3.3 Hz, 1H), 9.22 (s, 1H), 9.27 (s, 1H), 11.15 (s, 1H); 19F NMR (282 MHz, DMSO-d6): δ - 163.98; LCMS: ret. time: 10.38 min.; purity: 91.61%; MS (m/e): 417.14 (MH+). |
| 7.4.292 | N2-(3,5-Dichloro-4-methoxyphenyl)-5-fluoro-N4-(2H-3-oxo-4H-5-pyrid[1,4]oxazin-6-yl)-2,4-pyrimidinediamine (R945400) | In a manner similar to the preparation of N4-(3,4-ethylenedioxyphenyl)-5-fluoro-N2-(3-hydroxyphenyl)-2,4-pyrimidinediamine, 3,5-dichloro-4-methoxyaniline (100 mg) and 2-chloro-5-fluoro-N4-(2H-3-oxo-4H-5-pyrid[1,4]oxazin-6-yl)-4-pyrimidineamine (50 mg) were reacted to give N2-(3,5-dichloro-4-methoxyphenyl)-5-fluoro-N4-(2H-3-oxo-4H-5-pyrid[1,4]oxazin-6-yl)-2,4-pyrimidinediamine. 1H NMR (DMSO-d6): δ 3.72 (s, 3H), 4.55 (s, 2H), 7.30 (d, J= 8.4 Hz, 1H), 7.35 (d, J= 8.4 Hz, 1H), 7.75 (s, 2H), 8.14 (d, J= 3.6 Hz, 1H), 9.48 (s, 1H); 19F NMR (282 MHz, DMSO-d6): δ - 162.65. |
| 7.4.293 | N2-(3,5-Dimethoxyphenyl)-5-fluoro-N4-(2H-3-oxo-4H-5-pyrid[1,4]oxazin-6-yl)-2,4-pyrimidinediamine (R945401) | In a manner similar to the preparation of N4-(3,4-ethylenedioxyphenyl)-5-fluoro-N2-(3-hydroxyphenyl)-2,4-pyrimidinediamine, 3,5-dimethoxyaniline (100 mg) and 2-chloro-5-fluoro-N4-(2H-3-oxo-4H-5-pyrid[1,4]oxazin-6-yl)-4-pyrimidineamine (50 mg) were reacted to give N2-(3,5-dimethoxyphenyl)-5-fluoro-N4-(2H-3-oxo-4H-5-pyrid[1,4]oxazin-6-yl)-2,4-pyrimidinediamine. 1H NMR (DMSO-d6): δ 3.64 (s, 6H), 4.62 (s, 2H), 6.06 (t, J= 2.4 Hz, 1H), 6.92 (d, J= 2.4 Hz, 2H), 7.32 (d, J= 8.7 Hz, 1H), 7.60 (d, J= 8.7 Hz, 1H), 8.13 (d, J= 3.6 Hz, 1H), 9.18 (s, 1H), 9.24 (s, 1H), 11.11 (s, 1H); 19F NMR (282 MHz, DMSO-d6): δ - 163.28; LCMS: ret. time: 10.41 min.; purity: 97.00%; MS (m/e): 413.19 (MH+). |
| 7.4.294 | 5-Fluoro-N2-(3-fluoro-4-methoxyphenyl)-N4-(2H-3-oxo-4H-5-pyrid[1,4]oxazin-6-yl)-2,4-pyrimidinediamine (R945402) | In a manner similar to the preparation of N4-(3,4-ethylenedioxyphenyl)-5-fluoro-N2-(3-hydroxyphenyl)-2,4-pyrimidinediamine, 3-fluoro-4-methoxyaniline (100 mg) and 2-chloro-5-fluoro-N4-(2H-3-oxo-4H-5-pyrid[1,4]oxazin-6-yl)-4-pyrimidineamine (50 mg) were reacted to give 5-fluoro-N2-(3-fluoro-4-methoxyphenyl)-N4-(2H-3-oxo-4H-5-pyrid[1,4]oxazin-6-yl)-2,4-pyrimidinediamine. 1H NMR (DMSO-d6): δ 3.75 (s, 3H), 4.62 (s, 2H), 6.99 (t, J= 9.3 Hz, 1H), 7.26 (dd, J= 2.4 and 9.0 Hz, 1H), 7.36 (d, J= 8.4 Hz, 1H), 7.45 (d, J= 8.4 Hz, 1H), 7.66 (dd, J= 2.7 and 14.4 Hz, 1H), 8.11 (d, J= 3.3 Hz, 1H), 9.24 (s, 1H), 9.32 (s, 1H), 11.15 (s, 1H); 19F NMR (282 MHz, DMSO-d6): δ 163.98, - 134.90; LCMS: ret. time: 9.84 min.; purity: 93.66%; MS (m/e): 401.18 (MH+). |
| 7.4.296. | 5-Fluoro-N2-(4-methoxyphenyl)-N4-(2H-3-oxo-4H-5-pyrid[1,4]oxazin-6-yl)-2,4-pyrimidinediamine (R945404) | In a manner similar to the preparation of N4-(3,4-ethylenedioxyphenyl)-5-fluoro-N2-(3-hydroxyphenyl)-2,4-pyrimidinediamine, 4-methoxyaniline (100 mg) and 2-chloro-5-fluoro-N4-(2H-3-oxo-4H-5-pyrid[1,4]oxazin-6-yl)-4-pyrimidineamine (50 mg) were reacted to give 5-fluoro-N2-(4-methoxyphenyl)-N4-(2H-3-oxo-4H-5-pyrid[1,4]oxazin-6-yl)-2,4-pyrimidinediamine. 1H NMR (DMSO-d6): δ 3.69 (s, 3H), 4.63 (s, 2H), 6.79 (d, J= 9.0 Hz, 2H), 7.35 (d, J= 8.4 Hz, 1H), 7.50 (d, J= 9.0 Hz, 2H), 7.53 (d, J= 9.0 Hz, 1H), 8.07 (d, J= 3.3 Hz, 1H), 9.05 (s, 1H), 9.18 (s, 1H), 11.13 (s, 1H); 19F NMR (282 MHz, DMSO-d6): δ - 175.00; LCMS: ret. time: 8.85 min.; purity: 100%; MS (m/e): 383.25 (MH+). |
| 7.4.297 | N2-(3,4-Ethylenedioxyphenyl)-5-fluoro-N4-(2H-3-oxo-4H-5-pyrid[1,4]oxazin-6-yl)-2,4-pyrimidinediamine (R945405) | In a manner similar to the preparation of N4-(3,4-ethylenedioxyphenyl)-5-fluoro-N2-(3-hydroxyphenyl)-2,4-pyrimidinediamine, 3,4-ethylenedioxyaniline (100 mg) and 2-chloro-5-fluoro-N4-(2H-3-oxo-4H-5-pyrid[1,4]oxazin-6-yl)-4-pyrimidineamine (50 mg) were reacted to give N2-(3,4-ethylenedioxyphenyl)-5-fluoro-N4-(2H-3-oxo-4H-5-pyrid[1,4]oxazin-6-yl)-2,4-pyrimidinediamine. 1H NMR (DMSO-d6): δ 4.16 (q, J= 2.1 Hz, 4H), 4.62 (s, 2H), 6.67 (d, J= 8.7 Hz, 1H), 6.98 (dd, J= 2.4 and 9.0 Hz, 1H), 7.27 (d, J= 2.4 Hz, 1H), 7.34 (d, J= 8.4 Hz, 1H), 7.53 (d, J= 9.0 Hz, 1H), 8.07 (d, J= 3.6 Hz, 1H), 9.05 (s, 1H), 9.21 (s, 1H), 11.11 (s, 1H); 19F NMR (282 MHz, DMSO-d6): δ - 174.73; LCMS: ret. time: 8.94 min.; purity: 97.69%; MS (m/e): 411.26 (MH+). |
| 7.4.298 | 5-Fluoro-N4-(2H-3-oxo-4H-5-pyrid[1,4]oxazin-6-yl)-N2-(4-trifluoromethoxyphenyl)-2,4-pyrimidinediamine (R945406) | In a manner similar to the preparation of N4-(3,4-cthylenedioxyphenyl)-5-fluoro-N2-(3-hydroxyphenyl)-2,4-pyrimidinediamine, 4-trifluoromethoxyaniline (100 mg) and 2-chloro-5-fluoro-N4-(2H-3-oxo-4H-5-pyrid[1,4]oxazin-6-yl)-4-pyrimidineamine (50 mg) were reacted to give 5-fluoro-N4-(2H-3-oxo-4H-5-pyrid[1,4]oxazin-6-yl)-N2-(4-trifluoromethoxyphenyl)-2,4-pyrimidinediamine. 1H NMR (DMSO-d6): δ 4.64 (s, 2H), 7.18 (d, J= 8.1 Hz, 2H), 7.38 (d, J= 8.7 Hz, 1H), 7.47 (d, J= 8.7 Hz, 1H), 7.73 (d, J= 9.0 Hz, 2H), 8.14 (d, J= 3.3 Hz, 1H), 9.38 (s, 1H), 9.45 (s, 1H), 11.18 (s, 1H); 19F NMR (282 MHz, DMSO-d6): δ - 173.29, - 68.81; LCMS: ret. time: 12.95 min.; purity: 100%; MS (m/e): 437.25 (MH+). |
| 7.4.299 | N2-(4-Ethoxyphenyl)-5-fluoro-N4-(2H-3-oxo-4H-5-pyrid[1,4]oxazin-6-yl)-2,4-pyrimidinediamine (R945407) | In a manner similar to the preparation of N4-(3,4-ethylenedioxyphenyl)-5-fluoro-N2-(3-hydroxyphenyl)-2,4-pyrimidinediamine, 4-ethoxyaniline (100 mg) and 2-chloro-5-fluoro-N4-(2H-3-oxo-4H-5-pyrid[1,4]oxazin-6-yl)-4-pyrimidineamine (50 mg) were reacted to give N2-(4-ethoxyphenyl)-5-fluoro-N4-(2H-3-oxo-4H-5-pyrid[1,4]oxazin-6-yl)-2,4-pyrimidinediamine. 1H NMR (DMSO-d6): δ 1.30 (t, J= 6.9 Hz, 3H), 3.94 (q, J= 6.9 Hz, 2H), 4.63 (s, 2H), 6.77 (d, J= 8.7 Hz, 2H), 7.36 (d, J= 8.4 Hz, 1H), 7.48 (d, J= 9.0 Hz, 2H), 7.54 (d, J= 8.7 Hz, 1H), 8.06 (d, J= 3.6 Hz, 1H), 9.04 (s, 1H), 9.17 (s, 1H), 11.14 (s, 1H); 19F NMR (282 MHz, DMSO-d6): δ - 175.00; LCMS: ret. time: 9.87 min.; purity: 90.82%; MS (m/e): 397.28 (MH+). |
| 7.4.300 | N2-(4-Butoxyphenyl)-5-fluoro-N4-(2H-3-oxo-4H-5-pyrid[1,4]oxazin-6-yl)-2,4-pyrimidinediamine (R945408) | In a manner similar to the preparation of N4-(3,4-ethylenedioxyphenyl)-5-fluoro-N2-(3-hydroxyphenyl)-2,4-pyrimidinediamine, 4-butoxyaniline (100 mg) and 2-chloro-5-fluoro-N4-(2H-3-oxo-4H-5-pyrid[1,4]oxazin-6-yl)-4-pyrimidineamine (50 mg) were reacted to give N2-(4-butoxyphenyl)-5-fluoro-N4-(2H-3-oxo-4H-5-pyrid[1,4]oxazin-6-yl)-2,4-pyrimidinediamine. 1H NMR (DMSO-d6): δ 0.93 (t, J= 7.5 Hz, 3H), 1.42 (hept, J= 7.5 Hz, 2H), 1.66 (p, J= 6.9 Hz, 2H), 3.89 (t, J= 6.3 Hz, 2H), 4.62 (s, 2H), 6.78 (d, J= 8.7 Hz, 2H), 7.35 (d, J= 8.4 Hz, 1H), 7.48 (d, J= 9.3 Hz, 2H), 7.54 (d, J= 8.7 Hz, 1H), 8.06 (d, J= 3.6 Hz, 1H), 9.04 (s, 1H), 9.17 (s, 1H), 11.14 (s, 1H); 19F NMR (282 MHz, DMSO-d6): δ 175.02; LCMS: ret. time: 12.12 min.; purity: 95.36%; MS (m/e): 425.31 (MH+). |
| 7.4.301 | 5-Fluoro-N2-(4-phenoxyphenyl)-N4-(2H-3-oxo-4H-5-pyrid[1,4]oxazin-6-yl)-2,4-pyrimidinediamine (R945409) | In a manner similar to the preparation of N4-(3,4-ethylenedioxyphenyl)-5-fluoro-N2-(3-hydroxyphenyl)-2,4-pyrimidinediamine, 4-phenoxyaniline (100 mg) and 2-chloro-5-fluoro-N4-(2H-3-oxo-4H-5-pyrid[1,4]oxazin-6-yl)-4-pyrimidineamine (50 mg) were reacted to give 5-fluoro-N2-(4-phenoxyphenyl)-N4-(2H-3-oxo-4H-5-pyrid[1,4]oxazin-6-yl)-2,4-pyrimidinediamine. 1H NMR (DMSO-d6): δ 4.60 (s, 2H), 6.91 (d, J= 9.0 Hz, 4H), 7.05 (t, J= 7.2 Hz, 1H), 7.32 (m, 3H), 7.52 (d, J= 8.4 Hz, 1H), 7.63 (d, J= 9.0 Hz, 2H), 8.10 (d, J= 3.6 Hz, 1H), 9.27 (s, 2H), 11.14 (s, 1H); 19F NMR (282 MHz, DMSO-d6): δ - 174.19; LCMS: ret. time: 12.69 min.; purity: 100%; MS (m/e): 445.27 (MH+). |
| 7.4.302 | N2-(4-Benzyloxyphenyl)-5-fluoro-N4-(2H-3-oxo-4H-5-pyrid[1,4]oxazin-6-yl)-2,4-pyrimidinediamine (R945410) | In a manner similar to the preparation of N4-(3,4-ethylenedioxyphenyl)-5-fluoro-N2-(3-hydroxyphenyl)-2,4-pyrimidinediamine, 4-benzyloxyaniline (100 mg) and 2-chloro-5-fluoro-N4-(2H-3-oxo-4H-5-pyrid[1,4]oxazin-6-yl)-4-pyrimidineamine (50 mg) were reacted to give N2-(4-benzyloxyphenyl)-5-fluoro-N4-(2H-3-oxo-4H-5-pyrid[1,4]oxazin-6-yl)-2,4-pyrimidinediamine. 1H NMR (DMSO-d6): δ 4.62 (s, 2H), 5.03 (s, 2H), 6.86 (d, J= 9.0 Hz, 2H), 7.31-7.51 (m, 9H), 8.06 (d, J= 3.3 Hz, 1H), 9.05 (s, 1H), 9.15 (s, 1H), 11.13 (s, 1H); 19F NMR (282 MHz, DMSO-d6): δ - 170.56; LCMS: ret. time: 12.02 min.; MS (m/e): 459.33 (MH+). |
| 7.4.304 | 5-Fluoro-N2-(4-morpholinophenyl)-N4-(2H-3-oxo-4H-5-pyrid[1,4]oxazin-6-yl)-2,4-pyrimidinediamine (R945412) | In a manner similar to the preparation of N4-(3,4-ethylenedioxyphenyl)-5-fluoro-N2-(3-hydroxyphenyl)-2,4-pyrimidinediamine, 4-morpholinoaniline (100 mg) and 2-chloro-5-fluoro-N4-(2H-3-oxo-4H-5-pyrid[1,4]oxazin-6-yl)-4-pyrimidineamine (50 mg) were reacted to give 5-fluoro-N2-(4-morpholinophenyl)-N4-(2H-3-oxo-4H-5-pyrid[1,4]oxazin-6-yl)-2,4-pyrimidinediamine. 1H NMR (DMSO-d6): δ 3.00 (t, J= 4.8 Hz, 4H), 3.71 (t, J= 4.8 Hz, 4H), 4.62 (s, 2H), 6.81 (d, J= 9.0 Hz, 2H), 7.35 (d, J= 8.4 Hz, 1H), 7.46 (d, J= 9.3 Hz, 2H), 7.56 (d, J= 8.4 Hz, 1H), 8.05 (d, J= 3.6 Hz, 1H), 9.00 (s, 1H), 9.13 (s, 1H), 11.13 (s, 1H); 19F NMR (282 MHz, DMSO-d6): δ - 175.15; LCMS: ret. time: 8.08 min.; purity: 92.97%; MS (m/e): 438.32 (MH+). |
| 7.4.305 | 5-Fluoro-N2-(4-isopropoxyphenyl)-N4-(2H-3-oxo-4H-5-pyrid[1,4]oxazin-6-yl)-2,4-pyrimidinediamine (R945413) | In a manner similar to the preparation of N4-(3,4-ethylenedioxyphenyl)-5-fluoro-N2-(3-hydroxyphenyl)-2,4-pyrimidinediamine, 4-isopropoxyaniline (100 mg) and 2-chloro-5-fluoro-N4-(2H-3-oxo-4H-5-pyrid[1,4]oxazin-6-yl)-4-pyrimidineamine (50 mg) were reacted to give 5-fluoro-N2-(4-isopropoxyphenyl)-N4-(2H-3-oxo-4H-5-pyrid[1,4]oxazin-6-yl)-2,4-pyrimidinediamine. 1H NMR (DMSO-d6): δ 1.22 (d, J= 6.3 Hz, 6H), 4.48 (p, J= 6.0 Hz, 1H), 4.62 (s, 2H), 6.76 (d, J= 9.0 Hz, 2H), 7.34 (d, J= 8.7 Hz, 1H), 7.47 (d, J= 9.0 Hz, 2H), 7.53 (d, J= 8.4 Hz, 1H), 8.06 (d, J= 3.6 Hz, 1H), 9.02 (s, 1H), 9.15 (s, 1H), 11.12 (s, 1H); 19F NMR (282 MHz, DMSO-d6): δ - 175.03; LCMS: ret. time: 10.52 min.; purity: 100%; MS (m/e): 411.32 (MH+). |
| 7.4.308 | N4-(2,2-Dimethyl-2H-3-oxo-4H-5-pyrido[1,4]oxazin-6-yl]-5-fluoro-N2-[4-(4-methoxycarbonylpiperazi no)phenyl]-2,4-pyrimidinediamine (R945416) | In a manner similar to the preparation of N4-(3,4-ethylenedioxyphenyl)-5-fluoro-N2-(3-hydroxyphenyl)-2,4-pyrimidinediamine, 4-(4-methoxycarbonylpiperazino)aniline (100 mg) and 2-chloro-N4-(2,2-dimethyl-2H-3-oxo-4H-5-pyrid[1,4]oxazin-6-yl)-5-fluoro-4-pyrimidineamine (50 mg) were reacted to give N4-(2,2-dimethyl-2H-3-oxo-4H-5-pyrido[1,4]oxazin-6-yl]-5-fluoro-N2-[4-(4-methoxycarbonylpiperazino)phenyl]-2,4-pyrimidinediamine. 1H NMR (DMSO-d6): δ 1.43 (s, 6H), 2.99 (t, J= 5.1 Hz, 4H), 3.49 (t, J= 5.1 Hz, 4H), 3.61 (s, 3H), 6.82 (d, J= 9.0 Hz, 2H), 7.37 (d, J= 8.4 Hz, 1H), 7.47 (d, J= 9.0 Hz, 2H), 7.55 (d, J= 8.1 Hz, 1H), 8.06 (d, J= 3.6 Hz, 1H), 9.02 (s, 1H), 9.14 (s, 1H), 11.08 (s, 1H); 19F NMR (282 MHz, DMSO-d6): δ - 164.38; LCMS: ret. time: 10.24 min.; purity: 100%; MS (m/e): 523.45 (MH+). |
| 7.4.309 | N2-[4-(N-Acetyl-N-methylamino)phenyl]-N4-(2,2-dimethyl-2H-3-oxo-4H-5-pyrid[1,4]oxazin-6-yl)-5-fluoro-2,4-pyrimidinediamine (R945417) | In a manner similar to the preparation of N4-(3,4-ethylenedioxyphenyl)-5-fluoro-N2-(3-hydroxyphenyl)-2,4-pyrimidinediamine, 4-(N-acetyl-N-methylamino)aniline (100 mg) and 2-chloro-N4-(2,2-dimethyl-2H-3-oxo-4H-5-pyrid[1,4]oxazin-6-yl)-5-fluoro-4-pyrimidineamine (50 mg) were reacted to give N2-[4-(N-acetyl-N-methylamino)phenyl]-N4-(2,2-dimethyl-2H-3-oxo-4H-5-pyrid[1,4]oxazin-6-yl)-5-fluoro-2,4-pyrimidinediamine. 1H NMR (DMSO-d6): δ 1.43 (s, 6H), 1.73 (s, 3H), 3.08 (s, 3H), 7.11 (d, J= 8.7 Hz, 2H), 7.41 (d, J= 8.4 Hz, 1H), 7.49 (d, J= 8.7 Hz, 1H), 7.68 (d, J= 8.7 Hz, 2H), 8.13 (d, J= 3.6 Hz, 1H), 9.35 (s, 1H), 9.40 (s, 1H), 11.12 (s, 1H); 19F NMR (282 MHz, DMSO-d6): δ - 162.87; LCMS: ret. time: 10.03 min.; purity: 100%; MS (m/e): 452.26 (MH+). |
| 7.4.310 | N2-[4-(N-Acetyl-N-ethylamino)phenyl]-N4-(2,2-dimethyl-2H-3-oxo-4H-5-pyrid[1,4]oxazin-6-yl)-5-fluoro-2,4-pyrimidinediamine (R945418) | In a manner similar to the preparation of 4-(4-methoxycarbonylpiperazino)nitrobenzene, N-ethyl-4-nitroaniline (1 g) and acetyl chloride (1 mL) were reacted to yield N-acetyl-N-ethyl-4-nitroaniline. 1H NMR (CDCl3): δ 1.15 (t, J= 7.2 Hz, 3H), 1.94 (s, 3H), 3.81 (q, J= 7.2 Hz, 2H), 7.36 (d, J= 9.0 Hz, 2H), 8.30 (d, J= 8.7 Hz, 2H). |
| | | N-Acetyl-N-ethyl-4-nitroaniline was reduced under hydrogenolysis conditions using 10% Pd-C in methanol at 40 psi for 1h. The catalyst was filtered off. The filtrate was evaporated to give 4-(N-acetyl-N-ethylamino)aniline. 1H NMR (CDCl3): δ 1.09 (t, J= 7.2 Hz, 3H), 1.82 (s, 3H), 3.68 (q, J= 7.2 Hz, 2H), 6.79 (d, J= 8.4 Hz, 2H), 6.95 (d, J= 8.1 Hz, 2H). |
| | | In a manner similar to the preparation of N4-(3,4-ethylenedioxyphenyl)-5-fluoro-N2-(3-hydroxyphenyl)-2,4-pyrimidinediamine, 4-(N-acetyl-N-ethylamino)aniline (100 mg) and 2-chloro-N4-(2,2-dimethyl-2H-3-oxo-4H-5-pyrid[1,4]oxazin-6-yl]-5-fluoro-4-pyrimidineamine (50 mg) were reacted to give N2-[4-(N-acetyl-N-ethylamino)phenyl]-N4-(2,2-dimethyl-2H-3-oxo-4H-5-pyrid[1,4]oxazin-6-yl)-5-fluoro-2,4-pyrimidinediamine. |
| | | 1H NMR (DMSO-d6): δ 0.97 (t, J= 7.2 Hz, 3H), 1.43 (s, 6H), 1.68 (s, 3H), 3.56 (q, J= 6.9 Hz, 2H), 7.06 (d, J= 8.7 Hz, 2H), 7.40 (d, J= 8.7 Hz, 1H), 7.48 (d, J= 8.1 Hz, 1H), 7.69 (d, J= 8.7 Hz, 2H), 8.13 (d, J= 3.3 Hz, 1H), 9.35 (s, 1H), 9.40 (s, 1H), 11.12 (s, 1H); 19F NMR (282 MHz, DMSO-d6): δ - 162.90; LCMS: ret. time: 10.51 min.; purity: 100%; MS (m/e): 466.25 (MH+). |
| 7.4.311 | N2-[4-(4-Acetylpiperazino)phenyl]-N4-(2,2-dimethyl-2H-3-oxo-4H-5-pyrido[1,4]oxazin-6-yl)-5-fluoro-2,4-pyrimidinediamine (R945419) | In a manner similar to the preparation of N4-(3,4-ethylenedioxyphenyl)-5-fluoro-N2-(3-hydroxyphenyl)-2,4-pyrimidinediamine, 4-(4-acetylpiperazino)aniline (100 mg) and 2-chloro-N4-(2,2-dimethyl-2H-3-oxo-4H-5-pyrid[1,4]oxazin-6-yl)-5-fluoro-4-pyrimidineamine (50 mg) were reacted to give N2-[4-(4-acetylpiperazino)phenyl]-N4-(2,2-dimethyl-2H-3-oxo-4H-5-pyrid[1,4] oxazin-6-yl)-5-fluoro-2,4-pyrimidinediamine. 1H NMR (DMSO-d6): δ 1.43 (s, 6H), 2.03 (s, 3H), 2.96 (t, J= 5.1 Hz, 2H), 3.03 (t, J= 4.8 Hz, 2H), 3.56 (m, 4H), 6.82 (d, J= 9.0 Hz, 2H), 7.37 (d, J= 8.4 Hz, 1H), 7.47 (d, J= 8.7 Hz, 2H), 7.56 (d, J= 8.1 Hz, 1H), 8.06 (d, J= 3.6 Hz, 1H), 9.02 (s, 1H), 9.13 (s, 1H), 11.08 (s, 1H); 19F NMR (282 MHz, DMSO-d6): δ - 164.40; LCMS: ret. time: 8.70 min.; purity: 97.70%; MS (m/e): 507.55 (MH+). |
| 7.4.312 | N2-[4-(N-Acetyl-N-methylamino)phenyl]-5-fluoro-N4-(2H-3-oxo-4H-5-pyrid[1,4]oxazin-6-yl)-2,4-pyrimidinediamine (R945420) | In a manner similar to the preparation of N4-(3,4-ethylenedioxyphenyl)-5-fluoro-N2-(3-hydroxyphenyl)-2,4-pyrimidinediamine, 4-(N-acetyl-N-methylamino)aniline (100 mg) and 2-chloro-5-fluoro-N4-(2H-3-oxo-4H-5-pyrid[1,4]oxazin-6-yl)-4-pyrimidineamine (50 mg) were reacted to give N2-[4-(N-acetyl-N-methylamino)phenyl]-5-fluoro-N4-(2H-3-oxo-4H-5-pyrid[1,4]oxazin-6-yl)-2,4-pyrimidinediamine. 1H NMR (DMSO-d6): δ 1.74 (s, 3H), 3.09 (s, 3H), 4.64 (s, 2H), 7.13 (d, J= 8.7 Hz, 2H), 7.40 (d, J= 8.7 Hz, 1H), 7.50 (d, J= 8.7 Hz, 1H), 7.69 (d, J= 8.7 Hz, 2H), 8.13 (d, J= 3.3 Hz, 1H), 9.34 (s, 1H), 9.39 (s, 1H), 11.16 (s, 1H); 19F NMR (282 MHz, DMSO-d6): δ - 171.37; LCMS: ret. time: 9.14 min.; purity: 91.43%; MS (m/e): 424.50 (MH+). |
| 7.4.313 | N2-[4-(N-Acetyl-N-ethylamino)phenyl]-5-fluoro-N4-(2H-3-oxo-4H-5-pyrid[1,4]oxazin-6-yl)-2,4-pyrimidinediamine (R945421) | In a manner similar to the preparation of N4-(3,4-ethylenedioxyphenyl)-5-fluoro-N2-(3-hydroxyphenyl)-2,4-pyrimidinediamine, 4-(N-acetyl-N-ethylamino)aniline (100 mg) and 2-chloro-5-fluoro-N4-(2H-3-oxo-4H-5-pyrid[1,4]oxazin-6-yl)-4-pyrimidineamine (50 mg) were reacted to give N2-[4-(N-acetyl-N-ethylamino)phenyl]-5-fluoro-N4-(2H-3-oxo-4H-5-pyrid[1,4]oxazin-6-yl)-2,4-pyrimidinediamine. 1H NMR (DMSO-d6): δ 0.98 (t, J= 6.9 Hz, 3H), 1.69 (s, 3H), 3.57 (q, J= 6.9 Hz, 2H), 4.63 (s, 2H), 7.08 (d, J= 8.7 Hz, 2H), 7.39 (d, J= 8.4 Hz, 1H), 7.50 (d, J= 8.4 Hz, 1H), 7.69 (d, J= 9.0 Hz, 2H), 8.13 (d, J= 3.6 Hz, 1H), 9.34 (s, 1H), 9.40 (s, 1H), 11.16 (s, 1H); 19F NMR (282 MHz, DMSO-d6): δ - 171.36; LCMS: ret. time: 9.26 min.; purity: 91.13%; MS (m/e): 438.27 (MH+). |
| 7.4.314 | N2-(3,4-Dimethoxyphenyl)-5-fluoro-N4-(2H-3-oxo-4H-5-pyrid[1,4]oxazin-6-yl)-2,4-pyrimidinediamine (R945422) | In a manner similar to the preparation of N4-(3,4-ethylenedioxyphenyl)-5-fluoro-N2-(3-hydroxyphenyl)-2,4-pyrimidinediamine, 3,4-dimethoxyaniline (100 mg) and 2-chloro-5-fluoro-N4-(2H-3-oxo-4H-5-pyrid[1,4]oxazin-6-yl)-4-pyrimidineamine (50 mg) were reacted to give N2-(3,4-dimethoxyphenyl)-5-fluoro-N4-(2H-3-oxo-4H-5-pyrid[1,4]oxazin-6-yl)-2,4-pyrimidinediamine. 1H NMR (DMSO-d6): δ 3.64 (s, 3H), 3.68 (s, 3H), 4.63 (s, 2H), 6.79 (d, J= 9.0 Hz, 1H), 7.20-7.23 (m, 2H), 7.33 (d, J= 8.7 Hz, 1H), 7.59 (d, J= 8.7 Hz, 1H), 8.08 (d, J= 3.6 Hz, 1H), 9.03 (s, 1H), 9.16 (s, 1H), 11.13 (s, 1H); 19F NMR (282 MHz, DMSO-d6): δ - 172.45; LCMS: ret. time: 8.35 min.; purity: 94.21%; MS (m/e): 413.30 (MH+). |
| 7.4.315 | N4-(2,2-Dimethyl-2H-3-oxo-4H-5-pyrid[1,4]oxazin-6-yl)-5-fluoro-N2-(4-morpholinophenyl)-2,4-pyrimidinediamine (R945423) | In a manner similar to the preparation of N4-(3,4-ethylenedioxyphenyl)-5-fluoro-N2-(3-hydroxyphenyl)-2,4-pyrimidinediamine, 4-morpholinoaniline (100 mg) and 2-chloro-N4-(2,2-dimethyl-2H-3-oxo-4H-5-pyrid[1,4]oxazin-6-yl)-5-fluoro-4-pyrimidineamine (50 mg) were reacted to give N4-(2,2-dimethyl-2H-3-oxo-4H-5-pyrid[1,4]oxazin-6-yl)-5-fluoro-N2-(4-morpholinophenyl)-2,4-pyrimidinediamine. 1H NMR (DMSO-d6): δ 1.43 (s, 6H), 2.99 (t, J= 4.8 Hz, 4H), 3.72 (t, J= 4.8 Hz, 4H), 6.80 (d, J= 8.7 Hz, 2H), 7.36 (d, J= 8.4 Hz, 1H), 7.46 (d, J= 9.0 Hz, 2H), 7.55 (d, J= 8.7 Hz, 1H), 8.06 (d, J= 3.6 Hz, 1H), 9.00 (s, 1H), 9.13 (s, 1H), 11.09 (s, 1H); 19F NMR (282 MHz, DMSO-d6): δ - 173.16; LCMS: ret. time: 9.59 min.; purity: 100%; MS (m/e): 466.28 (MH+). |
| 7.4.316 | N2-[3-(N-Cyclobutylamino)carbony lmethyleneoxyphenyl]-N4-(2,2-dimethyl-2H-3-oxo-4H-5-pyrid[1,4]oxazin-6-yl)-5-fluoro-2,4-pyrimidinediamine (R945424) | In a manner similar to the preparation of N4-(3,4-ethylenedioxyphenyl)-5-fluoro-N2-(3-hydroxyphenyl)-2,4-pyrimidinediamine, 3-(cyclobutylaminocarbonylmethyleneoxy)aniline (100 mg) and 2-chloro-N4-(2,2-dimethyl-2H-3-oxo-4H-5-pyrid[1,4]oxazin-6-yl)-5-fluoro-4-pyrimidineamine (50 mg) were reacted to give N2-[3-(N-cyclobutylamino)carbonylmethyleneoxyphenyl]-N4-(2,2-dimethyl-2H-3-oxo-4H-5-pyrid[1,4]oxazin-6-yl)-5-fluoro-2,4-pyrimidinediamine. 1H NMR (DMSO-d6): δ 1.42 (s, 6H), 1.57-1.66 (m, 2H), 1.90-2.04 (m, 2H), 2.12 (m, 2H), 4.25 (q, J= 8.4 Hz, 1H), 4.33 (s, 2H), 6.46 (dd, J= 1.8 and 8.1 Hz, 1H), 7.08 (t, J= 8.1 Hz,1H), 7.25 (dd, J= 8.4 Hz, 1H), 7.35 (m, 1H), 7.36 (d, J= 9.0 Hz, 1H), 7.63 (d, J= 9.0 Hz, 1H), 8.12 (d, J= 3.6 Hz, 1H), 8.23 (d, J= 7.5 Hz, 1H), 9.22 (s, 1H), 9.26 (s, 1H), 11.06 (s, 1H); 19F NMR (282 MHz, DMSO-d6): δ - 171.41; LCMS: ret. time: 11.46 min.; purity: 97.65%; MS (m/e): 508.45 (MH+). |
| 7.4.317 | 5-Fluoro-N2-[4-(4-methylpiperazino)phenyl] -N4-(2H-3-oxo-4H-5-pyrid[1,4]oxazin-6-yl)-2,4-pyrimidinediamine (R945426) | 1-(4-Nitrophenyl)piperazine (1 g), iodomethane (0.3 mL) and sodium hydride(500 mg) in THF (10 mL) were reacted overnight at room temperature. The solution was diluted with water. The yellow precipitation was collected by filtration, washed with water to give 4-(4-methylpiperazino)nitrobenzene as yellow solid. 1H NMR (CDCl3): δ 2.45 (s, 3H), 2.69 (t, J= 5.1 Hz, 4H), 3.52 (t, J= 5.1 Hz, 4H), 6.83 (d, J= 9.3 Hz, 2H), 8.12 (d, J= 9.3 Hz, 2H). |
| | | 4-(4-Methylpiperazino)nitrobenzene was reduced under hydrogenolysis conditions using 10% Pd-C in methanol at 40 psi for 1h. The catalyst was filtered off. The filtrate was evaporated to give 4-(4-methylpiperazino)aniline. 1H NMR (CDCl3): δ 2.47 (s, 3H), 2.75 (t, J= 5.1 Hz, 4H), 3.16 (t, J= 5.1 Hz, 4H), 6.65 (d, J= 9.0 Hz, 2H), 6.81 (d, J= 8.7 Hz, 2H). |
| | | In a manner similar to the preparation of N4-(3,4-ethylenedioxyphenyl)-5-fluoro-N2-(3-hydroxyphenyl)-2,4-pyrimidinediamine, 4-(4-methylpiperazino)aniline (100 mg) and 2-chloro-5-fluoro-N4-(2H-3-oxo-4H-5-pyrid[1,4]oxazin-6-yl)-4-pyrimidineamine (50 mg) were reacted to give 5-fluoro-N2-[4-(4-methylpiperazino)phenyl]-N4-(2H-3-oxo-4H-5-pyrid[1,4]oxazin-6-yl)-2,4-pyrimidinediamine. 1H NMR (DMSO-d6): δ 2.85 (s, 3H), 3.16 (m, 2H), 3.48 (m, 4H), 3.69 (m, 2H), 4.63 (s, 2H), 6.87 (d, J= 9.0 Hz, 2H), 7.36 (d, J= 8.7 Hz, 1H), 7.50 (d, J= 9.0 Hz, 2H), 7.54 (d, J= 8.1 Hz, 1H), 8.07 (d, J= 3.6 Hz, 1H), 9.08 (s, 1H), 9.21 (s, 1H), 11.16 (s, 1H); 19F NMR (282 MHz, DMSO-d6): δ - 172.68; LCMS: ret. time: 5.67 min.; purity: 100%; MS (m/e): 451 (MH+). |
| 7.4.318 | N4-(2,2-Dimethyl-2H-3-oxo-4H-5-pyrid[1,4]oxazin-6-yl)-5-fluoro-N2-[4-(4-methylpiperazino)phenyl] -2,4-pyrimidinediamine (R945427) | In a manner similar to the preparation of N4-(3,4-ethylenedioxyphenyl)-5-fluoro-N2-(3-hydroxyphenyl)-2,4-pyrimidinediamine, 4-(4-methylpiperazino)aniline (100 mg) and 2-chloro-N4-(2,2-dimethyl-2H-3-oxo-4H-5-pyrid[1,4]oxazin-6-yl)-5-fluoro-4-pyrimidineamine (50 mg) were reacted to give N4-(2,2-dimethyl-2H-3-oxo-4H-5-pyrid[1,4]oxazin-6-yl)-5-fluoro-N2-[4-(4-methylpiperazino)phenyl]-2,4-pyrimidinediamine. 1H NMR (DMSO-d6): δ 1.43 (s, 6H), 2.71 (s, 3H), 3.16 (br, 8H), 6.84 (d, J= 9.0 Hz, 2H), 7.37 (d, J= 8.4 Hz, 1H), 7.49 (d, J= 9.0 Hz, 2H), 7.54 (d, J= 8.4 Hz, 1H), 8.07 (d, J= 3.6 Hz, 1H), 9.05 (s, 1H), 9.18 (s, 1H), 11.10 (s, 1H); 19F NMR (282 MHz, DMSO-d6): δ - 172.96; LCMS: ret. time: 7.08 min.; purity: 91.96%; MS (m/e): 479.25 (MH+). |
| 7.4.319 | N2-(3,5-Dimethylphenyl)-5-fluoro-N4-(2H-3-oxo-4H-5-pyrid[1,4]oxazin-6-yl)-2,4-pyrimidinediamine (R945432) | In a manner similar to the preparation of N4-(3,4-ethylenedioxyphenyl)-5-fluoro-N2-(3-hydroxyphenyl)-2,4-pyrimidinediamine, 3,5-dimethylaniline (100 mg) and 2-chloro-5-fluoro-N4-(2H-3-oxo-4H-5-pyrid[1,4]oxazin-6-yl)-4-pyrimidineamine (50 mg) were reacted to give N2-(3,5-dimethylphenyl)-5-fluoro-N4-(2H-3-oxo-4H-5-pyrid[1,4]oxazin-6-yl)-2,4-pyrimidinediamine. 1H NMR (DMSO-d6): δ 2.17 (s, 6H), 4.62 (s, 2H), 6.52 (s, 1H), 7.22 (s, 2H), 7.34 (d, J= 8.4 Hz, 1H), 7.57 (d, J= 8.4 Hz, 1H), 8.11 (d, J= 3.6 Hz, 1H), 9.10 (s, 1H), 9.19 (s, 1H), 11.14 (s, 1H); 19F NMR (282 MHz, DMSO-d6): δ - 172.16; LCMS: ret. time: 11.34 min.; purity: 90.04%; MS (m/e): 381.23 (MH+). |
| 7.4.320 | N2-(3,5-Dimethylphenyl)-N4-(2,2-dimethyl-2H-3-oxo-4H-5-pyrid[1,4]oxazin-6-yl)-5-fluoro-2,4-pyrimidinediamine (R945433) | In a manner similar to the preparation of N4-(3,4-ethylenedioxyphenyl)-5-fluoro-N2-(3-hydroxyphenyl)-2,4-pyrimidinediamine, 3,5-dimethylaniline (100 mg) and 2-chloro-N4-(2,2-dimethyl-2H-3-oxo-4H-5-pyrid[1,4]oxazin-6-yl)-5-fluoro-4-pyrimidineamine (50 mg) were reacted to give N2-(3,5-dimethylphenyl)-N4-(2,2-dimethyl-2H-3-oxo-4H-5-pyrid[1,4]oxazin-6-yl)-5-fluoro-2,4-pyrimidinediamine. 1H NMR (DMSO-d6): δ 1.42 (s, 6H), 2.16 (s, 6H), 6.51 (s, 1H), 7.23 (s, 2H), 7.35 (d, J= 8.4 Hz, 1H), 7.59 (d, J= 8.1 Hz, 1H), 8.11 (d, J= 3.6 Hz, 1H), 9.10 (s, 1H), 9.18 (s, 1H), 11.08 (s, 1H); 19F NMR (282 MHz, DMSO-d6): δ - 172.19; LCMS: ret. time: 13.05 min.; purity: 95.71%; MS (m/e): 409.30 (MH+). |
| 7.4.321 | 5-Fluoro-N2-(3-isopropylphenyl)-N4-(2H-3-oxo-4H-5-pyrid[1,4]oxazin-6-yl)-2,4-pyrimidinediamine (R945434) | In a manner similar to the preparation of N4-(3,4-ethylenedioxyphenyl)-5-fluoro-N2-(3-hydroxyphenyl)-2,4-pyrimidinediamine, 3-isopropylaniline (100 mg) and 2-chloro-5-fluoro-N4-(2H-3-oxo-4H-5-pyrid[1,4]oxazin-6-yl)-4-pyrimidineamine (50 mg) were reacted to give 5-fluoro-N2-(3-isopropylphenyl)-N4-(2H-3-oxo-4H-5-pyrid[1,4]oxazin-6-yl)-2,4-pyrimidinediamine. 1H NMR (DMSO-d6): δ 1.15 (d, J= 6.9 Hz, 6H), 2.74 (p, J= 6.9 Hz, 1H), 4.63 (s, 2H), 6.76 (d, J= 7.8 Hz, 1H), 7.10 (t, J= 7.8 Hz, 1H), 7.34 (d, J= 8.7 Hz, 1H), 7.42 (s, 1H), 7.51 (d, J= 9.0 Hz, 1H), 7.57 (d, J= 8.4 Hz, 1H), 8.11 (d, J= 3.6 Hz, 1H), 9.15 (s, 1H), 9.21 (s, 1H), 11.15 (s, 1H); 19F NMR (282 MHz, DMSO-d6): δ - 172.02; LCMS: ret. time: 12.40 min.; purity: 92.20%; MS (m/e): 395.28 (MH+). |
| 7.4.322 | N2-(3-Chloro-4-methylphenyl)-5-fluoro-N4-(2H-3-oxo-4H-5-pyrid[1,4]oxazin-6-yl)-2,4-pyrimidinediamine (R945439) | In a manner similar to the preparation of N4-(3,4-ethylenedioxyphenyl)-5-fluoro-N2-(3-hydroxyphenyl)-2,4-pyrimidinediamine, 3-chloro-4-methylaniline (100 mg) and 2-chloro-5-fluoro-N4-(2H-3-oxo-4H-5-pyrid[1,4]oxazin-6-yl)-4-pyrimidineamine (50 mg) were reacted to give N2-(3-chloro-4-methylphenyl)-5-fluoro-N4-(2H-3-oxo-4H-5-pyrid[1,4]oxazin-6-yl)-2,4-pyrimidinediamine. 1H NMR (DMSO-d6): δ 2.22 (s, 3H), 4.63 (s, 2H), 7.13 (d, J= 8.7 Hz, 1H), 7.38 (m, 2H), 7.48 (d, J= 8.4 Hz, 1H), 7.83 (d, J= 2.1 Hz, 1H), 8.13 (d, J= 3.3 Hz, 1H), 9.31 (s, 1H), 9.33 (s, 1H), 11.13 (s, 1H); 19F NMR (282 MHz, DMSO-d6): δ - 171.47; LCMS: ret. time: 12.66 min.; purity: 94.85%; MS (m/e): 401.13 (MH+). |
| 7.4.323 | 5-Fluoro-N2-(3-methoxy-5-trifluoromethylphenyl)-N4-(2H-3-oxo-4H-5-pyrid[1,4]oxazin-6-yl)-2,4-pyrimidinedianine (R945440) | In a manner similar to the preparation of N4-(3,4-ethylenedioxyphenyl)-5-fluoro-N2-(3-hydroxyphenyl)-2,4-pyrimidinediamine, 3-methoxy-5-trifluoromethylaniline (100 mg) and 2-chloro-5-fluoro-N4-(2H-3-oxo-4H-5-pyrid[1,4]oxazin-6-yl)-4-pyrimidineamine (50 mg) were reacted to give 5-fluoro-N2-(3-methoxy-5-trifluoromethylphenyl)-N4-(2H-3-oxo-4H-5-pyrid[1,4]oxazin-6-yl)-2,4-pyrimidinediamine. 1H NMR (DMSO-d6): δ 3.74 (s, 3H), 4.63 (s, 2H), 6.72 (s, 1H), 7.32 (d, J= 8.4 Hz, 1H), 7.51 (d, J= 8.7 Hz, 1H), 7.56 (s, 1H), 7.64 (s, 1H), 8.18 (d, J= 3.3 Hz, 1H), 9.36 (s, 1H), 9.55 (s, 1H), 11.12 (s, 1H); 19F NMR (282 MHz, DMSO-d6): δ - 170.42; LCMS: ret. time: 13.14 min.; purity: 86.65%; MS (m/e): 451.30 (MH+). |
| 7.4.324 | 5-Fluoro-N2-(indol-6-yl)-N4-(2H-3-oxo-4H-5-pyrid[1,4]oxazin-6-yl)-2,4-pyrimidinedianine (R945443) | In a manner similar to the preparation of N4-(3,4-ethylenedioxyphenyl)-S-fluoro-N2-(3-hydroxyphenyl)-2,4-pyrimidinediamine, 6-aminoindol (100 mg) and 2-chloro-5-fluoro-N4-(2H-3-oxo-4H-5-pyrid[1,4]oxazin-6-yl)-4-pyrimidineamine (50 mg) were reacted to give 5-fluoro-N2-(indol-6-yl)-N4-(2H-3-oxo-4H-5-pyrid[I,4]oxazin-6-yl)-2,4-pyrimidinediamine. 1H NMR (DMSO-d6): δ 4.62 (s, 2H), 6.30 (s, 1H), 7.17 (m, 2H), 7.29 (d, J= 8.7 Hz, 1H), 7.35 (d, J= 8.7 Hz, 1H), 7.75 (d, J= 8.7 Hz, 1H), 7.82 (s, 1H), 8.10 (d, J=3.6 Hz, 1H), 9.08 (s, 1H), 9.11 (s, 1H), 10.84 (s, 1H), 11.11 (s, 1H); 19F NMR (282 MHz, DMSO-d6): δ - 172.73; LCMS: ret. time: 8.52 min.; purity: 81.74%; MS (m/e): 392.30 (MH+). |
| 7.4.325 | N4-(2,2-dimethyl-2H-3-oxo-4H-5-pyrid[1,4]oxazin-6-yl)-5-fluoro-N2-(indol-6-yl)-2,4-pyrimidinediamine (R945444) | In a manner similar to the preparation of N4-(3,4-ethylenedioxyphenyl)-5-fluoro-N2-(3-hydroxyphenyl)-2,4-pyrimidinediamine, 6-aminoindol (100 mg) and 2-chloro-N4-(2,2-dimethyl-2H-3-oxo-4H-5-pyrid[1,4]oxazin-6-yl)-5-fluoro-4-pyrimidineamine (50 mg) were reacted to give N4-(2,2-dimethyl-2H-3-oxo-4H-5-pyrid[1,4]oxazin-6-yl)-5-fluoro-N2-(indol-6-yl)-2,4-pyrimidinediamine. 1H NMR (DMSO-d6): δ 1.42 (s, 6H), 6.30 (s, 1H), 7.18 (m, 2H), 7.29 (d, J= 8.4 Hz, 1H), 7.35 (d, J= 8.7 Hz, 1H), 7.77 (d, J= 8.7 Hz, 1H), 7.80 (s, 1H), 8.10 (d, J= 3.6 Hz, 1H), 9.02 (s, 1H), 9.09 (s, 1H), 10.84 (s, 1H), 11.04 (s, 1H); 19F NMR (282 MHz, DMSO-d6): δ - 172.86; LCMS: ret. time: 9.91 min.; purity: 98.01%; MS (m/e): 420.18 (MH+). |
| 7.4.326 | N2-(3,5-Dichlorophenyl)-5-fluoro-N4-(2H-3-oxo-4H-5-pyrid[1,4]oxazin-6-yl)-2,4-pyrimidinediamine (R945454) | In a manner similar to the preparation of N4-(3,4-ethylenedioxyphenyl)-5-fluoro-N2-(3-hydroxyphenyl)-2,4-pyrimidinediamine, 3,5-dichloroaniline (100 mg) and 2-chloro-5-fluoro-N4-(2H-3-oxo=4H-5-pyrid[1,4]oxazin-6-yl)-4-pyrimidineamine (50 mg) were reacted to give N2-(3,5-dichlorophenyl)-5-fluoro-N4-(2H-3-oxo-4H-5-pyrid[1,4]oxazin-6-yl)-2,4-pyrimidinediamine. 1H NMR (DMSO-d6): δ 4.62 (s, 2H), 6.99 (t, J= 1.8 Hz, 1H), 7.38 (s, 2H), 7.70 (d, J=2.1 Hz, 2H), 8.19 (d, J= 3.6Hz, 1H), 9.52 (s, 1H), 9.66 (s, 1H), 11.17 (s, 1H); 19F NMR (282 MHz, DMSO-d6): δ - 170.19; LCMS: ret. time: 14.05 min.; purity: 85.53%; MS (m/e): 421.21 (MH+). |
| 7.4.327 | N2-(3-Bromophenyl)-5-fluoro-N4-(2H-3-oxo-4H-5-pyrid[1,4]oxazin-6-yl)-2,4-pyrimidinediamine (R945455) | In a manner similar to the preparation of N4-(3,4-ethylenedioxyphenyl)-5-fluoro-N2-(3-hydroxyphenyl)-2,4-pyrimidinediamine, 3-bromoaniline (100 mg) and 2-chloro-5-fluoro-N4-(2H-3-oxo-4H-5-pyrid[1,4]oxazin-6-yl)-4-pyrimidineamine (50 mg) were reacted to give N2-(3-bromophenyl)-5-fluoro-N4-(2H-3-oxo-4H-5-pyrid[1,4]oxazin-6-yl)-2,4-pyrimidinediamine. 1H NMR (DMSO-d6): δ 4.63 (s, 2H), 7.02 (ddd, J= 0.9 and 1.8 and 7.8 Hz, 1H), 7.13 (t, J= 8.1 Hz, 1H), 7.39 (d, J= 8.7 Hz, 1H), 7.47 (d, J= 8.1 Hz, 1H), 7.52 (dd, J= 0.9 and 8.1 Hz, 1H), 7.99 (t, J= 1.8 Hz, 1H), 8.16 (d, J= 3.6 Hz, 1H), 9.40 (s, 1H), 9.47 (s, 1H), 11.17 (s, 1H); 19F NMR (282 MHz, DMSO-d6): δ - 170.91; LCMS: ret. time: 12.31 min.; purity: 100%; MS (m/e): 431.20 (MH+). |
| 7.4.328 | N2-(3-tert-Butylphenyl)-5-fluoro-N4-(2H-3-oxo-4H-5-pyrid[1,4]oxazin-6-yl)-2,4-pyrimidinediamine (R945456) | In a manner similar to the preparation of N4-(3,4-ethylenedioxyphenyl)-5-fluoro-N2-(3-hydroxyphenyl)-2,4-pyrimidinediamine, 3-tert-butylaniline (100 mg) and 2-chloro-5-fluoro-N4-(2H-3-oxo-4H-5-pyrid[1,4]oxazin-6-yl)-4-pyrimidineamine (50 mg) were reacted to give N2-(3-tert-butylphenyl)-5-fluoro-N4-(2H-3-oxo-4H-5-pyrid[1,4]oxazin-6-yl)-2,4-pyrimidinediamine. 1H NMR (DMSO-d6): δ 1.23 (s, 9H), 4.62 (s, 2H), 6.91 (d, J= 8.1 Hz, 1H), 7.11 (t, J= 8.1 Hz, 1H), 7.34 (d, J= 8.4 Hz, 1H), 7.48 (s, 1H), 7.58 (s, 1H), 7.63 (d, J= 9.9 Hz, 1H), 8.11 (d, J= 3.6 Hz, 1H), 9.12 (s, 1H), 9.16 (s, 1H), 11.12 (s, 1H); 19F NMR (282 MHz, DMSO-d6): δ - 171.99; LCMS: ret. time: 13.16 min.; purity: 93.03%; MS (m/e): 409.29 (MH+). |
| 7.4.329 | N2-(3,4-Difluorophenyl)-5-fluoro-N4-(2H-3-oxo-4H-5-pyrid[1,4]oxazin-6-yl)-2,4-pyrimidinediamine (R945458) | In a manner similar to the preparation of N4-(3,4-ethylenedioxyphenyl)-5-fluoro-N2-(3-hydroxyphenyl)-2,4-pyrimidinediamine, 3,4-difluoroaniline (100 mg) and 2-chloro-5-fluoro-N4-(2H-3-oxo-4H-5-pyrid[1,4]oxazin-6-yl)-4-pyrimidineamine (50 mg) were reacted to give N2-(3,4-difluorophenyl)-5-fluoro-N4-(2H-3-oxo-4H-5-pyrid[1,4]oxazin-6-yl)-2,4-pyrimidinediamine. 1H NMR (DMSO-d6): δ 4.63 (s, 2H), 7.27 (m, 2H), 7.38 (s, 2H), 7.88 (ddd, J= 2.7 and 8.1 and 14.1 Hz, 1H), 8.15 (d, J= 3.6 Hz, 1H), 9.46 (s, 1H), 9.48 (s, 1H), 11.17 (s, 1H); 19F NMR (282 MHz, DMSO-d6): δ - 162.44, - 148.50, - 138.13; LCMS: ret. time: 11.63 min.; purity: 84.89%; MS (m/e): 389.25 (MH+). |
| | Synthesis of Anilines | |
| 7.4.330 | (S)-2-Methyl-6-nitro-3-oxo-4H-benz[1,4]oxazine | To solution of 2-amino-4-nitrophenol (6.6 g) in DMF (100 mL) at 0 oC was added 95% NaH (1 g) solid all at once. The solution was stirred at 0 oC for 20 minutes then at room temperature for 1 hour. (S)-(-)-Methyl-2-chloropropionate (5 g) was added all at once and the reaction was heated with a reflux condenser attached at 85 oC overnight. The reaction mixture was concentrated and the residue was partitioned between EtOAc and water. The aqueous phase was extracted with EtOAc and the combined organic layers were washed three times with water and then once with brine, dried over MgSO4, filtered and the volume was minimized on the rotary evaporater to about 15 mL. The residue was chromatographed EtOAc/hexanes 1:4 isocratically. The pure fractions were combined and evaporated and the crude product recrystallized from EtOAc/hexanes to yield (S)-2-methyl-6-nitro-3-oxo-4H-benz[1,4]oxazine. 1H NMR (DMSO-d6): □ 7.82 (dd, 1H), 7.76 (d, 1H), 7.12 (d, 1H), 4.90 (q, 1H), 1.42 (d, 3H); LCMS: purity: 100 %; MS (m/e): 209 (MH+). |
| 7.4.331 | (S)-6-Amino-2-methyl-3-oxo-4H-benz[1,4]oxazine | To a solution of (S)-2-methyl-6-nitro-3-oxo-4H-benz[1,4]oxazine (2.5 g) in 250 mL EtOH/EtOAc (1:1; v/v) was added 500 mg of 10% Pd/C (Degussa) and the reaction was hydrogenated in the Parr apparatus at 50 PSI for 1 hour. The reaction was filtered through a bed of celite, evaporated and dried in vacuo to yield the 2.3 g of (S)-6-Amino-2-methyl-3-oxo-4H-benz[1,4]oxazine. 1H NMR (DMSO-d6): □ 6.60 (d, 1H), 6.12 (m, 2H), 4.40 (q, 1H), 1.32 (d, 3H); LCMS: purity: 100 %; MS (m/e): 179 (MH+). |
| 7.4.332 | (R)-2-Methyl-6-nitro-3-oxo-4H-benz[1,4]oxazine | In like manner to the synthesis of (S)-2-methyl-6-nitro-3-oxo-4H-benz[l,4Joxazine, the reaction of (R)-(+)-methyl-2-chloropropionate with 2-amino-4-nitrophenol gave (R)-2-methyl-6-nitro-3-oxo-4H-b cnzfl,4]oxazine. 1H NMR (DMSO-d6): □ 7,82 (dd, 1H), 7.76 (d, 1H), 7.12 (d, 1H), 4.90 (q, 1H), 1.42 (d, 3H); LCMS: purity: 100 %; MS (m/e): 209 (MH+). |
| 7.4.333 | (R)-6-Amino-2-methyl-3-oxo-4H-benz[1,4]oxazine | In like manner to the synthesis of (S)-6-amino-2-methyl-3-oxo-4H-benz[1,4]oxazine, the hydrogenation of (R)-2-methyl-6-nitro-3-oxo-4H-benz[1,4]oxazine gave (R)-6-amino-2-methyl-3-oxo-4H-benz[1 ,4]oxazine. 1H NMR (DMSO-d6): □ 6.60 (d, 1H), 6.12 (m, 2H), 4.40 (q, 1H), 1.32 (d, 3H); LCMS: purity: 100 %; MS (m/e): 179 (MH+). |
| 7.4.334 | 7-Amino-4,4-dimethyl-1,3-dioxo-2H,4H-isoquinoline | The material was prepared according to the procedure outlined in J. Med Chem, 2002, 45(16), 3394-3405. |
| 7.4.335 | (+)-2-(2-Hydroxyethyl)-6-nitro-3-oxo-4H-benz[1,4]oxazine | To solution of 2-Amino-4-nitrophenol (16.5 g) in DMF (100 mL) at 0 oC was added 95% NaH (3 g) solid all at once. The reaction mixture was stirred at 0 oC for 20 minutes then at room temperature for 1 hour. 2-Bromobutyrolactone (13.8 mL) was added to the reaction mixture and it was then heated at 85 oC for overnight period with a reflux condenser attached. The reaction mixturewas concentrated to approximately 25 mL and diluted with 25 mL of MeOH. 400 mL of DI water was added with stirring and the precipitated product was collected filtration and dried on the funnel for 4 h to yield (+)-2-(2-hydroxyethyl)-6-nitro-3-oxo-4H-benz[1,4]oxazine. 1H NMR (DMSO-d6): □ 7.82 (dd, 1H), 7.75 (d, 1H), 7.18 (d, 1H), 4.90 (m, 1H), 4.70 (t, 1H), 3.8 (m, 2H), 1.96 (m, 2H); LCMS: purity: 100 %; MS (m/e): 239 (MH+). |
| 7.4.336 | (+)-6-Amino-2-(2-hydroxyethyl)-3-oxo-4H-benz[1,4]oxazine | To a solution of (+)-2-(2-hydroxyethyl)-6-nitro-3-oxo-4H-benz[1,4]oxazine (1 g) in EtOH/EtOAc (100 mL; 1:1 v/v) was hydrogenated at 50 PSI in the presence of 200 mg of 10% Pd/C (Degussa) to give (+)-6-amino-2-(2-hydroxyethyl)-3-oxo-4H-benz[1,4]oxazine. 1H NMR (DMSO-d6): □ 6.60 (d, 1H), 6.12 (m" 2H), 4.58 (t, 1H), 4.40 (m, 1H), 3.57 (m, 2H), 1.76 (m, 2H); LCMS: purity: 98 %; MS (m/e): 209 (MH+) |
| | Preparation of Aminoindazolines | |
| 7.4.363 | 1-(2-Ethoxycarbonylethyl)-5-nitroindazoline and 2-(2-ethoxycarbonylethyl)-5-nitroindazoline | In like manner to the preparation of 1-(methoxycarbonyl)methyl-5-nitroindazoline, 1-(2-ethoxycarbonylethyl)-5-nitroindazoline was prepared by alkylation of 5-nitroindazoline with ethyl 3-bromopropionate in presence of K2CO3. The 1-(2-ethoxycarbonylethyl)-5-nitroindazoline (43%) with high Rf value on the TLC in 30% EtOAcn-hexanes was collected by silica gel column chromatographic purification. 1H NMR (CDCl3): □ 8.70 (d, 1H, J= 1.7 Hz), 8.27 (dd, 1H, J= 2.3 and 8.8 Hz), 8.20 (d, 1H, J=1.7 Hz), 7.59 (d, 1H, J= 8.8 Hz), 4.70 (t, 2H, J= 6.4 Hz), 4.07 (qt, 2H, J= 7.0 Hz), 3.01 (t, 2H, J= 6.4 Hz), 1.16 (t, 3H, J= 7.0 Hz). The lower Rf value by-product, 2-(2-ethoxycarbonylethyl)-5-nitroindazoline was also collected by eluting the column with 50% EtOAcn-hexanes. 1H NMR (CDCl3): □ 8.71 (d. 1H. J= 2.0 Hz), 8.32 (s, 1H), 8.08 (app dd, 1H, J= 2.0 and 9.7 Hz), 7.73 (dd, 1H, J= 0.8 and 9.7 Hz), 4.77 (t, 2H, J= 6.4 Hz), 4.12 (qt, 2H, J= 7.0 Hz), 3.08 (t, 2H, J= 6.4 Hz), 1.22 (t, 3H, J= 7.0 Hz). |
| 7.4.364 | 5-Amino-1-(2-ethoxycarbonylethyl)inda zoline | In like manner to the reduction of diethyl 2-methyl-2-(3-nitrophenoxy)malonate, 1-(2-ethoxycarbonylethyl)-5-nitroindazoline was reduced to provide 5-amino-1-(2-ethoxycarbonylethyl)indazoline. 1H NMR (CDCl3): □ 7.78 (s, 1H), 7.30 (d, 1H, J= 8.8 Hz), 6.91 (d, 1H, J= 2.3 Hz), 6.87 (dd, 1H, J= 2.3 and 8.8 Hz), 4.59 (t, 2H, J= 6.4 Hz), 4.08 (qt, 2H, J= 7.0 Hz), 3.02 (br s, 2H), 2.92 (t, 2H, J= 7.0 Hz), 1.16 (t, 3H, J= 7.0 Hz). |
| 7.4.365 | 5-Amino-2-(2-ethoxycarbonylethyl)inda zoline | In like manner to the reduction of diethyl 2-methyl-2-(3-nitrophenoxy)malonate, the reduction of 2-(2-ethoxycarbonylethyl)-5-nitroindazoline provided 5-amino-2-(2-ethoxycarbonylethyl)indazoline. 1H NMR (CDCl3): □ 7.64 (s, 1H), 7.45 (dd, 1H, J= 0.9 and 9.1 Hz), 6.74 (dd, 1H, J= 2.0 and 9.1 Hz), 6.67(d, 1H, J= 2.0 Hz), 4.57 (t, 2H, J= 6.7 Hz), 4.05 (qt, 2H, J= 7.0 Hz), 3.28 (br s, 2H), 2.93 (t, 2H, J= 6.7 Hz), 1.16 (t, 3H, J= 7.0 Hz). |
| 7.4.366 | 1-methyl-6-nitroindazoline and 2-methyl-6-nitroindazoline | In like manner to the preparation of 1-(methoxycarbonylmethyl)-5-nitroindazoline, 6-nitroindazole was alkylated with methyl iodide in presence of K2CO3. The reaction mixture was diluted with water upon completion of the reaction. The solid formed was filtered, dried and chromatographed with 15% EtOAc:n-n-hexanes on silica gel to provide high Rf value product 1-methyl-6-nitroindazoline. 1H NMR (CDCl3): □ 8.32 (s, 1H), 8.10 (s, 1H), 8.01 (dd, 1H, J= 2.7 and 8.8 Hz), 7.83 (d, 1H, J= 8.8 Hz), 4.18 (s, 3H). The lower Rf value by-product 2-methyl-6-nitroindazoline was also collected by eluting the column with 30% EtOAc:n-hexanes. 1H NMR (CDCl3): □ 8.69 (d, 1H, J= 2.0 Hz), 8.03 (s, 1H), 7.90 (dd, 1H, J= 2.0 and 9.1 Hz), 7.75 (d, 1H, J= 9.1 Hz), 4.31 (s, 13H). |
| 7.4.367 | 6-Amino-1-methylindazoline | In like manner to the reduction of diethyl 2-methyl-2-(3-nitrophenoxy)malonate, 1-methyl-6-nitroindazoline was reduced to give 6-amino-1-methylindazoline. 1H NMR (CDCl3): □ 7.80 (s, 1H), 7.48 (dd, 1H, J= 0.6 and 8.2 Hz), 6.58 (dd, 1H, J= 1.8 and 8.2 Hz), 6.54 (d, 1H, J= 0.6 Hz), 3.94 (s, 3H), 3.5 (br s, 2H). |
| 7.4.368 | 6-Amino-2-methylindazoline | In like manner to the reduction of diethyl 2-methyl-2-(3-nitrophenoxy)malonate, 2-methyl-6-nitroindazoline was reduced to give 6-amino-2-methylindazoline. 1H NMR (CDCl3): □ 7.71 (s, 1H), 7.43 (d, 1H, J= 8.8 Hz), 6.79 (app d, 1H, J= 1.7 Hz), 6.58 (dd, 1H, J= 1.7 and 8.8 Hz), 4.11 (s, 3H), 3.31 (br s, 2H). |

### The 2,4-Pyrimidinediamine Compounds of the Invention Inhibit FcεRI Receptor-Mediated Degranulation

The ability of the 2,4-pyrimidinediamine compounds of the invention to inhibit IgE-induced degranulation was demonstrated in a variety of cellular assays with cultured human mast cells (CHMC) and/or mouse bone marrow derived cells (BMMC). Inhibition of degranulation was measured at both low and high cell density by quantifying the release of the granule specific factors tryptase, histamine and hexosaminidase. Inhibition of release and/or synthesis of lipid mediators was assessed by measuring the release of leukotriene LTC4 and inhibition of release and/or synthesis of cytokines was monitored by quantifying TNF-α, IL-6 and IL-13. Tryptase and hexosaminidase were quantified using fluorogenic substrates as described in their respective examples. Histamine, TNFα, IL-6, IL-13 and LTC4 were quantified using the following commercial ELISA kits: histamine (Immunotech #2015, Beckman Coulter), TNFα (Biosource #KHC3011), IL-6 (Biosource #KMC0061), IL-13 (Biosource #KHC0132) and LTC4 (Cayman Chemical #520211). The protocols of the various assays are provided below.

### Culturing of Human Mast and Basophil Cells

Human mast and basophil cells were cultured from CD34-negative progenitor cells as described below (see also the methods described in copending U.S. application Serial No. 10/053,355, filed November 8, 2001).

### Preparation of STEMPRO-34 Complete Medium

To prepare STEMPRO-34 complete medium ("CM"), 250 mL STEMPRO-34TM serum free medium ("SFM"; GibcoBRL, Catalog No. 10640) was added to a filter flask. To this was added 13 mL STEMPRO-34 Nutrient Supplement ("NS"; GibcoBRL, Catalog No. 10641) (prepared as described in more detail, below). The NS container was rinsed with approximately 10 mL SFM and the rinse added to the filter flask. Following addition of 5 mL L-glutamine (200 mM; Mediatech, Catalog No. MT 25-005-CI and 5 mL 100X penicillin/streptomycin ("pen-strep"; HyClone, Catalog No. SV30010), the volume was brought to 500 mL with SFM and the solution was filtered.
The most variable aspect of preparing the CM is the method by which the NS is thawed and mixed prior to addition to the SFM. The NS should be thawed in a 37° C water bath and swirled, not vortexed or shaken, until it is completely in solution. While swirling, take note whether there are any lipids that are not yet in solution. If lipids are present and the NS is not uniform in appearance, return it to the water bath and repeat the swirling process until it is uniform in appearance. Sometimes this component goes into solution immediately, sometimes after a couple of swirling cycles, and sometimes not at all. If, after a couple of hours, the NS is still not in solution, discard it and thaw a fresh unit. NS that appears non-uniform after thaw should not be used.

### Expansion of CD34+ Cells

A starting population of CD34-positive (CD34+) cells of relatively small number (1-5 x 106 cells) was expanded to a relatively large number of CD34-negative progenitor cells (about 2-4 x 109 cells) using the culture media and methods described below. The CD34+ cells (from a single donor) were obtained from Allcells (Berkeley, CA). Because there is a degree of variation in the quality and number of CD34+ cells that Allcells typically provides, the newly delivered cells were transferred to a 15 mL conical tube and brought up to 10 mL in CM prior to use. On day 0, a cell count was performed on the viable (phase-bright) cells and the cells were spun at 1200 rpm to pellet. The cells were resuspended to a density of 275,000 cells/mL with CM containing 200 ng/mL recombinant human Stem Cell Factor ("SCF"; Peprotech, Catalog No. 300-07) and 20 ng/mL human flt-3 ligand (Peprotech, Catalog No. 300-19) ("CM/SCF/flt-3 medium"). On about day 4 or 5, the density of the culture was checked by performing a cell count and the culture was diluted to a density of 275,000 cells/mL with fresh CM/SCF/flt-3 medium. On about day 7, the culture was transferred to a sterile tube and a cell count was performed. The cells were spun at 1200 rpm and resuspended to a density of 275,000 cells/mL with fresh CM/SCF/flt-3 medium.
This cycle was repeated, starting from day 0, a total of 3-5 times over the expansion period.
When the culture is large and being maintained in multiple flasks and is to be resuspended, the contents of all of the flasks are combined into a single container prior to performing a cell count. This ensures that an accurate cell count is achieved and provides for a degree of uniformity of treatment for the entire population. Each flask is checked separately for contamination under the microscope prior to combining to prevent contamination of the entire population.
Between days 17-24, the culture can begin to go into decline (i.e., approximately 5-10% of the total number of cells die) and fail to expand as rapidly as before. The cells are then monitored on a daily basis during this time, as complete failure of the culture can take place in as little as 24 hours. Once the decline has begun, the cells are counted, spun down at 850 rpm for 15 minutes, and resuspended at a density of 350,000 cells/mL in CM/SCF/flt-3 medium to induce one or two more divisions out of the culture. The cells are monitored daily to avoid failure of the culture. When greater than 15% cell death is evident in the progenitor cell culture and some debris is present in the culture, the CD34-negative progenitor cells are ready to be differentiated.

### Differentiation of CD34-Negative Progenitor Cells into Mucosal Mast Cells

A second phase is performed to convert the expanded CD34-negative progenitor cells into differentiated mucosal mast cells. These mucosal cultured human mast cells ("CHMC") are derived from CD34+ cells isolated from umbilical cord blood and treated to form a proliferated population of CD34-negative progenitor cells, as described above. To produce the CD43-negative progenitor cells, the resuspension cycle for the culture was the same as that described above, except that the culture was seeded at a density of 425,000 cells/mL and 15% additional media was added on about day four or five without performing a cell count. Also, the cytokine composition of the medium was modified such that it contained SCF (200 ng/mL) and recombinant human IL-6 (200 ng/mL; Peprotech, Catalog No. 200-06 reconstituted to 100 ug/mL in sterile 10 mM acetic acid) ("CM/SCF/IL-6 medium").
Phases I and II together span approximately 5 weeks. Some death and debris in the culture is evident during weeks 1-3 and there is a period during weeks 2-5 during which a small percentage of the culture is no longer in suspension, but is instead attached to the surface of the culture vessel. As during Phase I, when the culture is to be resuspended on day seven of each cycle, the contents of all flasks are combined into a single container prior to performing a cell count to ensure uniformity of the entire population. Each flask is checked separately for contamination under the microscope prior to combining to prevent contamination of the entire population.
When the flasks are combined, approximately 75% of the volume is transferred to the communal container, leaving behind about 10 mL or so in the flask. The flask containing the remaining volume was rapped sharply and laterally to dislodge the attached cells. The rapping was repeated at a right angle to the first rap to completely dislodge the cells.
The flask was leaned at a 45 degree angle for a couple of minutes before the remaining volume was transferred to the counting vessel. The cells were spun at 950 rpm for 15 min prior to seeding at 35-50 mL per flask (at a density of 425,000 cells/mL). Differentiation of CD34-Negative Progenitor Cells into Connective Tissue-Type Mast Cells

A proliferated population of CD34-negative progenitor cells is prepared as above and treated to form a tryptase/chymase positive (connective tissue) phenotype. The methods are performed as described above for mucosal mast cells, but with the substitution of IL-4 for IL-6 in the culture medium. The cells obtained are typical of connective tissue mast cells.

### Differentiation of CD34-Negative Progenitor Cells into Basophil Cells

A proliferated population of CD34-negative progenitor cells is prepared as described in Section 7.5.1.3, above, and used to form a proliferated population of basophil cells. The CD34-negative cells are treated as described for mucosal mast cells, but with the substitution of IL-3 (at 20-50 ng/mL) for IL-6 in the culture medium.

### CHMC Low Cell Density IgE Activation: Tryptase and LTC4 Assays

To duplicate 96-well U-bottom plates (Costar 3799) add 65 ul of compound dilutions or control samples that have been prepared in MT [137 mM NaCl, 2.7 mM KCI, 1.8 mM CaCl2, 1.0 mM MgCl2, 5.6 mM Glucose, 20 mM Hepes (pH 7.4), 0.1% Bovine Serum Albumin, (Sigma A4503)] containing 2% MeOH and 1% DMSO. Pellet CHMC cells (980 rpm, 10 min) and resuspend in pre-warmed MT. Add 65 ul of cells to each 96-well plate. Depending on the degranulation activity for each particular CHMC donor, load 1000-1500 cells/well. Mix four times followed by a 1 hr incubation at 37oC. During the 1 hr incubation, prepare 6X anti-IgE solution [rabbit anti-human IgE (1 mg/ml, Bethyl Laboratories A80-109A) diluted 1:167 in MT buffer]. Stimulate cells by adding 25 ul of 6X anti-IgE solution to the appropriate plates. Add 25 ul MT to un-stimulated control wells. Mix twice following addition of the anti-IgE. Incubate at 37oC for 30 minutes. During the 30 minute incubation, dilute the 20 mM tryptase substrate stock solution [(Z-Ala-Lys-Arg-AMC.2TFA; Enzyme Systems Products, #AMC-246)] 1:2000 in tryptase assay buffer [0.1 M Hepes (pH 7.5), 10 % w/v Glycerol, 10 uM Heparin (Sigma H-4898) 0.01% NaN3]. Spin plates at 1000 rpm for 10 min to pellet cells. Transfer 25 ul of supernatant to a 96-well black bottom plate and add 100 ul of freshly diluted tryptase substrate solution to each well. Incubate plates at room temperature for 30 min. Read the optical density of the plates at 355nm/460nm on a spectrophotometric plate reader.
Leukotriene C4 (LTC4) is also quantified using an ELISA kit on appropriately diluted supernatant samples (determined empirically for each donor cell population so that the sample measurement falls within the standard curve) following the supplier's instructions.
CHMC High Cell Density IgE Activation: Degranulation (Tryptase, Histamine), Leukotriene (LTC4), and Cytokine (TNFalpha, IL-13) Assays
Cultured human mast cells (CHMC) are sensitized for 5 days with IL-4 (20 ng/ml), SCF (200 ng/ml), IL-6 (200 ng/ml), and Human IgE (CP 1035K from Cortx Biochem, 100-500ng/ml depending on generation) in CM medium After sensitizing, cells are counted, pelleted (1000 rpm, 5-10 minutes), and resuspended at 1-2 x106 cells/ml in MT buffer. Add 100 ul of cell suspension to each well and 100 ul of compound dilutions. The final vehicle concentration is 0.5% DMSO. Incubate at 37°C (5% CO2) for I hour. After 1hour of compound treatment, stimulate cells with 6X anti-IgE. Mix wells with the cells and allow plates to incubate at 37°C (5% CO2) for one hour. After 1 hour incubation, pellet cells (10 minutes, 1000 RPM) and collect 200 ul per well of the supernatant, being careful not to disturb pellet. Place the supernatant plate on ice. During the 7-hour step (see next) perform tryptase assay on supernatant that had been diluted 1:500. Resuspend cell pellet in 240 ul of CM media containing 0.5% DMSO and corresponding concentration of compound. Incubate CHMC cells for 7 hours at 37°C (5% CO2). After incubation, pellet cells (1000 RPM, 10 minutes) and collect 225 ul per well and place in -80°C until ready to perform ELISAS. ELISAS are performed on appropriately diluted samples (determined empirically for each donor cell population so that the sample measurement falls within the standard curve) following the supplier's instructions.
BMMC High Cell Density IgE Activation: Degranulation (Hexosiminidase, Histamine), Leukotriene (LTC4), and Cytokine (TNFalpha, IL-6) Assays

### Preparation of WEHI-Conditioned Medium

WEHI-conditioned medium was obtained by growing murine myelomonocytic WEHI-3B cells (American Type Culture Collection, Rockville, MD) in Iscove's Modified Eagles Media (Mediatech, Hernandon, VA) supplemented with 10% heat-inactivated fetal bovine serum (FBS; JRH Biosciences, Kansas City, MO), 50 µM 2-mercaptoethanol (Sigma, St. Louis, MO) and 100 IU/mL penicillin-steptomycin (Mediatech) in a humidified 37°C, 5% CO2/95% air incubator. An initial cell suspension was seeded at 200,000 cells/mL and then split 1:4 every 3-4 days over a period of two weeks. Cell-free supernatants were harvested, aliquoted and stored at -80°C until needed.

### Preparation of BMMC Medium

BMMC media consists of 20% WEHI-conditioned media, 10% heat-inactivated FBS (JHR Biosciences), 25 mM HEPES, pH7.4 (Sigma), 2mM L-glutamine (Mediatech), 0.1 mM non-essential amino acids (Mediatech), 1 mM sodium pyruvate (Mediatech), 50 µM 2-mercaptoethanol (Sigma) and 100 IU/mL penicillin-streptomycin (Mediatech) in RPMI 1640 media (Mediatech). To prepare the BMMC Media, all components are added to a sterile IL filter unit and filtered through a 0.2 µm filter prior to use.

### Protocol

Bone marrow derived mast cells (BMMC) are sensitized overnight with murine SCF (20 ng/ml) and monoclonal anti-DNP (10 ng/ml, Clone SPE-7, Sigma # D-8406) in BMMC media at a cell density of 666 x103 cells/ml. After sensitizing, cells are counted, pelleted (1000 rpm, 5-10 minutes), and resuspended at 1-3 x106 cells/ml in MT buffer. Add 100 ul of cell suspension to each well and 100 ul of compound dilutions. The final vehicle concentration is 0.5% DMSO. Incubate at 37°C (5% CO2) for 1 hour. After 1hour of compound treatment, stimulate cells with 6X stimulus (60 ng/ml DNP-BSA). Mix wells with the cells and allow plates to incubate at 37°C (5% CO2) for one hour. After 1hour incubation, pellet cells (10 minutes, 1000 RPM) and collect 200 ul per well of the supernatant, being careful not to disturb pellet, and transfer to a clean tube or 96-well plate. Place the supernatant plate on ice. During the 4-5 hour step (see next) perform the hexosiminidase assay. Resuspend cell pellet in 240 ul WEI-conditioned media containing 0.5% DMSO and corresponding concentration of compound. Incubate BMMC cells for 4-5 hours at 37°C (5% CO2). After incubation, pellet cells (1000 RPM, 10 minutes) and collect 225 ul per well and place in -80°C until ready to perform ELISAS. ELISAS are performed on appropriately diluted samples (determined empirically for each donor cell population so that the sample measurement falls within the standard curve) following the supplier's instructions. Hexosaminidase assay: In a solid black 96-well assay plate, add 50 uL hexosaminidase substrate (4-methylumbelliferyl-N-acetyl-β-D-glucosaminide; 2mM) to each well. Add 50 uL of BMMC cell supernatant (see above) to the hexoseaminidase substrate, place at 37°C for 30 minutes and read the plate at 5, 10, 15, and 30 minutes on a spectrophotometer.

### Basophil IgE or Dustmite Activation: Histamine Release Assay

The basophil activation assay was carried out using whole human peripheral blood from donors allergic to dust mites with the majority of the red blood cells removed by dextran sedimentation. Human peripheral blood was mixed 1:1 with 3% dextran T500 and RBCs were allowed to settle for 20-25min. The upper fraction was diluted with 3 volumes of D-PBS and cells were spun down for 10 min at 1500 rpm, RT. Supernatant was aspirated and cells were washed in an equal volume MT-buffer. Finally, cells were resuspended in MT-buffer containing 0.5% DMSO in the original blood volume. 80 uL cells were mixed with 20 uL compound in the presence of 0.5% DMSO, in triplicate, in a V-bottom 96-well tissue culture plate. A dose range of 8 compound concentrations was tested resulting in a 10-point dose response curve including maximum (stimulated) and minimum (unstimulated) response. Cells were incubated with compound for I hour at 37°C, 5% CO2 after which 20 uL of 6x stimulus [1 ug/mL anti-IgE (Bethyl Laboratories) 667 au/mL house dustmite (Antigen Laboratories)] was added. The cells were stimulated for 30 minutes at 37°C, 5% CO2. The plate was spun for 10 min at 1500 rpm at room temperature and 80 uL the supernatant was harvested for histamine content analysis using the histamine ELISA kit supplied by Immunotech. The ELISA was performed according to supplier's instructions.

### Results

The results of low density CHMC assays (Section 7.5.2), the high density BMMC assays (Section 7.5.4) and the basophil assays (Section 7.5.5) are provided in TABLE 1. The results of the high density CHMC assays (Section 7.5.3) are provided in TABLE 2. In TABLES 1 and 2, all reported values are IC50s (in µM). A value of "9999" indicates an IC50> 10µM, with no measurable activity at a 10µM concentration. Most compounds tested had IC50s of less than 10µM, with many exhibiting IC50s in the sub-micromolar range.
The 2,4-Pyrimidinediamine Compounds Inhibit FcγRI Receptor-Mediated Degranulation The ability of the 2,4-pyrimidinediamine compounds of the invention to inhibit FcγRI-mediated degranulation was demonstrated with Compound R940347 in assays similar to those described in Section 7.5, with the exception that the cells were not primed with IgE and were activated with rabbit anti-human IgG Fab fragment (Bethyl Laboratories, Catalog No. A80-105). All of the compounds tested exhibited IC50s in the sub micromolar range.

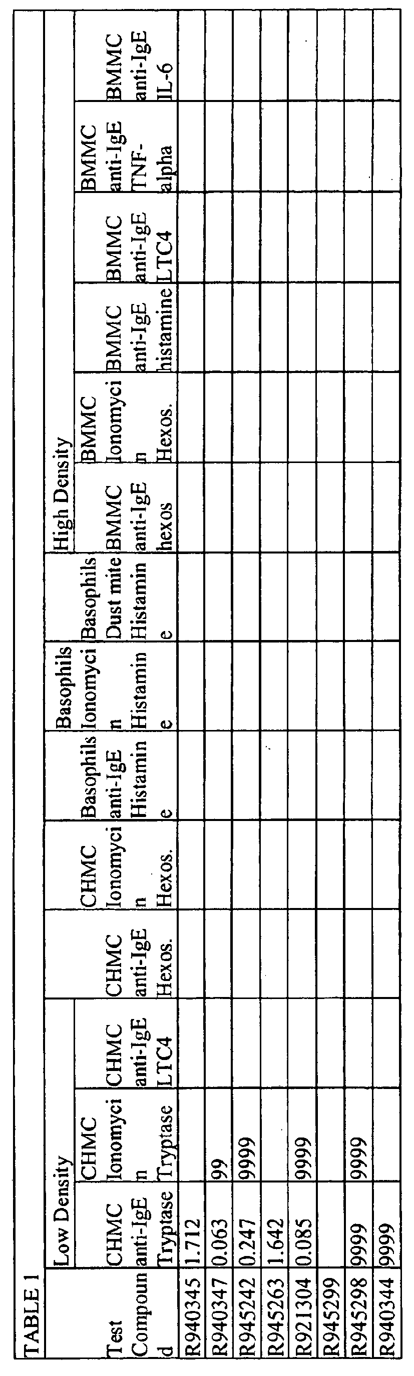

| TABLE 1B | | | | | |
|---|---|---|---|---|---|
| Compound | CHMC anti-IgE Tryptase | CHMC Ionomycin Tryptase | BMMC anti-IgE Hexos. | BMMC anti-IgE TNF-alpha | BMMC anti-IgE IL-6 |
| R940371 | 0.316 | | | | |
| R940372 | 0.094 | | | | |
| R940373 | 8888 | | | | |
| R940380 | 0.042 | | | | |
| R940381 | 8888 | | | | |
| R940382 | 0.104 | | | | |
| R940384 | 1.32 | | | | |
| R940386 | 3.42 | | | | |
| R940387 | 1.19 | | | | |
| R940389 | 0.06 | | | | |
| R940390 | 9999 | 9999 | | | |
| R940391 | 0.261 | | | | |
| R940392 | 0.145 | | | | |
| R940394 | 16.5353 | | | | |
| R940395 | 9999 | | | | |
| R945389 | 0.0748 | 9999 | | | |
| R945390 | 0.118 | 9999 | | | |
| R945391 | 0.094 | 9999 | | | |
| R945392 | 0.085 | 9999 | | | |
| R945398 | 7 | | | | |
| R945399 | 0.163 | 9999 | | | |
| R945400 | 9999 | | | | |
| R945401 | 8888 | 9999 | | | |
| R945402 | 0.112 | | | | |
| R945404 | 0.103 | | | | |
| R945405 | 0.131 | | | | |
| R945406 | 8888 | | | | |
| R945407 | 8888 | | | | |
| R945408 | 9999 | | | | |
| R945409 | 9999 | | | | |
| R945410 | 9999 | | | | |
| R945412 | 0.095 | | | | |
| R945413 | 1.698 | | | | |
| R945416 | 0.053 | | | | |
| R945417 | 2.52082 | 9999 | | | |
| R945418 | 8888 | 9999 | | | |
| R945419 | 0.125 | | | | |
| R945420 | 0.436 | | | | |
| R945421 | 0.371 | | | | |
| R945422 | 0.092 | | | | |
| R945423 | 0.145 | | | | |
| R945424 | 0.188 | | | | |
| R945426 | 0.256 | | | | |
| R945427 | 0.279 | | | | |
| R945432 | 0.049 | | | | |
| R945433 | 0.276 | | | | |
| R945434 | 8888 | | | | |
| R945439 | 8888 | | | | |
| R945440 | 8888 | | | | |
| R945443 | 0.081 | 9999 | | | |
| R945444 | 0.043 | 9999 | | | |
| R945454 | 20.6 | 9999 | | | |
| R945455 | 8888 | 9999 | | | |
| R945456 | 8888 | | | | |
| R945458 | 8888 | | | | |

The 2,4-Pyrimidinediamine Compounds of the Invention Selectively Inhibit the Upstream IgE

### Receptor Cascade

To confirm that many of the 2,4-pyrimidinediamine compounds of the invention exert their inhibitory activity by blocking or inhibiting the early IgE receptor signal transduction cascade, several of the compounds were tested in cellular assays for ionomycin-induced degranulation, as described below.

### CHMC Low Cell Density Ionomycin Activation: Tryptase Assay

Assays for ionomycin-induced mast cell degranulation were carried out as described for the CHMC Low Density IgE Activation assays (Section 7.5.2, supra), with the exception that during the 1 hour incubation, 6X ionomycin solution [5mM ionomycin (Signma 1-0634) in MeOH (stock) diluted 1:416.7 in MT buffer (2 µM final)] was prepared and cells were stimulated by adding 25 µl of the 6X ionomycin solution to the appropriate plates.

### Basophil Ionomycin Activation: Histamine Release Assay

Assays for ionomycin-induced basophil cell degranulation were carried out as described for the Basophil IgE or Dustmite Activation Assay (Section 7.5.5, supra), with the exception that following incubation with compound, cells were stimulated with 20 µl of 2 µM ionomycin.

### Results

The results of the ionomycin-induced degranulation assays, reported as IC50 values (in µM) are provided in TABLE 1, supra. Of the active compounds tested (i.e., those that inhibit IgE-induced degranulation), the vast majority do not inhibit ionomycin-induced degranulation, confirming that these active compounds selectively inhibit the early (or upstream) IgE receptor signal transduction cascade.

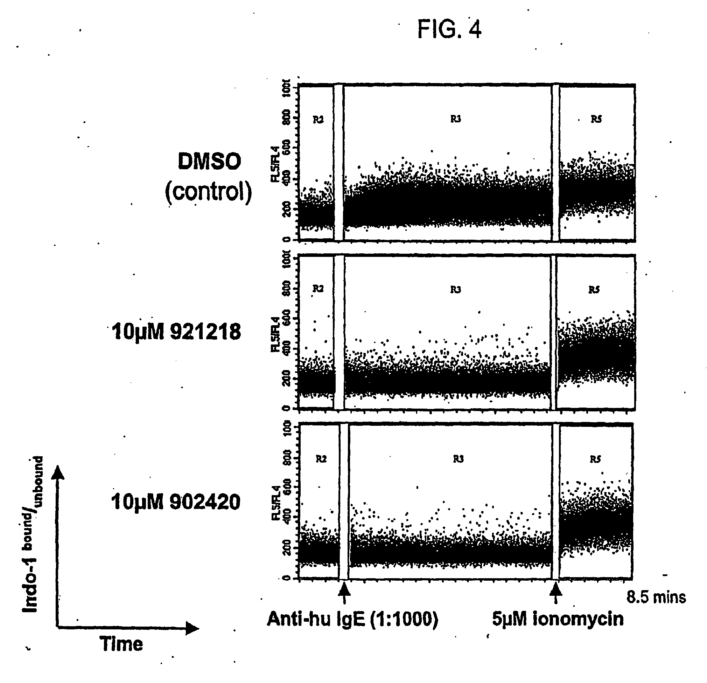

These results were confirmed for certain compounds by measuring anti-IgE-induced and ionomycin-induced calcium ion flux in CHMC cells. In these Ca2+ flux tests, 10 µM R921218 and 10 µM R902420 inhibited anti-IgE-induced Ca2+ flux, but had no effect on ionomycin-induced Ca2+ flux (See FIG. 4).

### The Inhibitory Effect of the 2,4-Pyrimidinediamine Compounds of the Invention is Immediate

To test the immediacy of their inhibitory effect, certain 2,4-pyrimidinediamines of the invention were added simultaneously with anti-IgE antibody activator in the cellular assays described above. All compounds tested blocked IgE-induced degranulation of CHMC cells to the same extent as observed when the compounds were pre-incubated with CHMC cells for 10 or 30 min. prior to receptor cross-linking.

### Kinetics of Pharmacological Activity In vitro

Compounds R921218, R921302, R921219, R926240, R940277, R926742, R926495, R909243 and R926782 were tested in washout experiments. In the experiments, CHMC cells were either activated immediately with anti-IgE antibody in the presence of 1.25 µM compound (time zero), or the compound was washed out followed by activation with anti-IgE antibody at 30, 60 or 120 min. The inhibitory activity of these compounds was greatly diminished 30 min. after compound removal, indicating that constant exposure of mast cells to these compounds is required for maximal inhibition of degranulation The other compounds tested yielded similar results.

### Toxicity: T- and B-Cells

The ability of the compounds of the invention to exert their inhibitory activity without being toxic to cells of the immune system was demonstrated in cellular assays with B- and T-cells. The protocols for the assays are provided below.

### Jurkat (T-Cell) Toxicity

Dilute Jurkat cells to 2x105 cells/ml in complete RPMI (10% heat-inactivated fetal bovine serum) media and incubate at 37°C, 5% CO2 for 18 hours. Add 65 ul cells at 7.7 x 105 cells/ml to a 96-well V-bottom plate (TC-treated, Costar) containing 65 ul 2X compound (final vehicle concentration is 0.5% DMSO, 1.5% MeOH). Mix, incubate plates for 18-24 hr at 37°C, 5% CO2.

### Toxicity was assessed by flow cytometric analysis of cellular light scatter

### BJAB (B-Cell) Toxicity

The B-cell line BJAB was cultured in log phase in RPMI1640 + 10% heat-inactivated fetal bovine serum, 1 x L-glutamine, 1 x penicillin, I x streptavidin and I x beta-mercaptoethanol at 37°C, 5% CO2. First, BJABs were harvested, spun and resuspended in culture medium to a concentration of 7.7x105 cells/mL. 65uL cells were mixed with 65 uL compound, in duplicate and in the presence of 0.1% DMSO in a V-bottomed 96-well tissue culture plate. Cells were incubated with compound at various dilutions at 37°C, 5% CO2. Toxicity was assessed by flow cytometric analysis of cellular light scatter.

### Toxicity: Cell Titer Glo Assay

Seed 50 µl cells (1x106/ml) into each well containing 50 µl compound. The final vehicle concentration is 0.5% DMSO, 1.5% MeOH. Shake plates for 1 minute to mix cells and compound. Incubate plates at 37°C (5% CO2) for 18 hours. Next day, harvest 50 µl cells from each well, add to 50 µl Cell Titer Glo reagent (Invitrogen). Shake plates for 1 minute. Read on luminometer.

### Results

The results of the T- and B-cell toxicity assays, reported as IC50 values (in µM), are presented in TABLE 2, supra. With a few exceptions (see TABLE 1), all compounds tested were non-toxic to both B- and T-cells at effective inhibitory concentrations. Assays performed with primary B-cells yielded similar results.

### The Compounds Are Effective In The Treatment of Asthma

The efficacy of compound R921304 in the treatment of asthma was also demonstrated in a mouse model of allergic asthma.

### Study protocol

Mice are sensitized to ovalbumin (chicken protein) in the presence of an adjuvant (Alum) by the intraperitoneal route on day 0 and day 7. One week later, mice are challenged intranasally with ovalbumin on Days 14, 15 and 16 (more stringent model) or on Day 14 (less stringent model). This sensitization and challenge regimen leads to airway hyperresponsiveness and inflammation in the lungs, which are two dominant characteristics of human allergic asthma. In the mouse model, the in vivo airway responses are measured using a whole body plethysmograph which determines the PENH (enhanced Pause, Buxco Electronics). The PENH is a dimensionless value comprised of the peak inspiratory flow (PIF), peak expiratory flow (PEF), time of inspiration, time of expiration and relaxation time, and is considered a validated parameter of airway responsiveness. Responses to allergen challenge (OVA) are compared with animals challenged with saline only. Twenty-four hours after challenge, mice are exposed to increasing doses of methacholine (muscarinic receptor agonist) which results in smooth muscle contraction. The ovalbumin-challenged mice demonstrate a significant airway hyperresponsiveness to methacholine when compared to the saline challenged mice. In addition, a cellular infiltrate in the airway is observed in ovalbumin challenged mice when compared with the saline challenged mice. This cellular infiltrate is mainly characterized by eosinophils, but a smaller influx of neutrophils and mononuclear cells is also present. The use of this model for the evaluation of small molecule inhibitors of mast cell degranulation has been validated is several ways. First, using mast cell deficient mice (W/Wv) it has been shown that the ovalbumin-induced responses are dependent upon the presence of mast cells. In the mast cell deficient mice, ovalbumin sensitization and challenge did not result in airway hyperresponsiveness and eosinophil influx. Second, the mast cell stabilizer, Cromolyn, was able to block the ovalbumin-induced airway hyperresponsiveness and inflammation (data not shown). The use of this model to evaluate compounds for the treatment of asthmatic responses that may be mediated by mechanisms other than mast cell stablization, is further supported by the inhibitory effect of the steroids, dexamethasone and budesonide, on methacoline-induced bronchocontriction.

### Results

The efficacy of R921304 was evaluated by intranasal administration on 10 consecutive days, from Day 7 through Day 16, at a dose level of 20 mg/kg, with the last 3 doses administered 30 minutes prior to either saline or ovalbumin challenge. R921304 was able to inhibit the ovalbumin-induced airway hyperresponsiveness to methacholine when compared to the vehicle treated mice. These results clearly demonstrate that and R921304 are efficacious in inhibiting the airway responses in a mouse model of allergic asthma.

### 2,4-Pyrimidinediamine Compounds Inhibit Phosphorylation of Proteins Downstream of Syk kinase in Activated Mast Cells

The inhibitory effect of the 2,4-pyrimidinediamine compounds on the phosphorylation of proteins downstream of Syk kinase was tested with compoundsR921304 in IgE receptor-activiated BMMC cells.

For the assay, BMMC cells were incubated in the presence of varying concentrations of test compound (0.08 µM, 0.4 µM, 2 µM and 10 µM) for I hr at 37°C. The cells were then stimulated with anti-IgE antibody as previously described. After 10 min, the cells were lysed and the cellular proteins separated by electrophoresis (SDS PAGE).

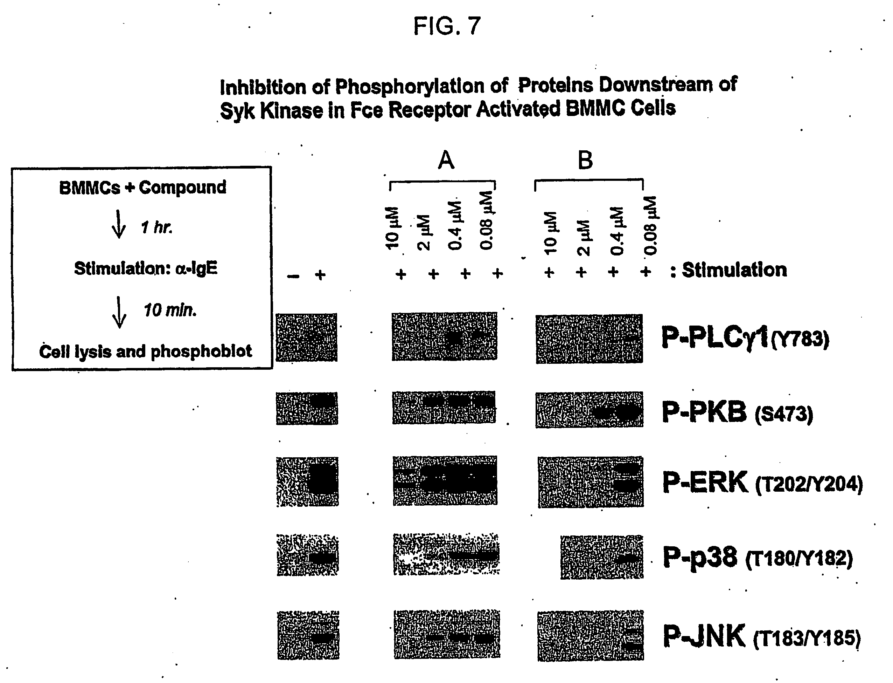

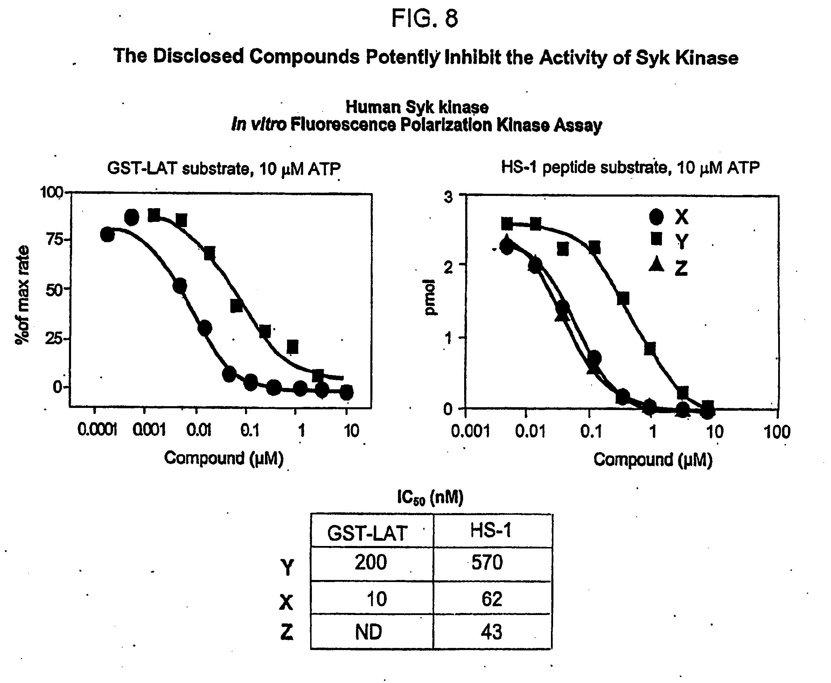

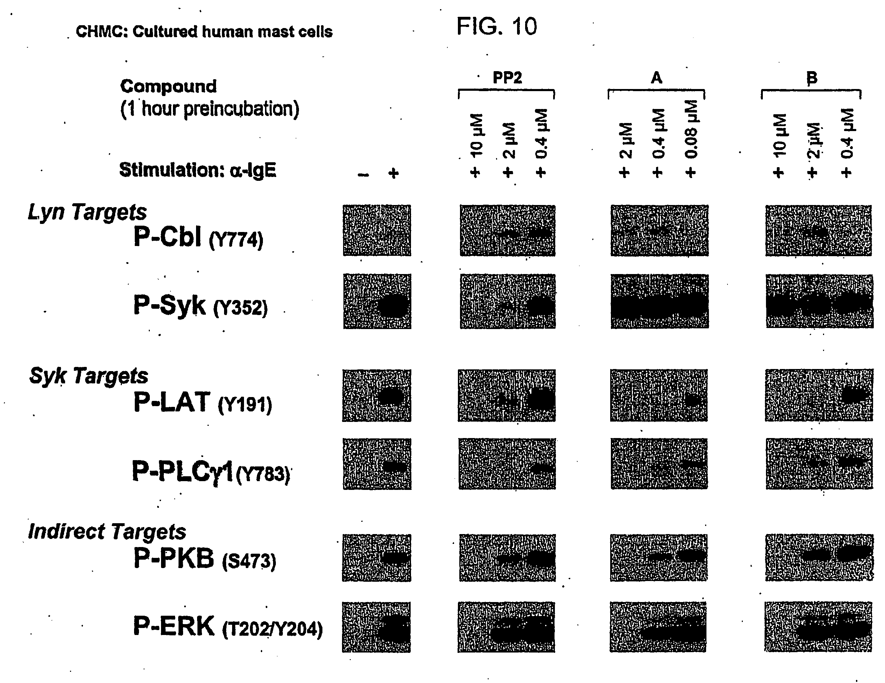

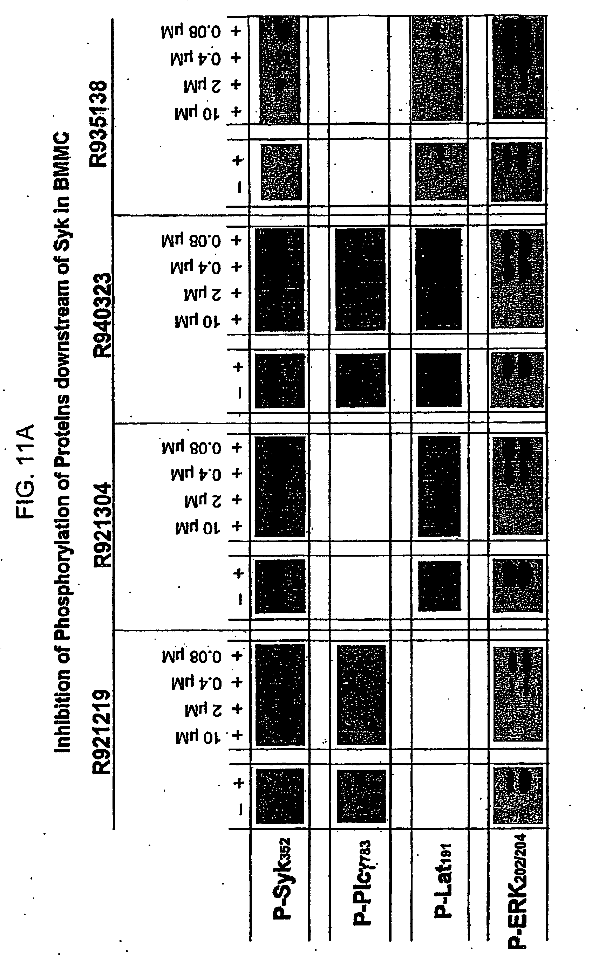

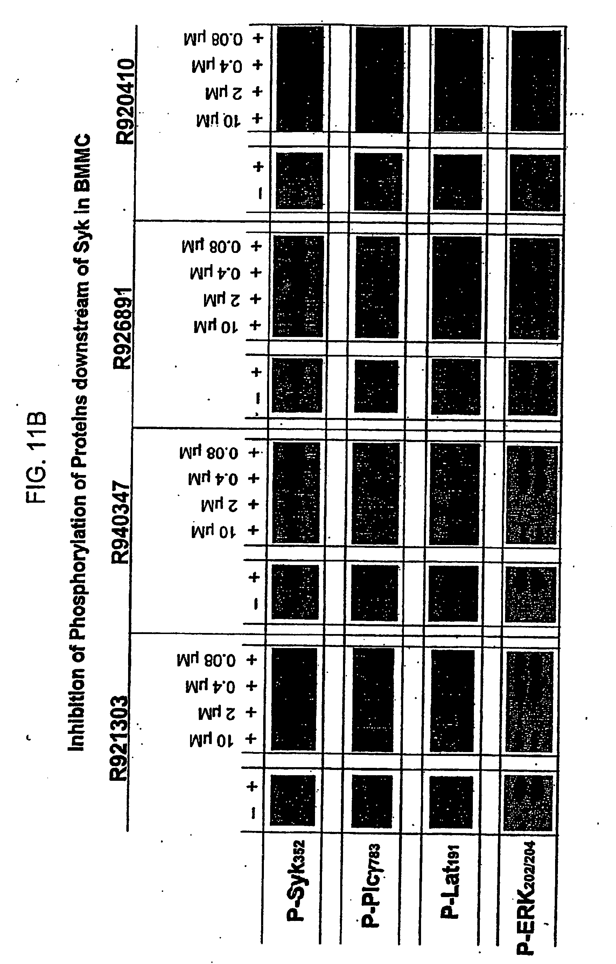

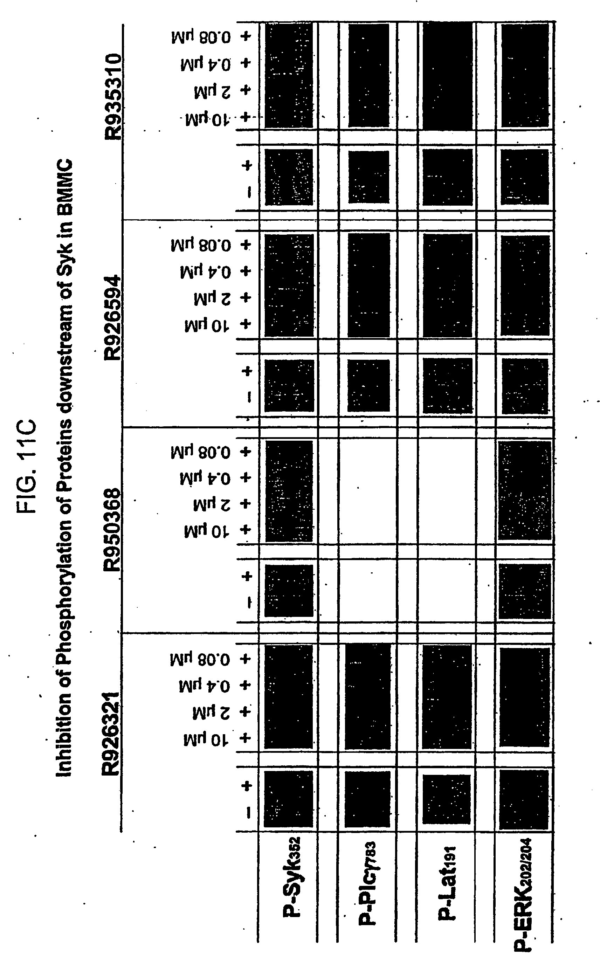

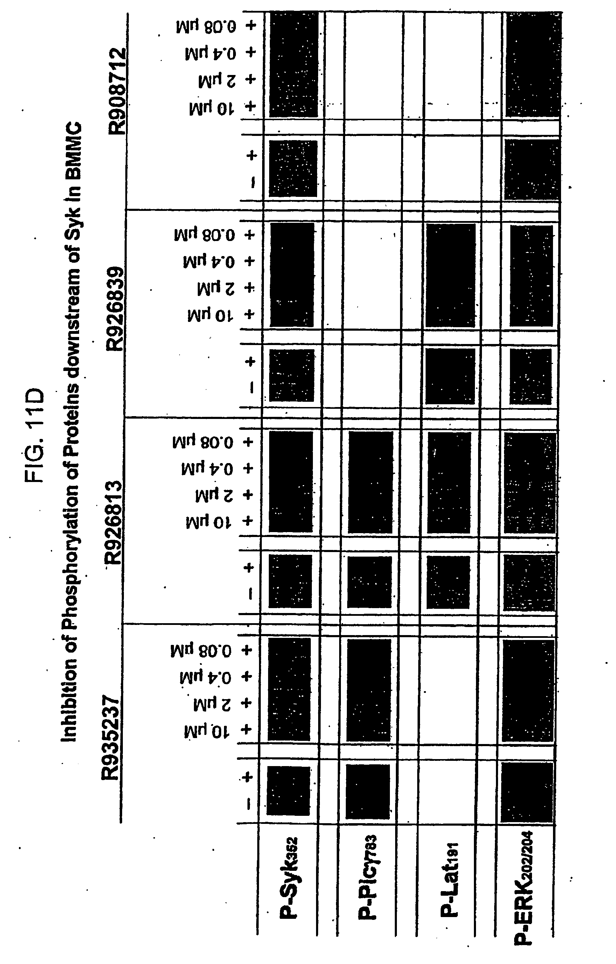

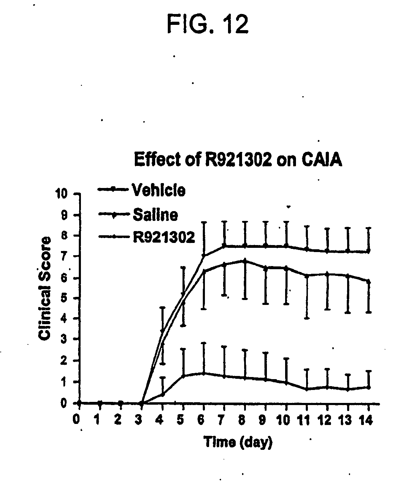

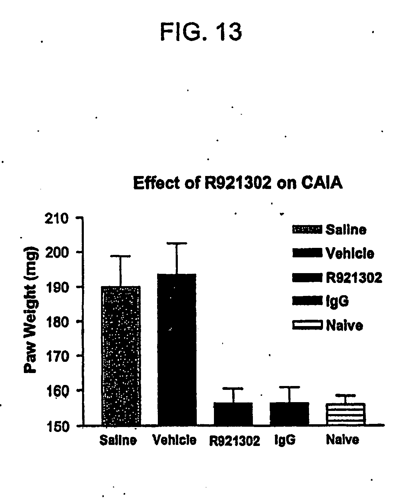

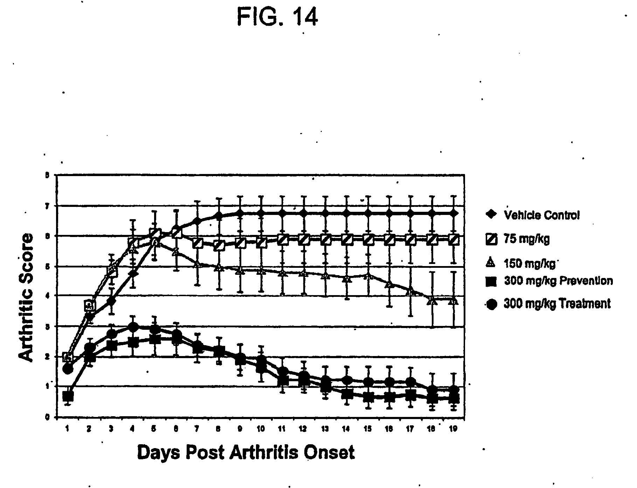

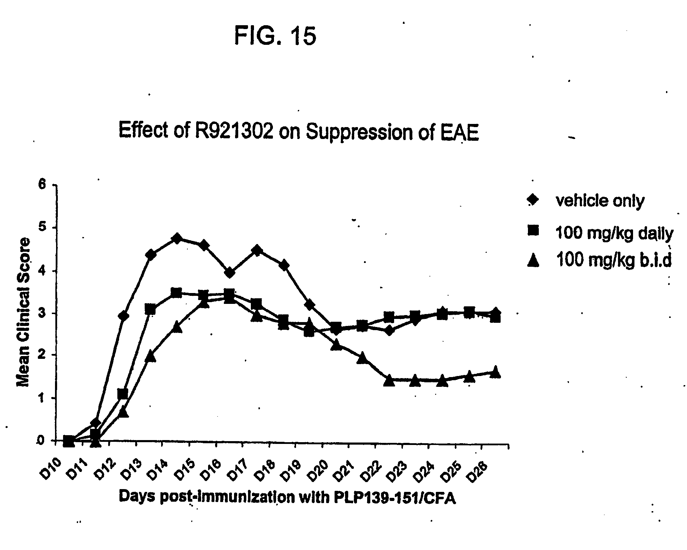

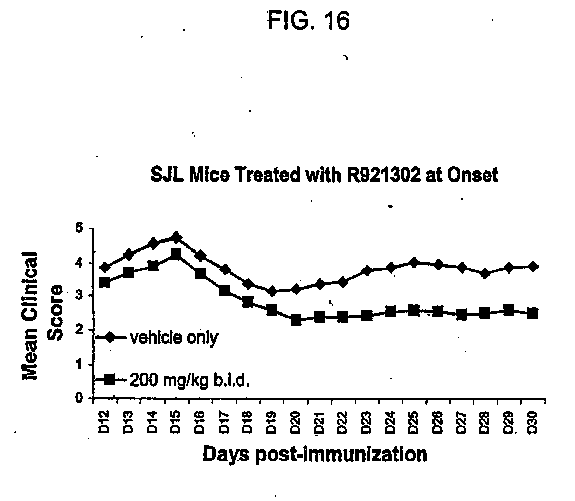

Following electrophoresis, the phosphorylation of the proteins indicated in FIGS. 7, 10 and 11A-D were assessed by immunoblot. Antibodies were purchased from Cell Signaling Technology, Beverley, MA.

Referring to FIGS. 7, 10 and 11A-D, the indicated compounds tested inhibited phosphorylation of proteins downstream of Syk, but not upstream of Syk, in the IgE receptor signaling cascade, confirming both that the compounds inhibit upstream IgE induced degranulation, and that the compounds exhert their inhibitory activity by inhibiting Syk kinase.

### 2,4-Pyrimidinediamine Compounds Inhibit Syk Kinase in Biochemical Assays

Several 2,4-pyrimidinediamine compounds were tested for the ability to inhibit Syk kinase catalyzed phosphorylation of a peptide substrate in a biochemical fluorescenced polarization assay with isolated Syk kinase. In this experiment, Compounds were diluted to 1% DMSO in kinase buffer (20 mM HEPES, pH 7.4, 5 mM MgCl2, 2 mM MnCl2, 1 mM DTT, 0.1 mg/mL acetylated Bovine Gamma Globulin). Compound in 1% DMSO (0.2% DMSO final) was mixed with ATP/substrate solution at room temperature. Syk kinase (Upstate, Lake Placid NY) was added to a final reaction volume of 20 uL, and the reaction was incubated for 30 minutes at room temperature. Final enzyme reaction conditions were 20 mM HEPES, pH 7.4, 5 mM MgCl2, 2 mM MnCl2, 1 mM DTT, 0.1 mg/mL acetylated Bovine Gamma Globulin, 0.125 ng Syk, 4 uM ATP, 2.5 uM peptide substrate (biotin-EQEDEPEGDYEEVLE-CONH2, SynPep Corporation). EDTA (10 mM final)/anti-phosphotyrosine antibody (1X final)/fluorescent phosphopeptide tracer (0.5X final) was added in FP Dilution Buffer to stop the reaction for a total volume of 40 uL according to manufacturer's instructions (PanVera Corporation) The plate was incubated for 30 minutes in the dark at room temperature. Plates were read on a Polarion fluorescence polarization plate reader (Tecan). Data were converted to amount of phosphopeptide present using a calibration curve generated by competition with the phosphopeptide competitor provided in the Tyrosine Kinase Assay Kit, Green (PanVera Corporation).

The results of the assay are shown in TABLE 6, below:

| Table 6 | | | | | |
|---|---|---|---|---|---|
| Compound | SYK Kinase IC50 (in µM) | Compound | SYK Kinase IC50 (in µM) | Compound | SYK Kinase IC50 (in µM) |
| R945398 | 0.182 | R945419 | 0.127 | R940380 | 0.029 |
| R945399 | 0.166 | R945422 | 0.087 | R940381 | 4999.846 |
| R945400 | 17.925 | R945423 | 0.273 | R940382 | 0.144 |
| R945401 | 0.007 | R945424 | 0.665 | R940384 | 9999 |
| R945402 | 0.418 | R945426 | 0.301 | R940386 | 19.49 |
| R945402 | 0.418 | R945427 | 0.479 | R940387 | 9999 |
| R945404 | 9999 | R945432 | 4444.247 | R940389 | 0.053 |
| R945405 | 0.168 | R945433 | 0.431 | R940390 | 9999 |
| R945407 | 9999 | R945434 | 9999 | R921304 | 0.017 |
| R945412 | 0.308 | R940347 | 0.038 | R945299 | 0.022 |
| R945413 | 9999 | R940371 | 3.643 | R945389 | 0.035 |
| R945416 | 0.515 | R940372 | 0.253 | R945390 | 0.009 |
| R945417 | 9999 | R940380 | 0.029 | R945391 | 0.01 |
| R945418 | 9999 | R940373 | 9999 | R945392 | 0.014 |

These data demonstrate that all of the compounds tested inhibit Syk kinase phosphorylation with IC50s in the submicromolar range. All compounds tested inhibit Syk kinase phosphorylation with IC50s in the micromolar range.

### The Compounds Are Effective for the Treatment of Autoimmunity

The in vivo efficacy of certain 2,4-pyrimidinediamine compounds towards autoimmune diseases was evaluated in the reverse passive Arthus reaction, an acute model of antigen-antibody mediated tissue injury, and in several disease models of autoimmunity and inflammation. These models are similar in that antibody to a specific antigen mediates immune complex-triggered (IC-triggered) inflammatory disease and subsequent tissue destruction. IC deposition at specific anatomic sites (central nervous system (CNS) for experimental autoimmune encephalomyelitis (EAE) and synovium for collagen-induced arthritis (CIA)) leads to activation of cells expressing surface FcγR and FcεR, notably mast cells, macrophages, and neutrophils, which results in cytokine release, and neutrophil chemotaxis. Activation of the inflammatory response is responsible for downstream effector responses, including edema, hemorrhage, neutrophil infiltration, and release of pro-inflammatory mediators. The consequences of these IC-triggered events are difficult to identify in autoimmune disorders; nonetheless, many investigators have demonstrated that inhibition of the FcγR signaling pathway in these animal models has resulted in a significant reduction in disease onset and severity.

### The Compounds Are Effective In Mouse Arthus Reaction

The in vivo efficacy of compoundR940347 to inhibit the IC-triggered inflammatory cascade was demonstrated in a mouse model of Reverse Passive Arthus Reaction (RPA reaction).

### Model

Immune complex (IC)-mediated acute inflammatory tissue injury is implicated in a variety of human autoimmune diseases, including vasculitis syndrome, sick serum syndrome, systemic lupus erythematosus (SLE), rheumatoid arthritis, Goodpasture's syndrome, and glomerulonephritis. The classical experimental model for IC-mediated tissue injury is the reverse passive Arthus reaction. The RPA reaction model is a convenient in vivo method to study localized inflammation, induced by ICs, without systemic effects. Intradermal injection of antibodies (Abs) specific to chicken egg albumin (rabbit anti-OVA IgG), followed by intravenous (IV) injection of antigens (Ags), specifically chicken egg albumin (ovalbumin, OVA), causes perivascular deposition of ICs and a rapid inflammatory response characterized by edema, neutrophil infiltration and hemorrhage at the injection sites. Aspects of the mouse RPA reaction model resemble the inflammatory response of patients with rheumatoid arthritis, SLE and glomerulonephritis.

### Study Protocol

In this model system, test compounds are administered at several timepoints prior to administration of Abs and Ags. A solution of rabbit anti-OVA IgG (50µg in 25µl/mouse) is injected intradermally, and immediately following is an intravenous injection of chicken egg albumin (20 mg/kg of body weight) in a solution containing 1% Evans blue dye. The degree of edema and hemorrhage is measured in the dorsal skin of C57BL/6 mice using the Evan's Blue dye as an indicator of local tissue damage. Purified polyclonal rabbit IgG is used as a control.

Pretreatment time, in which the test compounds are administered prior to Ab/Ag challenge, depends on the pharmacokinetic (PK) properties of each individual compound. Four hours after induction of Arthus reaction, mice are euthanized, and tissues are harvested for assessment of edema. This model system allows us to rapidly screen the in vivo activity of many inhibitors.

### Results

All compounds tested were administered by the oral route.

R940347 inhibited edema by 89% when administered at a dose level of 100 mg/kg, 2 hours prior to challenge.

Results for the compounds tested are summarized in Table 7.

| Table 7 | | | | |
|---|---|---|---|---|
| | | | % inhibition to vehicle control | Plasma Concentration ± SEM (ng/ml) |
| Compound Name | Dosage (mg/kg) | Pretreatment Time (hrs) | Edema Size ± SEM | Exposure = Pretreatment Time + 4 hours |
| R940347 | 100 | 0.5 | 36.7 ± 22.4 | 1596 ± 485 |
| | | 1 | 48.2 ± 5.7 | 3014 ± 590 |
| | | 2 | 88.9 ± 9.1 | 1992 ± 247 |
| R940347 | 100 | 1 | 48 | nd* |
| | 100 | 2 | 89 | nd |

| | | | | |
|---|---|---|---|---|
| *nd=not determined | | | | |

### The 2,4-Pyrimidinediamine Compounds of the Invention Inhibit Macrophage Activation Description

The ability of the 2,4-pyrimidinediamine compounds of the invention to inhibit activation of differentiated macrophages was shown by measuring the release of cytokines from stimulated macrophages. Release of tumor necrosis factor alpha (TNF) and interleukin 6 (IL-6) was quantified in response to IgG or LPS stimulation.

### Purification and Culture of Human Macrophages

CD 14+ monocytes were purified from from PBMC (Allcells # PB002) using the Monocyte Isolation kit (Miltenyi biotec #130-045-501) as per the manufacturer's instructions. Purity was assessed by measuring the percentage of CD 14+ cells by flow cytometry. Typically > 90% purity is achieved. The purified CD14+ cells are then plated out (6x106.cells/150 cm TC dish in 15mls media) in Macrophage-SFM (Gibco #12065-074) with 100ng/ml of M-CSF (Pepro Tech #300-25) and allowed to differentiate for five days. At the end of that period, cell morphology and cell surface markers (CD14, HLA-DR, B7.1, B7.2, CD64, CD32, and CD16) reflected the presence of mature differentiated macrophage.

### Stimulation with LPS

For stimulation with LPS, 10 uL of a 10X stock solution was added to the preincubated cell-compound mixture to a final concentration of 10 ng/mL. The cells were then incubated for 16hr at 37°C and supernatants were analyzed as described above.

Varied concentrations of compound were compared to solvent only to determine the IC50 of each compound for each cytokine. Representative IC50s for 2,4-pyrimidinediamine compounds of the invention are shown in Table 11.

| Table 11 | | | | | | | | | |
|---|---|---|---|---|---|---|---|---|---|
| Compound | Jurkat | 1° T-Cell | | | | | 1° B-Cell | Monocytes/Macrophage | |
| | CD69 IC50 (in µM) | TNF IC50 (in µM) | IL2 IC50 (in µM) | GMSCF IC50 (in µM) | IL4 IC50 (in µM) | IFNg IC50 (in µM) | CD69 IC50 (in µM) | TNF IC50 (in µM) | IL-6 IC50 (in µM) |
| R940344 | 7.735 | | | | | | | | |
| R940345 | 5.395 | | | | | | | | |
| R940347 | 0.581 | 0.0992 | 1.894 | 1.613 | 0.212 | 1.673 | 0.47 | 0.038 | 0.019 |
| R945242 | 1.707 | | | | | | | | |
| R945263 | 4.467 | | | | | | | | |
| R921304 | 0.141 | 1.497 | 2.772 | 1.567 | 0.366 | 2.894 | 0.167 | | |
| R945298 | 9.467 | | | | | | | | |
| R945299 | 1.063 | | | | | | | | |

Although the foregoing invention has been described in some detail to facilitate understanding, it will be apparent that certain changes and modifications may be practiced within the scope of the appended claims. Accordingly, the described embodiments are to be considered as illustrative and not restrictive, and the invention is not to be limited to the details given herein, but may be modified within the scope and equivalents of the appended claims.

## Claims

1. A compound of formula (1a) 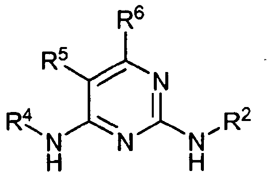 or a salt thereof wherein
R⁵ is selected from the group consisting of halogen, -OR^{d}, -SR^{d}, (C1-C3) haloalkyloxy, (C1-C3) perhaloalkyloxy, -NR^{c}R^{c}, (C1-C3) haloalkyl, (C1-C3) perhaloalkyl, -CN, -NC, -OCN, -SCN, -NO, -NO₂, -N₃, -S(O)R^{d}, -S(O)₂R^{d}, -S(O)₂OR^{d}, -S(O)NR^{c}R^{c}; -S(O)₂NR^{c}R^{c}, -OS(O)R^{d}, -OS(O)₂R^{d}, -OS(O)₂OR^{d}, -OS(O)NR^{c}R^{c}, -OS(O)₂NR^{c}R^{c}, -C(O)R^{d}, -C(O)OR^{d}, -C(O)NR^{c}R^{c}, -C(NH)NR^{c}R^{c}, -OC(O)R^{d}, -SC(O)R^{d}, -OC(O)OR^{d}, -SC(O)OR^{d}, -OC(O)NR^{c}R^{c}, -SC(O)NR^{c}R^{c}, -OC(NH)NR^{c}R^{c}, -SC(NH)NR^{c}R^{c}, -[NHC(O)]*ₙ*R^{d}, -[NHC(O)]*ₙ*OR^{d}, -[NHC(O)]*ₙ*NR^{c}R^{c} and -[NHC(NH)]*ₙ*NR^{c}R^{c}, (C5-C10) aryl optionally substituted with one or more of the same or different R⁸ groups, (C6-C16) arylalkyl optionally substituted with one or more of the same or different R⁸ groups, 5-10 membered heteroaryl optionally substituted with one or more of the same or different R⁸ groups and 6-16 membered heteroarylalkyl optionally substituted with one or more of the same or different R⁸ groups, (C1-C6) alkyl optionally substituted with one or more of the same or different R⁸ groups, (C1-C4) alkanyl optionally substituted with one or more of the same or different R⁸ groups, (C2-C4) alkenyl optionally substituted with one or more of the same or different R⁸ groups and (C2-C4) alkynyl optionally substituted with one or more of the same or different R⁸ groups;
each R⁶ is independently selected from the group consisting of hydrogen, halogen, -OR^{d}, -SR^{d}, (C1-C3) haloalkyloxy, (C1-C3) perhaloalkyloxy, -NR^{c}R^{c}, (C1-C3) haloalkyl, (C1-C3) perhaloalkyl, -CN, -NC, -OCN, -SCN, -NO, -NO₂, -N₃, -S(O)R^{d}, -S(O)₂R^{d}, -S(O)₂OR^{d}, -S(O)NR^{c}R^{c}; -S(O)₂NR^{c}R^{c}, -OS(O)R^{d}, -OS(O)₂R^{d}, -OS(O)₂OR^{d}, - OS(O)NR^{c}R^{c}, -OS(O)₂NR^{c}R^{c}, -C(O)R^{d}, -C(O)OR^{d}, -C(O)NR^{c}R^{c}, -C(NH)NR^{c}R^{c}, -OC(O)R^{d}, -SC(O)R^{d}, -OC(O)OR^{d}, -SC(O)OR^{d}, -OC(O)NR^{c}R^{c}, -SC(O)NR^{c}R^{c}, -OC(NH)NR^{c}R^{c}, -SC(NH)NR^{c}R^{c}, -[NHC(O)]*ₙ*R^{d}, -[NHC(O)]*ₙ*OR^{d}, -[NHC(O)]*ₙ*NR^{c}R^{c} and -[NHC(NH)]*ₙ*NR^{c}R^{c}, (C5-C10) aryl optionally substituted with one or more of the same or different R⁸ groups, (C6-C16) arylalkyl optionally substituted with one or more of the same or different R⁸ groups, 5-10 membered heteroaryl optionally substituted with one or more of the same or different R⁸ groups and 6-16 membered heteroarylalkyl optionally substituted with one or more of the same or different R⁸ groups;
R⁸ is selected from the group consisting of R^{a}, R^{a} substituted with one or more of the same or different R^{a} or R^{b}, R^{b}, -OR^{a} substituted with one or more of the same or different R^{a} or R^{b}, -B(OR^{a})₂, -B(NRCR^{c})₂, -(CH₂)*ₘ*-R^{b}, -(CHR^{a})*ₘ*-R^{b}, -O-(CH₂)*ₘ*-R^{b}, -S-(CH₂)*ₘ*-R^{b}, -O-CHR^{a}R^{b}, -O-CR^{a}(R^{b})₂, -O-(CHR^{a})*ₘ*-R^{b}, -O- (CH₂)*ₘ*-CH[(CH₂)*ₘ*R^{b}]R^{b}, -S-(CHR^{a})*ₘ*-R^{b}, -C(O)NH-(CH₂)*ₘ*-R^{b}, -C(O)NH-(CHR^{a})*ₘ*-R^{b}, -O-(CH₂)*ₘ*-C(O)NH-(CH₂)*ₘ*-R^{b}, -S-(CH₂)*ₘ*-C(O)NH-(CH₂)*ₘ*-R^{b}, -O-(CHR^{a})*ₘ*-C(O)NH-(CHR^{a})*ₘ*-R^{b}, -S-(CHR^{a})*ₘ*-C(O)NH-(CHR^{a})*ₘ*-R^{b}, -NH-(CH₂)*ₘ*-R^{b}, -NH-(CHR^{a})*ₘ*-R^{b}, -NH[(CH₂)*ₘ*R^{b}], -N[(CH₂)*ₘ*R^{b}]₂, -NH-C(O)-NH-(CH₂)*ₘ*-R^{b}, -NH-C(O)-(CH₂)*ₘ*-CHR^{b}R^{b} and -NH-(CH₂)*ₘ*-C(O)-NH-(CH₂)*ₘ*-R^{b};
each R^{a} is independently selected from the group consisting of (C1-C6) alkyl, hydrogen, (C3-C8) cycloalkyl, cyclohexyl, (C4-C11) cycloalkylalkyl, (C5-C10) aryl, (C6-C16) arylalkyl, 2-6 membered heteroalkyl, 3-8 membered cycloheteroalkyl, 4-11 membered cycloheteroalkylalkyl, 5-10 membered heteroaryl and 6-16 membered heteroarylalkyl;
each R^{b} is a suitable group independently selected from the group consisting of -C(O)R^{d}, -C(O)OR^{d}, =O, -OR^{d}, (C1-C3) haloalkyloxy, -OCF₃, =S, -SR^{d}, =NR^{d}, =NOR^{d}, -NR^{c}R^{c}, halogen, -CF₃, -CN, -NC, -OCN, -SCN, -NO, -NO₂, =N₂, -N₃, -S(O)R^{d}, -S(O)₂R^{d}, -S(O)₂OR^{d}, -S(O)NR^{c}R^{c}, -S(O)₂NR^{c}R^{c}, -OS(O)R^{d}, -OS(O)₂R^{d}, -OS(O)₂OR^{d}, -OS(O)₂NR^{c}R^{c}, -C(O)NR^{c}R^{c}, -C(NH)NR^{c}R^{c}, -C(NR^{a})NR^{c}R^{c}, -C(NOH)R^{a}, -C(NOH)NR^{c}R^{c}, -OC(O)R^{d}, -OC(O)OR^{d}, -OC(O)NR^{c}R^{c}, -OC(NH)NR^{c}R^{c}, -OC(NR^{a})NR^{c}R^{c}, -[NHC(O)]*ₙ*R^{d}, -[NR^{a}C(O)]*ₙ*R^{d}, -[NHC(O)]*ₙ*OR^{d}, -[NR^{a}C(O)]*ₙ*OR^{d}, -[NHC(O)]*ₙ*NR^{c}R^{c}, -[NR^{a}C(O)]*ₙ*NR^{c}R^{c}, -[NHC(NH)]*ₙ*NR^{c}R^{c} and -[NR^{a}C(NR^{a})]*ₙ*NR^{c}R^{c};
each R^{c} is independently a protecting group or R^{a}, or, alternatively, each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups;
each R^{d} is independently R^{a} or a protecting group;
each *m* is independently an integer from 1 to 3; and
each *n* is independently an integer from 0 to 3.
R² is selected from the group consisting (C1-C6) alkyl optionally substituted with one or more of the same or different R⁸ groups, (C3-C8) cycloalkyl optionally substituted with one or more of the same or different R⁸ groups, 3-8 membered cycloheteroalkyl optionally substituted with one or more of the same or different R⁸ groups, (C5-C15) aryl optionally substituted with one or more of the same or different R⁸ groups and 5-15 membered heteroaryl optionally substituted with one or more of the same or different R⁸ groups;
R⁴ is 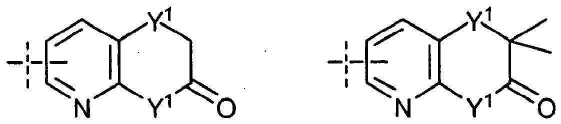 wherein:
each Y¹ is independently selected from the group consisting of O, NR³⁷, S, SO, SO₂, SONR³⁶ and NH;
R³⁶ is hydrogen or alkyl; and
R³⁷ is selected from the group consisting of hydrogen or a progroup selected from the group consisting of -CH₂-PO(OR⁸)₂, aryl, arylalkyl, heteroaryl, R^{a}, R^{b}, -CR^{a}R^{b}-O-C(O)R⁸, -CR^{a}R^{b}-O-PO(OR⁸)₂, -CH₂-O-PO(OR⁸)₂, -C(O)-CR^{a}R^{b}-N(CH₃)₂, -CR^{a}R^{b}-O-C(O)-CR^{a}R^{b}-N(CH₃)₂, -C(O)R⁸, and -C(O)-NR⁸-C(O)R⁸.

2. The compound according to claim 1, wherein R⁸ is independently selected from the group consisting of R^{d}, -NR^{c}R^{c}, -(CH₂)*ₘ*-NR^{c}R^{c}, -C(O)NR^{c}R^{c}, -(CH₂)*ₘ*-C(O)NR^{c}R^{c}, -C(O)OR^{d}, -(CH₂)*ₘ*-C(O)OR^{d} and -(CH₂)*ₘ*-OR^{d}.

3. The compound according to claim 1 or 2, wherein each R^{c} is taken together with the nitrogen atom to which it is bonded to form a 5 to 8-membered cycloheteroalkyl or heteroaryl which may optionally include one or more of the same or different additional heteroatoms and which may optionally be substituted with one or more of the same or different R^{a} or suitable R^{b} groups.

4. The compound according to any one of claims 1-3, wherein R⁵ is fluoro and R⁶ is H.

5. The compound according to claim 1 of formula 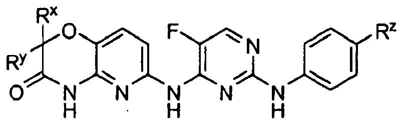 wherein:
R^{x} and R^{y} independently are H or methyl; and
R^{z} is (a) piperazin-1-yl substituted on the N at the 4 position with methoxycarbonyl, methylcarbonyl, or methyl, or (b) morpholinyl.

6. A compound according to claim 1 that is
5-Fluoro-N2-(4-morpholinophenyl)-N4-(2H-3-oxo-4H-5-pyrid[1,4]oxazin-6-yl)-2,4-pyrimidinediamine;
N4-(2,2-Dimethyl-2H-3-oxo-4H-5-pyrido[1,4]oxazin-6-yl]-5-fluoro-N2-[4-(4-methoxycarbonylpiperazino)phenyl]-2,4-pyrimidinediamine;
N2-[4-(4-Acetylpiperazino)phenyl]-N4-(2,2-dimethyl-2H-3-oxo-4H-5-pyrido[1,4]oxazin-6-yl)-5-fluoro-2,4-pyrimidinediamine;
N4-(2,2-Dimethyl-2H-3-oxo-4H-5-pyrid[1,4]oxazin-6-yl)-5-fluoro-N2-(4-morpholinophenyl)-2,4-pyrimidinediamine;
5-Fluoro-N2-[4-(4-methylpiperazino)phenyl]-N4-(2H-3-oxo-4H-5-pyrid[1,4]oxazin-6-yl)-2,4-pyrimidinediamine;
N4-(2,2-Dimethyl-2H-3-oxo-4H-5-pyrid[1,4]oxazin-6-yl)-5-fluoro-N2-[4-(4-methylpiperazino)phenyl]-2,4-pyrimidinediamine; or
a pharmaceutically acceptable salt of one of the foregoing.

7. A composition comprising a compound according to one of claims 1 to 6 and a pharmaceutically acceptable carrier, excipient or diluent.

8. A compound according to any one of claims 1 to 6 or a composition according to claim 7 for use in a method of treating or preventing an autoimmune disease and/or one or more symptoms associated therewith in a mammal, the method comprising administering to the mammal an effective amount of a compound according to any one of claims 1 to 6 or composition according to claim 7.

9. The compound or composition according to claim 8, wherein the mammal is a human.

10. The compound or composition according to claim 9, in which the autoimmune disease is selected from the group consisting autoimmune diseases that are frequently designated as single organ or single cell-type autoimmune disorders and autoimmune disease that are frequently designated as involving systemic autoimmune disorder.

11. The compound or composition according to claim 9, in which the autoimmune disease is selected from the group consisting of Hashimoto's thyroiditis, autoimmune hemolytic anemia, autoimmune atrophic gastritis of pernicious anemia, autoimmune encephalomyelitis, autoimmune orchitis, Goodpasture's disease, autoimmune thrombocytopenia, sympathetic ophthalmia, myasthenia gravis, Graves' disease, primary biliary cirrhosis, chronic aggressive hepatitis, ulcerative colitis and membranous glomerulopathy.

12. The compound or composition according to claim 9, in which the autoimmune disease is selected from the group consisting of systemic lupus erythematosis, rheumatoid arthritis, Sjogren's syndrome, Reiter's syndrome, polymyositis-dermatomyositis, systemic sclerosis, polyarteritis nodosa, multiple sclerosis and bullous pemphigoid.

13. The compound or composition according to claim 9, in which the autoimmune disease is systemic lupus erythematosis.

14. The compound or composition according to claim 9, in which the autoimmune disease is rheumatoid arthritis.

15. The compound or composition according to claim 9, in which the autoimmune disease is multiple sclerosis.

## Patentansprüche

1. Verbindung der Formel (1a) 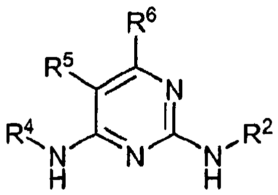 oder ein Salz davon, worin
R⁵ ausgewählt ist aus der Gruppe, die besteht aus Halogen, -OR^{d}, -SR^{d}, (C1-C3)-Halogenalkyloxy, (C1-C3)-Perhalogenalkyloxy, -NR^{c}R^{c}, (C1-C3)-Halogenalkyl, (C1-C3)-Perhalogenalkyl, -CN, -NC, -OCN, -SCN, -NO, -NO₂, -N₃, -S(O)R^{d}, -S(O)₂R^{d}, -S(O)₂OR^{d}, -S(O)NR^{c}R^{c}, -S(O)₂NR^{c}R^{c}, -OS(O)R^{d}, -OS(O)₂R^{d}, -OS(O)₂OR^{d}, -OS(O)NR^{c}R^{c}, -OS(O)₂NR^{c}R^{c}, -C(O)R^{d}, -C(O)OR^{d}, -C(O)NR^{c}R^{c}, -C(NH)NR^{c}R^{c}, -OC(O)R^{d}, -SC(O)R^{d}, -OC(O)OR^{d}, -SC(O)OR^{d}, -OC(O)NR^{c}R^{c}, -SC(O)NR^{c}R^{c}, -OC(NH)NR^{c}R^{c}, -SC(NH)NR^{c}R^{c}, -[NHC(O)]ₙR^{d}, -[NHC(O)]ₙOR^{d}, -[NHC(O)]ₙNR^{c}R^{c} und -[NHC(NH)]ₙNR^{c}R^{c}, (C5-C10)-Aryl, das optional substituiert ist mit einer oder mehreren R⁸-Gruppen, die gleich oder verschieden sind, (C6-C16)-Arylalkyl, das optional substituiert ist mit einer oder mehreren R⁸-Gruppen, die gleich oder verschieden sind, 5-10-gliedrigem Heteroaryl, das optional substituiert ist mit einer oder mehreren R⁸-Gruppen, die gleich oder verschieden sind, und 6-16-gliedrigem Heteroarylalkyl, das optional substituiert ist mit einer oder mehreren R⁸-Gruppen, die gleich oder verschieden sind, (C1-C6)-Alkyl, das optional substituiert ist mit einer oder mehreren R⁸-Gruppen, die gleich oder verschieden sind, (C1-C4)-Al-kanyl, das optional substituiert ist mit einer oder mehreren R⁸-Gruppen, die gleich oder verschieden sind, (C2-C4)-Alkenyl, das optional substituiert ist mit einer oder mehreren R⁸-Gruppen, die gleich oder verschieden sind, und (C2-C4)-Alkinyl, das optional substituiert ist mit einer oder mehreren R⁸-Gruppen, die gleich oder verschieden sind;
R⁶ jeweils unabhängig ausgewählt ist aus der Gruppe, die besteht aus Wasserstoff, Halogen, -OR^{d}, -SR^{d}, (C1-C3)-Halogenalkyloxy, (C1-C3)-Perhalogenalkyloxy, -NR^{c}R^{c}, (C1-C3)-Halogenalkyl, (C1-C3)-Per-halogenalkyl, -CN, -NC, -OCN, -SCN, -NO, -NO₂, -N₃, -S(O)R^{d}, -S(O)₂R^{d}, -S(O)₂OR^{d}, -S(O)NR^{c}R^{c}, -S(O)₂NR^{c}R^{c}, -OS(O)R^{d}, -OS(O)₂R^{d}, -OS(O)₂OR^{d}, -OS(O)NR^{c}R^{c}, -OS(O)₂NR^{c}R^{c}, -C(O)R^{d}, -C(O)OR^{d}, -C(O)NR^{c}R^{c}, -C(NH)NR^{c}R^{c}, -OC(O)R^{d}, -SC(O)R^{d}, -OC(O)OR^{d}, -SC(O)OR^{d}, -OC(O)NR^{c}R^{c}, -SC(O)NR^{c}R^{c}, -OC(NH)NR^{c}R^{c}, -SC(NH)NR^{c}R^{c}, -[NHC(O)]ₙR^{d}, -[NHC(O)]ₙOR^{d}, -[NHC(O)]ₙNR^{c}R^{c} und -[NHC(NH)]ₙNR^{c}R^{c}, (C5-C10)-Aryl, das optional substituiert ist mit einer oder mehreren R⁸-Gruppen, die gleich oder verschieden sind, (C6-C16)-Arylalkyl, das optional substituiert ist mit einer oder mehreren R⁸-Gruppen, die gleich oder verschieden sind, 5-10-glied-rigem Heteroaryl, das optional substituiert ist mit einer oder mehreren R⁸-Gruppen, die gleich oder verschieden sind, und 6-16-gliedrigem Heteroarylalkyl, das optional substituiert ist mit einer oder mehreren R⁸-Gruppen, die gleich oder verschieden sind;
R⁸ ausgewählt ist aus der Gruppe, die besteht aus R^{a}, R^{a}, das substituiert ist mit einem oder mehreren gleichen oder verschieden R^{a} oder R^{b}, R^{b}, -OR^{a}, das substituiert ist mit einem oder mehreren gleichen oder verschiedenen R^{a} oder R^{b}, -B(OR^{a})₂, -B(NR^{c}R^{c})₂, -(CH₂)ₘ-R^{b}, -(CHR^{a})ₘ-R^{b}, -O-(CH₂)ₘ-R^{b}, -S-(CH₂)ₘ-R^{b}, -O-CHR^{a}R^{b}, -O-CR^{a}(R^{b})₂, -O-(CHR^{a})ₘ-R^{b}, -O-(CH₂)ₘ-CH[(CH₂)ₘR^{b}]R^{b}, -S-(CHR^{a})ₘ-R^{b}, -C(O)NH-(CH₂)ₘ-R^{b}, -C(O)NH-(CHR^{a})ₘ-R^{b}, -O-(CH₂)ₘ-C(O)NH-(CH₂)ₘ-R^{b}, -S-(CH₂)ₘ-C(O)NH-(CH₂)ₘ-R^{b}, -O-(CHR^{a})ₘ-C(O)NH-(CHR^{a})ₘ-R^{b}, -S-(CHR^{a})ₘ-C(O)NH-(CHR^{a})ₘ-R^{b}, -NH-(CH₂)ₘ-R^{b}, -NH-(CHR^{a})ₘ-R^{b}, -NH[(CH₂)ₘR^{b}], -N[(CH₂)ₘR^{b}]₂, -NH-C(O)-NH-(CH₂)ₘ-R^{b}, -NH-C(O)-(CH₂)ₘ-CHR^{b}R^{b} und -NH-(CH₂)ₘ-C(O)-NH-(CH₂)ₘ-R^{b};
R^{a} jeweils unabhängig ausgewählt ist aus der Gruppe, die besteht aus (C1-C6)-Alkyl, Wasserstoff, (C3-C8)-Cycloalkyl, Cyclohexyl, (C4-C11)-Cycloalkylalkyl, (C5-C10)-Aryl, (C6-C16)-Arylalkyl, 2-6-gliedrigem Heteroalkyl, 3-8-gliedrigem Cycloheteroalkyl, 4-11-gliedrigem Cycloheteroalkylalkyl, 5-10-gliedrigem Heteroaryl und 6-16-gliedrigem Heteroarylalkyl;
R^{b} jeweils eine geeignete Gruppe ist, die unabhängig ausgewählt ist aus der Gruppe, die besteht aus -C(O)R^{d}, -C(O)OR^{d}, =O, -OR^{d}, (C1-C3)-Halogenalkyloxy, -OCF₃, =S, -SR^{d}, =NR^{d}, =NOR^{d}, -NR^{c}R^{c}, Halogen, -CF₃, -CN, -NC, -OCN, -SCN, -NO, NO₂, =N₂, -N₃, -S(O)R^{d}, -S(O)₂R^{d}, -S(O)₂OR^{d}, -S(O)NR^{c}R^{c}, -S(O)₂NR^{c}R^{c}, -OS(O)R^{d}, -OS(O)₂R^{d}, -OS(O)₂OR^{d}, -OS(O)₂NR^{c}R^{c}, -C(O)NR^{c}R^{c}, -C(NH)NR^{c}R^{c}, -(NR^{a})NR^{c}R^{c}, -C(NOH)R^{a}, -C(NOH)NR^{c}R^{c}, -OC(O)R^{d}, -OC(O)OR^{d}, -OC(O)NR^{c}R^{c}, -OC(NH)NR^{c}R^{c}, -OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙOR^{d}, -[NHC(O)]ₙNR^{c}R^{c}, -[NR^{a}C(O)]ₙNR^{c}R^{c}, -[NHC(NH)]ₙNR^{c}R^{c} und -[NR^{a}C(NR^{a})]ₙNR^{c}R^{c};
R^{c} jeweils unabhängig eine Schutzgruppe oder R^{a} ist, oder alternativ R^{c} jeweils mit dem Stickstoffatom, an das es gebunden ist, zusammengenommen wird, um ein 5-8-gliedriges Cycloheteroalkyl oder Heteroaryl zu bilden, das optional ein oder mehrere gleiche oder verschiedene zusätzliche Heteroatome enthalten kann, und das optional mit einer oder mehreren gleichen oder verschiedenen R^{a}-Gruppen oder geeigneten R^{b}-Gruppen substituiert sein kann;
R^{d} jeweils unabhängig R^{a} oder eine Schutzgruppe ist;
m jeweils unabhängig eine ganze Zahl von 1 bis 3 ist; und
n jeweils unabhängig eine ganze Zahl von 0 bis 3 ist;
R² ausgewählt ist aus der Gruppe, die besteht aus (C1-C6)-Alkyl, das optional substituiert ist mit einer oder mehreren R⁸-Gruppen, die gleich oder verschieden sind, (C3-C8)-Cycloalkyl, das optional substituiert ist mit einer oder mehreren R⁸-Gruppen, die gleich oder verschieden sind, 3-8-gliedrigem Cycloheteroalkyl, das optional substituiert ist mit einer oder mehreren R⁸-Gruppen, die gleich oder verschieden sind, (C5-C15)-Aryl, das optional substituiert ist mit einer oder mehreren R⁸-Guppen, die gleich oder verschieden sind, und 5-15-gliedrigem Heteroaryl, das optional substituiert ist mit einer oder mehreren R⁸-Gruppen, die gleich oder verschieden sind;
R⁴ 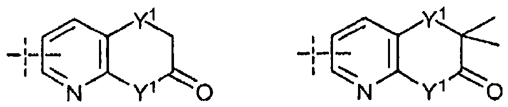 ist, worin:
Y¹ jeweils unabhängig ausgewählt ist aus der Gruppe, die besteht aus O, NR³⁷, S, SO, SO₂, SONR³⁶ und NH;
R³⁶ Wasserstoff oder Alkyl ist; und
R³⁷ ausgewählt ist aus der Gruppe, die besteht aus Wasserstoff oder einer Progruppe, die ausgewählt ist aus der Gruppe, die besteht aus -CH₂-PO(OR⁸)₂, Aryl, Arylalkyl, Heteroaryl, R^{a}, R^{b}, -CR^{a}R^{b}-O-C(O)R⁸, -CR^{a}R^{b}-O-PO(OR⁸)₂, -CH₂-O-PO(OR⁸)₂, -C(O)-CR^{a}R^{b}-N(CH₃)₂, -CR^{a}R^{b}-O-C(O)-CR^{a}R^{b}-N(CH₃)₂, -C(O)R⁸ und -C(O)-NR⁸-C(O)R⁸.

2. Verbindung nach Anspruch 1, worin R⁸ unabhängig ausgewählt ist aus der Gruppe, die besteht aus R^{d}, -NR^{c}R^{c}, -(CH₂)ₘ-NR^{c}R^{c}, -C(O)NR^{c}R^{c}, -(CH₂)ₘ-C(O)NR^{c}R^{c}, -C(O)OR^{d}, -(CH₂)ₘ-C(O)OR^{d} und -(CH₂)ₘ-OR^{d}.

3. Verbindung nach Anspruch 1 oder 2, worin R^{c} jeweils mit dem Stickstoffatom, an das es gebunden ist, zusammengenommen wird, um ein 5-8-gliedriges Cycloheteroalkyl oder Heteroaryl zu bilden, das optional ein oder mehrere gleiche oder verschiedene zusätzliche Heteroatome enthalten kann und das optional mit einer oder mehreren gleichen oder verschiedenen R^{a}-Gruppen oder geeigneten R^{b}-Gruppen substituiert sein kann.

4. Verbindung nach einem der Ansprüche 1 bis 3, worin R⁵ Fluor ist und R⁶ H ist.

5. Verbindung nach Anspruch 1 der Formel 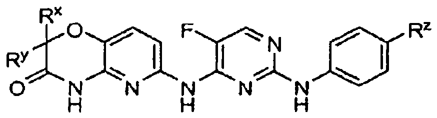 worin:
R^{x} und R^{y} unabhängig H oder Methyl sind; und
R^{z} (a) Piperazin-1-yl, das am N in Position 4 mit Methoxycarbonyl, Methylcarbonyl oder Methyl substituiert ist, oder (b) Morpholinyl ist.

6. Verbindung nach Anspruch 1, die
5-Fluor-N2-(4-morpholinophenyl)-N4-(2H-3-oxo-4H-5-pyrid[1,4]oxazin-6-yl)-2,4-pyrimidindiamin;
N4-(2,2-Dimethyl-2H-3-oxo-4H-5-pyrido[1,4]oxazin-6yl)-5-fluor-N2-[4-(4-methoxycarbonylpiperazino)phenyl]-2,4-pyrimidindiamin;
N2-[4-(4-Acetylpiperazino)phenyl]-N4-(2,2-dimethyl-2H-3-oxo-4H-5-pyrido[1,4]oxazin-6-yl)-5-fluor-2,4-pyrimidindiamin;
N4-(2,2-Dimethyl-2H-3-oxo-4H-5-pyrid[1,4]oxazin-6-yl)-5-fluor-N2-(4-morpholinophenyl)-2,4-pyrimidindiamin;
5-Fluor-N2-[4-(4-methylpiperazino)phenyl]-N4-(2H-3-oxo-4H-5-pyrid[1,4]oxazin-6-yl)-2,4-pyrimidindiamin;
N4-(2,2-Dimethyl-2H-3-oxo-4H-5-pyrid[1,4]oxazin-6-yl)-5-fluor-N2-[4-(4-methylpiperazino)phenyl]-2,4-pyrimidindiamin; oder
ein pharmazeutisch annehmbares Salz einer der vorstehenden ist.

7. Zusammensetzung aufweisend eine Verbindung nach einem der Ansprüche 1 bis 6 und eine pharmazeutisch annehmbare Trägersubstanz, ein pharmazeutisch annehmbares Vehikel oder Verdünnungsmittel.

8. Verbindung nach einem der Ansprüche 1 bis 6 oder Zusammensetzung nach Anspruch 7 zur Verwendung in einem Verfahren zur Behandlung oder Verhinderung einer Autoimmunkrankheit und/oder eines oder mehrerer Symptome, die damit einhergehen, bei einem Säugetier, wobei das Verfahren ein Verabreichen einer wirksamen Menge einer Verbindung nach einem der Ansprüche 1 bis 6 oder einer Zusammensetzung nach Anspruch 7 an das Säugetier aufweist.

9. Verbindung oder Zusammensetzung nach Anspruch 8, wobei das Säugetier ein Mensch ist.

10. Verbindung oder Zusammensetzung nach Anspruch 9, bei der die Autoimmunkrankheit ausgewählt ist aus der Gruppe, die aus Autoimmunkrankheiten, die häufig als Einzelorgan-Autoimmunerkrankungen oder Autoimmunerkrankungen vom Einzelzellen-Typ bezeichnet werden, und Autoimmunkrankheiten, die häufig als eine systemische Autoimmunerkrankung umfassend bezeichnet werden, besteht.

11. Verbindung oder Zusammensetzung nach Anspruch 9, bei der die Autoimmunkrankheit ausgewählt ist aus der Gruppe, die besteht aus Hashimoto-Thyreoiditis, autoimmunhämolytischer Anämie, autoimmunatrophischer Gastritis pernitiöser Anämie, Autoimmun-Enzephalomyelitis, Autoimmun-Orchitis, Goodpasture-Syndrom, Autoimmun-Thrombozytopenie, sympathischer Ophthalmie, Myasthenia gravis, Basedow-Krankheit, primärer biliärer Zirrhose, chronisch aggressiver Hepatitis, Colitis ulcerosa und membranöser Glomerulopathie.

12. Verbindung oder Zusammensetzung nach Anspruch 9, bei der die Autoimmunkrankheit ausgewählt ist aus der Gruppe, die besteht aus systemischem Lupus erythematodes, rheumatoider Arthritis, Sjögren-Syndrom, Reiter-Syndrom, Polymyositis-Dermatomyositis, systemischer Sklerose, Polyarteritis nodosa, multipler Sklerose und bullösem Pemphigoid.

13. Verbindung oder Zusammensetzung nach Anspruch 9, bei der die Autoimmunkrankheit systemischer Lupus erythematodes ist.

14. Verbindung oder Zusammensetzung nach Anspruch 9, bei der die Autoimmunkrankheit rheumatoide Arthritis ist.

15. Verbindung oder Zusammensetzung nach Anspruch 9, bei der die Autoimmunkrankheit multiple Sklerose ist.

## Revendications

1. Composé de formule (Ia) : 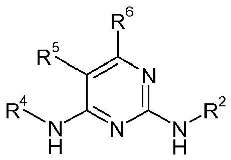 ou sel d'un tel composé, dans laquelle formule :
- R⁵ représente une entité choisie dans l'ensemble constitué par les atomes d'halogène, les groupes halogéno-alcoxy en C₁-C₃, perhalogéno-alcoxy en C₁-C₃, halogéno-alkyle en C₁-C₃ et perhalogéno-alkyle en C₁-C₃, les grou-pes symbolisés par -OR^{d}, -SR^{d}, -NR^{c}R^{c}, -CN, -NC, -OCN, -SCN, -NO, -NO₂, -N₃, -S(O)R^{d}, -S(O)₂R^{d}, -S(O)₂OR^{d}, S(O)NR^{c}R^{c}, -S(O)₂NR^{c}R^{c}, -OS(O)R^{d}, -OS(O)₂R^{d}, -OS(O)₂OR^{d}, -OS(O)NR^{c}R^{c}, -OS(O)₂NR^{c}R^{c}, -C(O)R^{d}, -C(O)OR^{d}, -C(O)NR^{c}R^{c}, -C(NH)NR^{c}R^{c}, -OC(O)R^{d}, -SC(O)R^{d}, -OC(O)OR^{d}, -SC(O)OR^{d}, -OC(O)NR^{c}R^{c}, -SC(O)NR^{c}R^{c}, -OC(NH)NR^{c}R^{c}, -SC(NH)NR^{c}R^{c}, -[NHC(O)]ₙR^{d}, -[NHC(O)]ₙOR^{d}, -[NHC(O)]ₙNR^{c}R^{c} ou -[NHC(NH)]ₙNR^{c}R^{c}, les groupes aryle en C₅-C₁₀, en option porteurs d'un substituant ou de plusieurs substituants identiques ou différents, symbolisé(s) par R⁸, les groupes aryl-alkyle en C₆-C₁₆, en option porteurs d'un substituant ou de plusieurs substituants identiques ou différents, symbolisé(s) par R⁸, les groupes hétéroaryle comportant de 5 à 10 chaînons, en option porteurs d'un substituant ou de plusieurs substituants identiques ou différents, symbolisé(s) par R⁸, les groupes hétéroaryl-alkyle comportant de 6 à 16 chaînons, en option porteurs d'un substituant ou de plusieurs substituants identiques ou différents, symbolisé(s) par R⁸, les groupes alkyle en C₁-C₆, en option porteurs d'un substituant ou de plusieurs substituants identiques ou différents, symbolisé(s) par R⁸, les groupes alcanyle en C₁-C₄, en option porteurs d'un substituant ou de plusieurs substituants identiques ou différents, symbolisé(s) par R⁸, les groupes alcényle en C₂-C₄, en option porteurs d'un substituant ou de plusieurs substituants identiques ou différents, symbolisé(s) par R⁸, et les groupes alcynyle en C₂-C₄, en option porteurs d'un substituant ou de plusieurs substituants identiques ou différents, symbolisé(s) par R⁸ ;
- et R⁶ représente une entité choisie dans l'ensemble constitué par un atome d'hydrogène, les atomes d'halogène, les groupes halogéno-alcoxy en C₁-C₃, perhalogéno-alcoxy en C₁-C₃, halogéno-alkyle en C₁-C₃ et perhalogéno-alkyle en C₁-C₃, les groupes symbolisés par -OR^{d}, -SR^{d}, -NR^{c}R^{c}, -CN, -NC, -OCN, -SCN, -NO, -NO₂, -N₃, -S(O)R^{d}, -S(O)₂R^{d}, -S(O)₂OR^{d}, -S(O)NR^{c}R^{c}, -S(O)₂NR^{c}R^{c}, -OS(O)R^{d}, -OS(O)₂R^{d}, -OS(O)₂OR^{d}, -OS(O)NR^{c}R^{c}, -OS(O)₂NR^{c}R^{c}, -C(O)R^{d}, -C(O)OR^{d}, -C(O)NR^{c}R^{c}, -C(NH)NR^{c}R^{c}, -OC(O)R^{d}, -SC(O)R^{d}, -OC(O)OR^{d}, -SC(O)OR^{d}, -OC(O)NR^{c}R^{c}, -SC(O)NR^{c}R^{c}, -OC(NH)NR^{c}R^{c}, -SC(NH)NR^{c}R^{c}, -[NHC(O)]ₙR^{d}, -[NHC(O)]ₙOR^{d}, -[NHC(O)]ₙNR^{c}R^{c} ou -[NHC(NH)]ₙNR^{c}R^{c}, les groupes aryle en C₅-C₁₀, en option porteurs d'un substituant ou de plusieurs substituants identiques ou différents, symbolisé(s) par R⁸, les groupes aryl-alkyle en C₆-C₁₆, en option porteurs d'un substituant ou de plusieurs substituants identiques ou différents, symbolisé(s) par R⁸, les groupes hétéroaryle comportant de 5 à 10 chaînons, en option porteurs d'un substituant ou de plusieurs substituants identiques ou différents, symbolisé(s) par R⁸, et les groupes hétéroaryl-alkyle comportant de 6 à 16 chaînons, en option porteurs d'un substituant ou de plusieurs substituants identiques ou différents, symbolisé(s) par R⁸ ;
étant entendu que
- R⁸ représente une entité choisie dans l'ensemble constitué par les groupes symbolisés par R^{a}, les groupes symbolisés par R^{a} et porteurs d'un substituant ou de plusieurs substituants identiques ou différents, symbolisé(s) par R^{a} ou R^{b}, les groupes symbolisés par R^{b}, les groupes symbolisés par -OR^{a} et porteurs d'un substituant ou de plusieurs substituants identiques ou différents, symbolisé(s) par R^{a} ou R^{b}, et les groupes symbolisés par -B(OR^{a})₂, -B(NR^{c}R^{c})₂, -(CH₂)ₘ-R^{b}, -(CHR^{a})ₘ-R^{b}, -O-(CH₂)ₘ-R^{b}, -S-(CH₂)ₘ-R^{b}, -O-CHR^{a}R^{b}, -O-CR^{a}(R^{b})₂, -O-(CHR^{a})ₘ-R^{b}, -O-(CH₂)ₘ-CH[(CH₂)ₘ-R^{b}]R^{b}, -S-(CHR^{a})ₘ-R^{b}, -C(O)NH-(CH₂)ₘ-R^{b}, -C(O)NH-(CHR^{a})ₘ-R^{b}, -O-(CH₂)ₘ-C(O)NH-(CH₂)ₘ-R^{b}, -O-(CHR^{a})ₘ-C(O)NH-(CHR^{a})ₘ-R^{b}, -S-(CH₂)ₘ-C(O)NH-(CH₂)ₘ-R^{b}, -S-(CHR^{a})ₘ-C(O)NH-(CHR^{a})ₘ-R^{b}, -NH-(CH₂)ₘ-R^{b}, -NH-(CHR^{a})ₘ-R^{b}, -NH[(CH₂)ₘ-R^{b}], -N[(CH₂)ₘ-R^{b}]₂, -NH-C(O)-NH-(CH₂)ₘ-R^{b}, -NH-C(O)-(CH₂)ₘ-CHR^{b}R^{b}, ou -NH-(CH₂)ₘ-C(O)-NH-(CH₂)ₘ-R^{b} ;
- chaque symbole R^{a} représente une entité choisie indépendamment dans l'ensemble constitué par un atome d'hydrogène et les groupes alkyle en C₁-C₆, cycloalkyle en C₃-C₈, cyclohexyle, cycloalkyl-alkyle en C₄-C₁₁, aryle en C₅-C₁₀, aryl-alkyle en C₆-C₁₆, hétéroalkyle comportant de 2 à 6 chaînons, cyclohétéroalkyle comportant de 3 à 8 chaînons, cyclohétéroalkyl-alkyle comportant de 4 à 11 chaînons, hétéroaryle comportant de 5 à 10 chaînons, et hétéroaryl-alkyle comportant de 6 à 16 chaînons ;
- chaque symbole R^{b} représente une entité appropriée choisie indépendamment dans l'ensemble constitué par les atomes d'halogène, les groupes halogéno-alcoxy en C₁-C₃, et les entités symbolisées par -C(O)R^{d}, -C(O)OR^{d}, =O, -OR^{d}, -OCF₃, =S, -SR^{d}, =NR^{d}, =NOR^{d}, -NR^{c}R^{c}, -CF₃, -CN, -NC, -OCN, -SCN, -NO, -NO₂, =N₂, -N₃, -S(O)R^{d}, -S(O)₂R^{d}, -S(O)₂OR^{d}, -S(O)NR^{c}R^{c}, -S(O)₂NR^{c}R^{c}, -OS(O)R^{d}, -OS(O)₂R^{d}, -OS(O)₂OR^{d}, -OS(O)₂NR^{c}R^{c}, -C(O)NR^{c}R^{c}, -C(NH)NR^{c}R^{c}, -C(NR^{a})NR^{c}R^{c}, -C(NOH)R^{a}, -C(NOH)NR^{c}R^{c}, -OC(O)R^{d}, -OC(O)OR^{d}, -OC(O)NR^{c}R^{c}, -OC(NH)NR^{c}R^{c}, -OC(NR^{a})NR^{c}R^{c}, -[NHC(O)]ₙR^{d}, -[NR^{a}C(O)]ₙR^{d}, -[NHC(O)]ₙOR^{d}, -[NR^{a}C(O)]ₙOR^{d}, -[NHC(O)]ₙNR^{c}R^{c}, -[NR^{a}C(O)]ₙNR^{c}R^{c}, -[NHC(NH)]ₙNR^{c}R^{c} ou -[NR^{a}C(NR^{a})]ₙNR^{c}R^{c} ;
- chaque symbole R^{c} représente, indépendamment, un groupe protecteur ou une entité symbolisée par R^{a}, ou encore chacun des symboles R^{c} représente une entité formant, conjointement avec l'atome d'azote auquel elle est liée, un groupe cyclohétéroalkyle ou hétéroaryle comportant de 5 à 8 chaînons, qui peut, en option, comporter un ou plusieurs hétéroatome(s) supplémentaire(s), identiques ou différents, et qui peut, en option, porter un ou plusieurs substituant(s), identiques ou différents, symbolisé(s) par R^{a} ou approprié(s) et symbolisé(s) par R^{b} ;
- chaque symbole R^{d} représente, indépendamment, une entité symbolisée par R^{a} ou un groupe protecteur ;
- chaque indice m est un nombre entier valant, indépendamment, de 1 à 3 ;
- et chaque indice n est un nombre entier valant, indépendamment, de 0 à 3 ;
- R² représente une entité choisie parmi les groupes alkyle en C₁-C₆, en option porteurs d'un substituant ou de plusieurs substituants identiques ou différents, symbolisé(s) par R⁸, les groupes cycloalkyle en C₃-C₈, en option porteurs d'un substituant ou de plusieurs substituants identiques ou différents, symbolisé(s) par R⁸, les groupes cyclohétéroalkyle comportant de 3 à 8 chaînons, en option porteurs d'un substituant ou de plusieurs substituants identiques ou différents, symbolisé(s) par R⁸, les groupes aryle en C₅-C₁₅, en option porteurs d'un substituant ou de plusieurs substituants identiques ou différents, symbolisé(s) par R⁸, et les groupes hétéroaryle comportant de 5 à 15 chaînons, en option porteurs d'un substituant ou de plusieurs substituants identiques ou différents, symbolisé(s) par R⁸ ;
- et R⁴ représente un groupe de l'une ou l'autre des formules 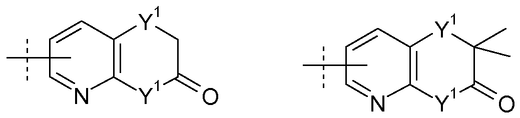 dans lesquelles
chaque symbole Y¹ représente indépendamment un chaînon choisi dans l'ensemble de ceux qui sont symbolisés par O, NR³⁷, S, SO, SO₂, SONR³⁶ et NH,
étant entendu que R³⁶ représente un atome d'hydrogène ou un groupe alkyle, et que R³⁷ représente un atome d'hydrogène ou un progroupe choisi dans l'ensemble constitué par les groupes aryle, aryl-alkyle et hétéroaryle et les groupes symbolisés par -CH₂-PO(OR⁸)₂, R^{a}, R^{b}, -CR^{a}R^{b}-O-C(O)R⁸, -CR^{a}R^{b}-O-PO(OR⁸)₂, -CH₂-O-PO(OR⁸)₂, -C(O)-CR^{a}R^{b}-N(CH₃)₂, -CR^{a}R^{b}-O-C(O)-CR^{a}R^{b}-N(CH₃)₂, -C(O)R⁸ et -C(O)-NR⁸-C(O)R⁸.

2. Composé conforme à la revendication 1, dans lequel R⁸ représente une entité choisie, indépendamment, dans l'ensemble constitué par les groupes symbolisés par R^{d}, -NR^{c}R^{c}, -(CH₂)ₘ-NR^{c}R^{c}, -C(O)NR^{c}R^{c}, -(CH₂)ₘ-C(O)NR^{c}R^{c}, -C(O)OR^{d}, -(CH₂)ₘ-C(O)OR^{d}, et -(CH₂)ₘ-OR^{d}.

3. Composé conforme à la revendication 1 ou 2, dans lequel chacun des symboles R^{c} représente une entité formant, conjointement avec l'atome d'azote auquel elle est liée, un groupe cyclohétéroalkyle ou hétéroaryle comportant de 5 à 8 chaînons, qui peut, en option, comporter un ou plusieurs hétéroatome(s) supplémentaire(s), identiques ou différents, et qui peut, en option, porter un ou plusieurs substituant(s) identiques ou différents, symbolisé(s) par R^{a} ou approprié(s) et symbolisé(s) par R^{b}.

4. Composé conforme à l'une des revendications 1 à 3, dans lequel R⁵ représente un atome de fluor et R⁶ représente un atome d'hydrogène.

5. Composé conforme à la revendication 1, de formule 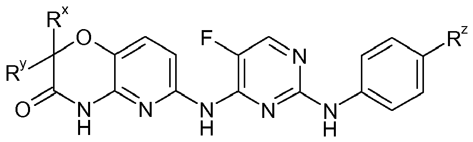 dans laquelle
- R^{x} et R^{y} représentent, indépendamment, un atome d'hydrogène ou un groupe méthyle,
- et R^{z} représente
a) un groupe pipérazin-1-yle dont l'atome d'azote en position 4 porte un substituant méthoxy-carbonyle, méthyl-carbonyle ou méthyle,
b) ou un groupe morpholinyle.

6. Composé conforme à la revendication 1, qui est l'un des suivants :
- 5-fluoro-N2-(4-morpholino-phényl)-N4-(2H-3-oxo-4H-5-pyrido[1,4]-oxazin-6-yl)-pyrimidine-2,4-diamine
- N4-(2,2-diméthyl-2H-3-oxo-4H-5-pyrido[1,4]oxazin-6-yl)-5-fluoro-N2-[4-(4-méthoxycarbonyl-pipérazino)-phényl)-pyrimidine-2,4-diamine
- N2-[4-(4-acétyl-pipérazino)-phényl)-N4-(2,2-diméthyl-2H-3-oxo-4H-5-pyrido[1,4]oxazin-6-yl)-5-fluoro-pyrimidine-2,4-diamine
- N4-(2,2-diméthyl-2H-3-oxo-4H-5-pyrido[1,4]oxazin-6-yl)-5-fluoro-N2-(4-morpholino-phényl)-pyrimidine-2,4-diamine
- 5-fluoro-N2-[4-(4-méthyl-pipérazino)-phényl)-N4-(2H-3-oxo-4H-5-pyrido[1,4]-oxazin-6-yl)-pyrimidine-2,4-diamine
- N4-(2,2-diméthyl-2H-3-oxo-4H-5-pyrido[1,4]oxazin-6-yl)-5-fluoro-N2-[4-(4-méthyl-pipérazino)-phényl)-pyrimidine-2,4-diamine
ou un sel, pharmacologiquement admissible, de l'un de ces composés.

7. Composition comprenant un composé, conforme à l'une des revendications 1 à 6, et un véhicule, excipient ou diluant, pharmacologiquement admissible.

8. Composé conforme à l'une des revendications 1 à 6, ou composition conforme à la revendication 7, pour utilisation dans un procédé de traitement ou de prévention, chez un mammifère, d'une maladie auto-immune et/ou d'un ou de plusieurs symptôme(s) associé(s) à une telle maladie, lequel procédé comporte le fait d'administrer au mammifère, en une quantité efficace, un composé conforme à l'une des revendications 1 à 6 ou une composition conforme à la revendication 7.

9. Composé ou composition conforme à la revendication 8, pour lequel ou laquelle le mammifère est un humain.

10. Composé ou composition conforme à la revendication 9, pour lequel ou laquelle la maladie auto-immune est choisie dans l'ensemble formé par les maladies auto-immunes qui sont fréquemment désignées comme des troubles auto-immuns affectant un seul organe ou un seul type cellulaire et les maladies auto-immunes qui sont fréquemment désignées comme impliquant des troubles auto-immuns systémiques.

11. Composé ou composition conforme à la revendication 9, pour lequel ou laquelle la maladie auto-immune est choisie dans l'ensemble formé par les suivantes : thyroïdite chronique de Hashimoto, anémie hémolytique auto-immune, gastrite atrophique auto-immune d'anémie pernicieuse, encéphalomyélite auto-immune, orchite autoimmune, maladie de Goodpasture, thrombocytopénie auto-immune, ophtalmie sympathique, myasthénie grave, maladie de Basedow-Graves, cirrhose biliaire primitive, hépatite chronique agressive, colite ulcéreuse, et glomérulopathie membraneuse.

12. Composé ou composition conforme à la revendication 9, pour lequel ou laquelle la maladie auto-immune est choisie dans l'ensemble formé par les suivantes : lupus érythémateux aigu disséminé, polyarthrite rhumatoïde, syndrome de Sjögren, syndrome de Fiessinger-Leroy-Reiter, polymyosite/dermatomyosite, sclérodermie généralisée, polyartérite noueuse, sclérose en plaques, et pemphigoïde bulleuse.

13. Composé ou composition conforme à la revendication 9, pour lequel ou laquelle la maladie auto-immune est le lupus érythémateux aigu disséminé.

14. Composé ou composition conforme à la revendication 9, pour lequel ou laquelle la maladie auto-immune est la polyarthrite rhumatoïde.

15. Composé ou composition conforme à la revendication 9, pour lequel ou laquelle la maladie auto-immune est la sclérose en plaques.
